# EUROPEAN PATENT APPLICATION

(11) **EP 4 585 601 A1**
(43) Date of publication of application: **16.07.2025**
(21) Application number: 23863112.1
(22) Date of filing: 01.09.2023
(51) Int. Cl.: C07H 1/00, C07H 3/06, C07H 15/203, C08B 37/00

(54) **NOVEL OLIGOSACCHARIDE, PRODUCTION INTERMEDIATE FOR NOVEL OLIGOSACCHARIDE, PRODUCTION METHOD FOR NOVEL OLIGOSACCHARIDE, AND PRODUCTION METHOD FOR PRODUCTION INTERMEDIATE FOR NOVEL OLIGOSACCHARIDE**

(30) Priority: 09.09.2022 JP 2022143814
(71) Applicant: Daiichi Sankyo Company, Limited, Tokyo 103-8426 (JP)
(72) Inventor: UEDA Tsuyoshi, Tokyo 103-8426 (JP); ITOH Ryusei, Tokyo 103-8426 (JP); NAKAMURA Tatsuya, Tokyo 103-8426 (JP)
(74) Representative: Marks & Clerk LLP
(86) International application number: PCT/JP2023/032064
(87) International publication number: WO 2024/053574

(57) **Abstract**

The present invention addresses the problem of providing a novel oligosaccharide, which is a biantennary glycan having an α2,6-sialic acid structure at a nonreducing end, and a method for producing the oligosaccharide, and an intermediate thereof, and a method for producing the intermediate. Provided are a novel oligosaccharide represented by the following formula A-22, a method for producing the oligosaccharide shown in Figure 1, and an intermediate thereof and a method for producing the intermediate.

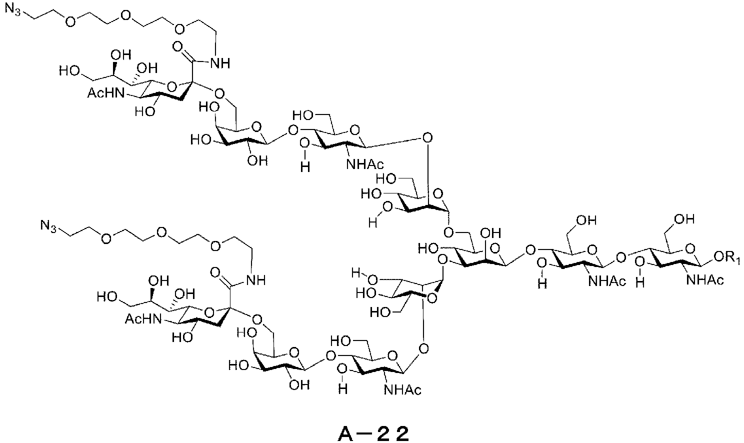

## Description

### Technical Field

The present invention relates to a novel oligosaccharide, which is a biantennary glycan having an α2,6-sialic acid structure at a non-reducing end, and a method for producing the oligosaccharide, and an intermediate thereof, and a method for producing the intermediate.

### Background Art

Protein glycosylation is known to significantly affect the function and structure of the protein. N-linked glycans, in particular, are deeply involved in physiological activities of proteins. Among them, a biantennary N-glycan with an α2,6-sialic acid structure at each non-reducing end has been reported to be an optimal structure for increasing antibody-dependent cellular cytotoxic activity (ADCC activity) and complement-dependent cytotoxic activity (CDC activity) (Non-Patent Literature 1).

For the development and commercialization of pharmaceuticals using glycans, it is desirable to be able to stably produce large quantities of highly pure glycans at an industrially applicable price. The synthesis of α2,6-sialyl glycan reported so far can be roughly classified into two methods: 1) semi-chemical synthesis involving isolation and purification from natural extracts or chemical synthesis of a main backbone precursors in combination with enzymatic/chemical conversion and 2) pure chemical synthesis.

Examples of the semi-chemical synthesis reported include a method in which an enzymatic process and a chemical process are combined to obtain an N-linked glycan from the yolks of chicken eggs (Non-Patent Literature 2). These processes can be used to synthesize a target glycan with fewer steps than pure chemical synthesis. On the other hand, a large amount of egg yolks must be provided. In many cases, special techniques and purification equipment are required for the subsequent isolated purification from egg yolks and purification of water-soluble unprotected glycans after chemical conversion (Patent Literatures 1 to 4).

Meanwhile, examples of the α2,6-sialyl glycan pure-chemical synthesis include the following reported cases:
(1) total synthesis of α2,6-sialyl moiety-containing complex glycan with 11 sugars (Non-Patent Literature 3);
(2) total synthesis of α2,6-sialyl moiety-containing immunoglobulin G peptide with 13 sugars (Non-Patent Literature 4);
(3) total synthesis of core fucose-containing α2,6-sialyl N-linked glycan with 12 sugars (Non-Patent Literature 5);
(4) total synthesis of position 3-fluoridated α2,6-sialyl oligosaccharide chain with 10 sugars (Non-Patent Literature 6);
(5) total synthesis of asymmetrically deuterated α2,6-sialyl biantennary oligosaccharide chain with 11 sugars and tetraantennary oligosaccharide chain with 17 sugars (Non-Patent Literature 7)

For pure chemical synthesis of glycan, in the case that a robust production method from monosaccharides is established, the flexibility of the production quantity is considered to be extremely high as well as ordinary low-molecular-weight compounds. Moreover, since it involves conversion of a protecting group-modified sugar, most of the purification operation is the same as nonaqueous compound handling. The complexity of the operation and the number of operation steps should be significantly less than those for the semi-chemical synthesis. Further, established chemical synthesis methods may be applied to facilitate synthesis of a wide variety of unnatural glycans.

Meanwhile, in the past reported cases described above, 1) difficult-to-construct sugar parts such as β-mannoside and α-sialyl moieties should be converted, and in their linkage process, a step with low selectivity and low yield is present; and 2) in any of the sugar-part conversion or the linkage process, silica gel column chromatography purification, which is not suitable for scale-up, is frequently used and a precision chromatographic preparation and a purification operation are essential in many steps to remove isomers and impurities generated as byproducts in the reaction. These two points are listed as big issues on the synthesis.

As described above, the pure chemical synthesis of glycan has potential advantages over the semi-chemical synthesis when the mass synthesis is intended. However, it is difficult to deem that the technology is sufficiently advanced in terms of yield, selectivity, efficiency, and cost. There are very few cases where the pure chemical synthesis has been adopted as an actual mass synthesis method.

In each sugar derivative protected by a phthaloyl group(s), deacylation is sometimes necessary. However, in the presence of a trace amount of water in the system, the phthalimide group(s) readily undergoes a ring-opening reaction under basic conditions. This necessitates strict control of water content of the system. Even at a moisture level on the order of ppm, it is difficult to completely suppress the ring-opening. Therefore, there is a need for a method that allows deacylation to proceed in high yield while inhibiting ring-opening of the phthalimide group.

In the chemical synthesis of, for instance, an oligosaccharide chain having hydroxyl group(s), it is necessary to make rational use of a method for selectively deprotecting and deprotecting such hydroxyl groups in order to efficiently obtain a target compound. In particular, a 2-naphthylmethyl group has been widely used as a common protecting group for a hydroxyl group. Meanwhile, as the 2-naphthylmethyl group deprotection method, the method is known in which 2,3-dichloro-5,6-dicyano-p-benzoquinone is used in dichloromethane-water. The method gives the desired deprotected product in moderate to high yields for a wide range of substrates. However, it is generally known that dichloromethane and water are immiscible and that 2,3-dichloro-5,6-dicyano-p-benzoquinone and its byproduct 2,3-dichloro-5,6-dicyano-p-benzohydroquinone are almost insoluble in dichloromethane-water. This affected the stirring properties, making it difficult to apply this method to mass synthesis. In addition, it has been reported that the desired deprotected product cannot always be obtained in a satisfactory yield depending on the substrate applied (Non-Patent Literature 8). In addition, an increase in the number of benzyl groups in the substrate tends to decrease the reaction yield (Non-Patent Literature 9). It has been sought to develop a milder and more efficient method applicable to complex substrates. As a means for achieving the objective, improved conditions using β-pinene as an additive have been reported (Non-Patent Literature 10). Even with this method, yields are only moderate for complex substrates with multiple benzyl groups (Non-Patent Literature 11). In view of the above background, it is desirable to develop a de-2-naphthylmethylation reaction that can deprotect the protecting group of the 2-naphthylmethyl group under milder conditions to obtain the deprotected product in high yield.

Further, liquid-phase and solid-phase synthesis methods are known for oligosaccharide chain chemical synthesis. Here, the liquid-phase synthesis method allows for the use of ordinary organic synthesis techniques, making it easy to track and scale up the reaction. However, every step requires post-processing and purification. This results in the disadvantage of being time-consuming and labor-intensive. In addition, the solid-phase synthesis method is also advantageous in that it can be automated and the production can be quick. However, the scale-up is limited due to facility constraints. Moreover, the low reactivity necessitates the use of an excessive amount of glycosyl donor used in the sugar elongation reaction. This is unfit for industrial mass synthesis. Further, the disadvantage is that it is difficult to check the progress of the reaction during the process (Patent Literature 5). To solve these problems, several methods have been developed for the production of oligosaccharide using a substrate tagged with a molecular structure (tag) that can induce, for instance, specific precipitation, distribution, or adsorption in a certain environment (Patent Literature 6 and Non-Patent Literatures 12 to 16). These methods combine the advantages of liquid-phase and solid-phase synthesis methods. In other words, the reaction can be performed in a homogeneous system, so that analysis is easy. Moreover, the tag features enable separation from, for instance, reagent debris. For example, a method is known which separates an untagged compound by adsorption on octadecyl-modified silica gel in a post-reaction solution while using a branched long-chain alkane as a hydrophobic tag (Non-Patent Literature 16). However, there have been disadvantages: any of these methods requires the use of a tag, necessitating the desorption process and as oligomerization proceeds, the substrate moiety becomes larger than the tag, gradually making the physical properties of the substrate dominant, thereby weakening the function of the tag. Therefore, a more efficient oligosaccharide purification method is required in the method for producing an oligosaccharide chain.

In the glycosylation reaction, a -NHAc group, if present in the reaction substrate, interacts with a Lewis acid, causing a significant decrease in reactivity during the desired glycosylation reaction, and an excess amount of glycosyl donor is often required to complete the reaction. Therefore, in the case of synthesizing an acetylglucosamine-containing oligosaccharide chain, a method has been adopted in which a Troc group (Non-Patent Literatures 5, 6 and 7), phthaloyl group (Non-Patent Literature 15), or sulfonyl group (Non-Patent Literature 4) is used as a temporary protecting group on the nitrogen of glucosamine during the glycosylation reaction, followed by deprotection and then N-acetylation. However, the Troc group requires zinc/AcOH-mediated deprotection reaction conditions or a long reaction time due to an excess amount of lithium hydroxide. In complex glycans, substrate degradation is accompanied under the deprotection reaction conditions. In the deprotection of the phthaloyl group, the use of an excess amount of ethylenediamine has a problem of amidation of the sialic acid ester moiety proceeding as a side reaction. In order to avoid this, a two-step process is required, in which the ester moiety is selectively hydrolyzed in advance, followed by deprotection. In addition, the sulfonyl group is deprotected under reaction conditions that are difficult to scale up by using metallic sodium. Hence, in the method for producing an oligosaccharide chain containing acetylglucosamine, it has been sought to develop a protecting group that can be easily replaced with an acetyl group without degrading the reactivity of the glycosylation reaction.

### Citation List

### Patent Literature

Patent Literature 1: WO 2011/027868
Patent Literature 2: WO 96/02255
Patent Literature 3: WO 2014/208742
Patent Literature 4: WO 2017/110984
Patent Literature 5: WO 2002/16384
Patent Literature 6: Japanese Patent No. 6001267

### Non-Patent Literature

Non-Patent Literature 1: Proc. Natl. Acad. Sci. U.S.A. 2015, 112, 10611
Non-Patent Literature 2: Beilstein J. Org. Chem. 2018, 14, 416
Non-Patent Literature 3: TetrahedronLett. 1986, 27, 5739
Non-Patent Literature 4: J. Am. Chem. Soc. 2009, 131, 16669-16671
Non-Patent Literature 5: J. Org. Chem. 2016, 81, 10600-10616
Non-Patent Literature 6: J. Am. Chem. Soc. 2019, 141, 6484-6488
Non-Patent Literature 7: Angew. Chem. Int. Ed. 2021, 60, 24686-24693
Non-Patent Literature 8: Org. Lett. 2002, 4, 4551
Non-Patent Literature 9: J. Am. Chem. Soc. 2018, 140, 4632
Non-Patent Literature 10: J. Org. Chem., 2017, 82, 3926
Non-Patent Literature 11: Angew. Chem. Int. Ed. 2021, 60, 19287
Non-Patent Literature 12: Tetrahedron Lett., 40, 7479(1999)
Non-Patent Literature 13: Synlett, 2001, 590
Non-Patent Literature 14: Synlett, 2001, 1693
Non-Patent Literature 15: Org. Biomol. Chem. 2018, 16, 4720
Non-Patent Literature 16: J. AM. CHEM. SOC. 2005, 127, 7296

### Summary of Invention

### Technical Problem

The present invention addresses the problem of providing a novel oligosaccharide, which is a biantennary glycan having an α2,6-sialic acid structure at a non-reducing end, and a method for producing the oligosaccharide, and an intermediate thereof, and a method for producing the intermediate.

### Solution to Problem

The present inventors have conducted intensive research to solve the above-mentioned problems and, as a result, have found a novel oligosaccharide represented by formula A-22 below, which is a biantennary glycan having an α2,6-sialic acid structure at a non-reducing end, and a novel method for efficiently producing the oligosaccharide, and an intermediate thereof, and a method for producing the intermediate. In this way, the present invention has been completed.

Specifically, the present invention pertains to the following items, but is not limited to them.
[1] A method for producing an oligosaccharide represented by the following formula A-22: 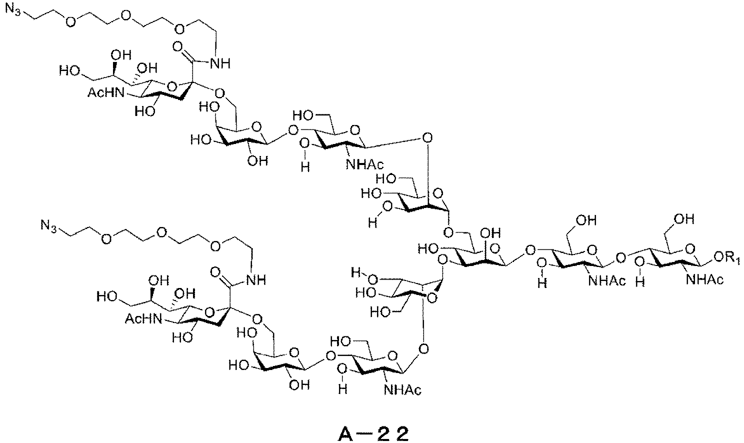 wherein R₁ is a C₁ to C₆ alkyl group or a phenyl group, and the phenyl group is optionally substituted with one to three C₁ to C₆ alkoxy groups, the method comprising the steps of:
   (step I-1) producing a compound represented by the following formula A-8: 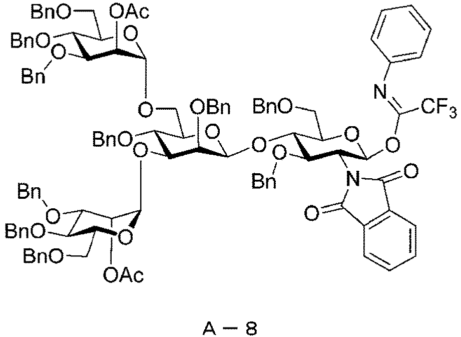 the step comprising the step of:
      subjecting a compound represented by formula A-3: 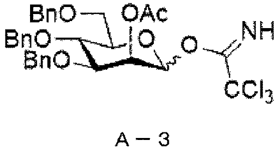
      and a compound represented by the following formula A-5: 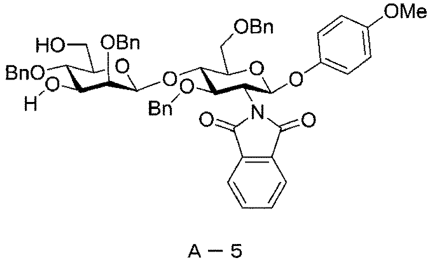
      to α-1,3-glycosidic linkage and α-1,6-glycosidic linkage to give a compound represented by the following formula A-6: 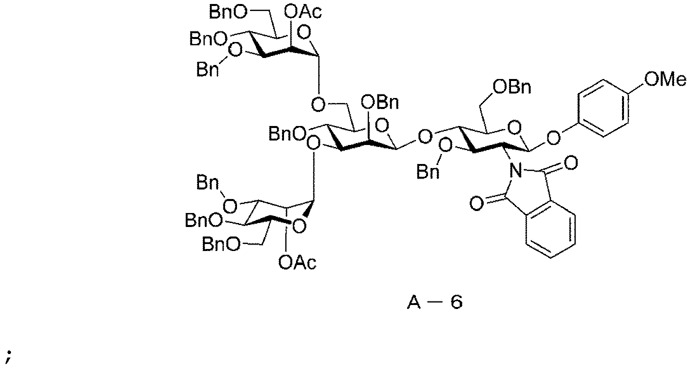
   (step I-2) producing a compound represented by the following formula A-11: 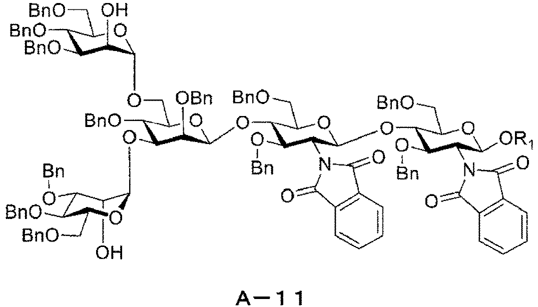 wherein R₁ is a C₁ to C₆ alkyl group or a phenyl group, and the phenyl group is optionally substituted with one to three C₁ to C₆ alkoxy groups,
      the step comprising the step of:
      subjecting the compound represented by formula A-8 and a compound represented by the following formula A-9: 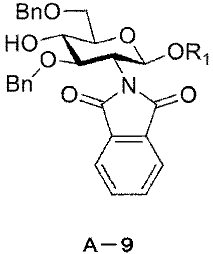
      wherein is a C₁ to C₆ alkyl group or a phenyl group, and the phenyl group is optionally substituted with one to three C₁ to C₆ alkoxy groups,
      to β-1,4-glycosidic linkage to give a compound represented by the following formula A-10: 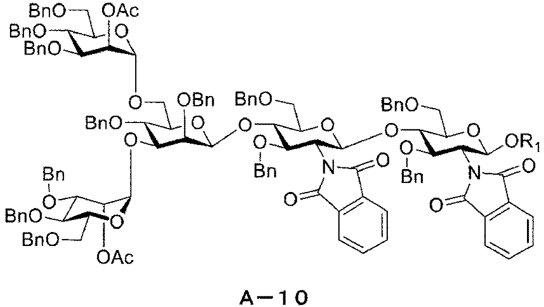
      wherein R₁ is a C₁ to C₆ alkyl group or a phenyl group, and the phenyl group is optionally substituted with one to three C₁ to C₆ alkoxy groups;
   (step I-3) subjecting the compound represented by formula A-11 and a compound represented by the following formula A-12: 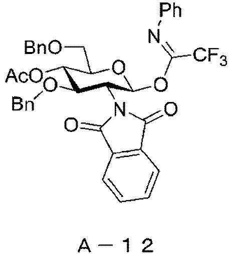 to β-1,2-glycosidic linkage to give a compound represented by the following formula A-13: 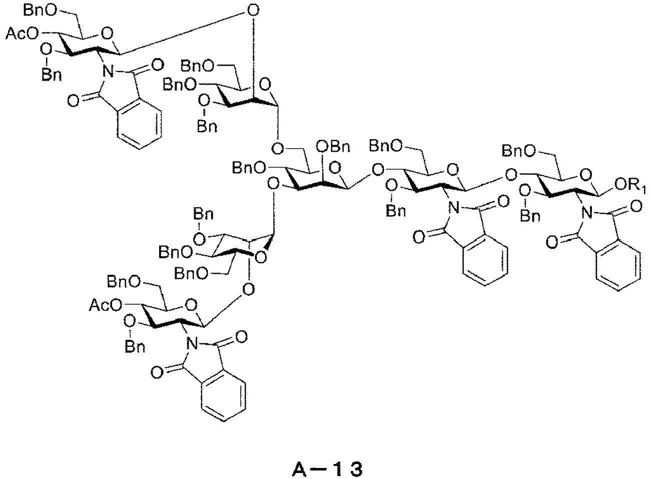
      wherein R₁ is a C₁ to C₆ alkyl group or a phenyl group, and the phenyl group is optionally substituted with one to three C₁ to C₆ alkoxy groups,
      then eliminating each phthaloyl group as a protecting group of an amino group and each acetyl group on a hydroxyl group on the compound represented by formula A-13 to give a compound represented by the following formula A-14: 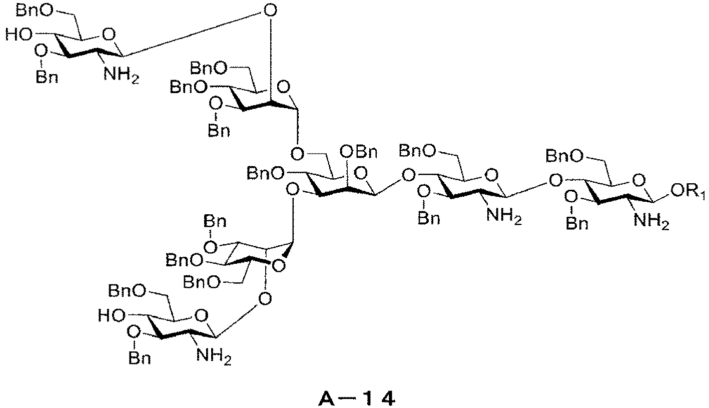
      wherein R₁ is a C₁ to C₆ alkyl group or a phenyl group, and the phenyl group is optionally substituted with one to three C₁ to C₆ alkoxy groups,
      and then protecting the amino group in the compound represented by formula A-14 by a protecting group selected from an aryloxycarbonyl (COOAr) group, an acetyl (Ac) group, a 2,2,2-trichloroethoxycarbonyl (Troc) group, or a phthaloyl (Phth) group to give a compound represented by the following formula A-15: 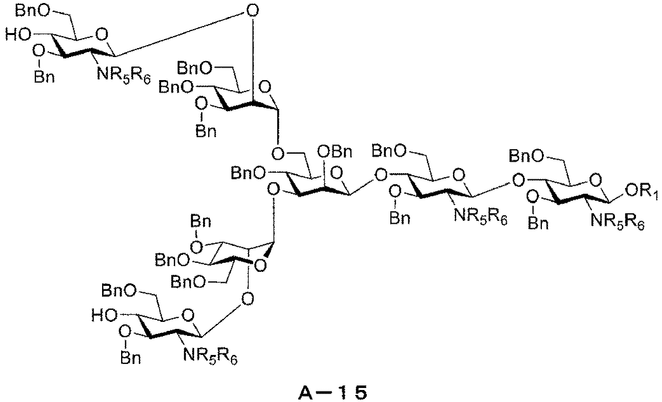
      wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
      or eliminating each acetyl (Ac) group on the compound represented by formula A-13 to give the compound represented by formula A-15 wherein R₅ and R₆ together with a nitrogen atom attached thereto form a phthalimide group;
   (step I-4) producing a compound represented by the following formula A-20: 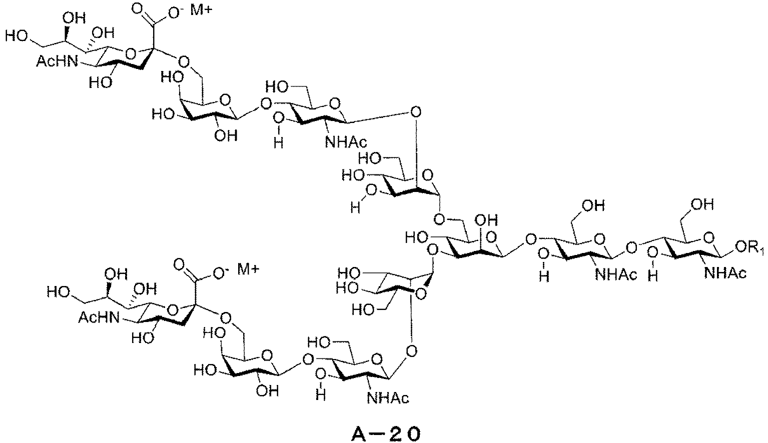 wherein R₁ is a C₁ to C₆ alkyl group or a phenyl group, the phenyl group is optionally substituted with one to three C₁ to C₆ alkoxy groups, and M⁺ is a sodium ion, lithium ion, potassium ion, or protonated triethylamine cation,
      the step comprising:
      subjecting the compound represented by A-15 and a compound represented by the following formula A-16: 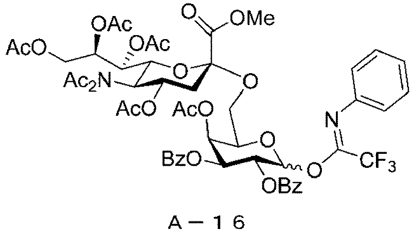
      to β-1,4-glycosidic linkage to give a compound represented by the following formula A-17: 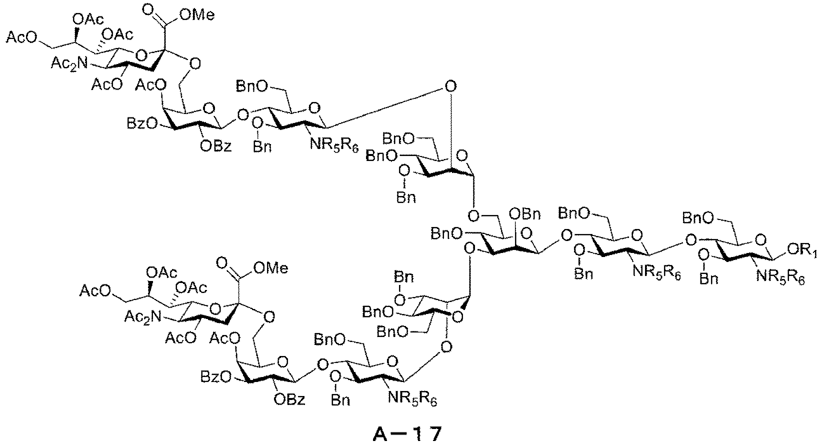
      wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
      and then eliminating each protecting group of an amino group and each acyl-based protecting group of a hydroxyl group in the compound represented by formula A-17 to give a compound represented by formula A-18: 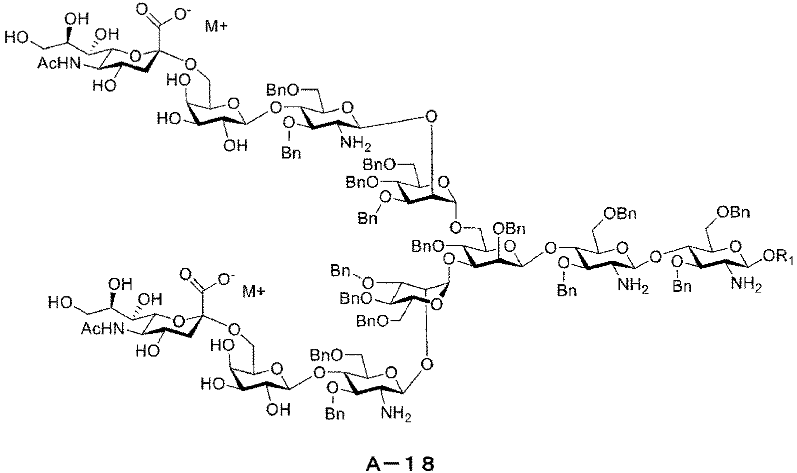
      wherein R₁ is a C₁ to C₆ alkyl group or a phenyl group, the phenyl group is optionally substituted with one to three C₁ to C₆ alkoxy groups, and M⁺ is a sodium ion, lithium ion, potassium ion, or protonated triethylamine cation; and
   (step I-5) producing the oligosaccharide represented by formula A-22 by reacting the compound represented by formula A-20 with a compound represented by the following formula A-21: 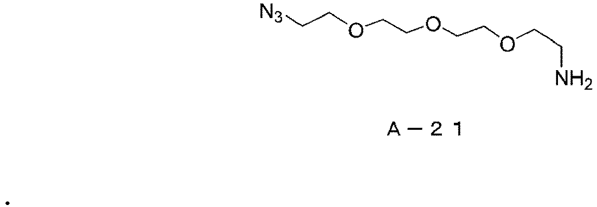
[2] The method according to [1], wherein the method comprises reacting a compound represented by the following formula A-4: 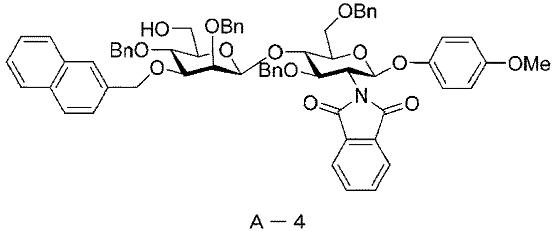 with 2,3-dichloro-5,6-dicyano-p-benzoquinone (DDQ) in a mixed solvent of fluorous alcohol and water to eliminate a 2-naphthylmethyl group in the compound represented by formula A-4 to give the compound represented by formula A-5.
[3] The method according to [2], wherein the fluorous alcohol is selected from the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and combinations thereof.
[4] The method according to [2] or [3], wherein the reaction is carried out at -35°C to 70°C.
[5] The method according to [2] or [3], wherein the reaction is carried out at -30°C to -10°C.
[6] The method according to any one of [1] to [5], wherein step I-2 comprises reacting the compound represented by formula A-10 with a strong base in the presence of an alkyl ester of perfluorocarboxylic acid to give the compound represented by formula A-11.
[7] The method according to [6], wherein the alkyl ester of perfluorocarboxylic acid is methyl trifluoroacetate, ethyl trifluoroacetate, propyl trifluoroacetate, isopropyl trifluoroacetate, butyl trifluoroacetate, methyl pentafluoropropionate, ethyl pentafluoropropionate, propyl pentafluoropropionate, isopropyl pentafluoropropionate, butyl pentafluoropropionate, methyl heptafluorobutyrate, ethyl heptafluorobutyrate, propyl heptafluorobutyrate, isopropyl heptafluorobutyrate, butyl heptafluorobutyrate, methyl nonafluorovalerate, ethyl nonafluorovalerate, propyl nonafluorovalerate, isopropyl nonafluorovalerate, butyl nonafluorovalerate, methyl undecafluorocaproate, ethyl undecafluorocaproate, propyl undecafluorocaproate, isopropyl undecafluorocaproate, or butyl undecafluorocaproate.
[8] The method according to [6] or [7], wherein the strong base is selected from the group consisting of sodium, lithium, and potassium salts of metal amides; sodium, lithium, potassium, cesium, and barium salts of C1-C20 alkoxides; sodium hydride, potassium hydride, lithium hydride, butyllithium, potassium carbonate, sodium carbonate, cesium carbonate, lithium carbonate, potassium phosphate, sodium phosphate, cesium phosphate, lithium phosphate, diazabicycloundecene (DBU), diazabicyclononene (DBN), and 1,1,3,3-tetramethylguanidine (TMG); and combinations thereof.
[9] The method according to [6] or [7], wherein the strong base is potassium tert-butoxide, sodium tert-butoxide, lithium tert-butoxide, or lithium hexamethyldisilazide(LHMDS).
[10] The method according to any one of [6] to [9], wherein the step of reacting the compound represented by formula A-10 with a strong base in the presence of an alkyl ester of perfluorocarboxylic acid to give the compound represented by formula A-11 is carried out in a C₁-C₁₀ alcohol solvent alone or in a mixture of a C₁-C₁₀ alcohol solvent and an amide-based solvent, ether-based solvent, ester-based solvent, aromatic solvent, halogen-based solvent, hydrocarbon-based solvent, or nitrile-based solvent.
[11] The method according to any one of [1] to [10], wherein in step I-3, each amino group in the compound represented by formula A-14 is protected with an aryloxycarbonyl (COOAr) group to give the compound represented by formula A-15.
[12] The method according to any one of [1] to [11], wherein in step I-3, the step of producing the compound represented by formula A-15 from the compound represented by formula A-14 is carried out in an aqueous solution containing sodium bicarbonate, potassium bicarbonate, disodium hydrogen phosphate, or dipotassium hydrogen phosphate.
[13] The method according to any one of [1] to [12], wherein R₁ is isopropyl or 4-methoxyphenyl.
[14] The method according to any one of [1] to [13], wherein the method comprises a step for producing the compound represented by formula A-16;
   the step comprising:
   stirring a solution containing a compound represented by the following formula G-11: 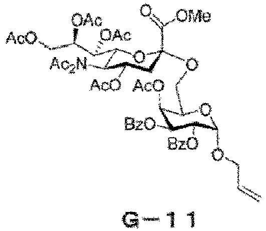
   and carbonylchlorohydridotris(triphenylphosphine) ruthenium(II), and then adding water and 1,3-dibromo-5,5-dimethylhydantoin, iodide, or N-bromosuccinimide to the solution and stirring the mixture to eliminate the allyl group attached via oxygen to the carbon at the 1-position of D-galactopyranoside in the compound represented by formula G-11 to give a compound represented by the following formula G-12: 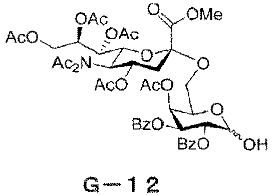
   and further converting the hydroxyl group on the carbon at the 1-position of D-galactopyranoside in the compound represented by formula G-12 to a 2,2,2-trifluoro-N-phenylacetimidate group to give the compound represented by formula A-16.
[15] A method for producing a compound represented by the following formula A-5: 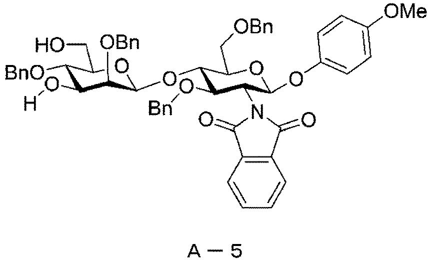 the method comprising reacting a compound represented by the following formula A-4: 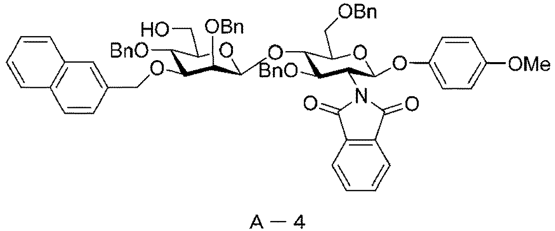 with DDQ (2,3-dichloro-5,6-dicyano-p-benzoquinone) in a mixed solvent of fluorous alcohol and water to eliminate a 2-naphthylmethyl group in the compound represented by formula A-4 to give the compound represented by formula A-5.
[16] The method according to [15], wherein the fluorous alcohol is selected from the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and combinations thereof.
[17] The method according to [15] or [16], wherein the reaction is carried out at -35°C to 70°C.
[18] The method according to [15] or [16], wherein the reaction is carried out at -30°C to -10°C.
[19] A method for producing a compound represented by the following formula A-11: 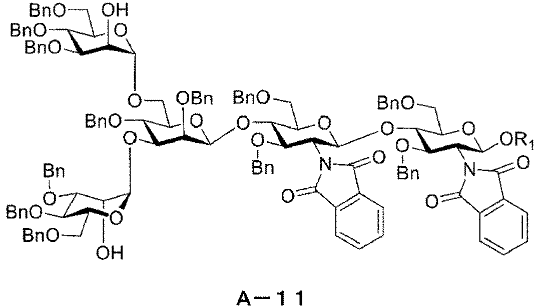
   wherein R₁ is a C₁ to C₆ alkyl group or a phenyl group, and the phenyl group is optionally substituted with one to three C₁ to C₆ alkoxy groups,
   the method comprising the step of:
      reacting a compound represented by the following formula A-10: 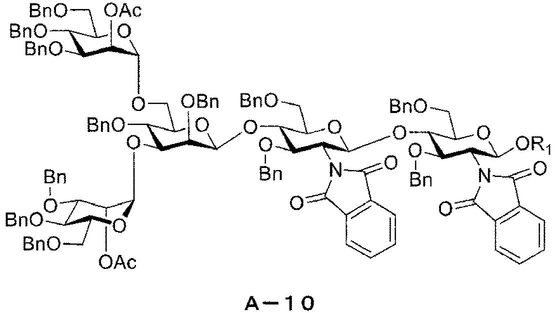
      wherein R₁ is a C₁ to C₆ alkyl group or a phenyl group, and the phenyl group is optionally substituted with one to three C₁ to C₆ alkoxy groups,
      with a strong base in the presence of an alkyl ester of perfluorocarboxylic acid.
[20] The method according to [19], wherein the alkyl ester of perfluorocarboxylic acid is methyl trifluoroacetate, ethyl trifluoroacetate, propyl trifluoroacetate, isopropyl trifluoroacetate, butyl trifluoroacetate, methyl pentafluoropropionate, ethyl pentafluoropropionate, propyl pentafluoropropionate, isopropyl pentafluoropropionate, butyl pentafluoropropionate, methyl heptafluorobutyrate, ethyl heptafluorobutyrate, propyl heptafluorobutyrate, isopropyl heptafluorobutyrate, butyl heptafluorobutyrate, methyl nonafluorovalerate, ethyl nonafluorovalerate, propyl nonafluorovalerate, isopropyl nonafluorovalerate, butyl nonafluorovalerate, methyl undecafluorocaproate, ethyl undecafluorocaproate, propyl undecafluorocaproate, isopropyl undecafluorocaproate, or butyl undecafluorocaproate.
[21] The method according to [19] or [20], wherein the strong base is selected from the group consisting of sodium, lithium, and potassium salts of metal amides; sodium, lithium, potassium, cesium, and barium salts of C₁-C₂₀ alkoxides; sodium hydride, potassium hydride, lithium hydride, butyllithium, potassium carbonate, sodium carbonate, cesium carbonate, lithium carbonate, potassium phosphate, sodium phosphate, cesium phosphate, lithium phosphate, diazabicycloundecene (DBU), diazabicyclononene (DBN), and 1,1,3,3-tetramethylguanidine (TMG); and combinations thereof.
[22] The method according to [19] or [20], wherein the strong base is potassium tert-butoxide, sodium tert-butoxide, lithium tert-butoxide, or lithium hexamethyldisilazide (LHMDS).
[23] The method according to any one of [19] to [22], wherein the reaction is carried out in a C₁-C₁₀ alcohol solvent alone or in a mixture of a C₁-C₁₀ alcohol solvent and an amide-based solvent, ether-based solvent, ester-based solvent, aromatic solvent, halogen-based solvent, hydrocarbon-based solvent, or nitrile-based solvent.
[24] A method for producing a compound represented by the following formula A-17: 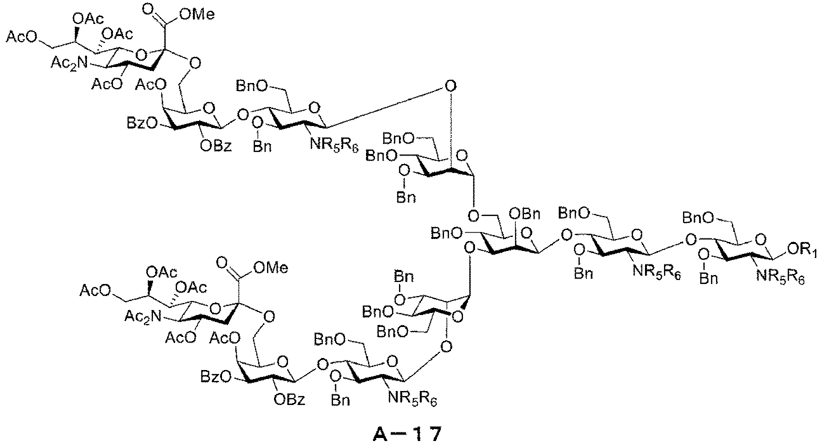 wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
   the method comprising the step of:
   producing a compound represented by the following formula A-15: 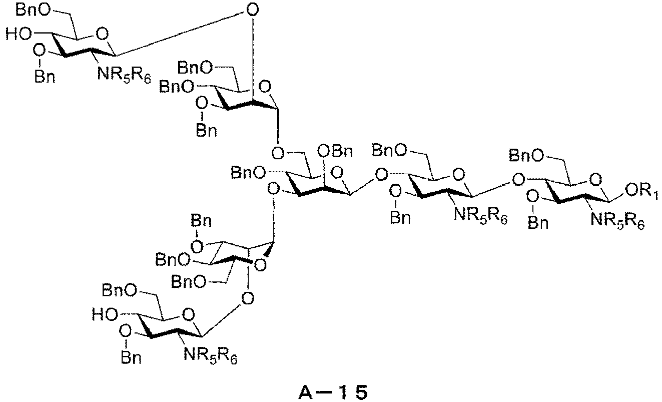
   wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
   and then subjecting the compound and a compound represented by the following formula A-16: 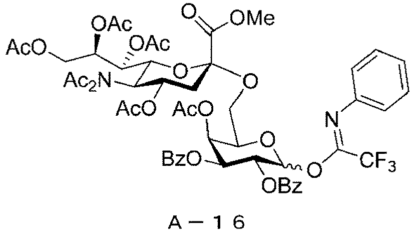
   to β-1,4-glycosidic linkage to give the compound represented by formula A-17.
[25] The method according to [24], wherein R₅ is an aryloxycarbonyl (COOAr) group.
[26] A method for producing a compound represented by the following formula A-18: 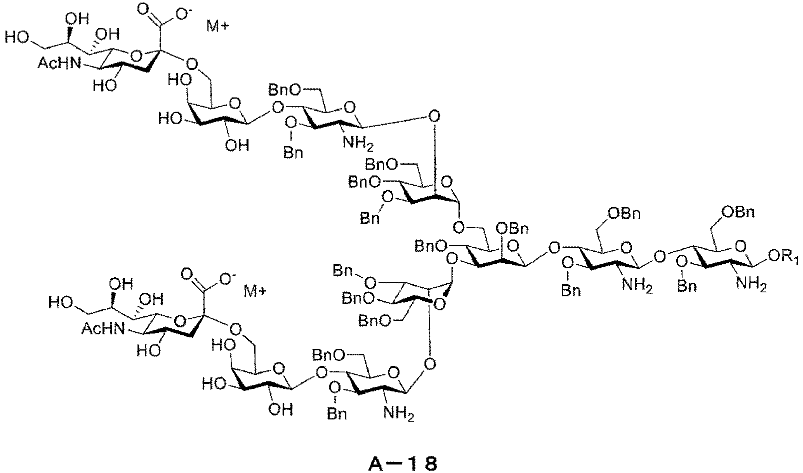
   wherein R₁ is a C₁ to C₆ alkyl group or a phenyl group, the phenyl group is optionally substituted with one to three C₁ to C₆ alkoxy groups, and M⁺ is a sodium ion, lithium ion, potassium ion, or protonated triethylamine cation,
   the method comprising:
      eliminating each protecting group of an amino group and each acyl-based protecting group of a hydroxyl group in a compound represented by the following formula A-17: 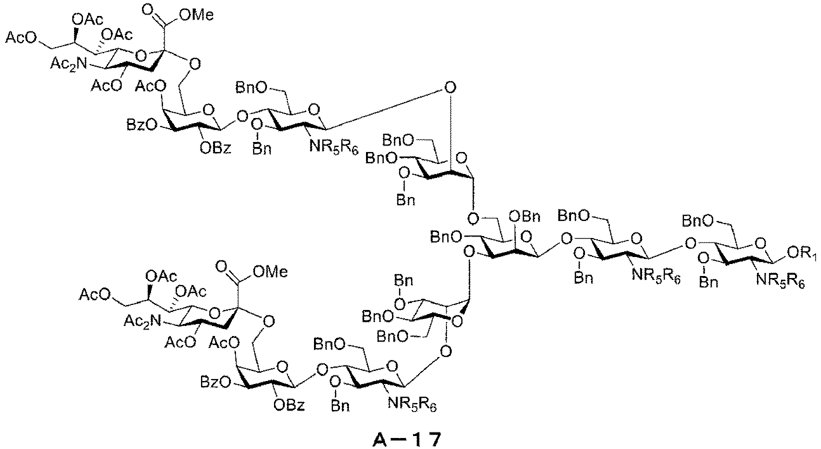
      wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group.
[27] An oligosaccharide represented by the following formula A-22: 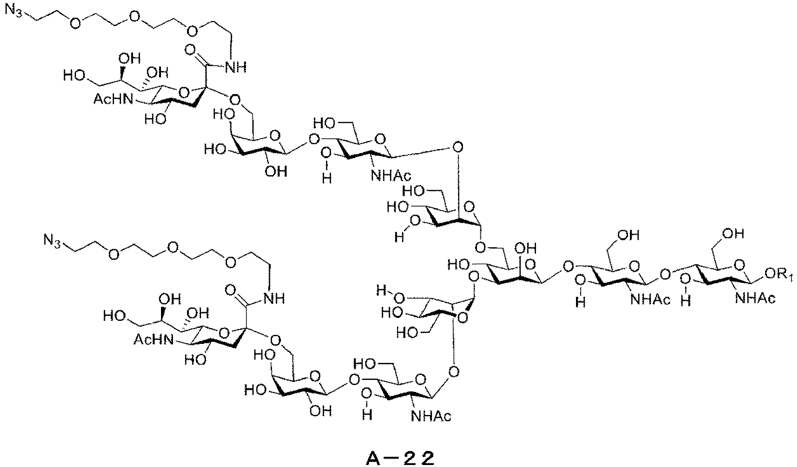 wherein R₁ is a C₁ to C₆ alkyl group or a phenyl group, and the phenyl group is optionally substituted with one to three C₁ to C₆ alkoxy groups.
[28] An oligosaccharide represented by the following formula A'-22: 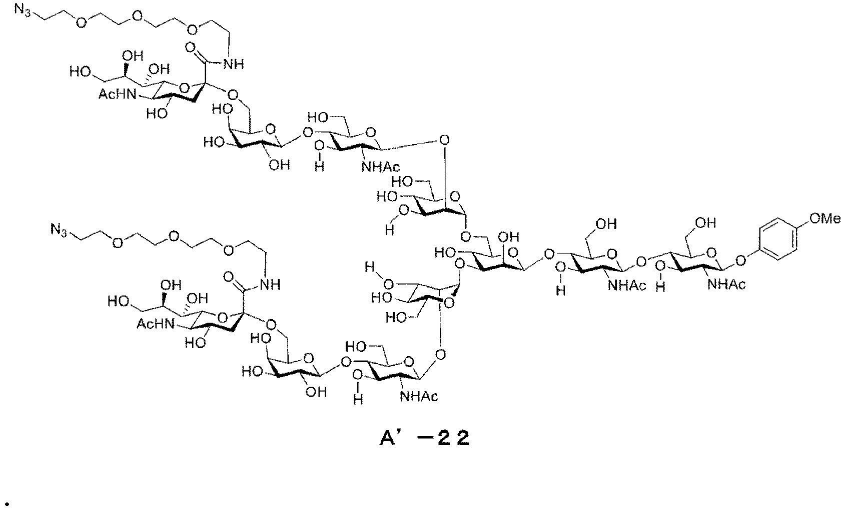
[29] A compound represented by the following formula A-8: 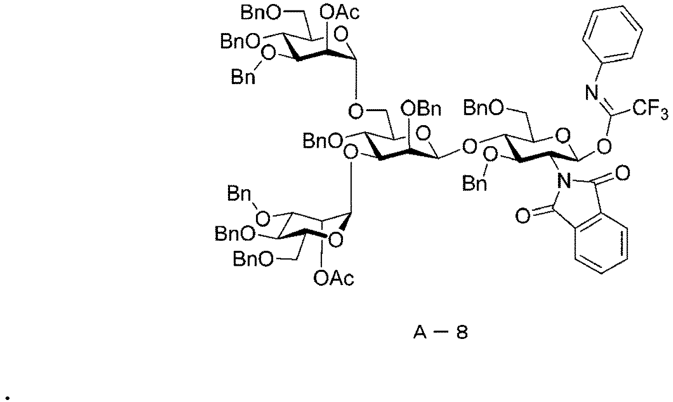
[30] A compound represented by the following formula A-10: 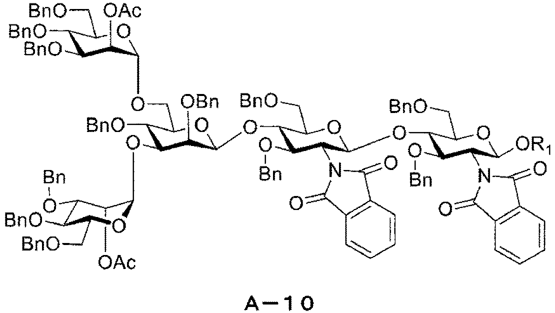 wherein R₁ is a C₁ to C₆ alkyl group or a phenyl group, and the phenyl group is optionally substituted with one to three C₁ to C₆ alkoxy groups.
[31] A compound represented by the following formula A-11: 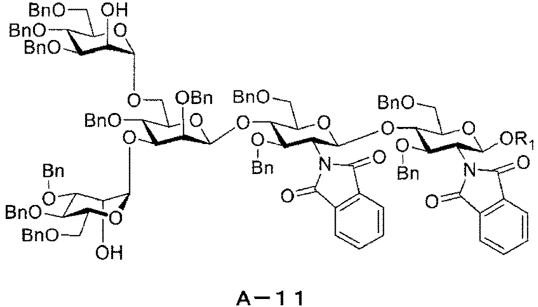 wherein R₁ is a C₁ to C₆ alkyl group or a phenyl group, and the phenyl group is optionally substituted with one to three C₁ to C₆ alkoxy groups.
[32] A compound represented by the following formula A-13: 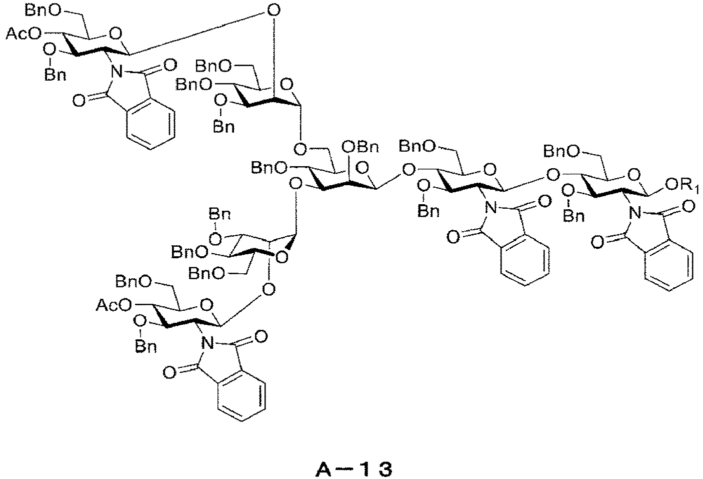 wherein R₁ is a C₁ to C₆ alkyl group or a phenyl group, and the phenyl group is optionally substituted with one to three C₁ to C₆ alkoxy groups.
[33] A compound represented by the following formula A-14: 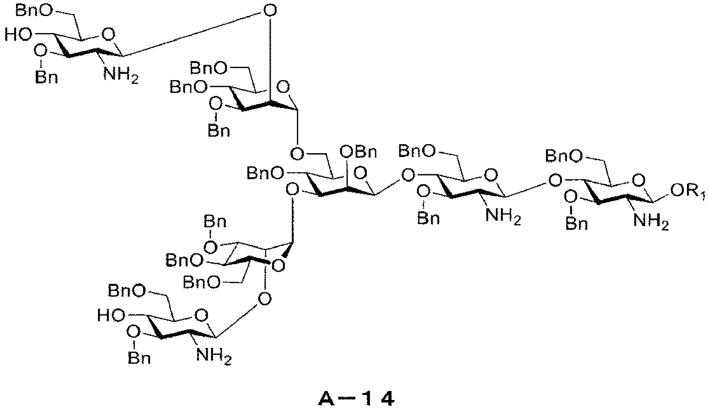 wherein R₁ is a C₁ to C₆ alkyl group or a phenyl group, and the phenyl group is optionally substituted with one to three C₁ to C₆ alkoxy groups.
[34] A compound represented by the following formula A-14-FMA: 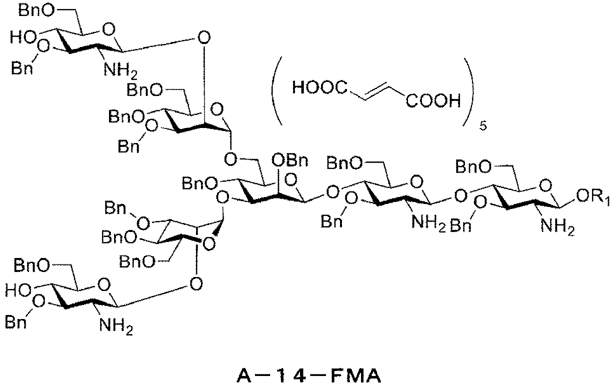 wherein R₁ is a C₁ to C₆ alkyl group or a phenyl group, and the phenyl group is optionally substituted with one to three C₁ to C₆ alkoxy groups.
[35] A compound represented by the following formula A-15: 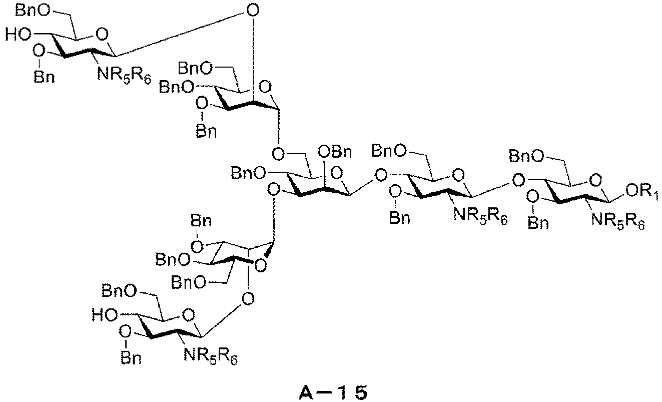 wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group.
[36] A compound represented by the following formula A-17: 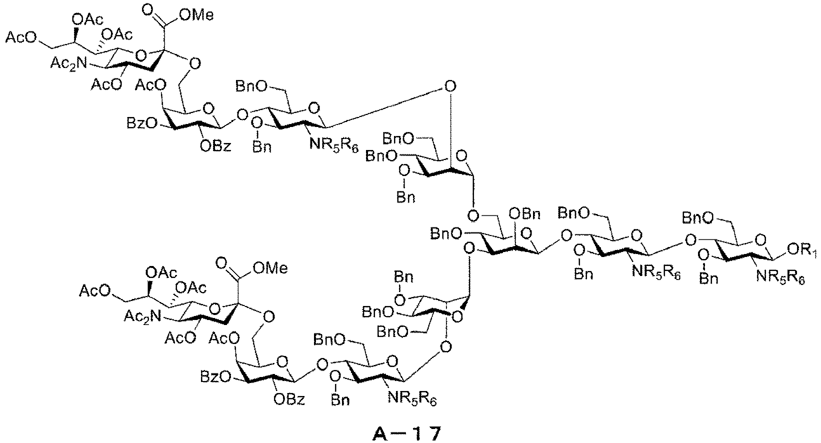 wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group.
[37] A compound represented by the following formula A-18-FF: 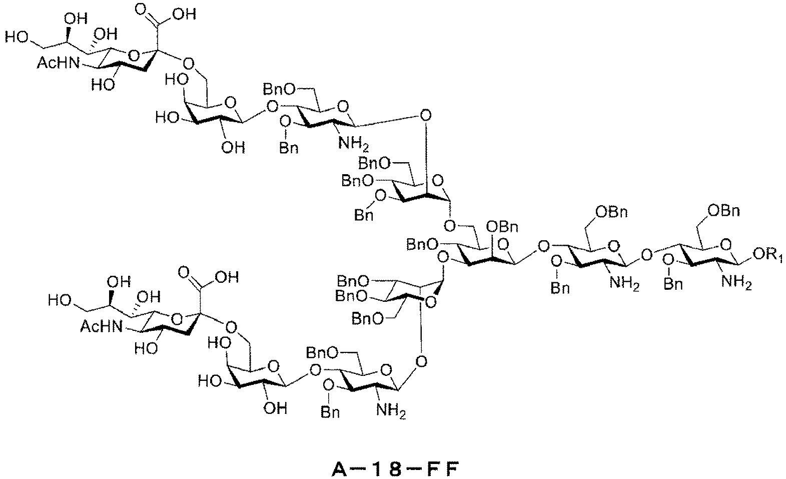
   wherein R₁ is a C₁ to C₆ alkyl group or a phenyl group, and the phenyl group is optionally substituted with one to three C₁ to C₆ alkoxy groups,
   or a salt thereof.
[38] A compound represented by the following formula A-19-FF: 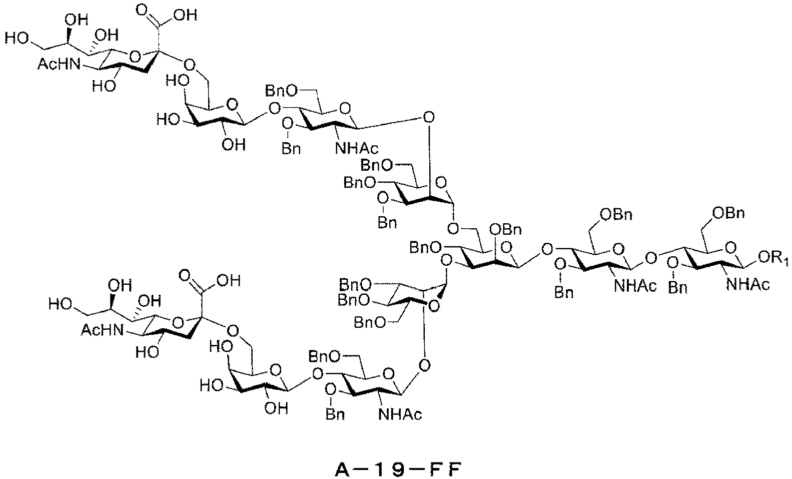
   wherein R₁ is a C₁ to C₆ alkyl group or a phenyl group, and the phenyl group is optionally substituted with one to three C₁ to C₆ alkoxy groups,
   or a salt thereof.
[39] A compound represented by the following formula A-20-FF: 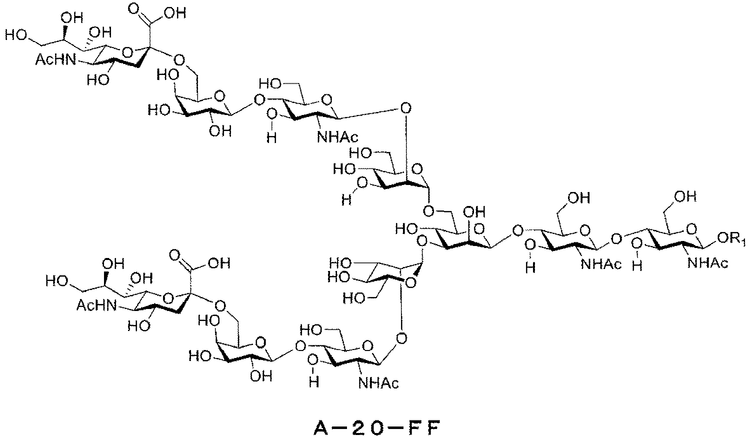
   wherein R₁ is a C₁ to C₆ alkyl group or a phenyl group, and the phenyl group is optionally substituted with one to three C₁ to C₆ alkoxy groups,
   or a salt thereof.
[40] A compound represented by the following formula A'-20-FF: 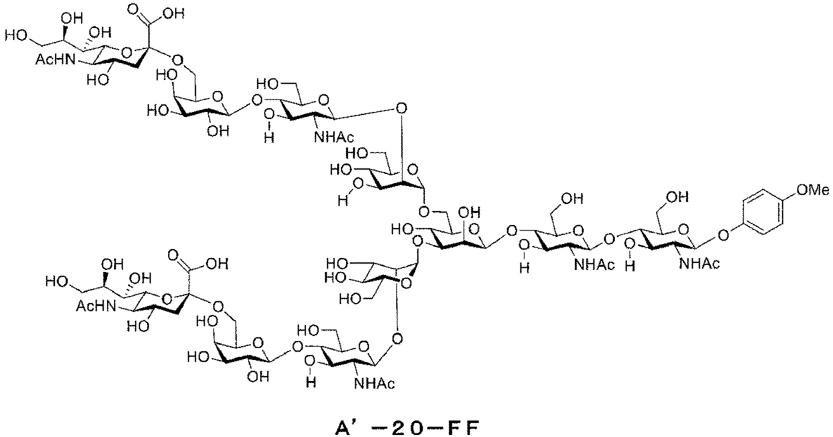 or a salt thereof.
[41] A method for producing a glycan-remodeled antibody or a molecule containing an Fc region thereof, the method comprising the steps of: obtaining the compound represented by formula A-22, the step comprising the method according to any one of [1] to [14]; and reacting the resulting compound represented by formula A-22 with an acceptor molecule that is an antibody having core GlcNAc to which fucose is optionally added as an N297-linked glycan or a molecule containing an Fc region thereof to obtain the glycan-remodeled antibody or molecule containing an Fc region thereof.
[42] The method according to [41], further comprising the step of reacting an azide group (N₃-) with a molecule having an alkyne structure.
[43] The method according to [42], wherein the molecule having an alkyne structure is selected from chemotherapeutic agents, molecular target drugs, immunostimulatory agents, toxins, antimicrobial agents, antiviral agents, diagnostic agents, proteins, peptides, amino acids, nucleic acids, antigens, vitamins, and hormones.
[44] A method for producing an antibody-drug conjugate, comprising the method according to any one of [41] to [43].
[45] The method according to any one of [1] to [14], wherein step I-1 comprises purifying the compound represented by formula A-6 by stopping the reaction of the compound represented by formula A-3 with the compound represented by formula A-5; then adding a hydrophobic carrier and water to a water-soluble organic solvent containing the resulting compound represented by formula A-6 and contaminants to adsorb the compound represented by formula A-6 onto the hydrophobic carrier; then filtering the resultant and washing the hydrophobic carrier with a mixed solution of the water-soluble organic solvent and the water to remove the contaminants; and then eluting the compound represented by formula A-6 using an organic solvent, from the hydrophobic carrier.
[46] The method according to any one of [1] to [14] and [45], wherein step I-2 comprises purifying the compound represented by formula A-10 by stopping the reaction of the compound represented by formula A-8 with the compound represented by formula A-9; then adding a hydrophobic carrier and water to a water-soluble organic solvent containing the resulting compound represented by formula A-10 and contaminants to adsorb the compound represented by formula A-10 onto the hydrophobic carrier; then filtering the resultant and washing the hydrophobic carrier with a mixed solution of the water-soluble organic solvent and the water to remove the contaminants; and then eluting the compound represented by formula A-10 using an organic solvent, from the hydrophobic carrier.
[47] The method according to any one of [1] to [14], [45], and [46], wherein step I-3 comprises purifying the compound represented by formula A-13 by stopping the reaction of the compound represented by formula A-11 with the compound represented by formula A-12; then adding a hydrophobic carrier and water to a water-soluble organic solvent containing the resulting compound represented by formula A-13 and contaminants to adsorb the compound represented by formula A-13 onto the hydrophobic carrier; then filtering the resultant and washing the hydrophobic carrier with a mixed solution of the water-soluble organic solvent and the water to remove the contaminants; and then eluting the compound represented by formula A-13 using an organic solvent, from the hydrophobic carrier.
[48] The method according to any one of [1] to [14] and [45] to [47], wherein step I-3 comprises purifying the compound represented by formula A-14 by adding a hydrophobic carrier and water to a water-soluble organic solvent containing the resulting compound represented by formula A-14 and contaminants to adsorb the compound represented by formula A-14 onto the hydrophobic carrier; then filtering the resultant and washing the hydrophobic carrier with a mixed solution of the water-soluble organic solvent and the water to remove the contaminants; and then eluting the compound represented by formula A-14 using an organic solvent, from the hydrophobic carrier.
[49] The method according to any one of [1] to [14], and [45] to [48], wherein step I-4 comprises purifying the compound represented by formula A-17 by stopping the reaction of the compound represented by formula A-15 with the compound represented by formula A-16; then adding a hydrophobic carrier and water to a water-soluble organic solvent containing the resulting compound represented by formula A-17 and contaminants to adsorb the compound represented by formula A-17 onto the hydrophobic carrier; then filtering the resultant and washing the hydrophobic carrier with a mixed solution of the water-soluble organic solvent and the water to remove the contaminants; and then eluting the compound represented by formula A-17 using an organic solvent, from the hydrophobic carrier.
[50] The method according to [45], wherein the contaminants comprise a sugar compound other than the compound represented by formula A-6, and/or a compound derived from a reaction reagent to obtain the compound to be purified.
[51] The method according to [46], wherein the contaminants comprise a sugar compound other than the compound represented by formula A-10, and/or a compound derived from a reaction reagent to obtain the compound to be purified.
[52] The method according to [47], wherein the contaminants comprise a sugar compound other than the compound represented by formula A-13, and/or a compound derived from a reaction reagent to obtain the compound to be purified.
[53] The method according to [48], wherein the contaminants comprise a sugar compound other than the compound represented by formula A-14, and/or a compound derived from a reaction reagent to obtain the compound to be purified.
[54] The method according to [49], wherein the contaminants comprise a sugar compound other than the compound represented by formula A-17, and/or a compound derived from a reaction reagent to obtain the compound to be purified.
[55] The method according to any one of [45] to [54], wherein the hydrophobic carrier is a resin for packing in reversed-phase partition chromatography.
[56] The method according to [55], wherein the resin for packing in reversed-phase partition chromatography is selected from the group consisting of poly(styrene/divinylbenzene) polymer gel resins, polystyrene-divinylbenzene resins, polyhydroxymethacrylate resins, styrene-vinylbenzene copolymer resins, polyvinyl alcohol resins, polystyrene resins, polymethacrylate resins, chemically bonded silica gel resins, and combinations thereof.
[57] The method according to [56], wherein the chemically bonded silica gel resin is selected from the group consisting of (1) resins obtained by reacting silica gel with a silane coupling agent, (2) resins obtained by chemically bonding a dimethyloctadecyl, octadecyl, trimethyloctadecyl, dimethyloctyl, octyl, butyl, ethyl, methyl, phenyl, cyanopropyl, or aminopropyl group to silica gel, and (3) resins obtained by chemically bonding a docosyl or triacontyl group to silica gel, and (4) combinations of the resins (1) to (3).
[58] The method according to [56], wherein the chemically bonded silica gel resin is an octadecyl group-bonded silica gel resin (ODS resin).
[59] The method according to any one of [45] to [56], wherein the water-soluble organic solvent is a water-soluble alcohol-based solvent, a water-soluble nitrile-based solvent, a water-soluble ether-based solvent, a water-soluble ketone-based solvent, a water-soluble amide-based solvent, or a water-soluble sulfoxide-based solvent, or a mixed solvent containing at least one of the water-soluble organic solvent-based ones.
[60] The method according to [59], wherein the water-soluble nitrile-based solvent is acetonitrile.
[61] The method according to any one of [45] to [60], wherein the organic solvent used in the step of eluting a target substance from the hydrophobic carrier is a nitrile-based solvent, an ether-based solvent, an ester-based solvent, a ketone-based solvent, a halogen-based solvent, an aromatic solvent, or a mixed solvent containing at least one of the solvent systems.
[62] A method of purifying a compound represented by the following formula A-6: 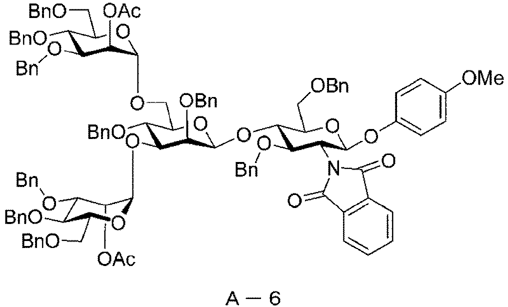 the method comprising the steps of: adding a hydrophobic carrier and water to a water-soluble organic solvent containing the compound represented by formula A-6 and contaminants to adsorb the compound represented by formula A-6 onto the hydrophobic carrier; then filtering the resultant and washing the hydrophobic carrier with a mixed solution of the water-soluble organic solvent and the water to remove the contaminants; and then eluting the compound represented by formula A-6 using an organic solvent, from the hydrophobic carrier to purify the compound represented by formula A-6.
[63] A method of purifying a compound represented by the following formula A-10: 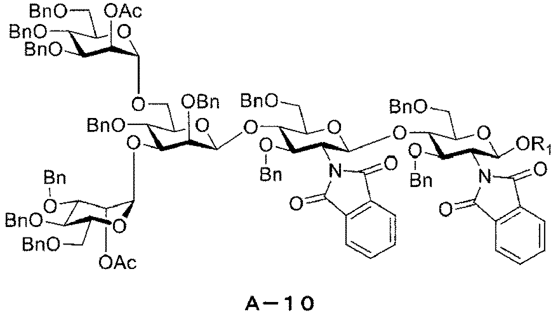
   wherein R₁ is a C₁ to C₆ alkyl group or a phenyl group, and the phenyl group is optionally substituted with one to three C₁ to C₆ alkoxy groups,
   the method comprising the steps of: adding a hydrophobic carrier and water to a water-soluble organic solvent containing the compound represented by formula A-10 and contaminants to adsorb the compound represented by formula A-10 onto the hydrophobic carrier; then filtering the resultant and washing the hydrophobic carrier with a mixed solution of the water-soluble organic solvent and the water to remove the contaminants; and then eluting the compound represented by formula A-10 using an organic solvent, from the hydrophobic carrier to purify the compound represented by formula A-10.
[64] A method of purifying a compound represented by the following formula A-13: 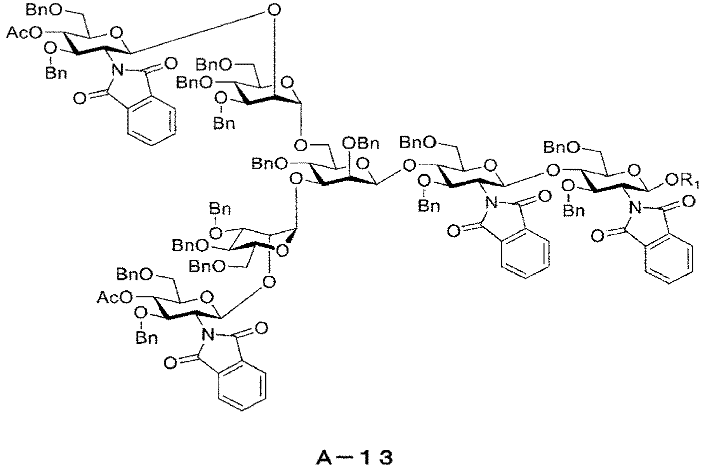
   wherein R₁ is a C₁ to C₆ alkyl group or a phenyl group, and the phenyl group is optionally substituted with one to three C₁ to C₆ alkoxy groups,
   the method comprising the steps of: adding a hydrophobic carrier and water to a water-soluble organic solvent containing the compound represented by formula A-13 and contaminants to adsorb the compound represented by formula A-13 onto the hydrophobic carrier; then filtering the resultant and washing the hydrophobic carrier with a mixed solution of the water-soluble organic solvent and the water to remove the contaminants; and then eluting the compound represented by formula A-13 using an organic solvent, from the hydrophobic carrier to purify the compound represented by formula A-13.
[65] A method of purifying a compound represented by the following formula A-14: 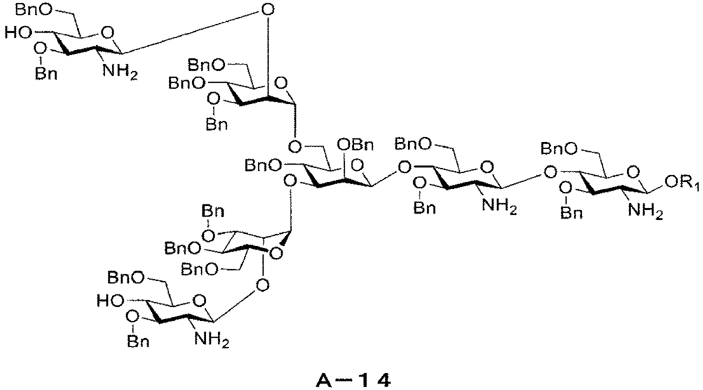
   wherein R₁ is a C₁ to C₆ alkyl group or a phenyl group, and the phenyl group is optionally substituted with one to three C₁ to C₆ alkoxy groups,
   the method comprising the steps of: adding a hydrophobic carrier and water to a water-soluble organic solvent containing the compound represented by formula A-14 and contaminants to adsorb the compound represented by formula A-14 onto the hydrophobic carrier; then filtering the resultant and washing the hydrophobic carrier with a mixed solution of the water-soluble organic solvent and the water to remove the contaminants; and then eluting the compound represented by formula A-14 using an organic solvent, from the hydrophobic carrier to purify the compound represented by formula A-14.
[66] A method of purifying a compound represented by the following formula A-17: 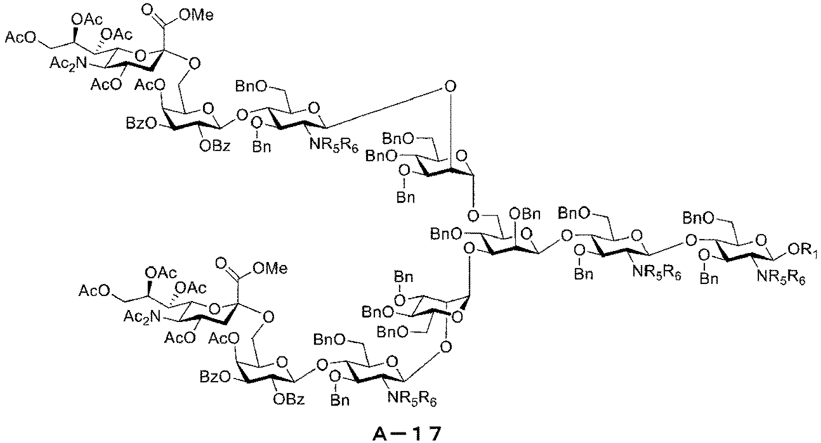
   wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
   the method comprising the steps of: adding a hydrophobic carrier and water to a water-soluble organic solvent containing the compound represented by formula A-17 and contaminants to adsorb the compound represented by formula A-17 onto the hydrophobic carrier; then filtering the resultant and washing the hydrophobic carrier with a mixed solution of the water-soluble organic solvent and the water to remove the contaminants; and then eluting the compound represented by formula A-17 using an organic solvent, from the hydrophobic carrier to purify the compound represented by formula A-17.
[67] The method according to [62], wherein the contaminants comprise a sugar compound other than the compound represented by formula A-6, and/or a compound derived from a reaction reagent to obtain the compound to be purified.
[68] The method according to [63], wherein the contaminants comprise a sugar compound other than the compound represented by formula A-10, and/or a compound derived from a reaction reagent to obtain the compound to be purified.
[69] The method according to [64], wherein the contaminants comprise a sugar compound other than the compound represented by formula A-13, and/or a compound derived from a reaction reagent to obtain the compound to be purified.
[70] The method according to [65], wherein the contaminants comprise a sugar compound other than the compound represented by formula A-14, and/or a compound derived from a reaction reagent to obtain the compound to be purified.
[71] The method according to [66], wherein the contaminants comprise a sugar compound other than the compound represented by formula A-17, and/or a compound derived from a reaction reagent to obtain the compound to be purified.
[72] The method according to any one of [62] to [71], wherein the hydrophobic carrier is a resin for packing in reversed-phase partition chromatography.
[73] The method according to [72], wherein the resin for packing in reversed-phase partition chromatography is selected from the group consisting of poly(styrene/divinylbenzene) polymer gel resins, polystyrene-divinylbenzene resins, polyhydroxymethacrylate resins, styrene-vinylbenzene copolymer resins, polyvinyl alcohol resins, polystyrene resins, polymethacrylate resins, chemically bonded silica gel resins, and combinations thereof.
[74] The method according to [73], wherein the chemically bonded silica gel resin is selected from the group consisting of (1) resins obtained by reacting silica gel with a silane coupling agent, (2) resins obtained by chemically bonding a dimethyloctadecyl, octadecyl, trimethyloctadecyl, dimethyloctyl, octyl, butyl, ethyl, methyl, phenyl, cyanopropyl, or aminopropyl group to silica gel, and (3) resins obtained by chemically bonding a docosyl or triacontyl group to silica gel, and (4) combinations of the resins (1) to (3).
[75] The method according to [73], wherein the chemically bonded silica gel resin is an octadecyl group-bonded silica gel resin (ODS resin).
[76] The method according to any one of [62] to [75], wherein the water-soluble organic solvent is a water-soluble alcohol-based solvent, a water-soluble nitrile-based solvent, a water-soluble ether-based solvent, a water-soluble ketone-based solvent, a water-soluble amide-based solvent, a water-soluble sulfoxide-based solvent, or a mixed solvent containing at least one of the water-soluble organic solvent-based ones.
[77] The method according to [76], wherein the water-soluble nitrile-based solvent is acetonitrile.
[78] The method according to any one of [62] to [77], wherein the organic solvent used in the step of eluting a target substance from the hydrophobic carrier is a nitrile-based solvent, an ether-based solvent, an ester-based solvent, a ketone-based solvent, a halogen-based solvent, an aromatic solvent, or a mixed solvent containing at least one of the solvent systems.

Further, an embodiment of the present invention pertains to the following items, but is not limited to them.
[1] A method for producing an oligosaccharide represented by the following formula A'-22: 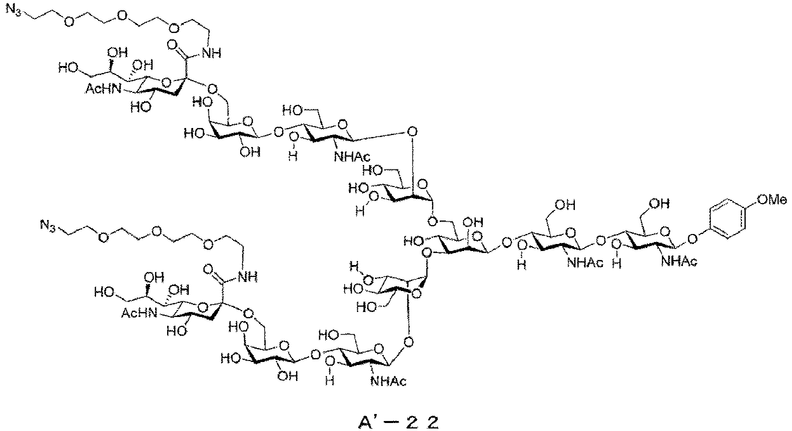 the method comprising the steps of:
   (step I-1)
      producing a compound represented by the following formula A-8: 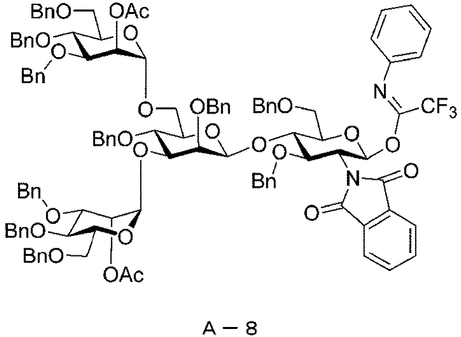 the step comprising the step of:
      subjecting a compound represented by formula A-3: 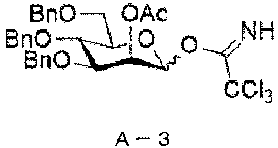
      and a compound represented by the following formula A-5: 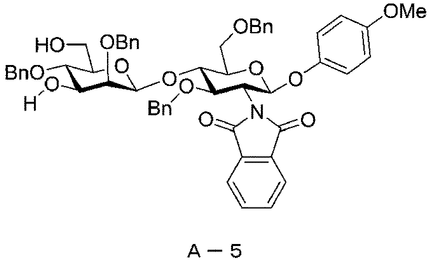
      to α-1,3-glycosidic linkage and α-1,6-glycosidic linkage to give a compound represented by the following formula A-6: 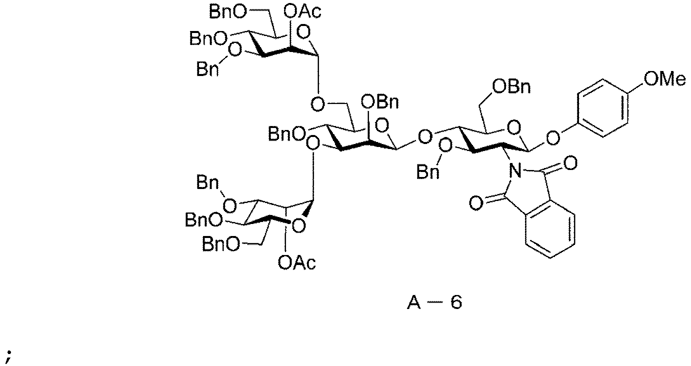
   (step I-2) producing a compound represented by the following formula A'-11: 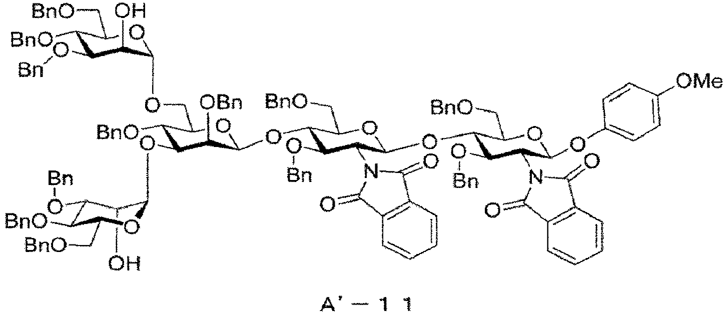 the step comprising the step of:
      subjecting the compound represented by formula A-8 and a compound represented by the following formula A'-9: 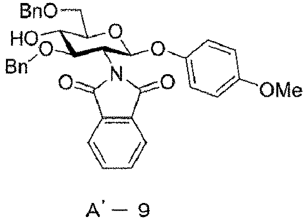
      to β-1,4-glycosidic linkage to give a compound represented by the following formula A'-10: 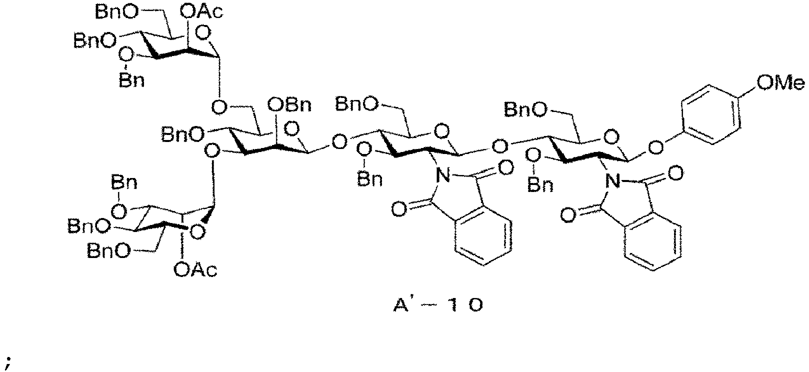
   (step I-3) subjecting the compound represented by formula A'-11 and a compound represented by the following formula A-12: 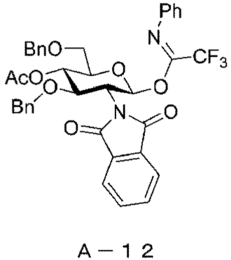 to β-1,2-glycosidic linkage to give a compound represented by the following formula A'-13: 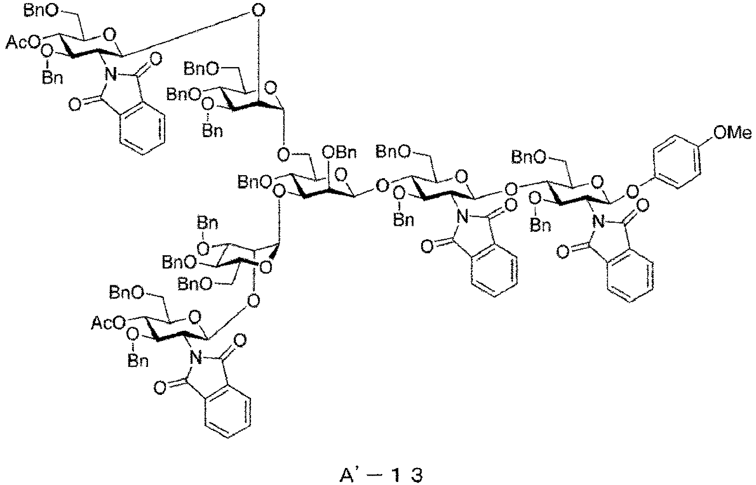 then eliminating each phthaloyl group as a protecting group of an amino group and each acetyl group on a hydroxyl group on the compound represented by formula A'-13 to give a compound represented by the following formula A'-14: 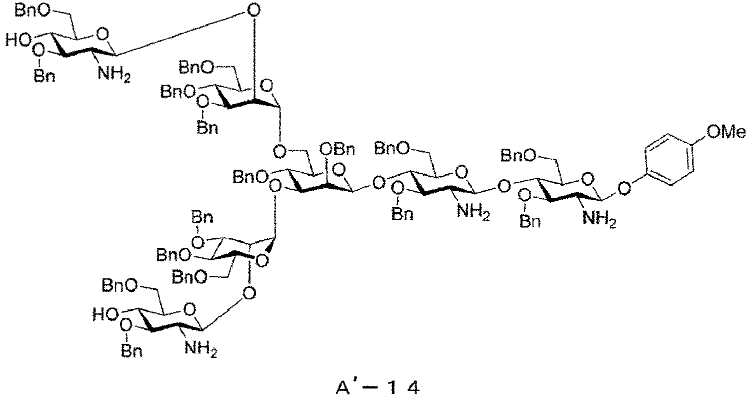 and then protecting the amino group in the compound represented by formula A'-14 by a protecting group selected from an aryloxycarbonyl (COOAr) group, an acetyl (Ac) group, a 2,2,2-trichloroethoxycarbonyl (Troc) group, or a phthaloyl (Phth) group to give a compound represented by the following formula A'-15: 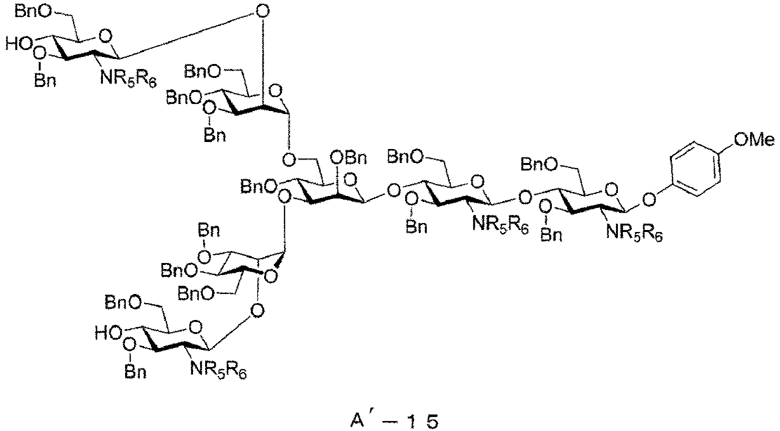 wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
      or eliminating each acetyl (Ac) group on the compound represented by formula A'-13 to give the compound represented by formula A'-15 wherein R₅ and R₆ together with a nitrogen atom attached thereto form a phthalimide group;
   (step I-4) producing a compound represented by the following formula A'-20: 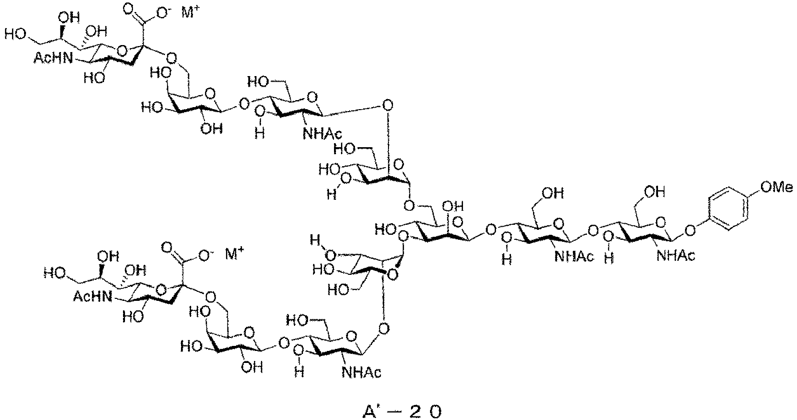 the step comprising:
      subjecting the compound represented by A'-15 and a compound represented by the following formula A-16: 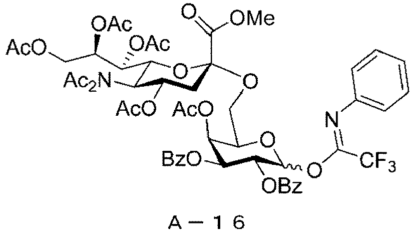
      to β-1,4-glycosidic linkage to give a compound represented by the following formula A'-17: 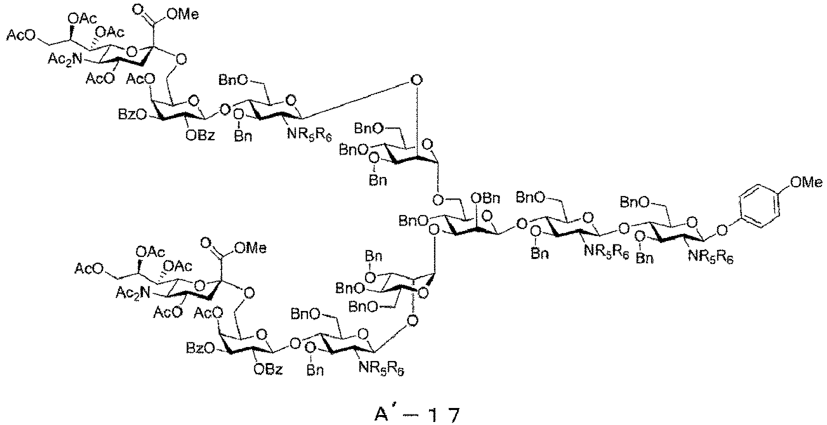
      wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
      and then eliminating each protecting group of an amino group and each acyl-based protecting group of a hydroxyl group in the compound represented by formula A'-17 to give a compound represented by formula A'-18: 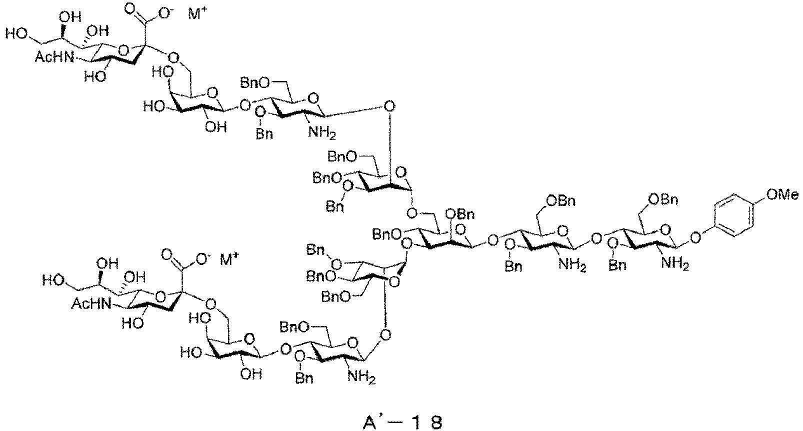
      wherein M⁺ is a sodium ion, lithium ion, potassium ion, or protonated triethylamine cation; and
   (step I-5) producing the oligosaccharide represented by formula A'-22 by reacting the compound represented by formula A'-20 with a compound represented by the following formula A-21: 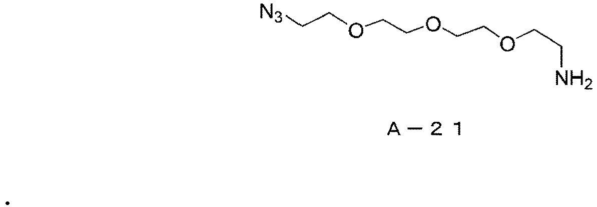
[2] The method according to [1], wherein the method comprises reacting a compound represented by the following formula A-4: 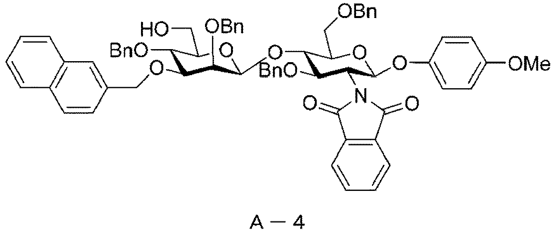 with 2,3-dichloro-5,6-dicyano-p-benzoquinone (DDQ) in a mixed solvent of fluorous alcohol and water to eliminate a 2-naphthylmethyl group in the compound represented by formula A-4 to give the compound represented by formula A-5.
[3] The method according to [2], wherein the fluorous alcohol is selected from the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and combinations thereof.
[4] The method according to [2] or [3], wherein the reaction is carried out at -35°C to 70°C.
[5] The method according to [2] or [3], wherein the reaction is carried out at -30°C to -10°C.
[6] The method according to any one of [1] to [5], wherein step I-1 comprises purifying the compound represented by formula A-6 by stopping the reaction of the compound represented by formula A-3 with the compound represented by formula A-5; then adding a hydrophobic carrier and water to a water-soluble organic solvent containing the resulting compound represented by formula A-6 and contaminants to adsorb the compound represented by formula A-6 onto the hydrophobic carrier; then filtering the resultant and washing the hydrophobic carrier with a mixed solution of the water-soluble organic solvent and the water to remove the contaminants; and then eluting the compound represented by formula A-6 using an organic solvent, from the hydrophobic carrier.
[7] The method according to any one of [1] to [6], wherein step I-2 comprises purifying the compound represented by formula A'-10 by stopping the reaction of the compound represented by formula A-8 with the compound represented by formula A'-9; then adding a hydrophobic carrier and water to a water-soluble organic solvent containing the resulting compound represented by formula A'-10 and contaminants to adsorb the compound represented by formula A'-10 onto the hydrophobic carrier; then filtering the resultant and washing the hydrophobic carrier with a mixed solution of the water-soluble organic solvent and the water to remove the contaminants; and then eluting the compound represented by formula A'-10 using an organic solvent, from the hydrophobic carrier.
[8] The method according to any one of [1] to [7], wherein step I-3 comprises purifying the compound represented by formula A'-13 by stopping the reaction of the compound represented by formula A'-11 with the compound represented by formula A-12; then adding a hydrophobic carrier and water to a water-soluble organic solvent containing the resulting compound represented by formula A'-13 and contaminants to adsorb the compound represented by formula A'-13 onto the hydrophobic carrier; then filtering the resultant and washing the hydrophobic carrier with a mixed solution of the water-soluble organic solvent and the water to remove the contaminants; and then eluting the compound represented by formula A'-13 using an organic solvent, from the hydrophobic carrier.
[9] The method according to any one of [1] to [8], wherein step I-3 comprises purifying the compound represented by formula A'-14 by adding a hydrophobic carrier and water to a water-soluble organic solvent containing the resulting compound represented by formula A'-14 and contaminants to adsorb the compound represented by formula A'-14 onto the hydrophobic carrier; then filtering the resultant and washing the hydrophobic carrier with a mixed solution of the water-soluble organic solvent and the water to remove the contaminants; and then eluting the compound represented by formula A'-14 using an organic solvent, from the hydrophobic carrier.
[10] The method according to any one of [1] to [9], wherein step I-4 comprises purifying the compound represented by formula A'-17 by stopping the reaction of the compound represented by formula A'-15 with the compound represented by formula A-16; then adding a hydrophobic carrier and water to a water-soluble organic solvent containing the resulting compound represented by formula A'-17 and contaminants to adsorb the compound represented by formula A'-17 onto the hydrophobic carrier; then filtering the resultant and washing the hydrophobic carrier with a mixed solution of the water-soluble organic solvent and the water to remove the contaminants; and then eluting the compound represented by formula A'-17 using an organic solvent, from the hydrophobic carrier.
[11] The method according to [6], wherein the contaminants comprise a sugar compound other than the compound represented by formula A-6, and/or a compound derived from a reaction reagent to obtain the compound to be purified.
[12] The method according to [7], wherein the contaminants comprise a sugar compound other than the compound represented by formula A'-10, and/or a compound derived from a reaction reagent to obtain the compound to be purified.
[13] The method according to [8], wherein the contaminants comprise a sugar compound other than the compound represented by formula A'-13, and/or a compound derived from a reaction reagent to obtain the compound to be purified.
[14] The method according to [9], wherein the contaminants comprise a sugar compound other than the compound represented by formula A'-14, and/or a compound derived from a reaction reagent to obtain the compound to be purified.
[15] The method according to [10], wherein the contaminants comprise a sugar compound other than the compound represented by formula A'-17, and/or a compound derived from a reaction reagent to obtain the compound to be purified.
[16] The method according to any one of [6] to [15], wherein the hydrophobic carrier is a resin for packing in reversed-phase partition chromatography.
[17] The method according to [16], wherein the resin for packing in reversed-phase partition chromatography is selected from the group consisting of poly(styrene/divinylbenzene) polymer gel resins, polystyrene-divinylbenzene resins, polyhydroxymethacrylate resins, styrene-vinylbenzene copolymer resins, polyvinyl alcohol resins, polystyrene resins, polymethacrylate resins, chemically bonded silica gel resins, and combinations thereof.
[18] The method according to [17], wherein the chemically bonded silica gel resin is selected from the group consisting of (1) resins obtained by reacting silica gel with a silane coupling agent, (2) resins obtained by chemically bonding a dimethyloctadecyl, octadecyl, trimethyloctadecyl, dimethyloctyl, octyl, butyl, ethyl, methyl, phenyl, cyanopropyl, or aminopropyl group to silica gel, and (3) resins obtained by chemically bonding a docosyl or triacontyl group to silica gel, and (4) combinations of the resins (1) to (3).
[19] The method according to [17], wherein the chemically bonded silica gel resin is an octadecyl group-bonded silica gel resin (ODS resin).
[20] The method according to any one of [6] to [19], wherein the water-soluble organic solvent is a water-soluble alcohol-based solvent, a water-soluble nitrile-based solvent, a water-soluble ether-based solvent, a water-soluble ketone-based solvent, a water-soluble amide-based solvent, or a water-soluble sulfoxide-based solvent, or a mixed solvent containing at least one of the water-soluble organic solvent-based ones.
[21] The method according to [20], wherein the water-soluble nitrile-based solvent is acetonitrile.
[22] The method according to any one of [6] to [21], wherein the organic solvent used in the step of eluting a target substance from the hydrophobic carrier is a nitrile-based solvent, an ether-based solvent, an ester-based solvent, a ketone-based solvent, a halogen-based solvent, an aromatic solvent, or a mixed solvent containing at least one of the solvent systems.
[23] The method according to any one of [1] to [22], wherein step I-2 comprises reacting the compound represented by formula A'-10 with a strong base in the presence of an alkyl ester of perfluorocarboxylic acid to give the compound represented by formula A'-11.
[24] The method according to [23], wherein the alkyl ester of perfluorocarboxylic acid is methyl trifluoroacetate, ethyl trifluoroacetate, propyl trifluoroacetate, isopropyl trifluoroacetate, butyl trifluoroacetate, methyl pentafluoropropionate, ethyl pentafluoropropionate, propyl pentafluoropropionate, isopropyl pentafluoropropionate, butyl pentafluoropropionate, methyl heptafluorobutyrate, ethyl heptafluorobutyrate, propyl heptafluorobutyrate, isopropyl heptafluorobutyrate, butyl heptafluorobutyrate, methyl nonafluorovalerate, ethyl nonafluorovalerate, propyl nonafluorovalerate, isopropyl nonafluorovalerate, butyl nonafluorovalerate, methyl undecafluorocaproate, ethyl undecafluorocaproate, propyl undecafluorocaproate, isopropyl undecafluorocaproate, or butyl undecafluorocaproate.
[25] The method according to [23] or [24], wherein the strong base is selected from the group consisting of sodium, lithium, and potassium salts of metal amides; sodium, lithium, potassium, cesium, and barium salts of C₁-C₂₀ alkoxides; sodium hydride, potassium hydride, lithium hydride, butyllithium, potassium carbonate, sodium carbonate, cesium carbonate, lithium carbonate, potassium phosphate, sodium phosphate, cesium phosphate, lithium phosphate, diazabicycloundecene (DBU), diazabicyclononene (DBN), and 1,1,3,3-tetramethylguanidine (TMG); and combinations thereof.
[26] The method according to [23] or [24], wherein the strong base is potassium tert-butoxide, sodium tert-butoxide, lithium tert-butoxide, or lithium hexamethyldisilazide (LHMDS).
[27] The method according to any one of [23] to [26], wherein the step of reacting the compound represented by formula A'-10 with a strong base in the presence of an alkyl ester of perfluorocarboxylic acid to give the compound represented by formula A'-11 is carried out in a C₁-C₁₀ alcohol solvent alone or in a mixture of a C₁-C₁₀ alcohol solvent and an amide-based solvent, ether-based solvent, ester-based solvent, aromatic solvent, halogen-based solvent, hydrocarbon-based solvent, or nitrile-based solvent.
[28] The method according to any one of [1] to [27], wherein in step I-3, each amino group in the compound represented by formula A'-14 is protected with an aryloxycarbonyl (COOAr) group to give the compound represented by formula A'-15.
[29] The method according to any one of [1] to [28], wherein in step I-3, the step of producing the compound represented by formula A'-15 from the compound represented by formula A'-14 is carried out in an aqueous solution containing sodium bicarbonate, potassium bicarbonate, disodium hydrogen phosphate, or dipotassium hydrogen phosphate.
[30] A method for producing a compound represented by the following formula A-5: 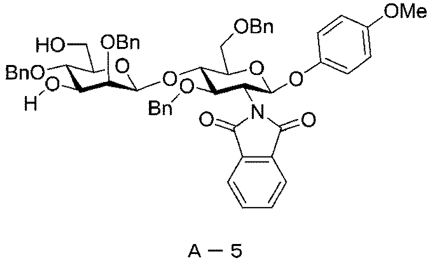 the method comprising reacting a compound represented by the following formula A-4: 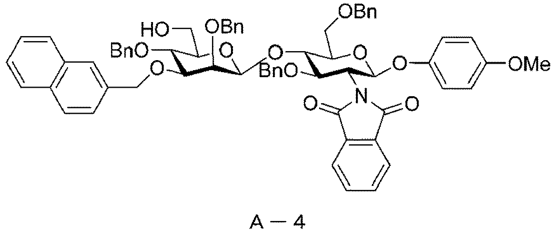 with 2,3-dichloro-5,6-dicyano-p-benzoquinone (DDQ) in a mixed solvent of fluorous alcohol and water to eliminate a 2-naphthylmethyl group in the compound represented by formula A-4 to give the compound represented by formula A-5.
[31] The method according to [30], wherein the fluorous alcohol is selected from the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and combinations thereof.
[32] The method according to [30] or [31], wherein the reaction is carried out at -35°C to 70°C.
[33] The method according to [30] or [31], wherein the reaction is carried out at -30°C to -10°C.
[34] A method of purifying a compound represented by the following formula A-6: 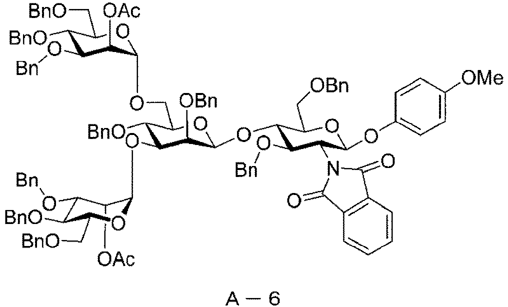 , the method comprising the steps of: adding a hydrophobic carrier and water to a water-soluble organic solvent containing the compound represented by formula A-6 and contaminants to adsorb the compound represented by formula A-6 onto the hydrophobic carrier; then filtering the resultant and washing the hydrophobic carrier with a mixed solution of the water-soluble organic solvent and the water to remove the contaminants; and then eluting the compound represented by formula A-6 using an organic solvent, from the hydrophobic carrier to purify the compound represented by formula A-6.
[35] A method of purifying a compound represented by the following formula A'-10: 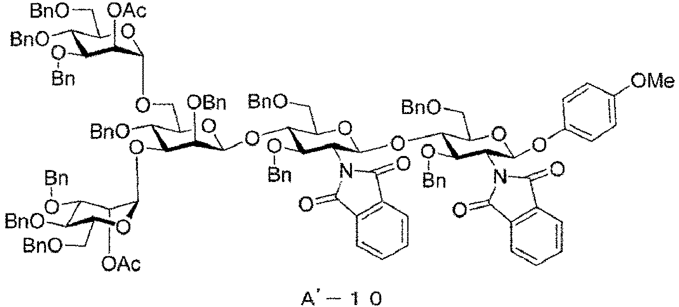 , the method comprising the steps of: adding a hydrophobic carrier and water to a water-soluble organic solvent containing the compound represented by formula A'-10 and contaminants to adsorb the compound represented by formula A'-10 onto the hydrophobic carrier; then filtering the resultant and washing the hydrophobic carrier with a mixed solution of the water-soluble organic solvent and the water to remove the contaminants; and then eluting the compound represented by formula A'-10 using an organic solvent, from the hydrophobic carrier to purify the compound represented by formula A'-10.
[36] A method of purifying a compound represented by the following formula A'-13: 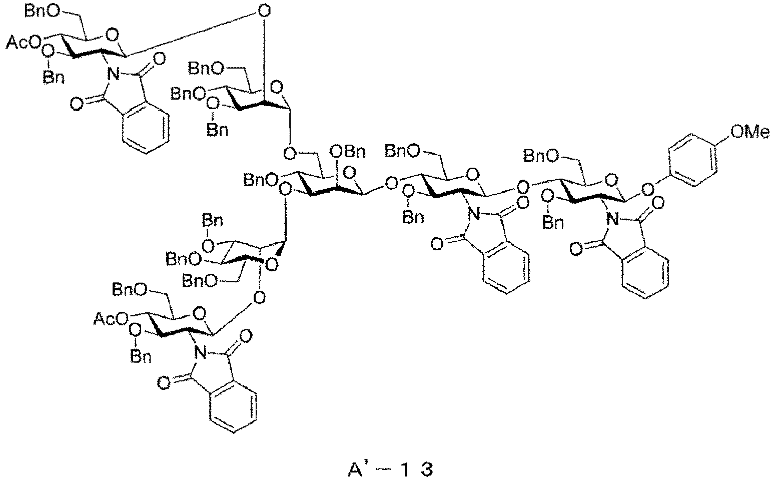 , the method comprising the steps of: adding a hydrophobic carrier and water to a water-soluble organic solvent containing the compound represented by formula A'-13 and contaminants to adsorb the compound represented by formula A'-13 onto the hydrophobic carrier; then filtering the resulting material and washing the hydrophobic carrier with a mixed solution of the water-soluble organic solvent and the water to remove the contaminants; and then eluting the compound represented by formula A'-13 using an organic solvent, from the hydrophobic carrier to purify the compound represented by formula A'-13.
[37] A method of purifying a compound represented by the following formula A'-14: 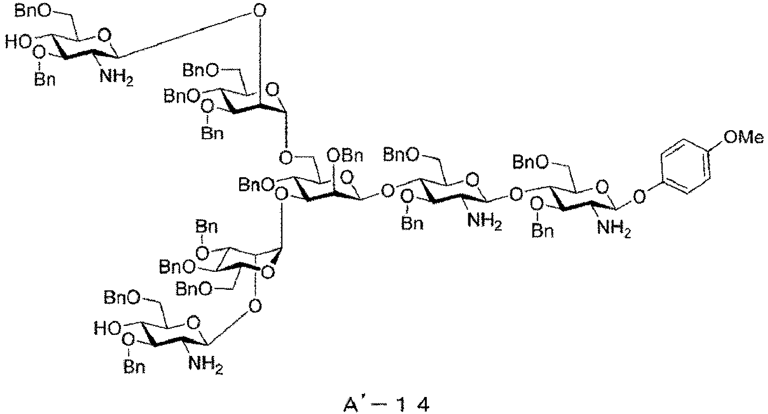 , the method comprising the steps of: adding a hydrophobic carrier and water to a water-soluble organic solvent containing the compound represented by formula A'-14 and contaminants to adsorb the compound represented by formula A'-14 onto the hydrophobic carrier; then filtering the resulting material and washing the hydrophobic carrier with a mixed solution of the water-soluble organic solvent and the water to remove the contaminants; and then eluting the compound represented by formula A'-14 using an organic solvent, from the hydrophobic carrier to purify the compound represented by formula A'-14.
[38] A method of purifying the compound represented by the following formula A'-17: 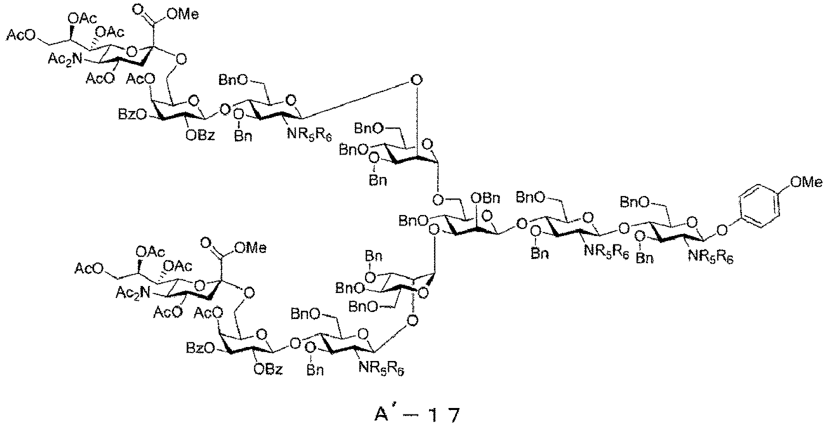 wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group, the method comprising the steps of: adding a hydrophobic carrier and water to a water-soluble organic solvent containing the compound represented by formula A'-17 and contaminants to adsorb the compound represented by formula A'-17 onto the hydrophobic carrier; then filtering the resultant and washing the hydrophobic carrier with a mixed solution of the water-soluble organic solvent and the water to remove the contaminants; and then eluting the compound represented by formula A'-17 using an organic solvent, from the hydrophobic carrier to purify the compound represented by formula A'-17.
[39] The method according to [34], wherein the contaminants comprise a sugar compound other than the compound represented by formula A-6, and/or a compound derived from a reaction reagent to obtain the compound to be purified.
[40] The method according to [35], wherein the contaminants comprise a sugar compound other than the compound represented by formula A'-10, and/or a compound derived from a reaction reagent to obtain the compound to be purified.
[41] The method according to [36], wherein the contaminants comprise a sugar compound other than the compound represented by formula A'-13, and/or a compound derived from a reaction reagent to obtain the compound to be purified.
[42] The method according to [37], wherein the contaminants comprise a sugar compound other than the compound represented by formula A'-14, and/or a compound derived from a reaction reagent to obtain the compound to be purified.
[43] The method according to [38], wherein the contaminants comprise a sugar compound other than the compound represented by formula A'-17, and/or a compound derived from a reaction reagent to obtain the compound to be purified.
[44] The method according to any one of [34] to [43], wherein the hydrophobic carrier is a resin for packing in reversed-phase partition chromatography.
[45] The method according to [44], wherein the resin for packing in reversed-phase partition chromatography is selected from the group consisting of poly(styrene/divinylbenzene) polymer gel resins, polystyrene-divinylbenzene resins, polyhydroxymethacrylate resins, styrene-vinylbenzene copolymer resins, polyvinyl alcohol resins, polystyrene resins, polymethacrylate resins, chemically bonded silica gel resins, and combinations thereof.
[46] The method according to [45], wherein the chemically bonded silica gel resin is selected from the group consisting of (1) resins obtained by reacting silica gel with a silane coupling agent, (2) resins obtained by chemically bonding a dimethyloctadecyl, octadecyl, trimethyloctadecyl, dimethyloctyl, octyl, butyl, ethyl, methyl, phenyl, cyanopropyl, or aminopropyl group to silica gel, and (3) resins obtained by chemically bonding a docosyl or triacontyl group to silica gel, and (4) combinations of the resins (1) to (3).
[47] The method according to [45], wherein the chemically bonded silica gel resin is an octadecyl group-bonded silica gel resin (ODS resin).
[48] The method according to any one of [34] to [47], wherein the water-soluble organic solvent is a water-soluble alcohol-based solvent, a water-soluble nitrile-based solvent, a water-soluble ether-based solvent, a water-soluble ketone-based solvent, a water-soluble amide-based solvent, a water-soluble sulfoxide-based solvent, or a mixed solvent containing at least one of the water-soluble organic solvent-based ones.
[49] The method according to [48], wherein the water-soluble nitrile-based solvent is acetonitrile.
[50] The method according to any one of [34] to [49], wherein the organic solvent used in the step of eluting a target substance from the hydrophobic carrier is a nitrile-based solvent, an ether-based solvent, an ester-based solvent, a ketone-based solvent, a halogen-based solvent, an aromatic solvent, or a mixed solvent containing at least one of the solvent systems.
[51] A method for producing a compound represented by the following formula A'-11: 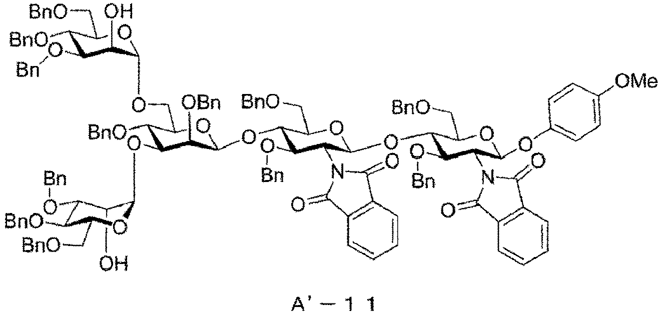 the method comprising the step of reacting a compound represented by the following formula A'-10: 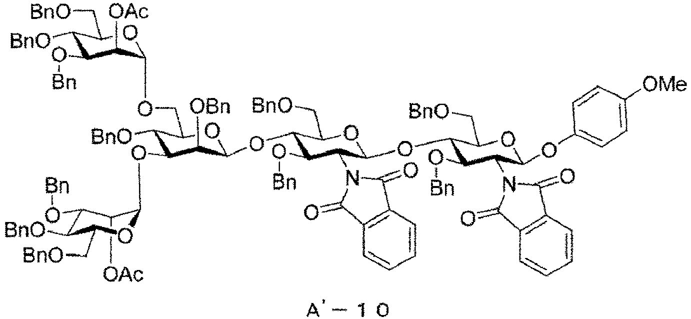 with a strong base in the presence of an alkyl ester of perfluorocarboxylic acid.
[52] The method according to [51], wherein the alkyl ester of perfluorocarboxylic acid is methyl trifluoroacetate, ethyl trifluoroacetate, propyl trifluoroacetate, isopropyl trifluoroacetate, butyl trifluoroacetate, methyl pentafluoropropionate, ethyl pentafluoropropionate, propyl pentafluoropropionate, isopropyl pentafluoropropionate, butyl pentafluoropropionate, methyl heptafluorobutyrate, ethyl heptafluorobutyrate, propyl heptafluorobutyrate, isopropyl heptafluorobutyrate, butyl heptafluorobutyrate, methyl nonafluorovalerate, ethyl nonafluorovalerate, propyl nonafluorovalerate, isopropyl nonafluorovalerate, butyl nonafluorovalerate, methyl undecafluorocaproate, ethyl undecafluorocaproate, propyl undecafluorocaproate, isopropyl undecafluorocaproate, or butyl undecafluorocaproate.
[53] The method according to [51] or [52], wherein the strong base is selected from the group consisting of sodium, lithium, and potassium salts of metal amides; sodium, lithium, potassium, cesium, and barium salts of C₁-C₂₀ alkoxides; sodium hydride, potassium hydride, lithium hydride, butyllithium, potassium carbonate, sodium carbonate, cesium carbonate, lithium carbonate, potassium phosphate, sodium phosphate, cesium phosphate, lithium phosphate, diazabicycloundecene (DBU), diazabicyclononene (DBN), and 1,1,3,3-tetramethylguanidine (TMG); and combinations thereof.
[54] The method according to [51] or [52], wherein the strong base is potassium tert-butoxide, sodium tert-butoxide, lithium tert-butoxide, or lithium hexamethyldisilazide (LHMDS).
[55] The method according to any one of [51] to [54], wherein the reaction is carried out in a C₁-C₁₀ alcohol solvent alone or in a mixture of a C₁-C₁₀ alcohol solvent and an amide-based solvent, ether-based solvent, ester-based solvent, aromatic solvent, halogen-based solvent, hydrocarbon-based solvent, or nitrile-based solvent.
[56] A method for producing a compound represented by the following formula A'-17: 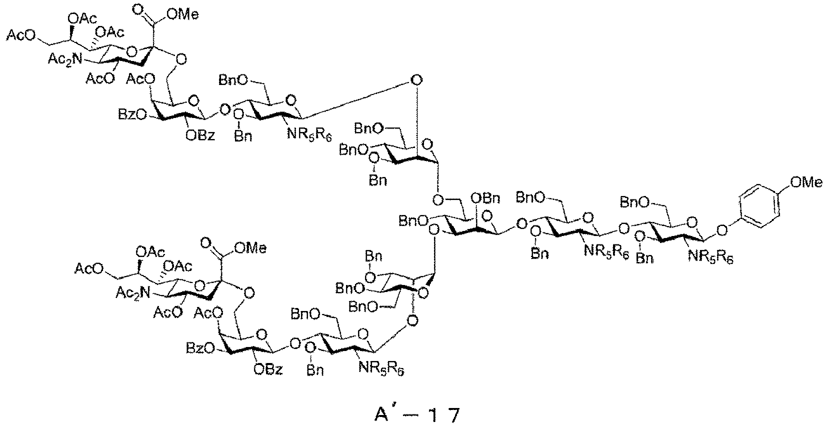
   wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
   the method comprising the step of producing a compound represented by the following formula A'-15: 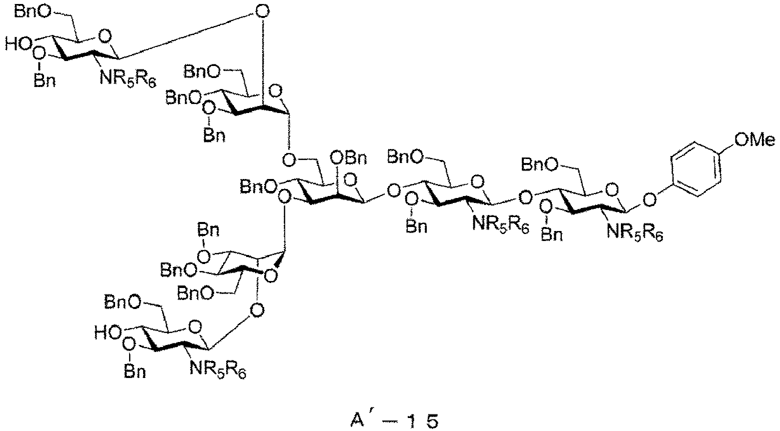
   wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group, and then subjecting the compound and a compound represented by the following formula A-16: 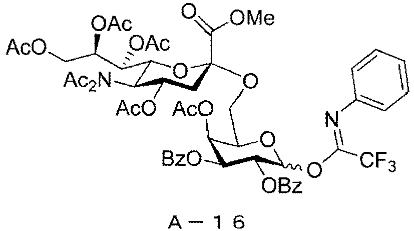
   to β-1,4-glycosidic linkage to give the compound represented by formula A'-17.
[57] The method according to [56], wherein R₅ is an aryloxycarbonyl (COOAr) group.
[58] A method for producing a compound represented by the following formula A'-18: 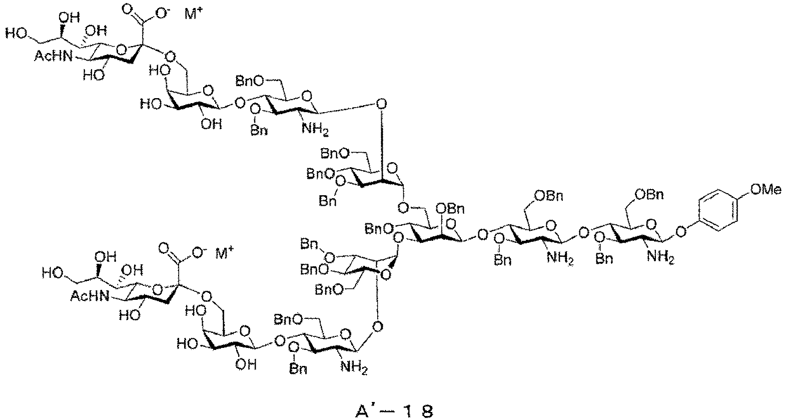
   wherein M⁺ is a sodium ion, lithium ion, potassium ion, or protonated triethylamine cation,
   the method comprising eliminating each protecting group of an amino group and each acyl-based protecting group of a hydroxyl group in a compound represented by the following formula A'-17: 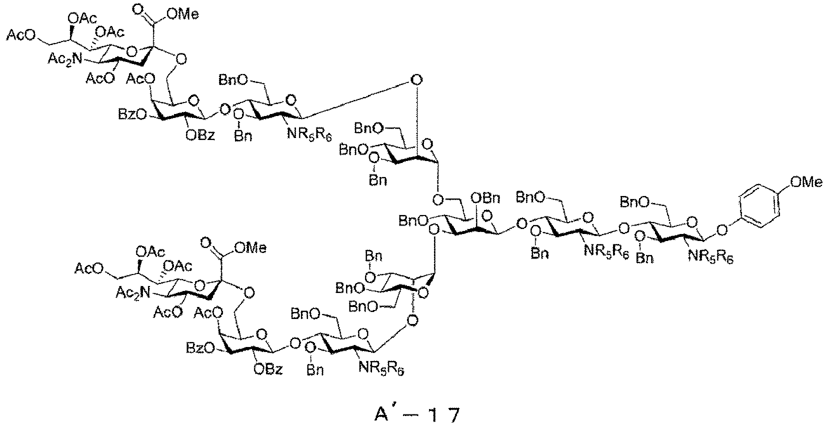
   wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group.
[59] An oligosaccharide represented by the following formula A'-22: 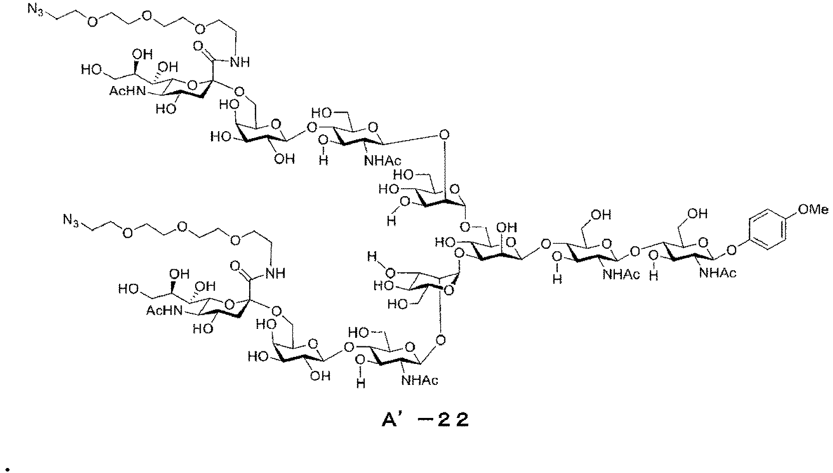
[60] A compound represented by the following formula A'-8: 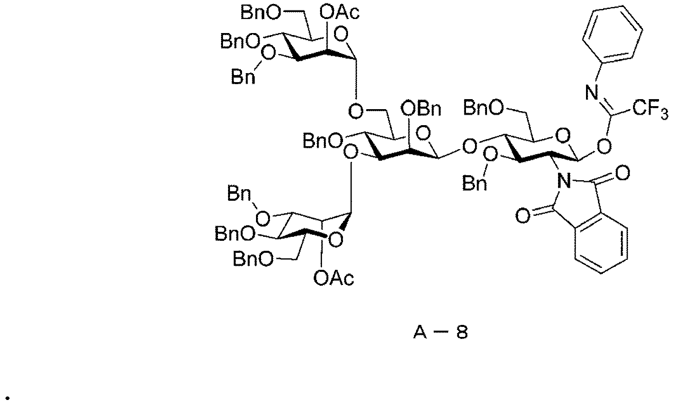
[61] A compound represented by the following formula A'-10: 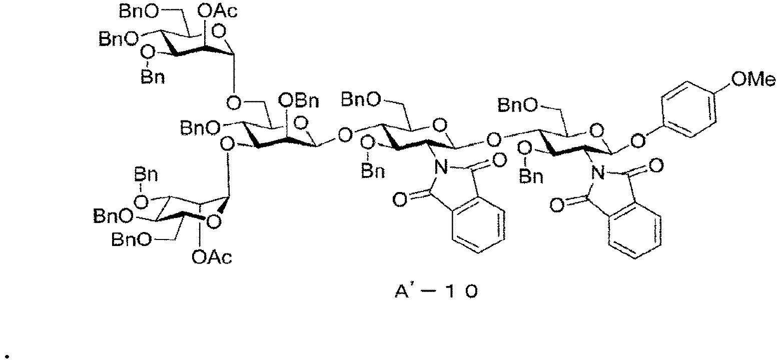
[62] A compound represented by the following formula A'-11: 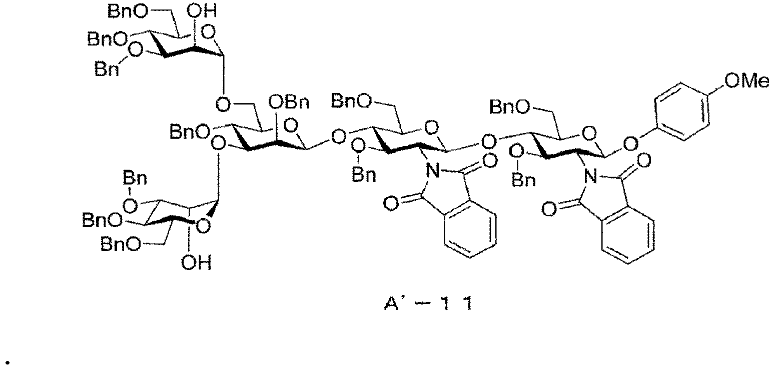
[63] A compound represented by the following formula A'-13: 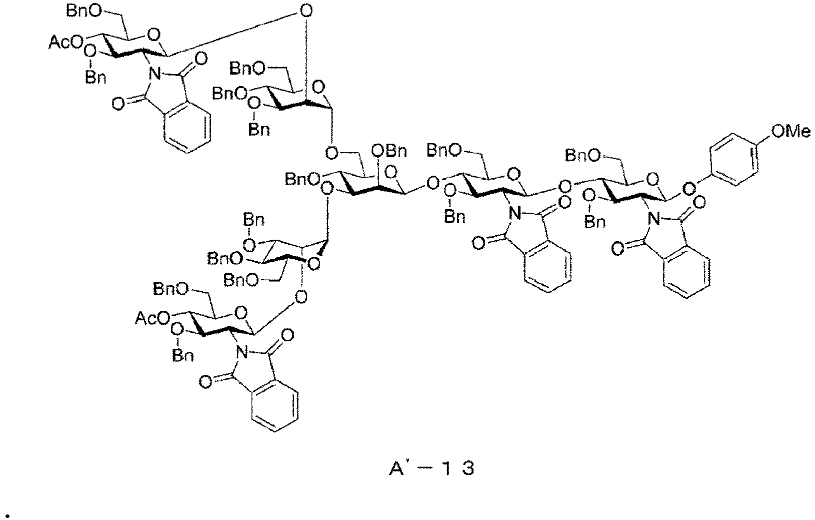
[64] A compound represented by the following formula A'-14: 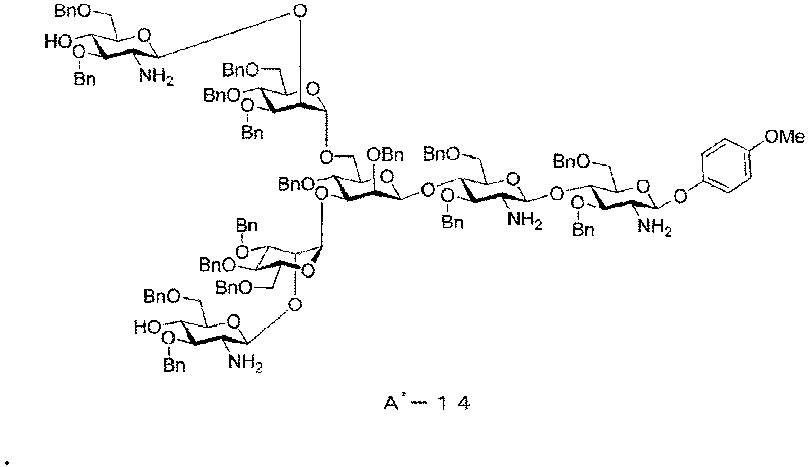
[65] A compound represented by the following formula A'-15: 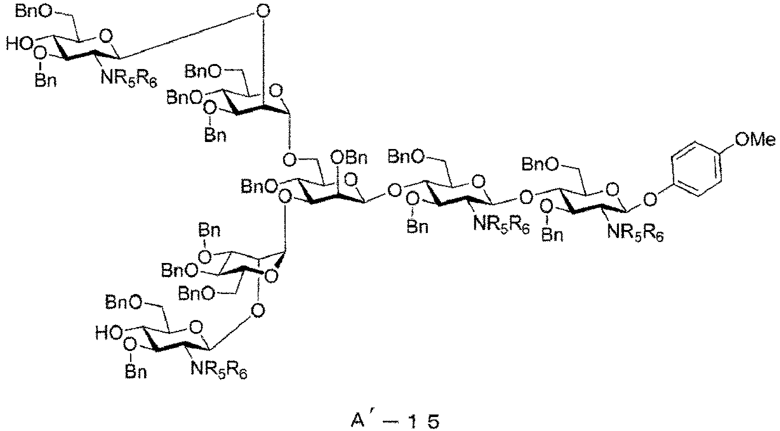 wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group.
[66] A compound represented by the following formula A'-17: 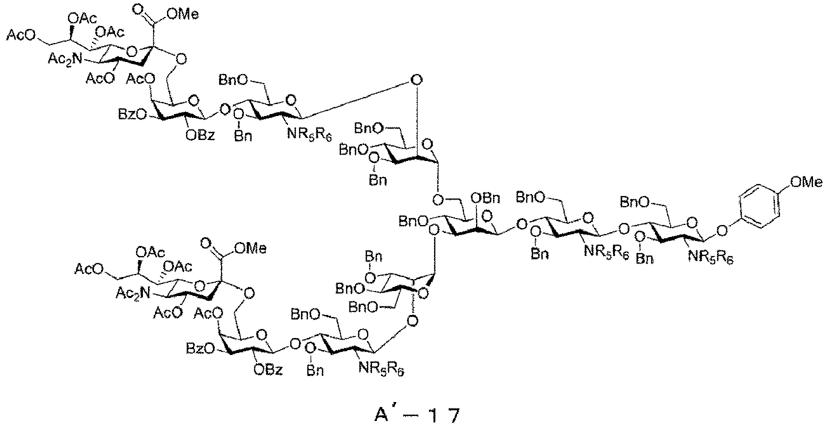 wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group.
[67] A compound represented by the following formula A'-18: 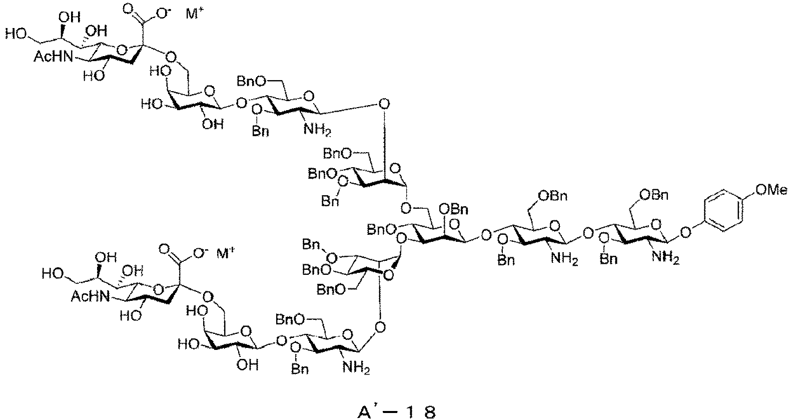 wherein M⁺ is a sodium ion, lithium ion, potassium ion, or protonated triethylamine cation.
[68] A compound represented by the following formula A'-19: 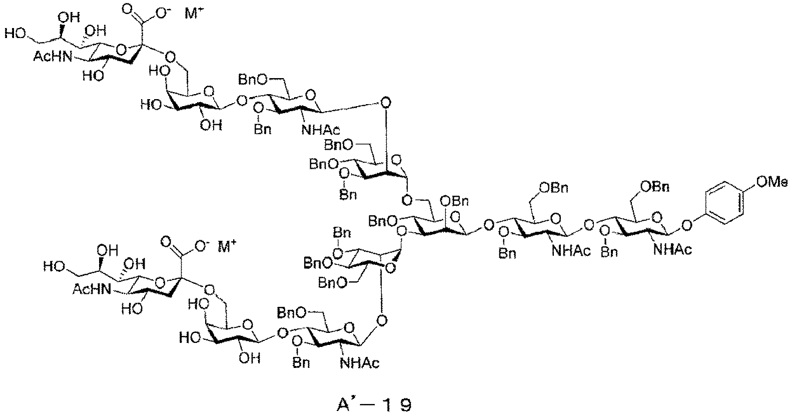 wherein M⁺ is a sodium ion, lithium ion, potassium ion, or protonated triethylamine cation.
[69] A compound represented by the following formula A'-20: 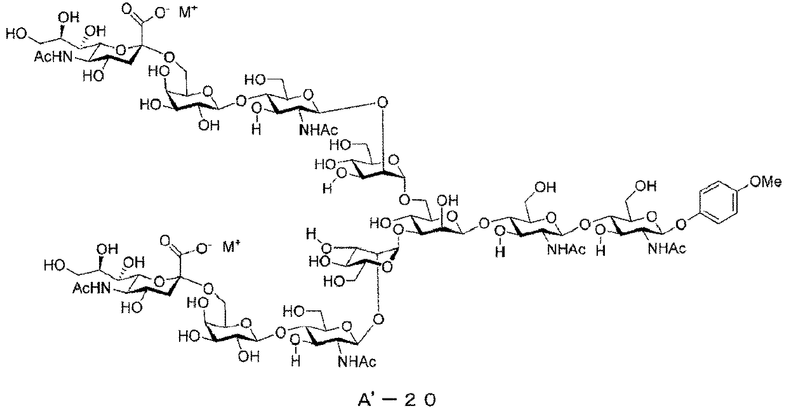 wherein M⁺ is a sodium ion, lithium ion, potassium ion, or protonated triethylamine cation.

### Advantageous Effects of Invention

The present invention provides an oligosaccharide represented by formula A-22 above and a novel production method therefor, and an intermediate for production of the oligosaccharide and a production method therefor.

### Brief Description of Drawings

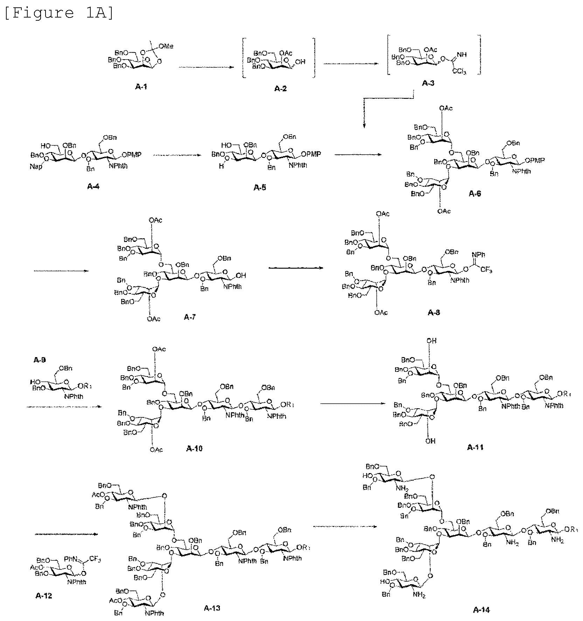

[Figure 1A] Figure 1A shows a simplified example of a part of a novel method for producing an oligosaccharide represented by formula A-22, which is provided in the present invention; and more specifically shows a simplified method for producing compounds represented by formulas A-2 to A-14 using a compound represented by formula A-1 as a starting material.

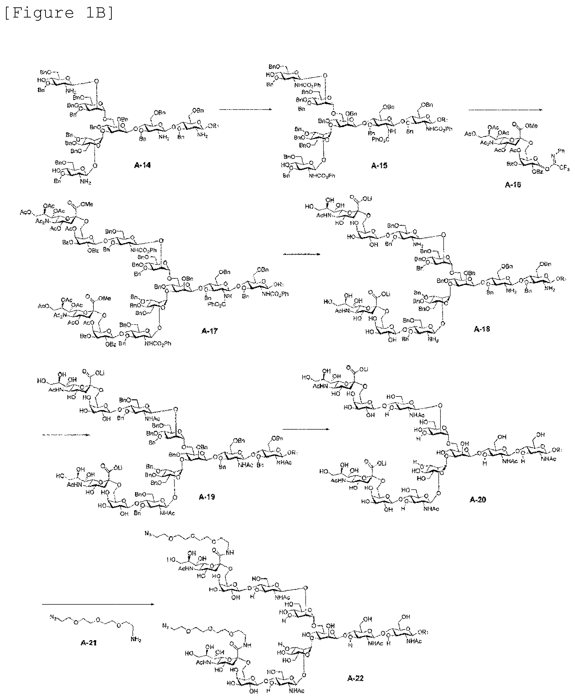

[Figure 1B] Figure 1B shows a simplified example of a part of a novel method for producing an oligosaccharide represented by formula A-22, which is provided in the present invention; and more specifically shows a simplified method for producing compounds represented by formulas A-15 to A-22 using a compound represented by formula A-14 as a starting material.

### Description of Embodiments

Hereinafter, preferable embodiments of the present invention will be described. Note that the below-described embodiments are representative examples of the embodiments of the present invention. The scope of the present invention should not be narrowly construed due to them.

### <1. Method for producing oligosaccharide represented by formula A-22>

An embodiment of the present invention provides a novel oligosaccharide represented by formula A-22 and a novel production method therefor. In the present invention, the oligosaccharide represented by formula A-22 means the following oligosaccharide.

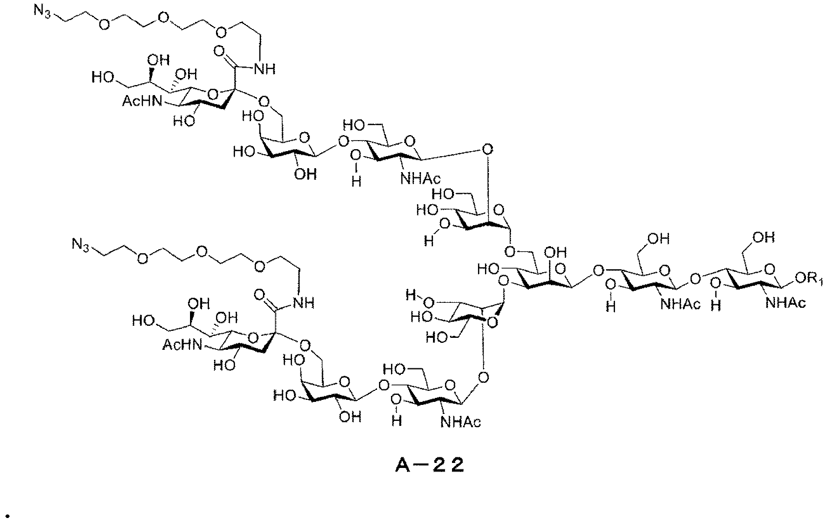

The new synthetic scheme for the oligosaccharide represented by formula A-22 above comprises the following steps I-1 to I-5.

Throughout the specification, the substituent "R₁" in oligosaccharides and compounds is a C₁ to C₆ alkyl group or a phenyl group, and the phenyl group is optionally substituted with one to three (i.e., one, two, or three) C₁ to C₆ alkoxy groups. The phenyl group may be unsubstituted.

As used herein, the term "Cₓ to C_{y} alkyl group" refers to a linear, branched, or cyclic saturated aliphatic hydrocarbon group containing x to y carbons. Examples of the linear saturated aliphatic hydrocarbon group include methyl, ethyl, n-propyl, n-butyl, n-pentyl, and n-hexyl; and examples of the branched saturated aliphatic hydrocarbon group include isopropyl, isobutyl, tert-butyl, sec-butyl, isopentyl, tert-pentyl, sec-pentyl, isohexyl, tert-hexyl, and sec-hexyl. As described above, the "alkyl group" also includes a cyclic saturated aliphatic hydrocarbon group, i.e., "cycloalkyl", and the "cycloalkyl" may include a fused or bridged ring system. Examples of cycloalkyl include cyclopropyl, cyclobutyl, cyclopentyl, and cyclohexyl.

As used herein, the term "alkoxy group" refers to an alkyl group attached via oxygen (i.e., alkyl-O-), wherein the alkyl group is as defined above. Examples of the alkoxy group include methoxy, ethoxy, propoxy, and tert-butoxy.

In an embodiment of the present invention, the substituent "R₁" in oligosaccharides and compounds is preferably n-propyl, n-butyl, n-pentyl, n-hexyl, isopropyl, isobutyl, tert-butyl, isopentyl, tert-pentyl, sec-pentyl, cyclopropyl, cyclobutyl, cyclopentyl, cyclohexyl, 4-methoxyphenyl, 3-methoxyphenyl, 2-methoxyphenyl, 3,4-dimethoxyphenyl, 2,4-dimethoxyphenyl, 2,3-dimethoxyphenyl, 3,4,5-trimethoxyphenyl, or phenyl; more preferably isopropyl, isobutyl, tert-butyl, 4-methoxyphenyl, 3-methoxyphenyl, or 2-methoxyphenyl; and still more preferably isopropyl or 4-methoxyphenyl.

Throughout the specification, an embodiment of the present invention also includes "salts" of the oligosaccharide represented by formula A-22 and novel production intermediates thereof (e.g., compounds represented by formulas A-2 to A-20). For example, a salt can be formed with a carboxyl group, an amino group, etc., in the oligosaccharide represented by formula A-22 and each compound used for producing the oligosaccharide. Examples of the salt that may be formed include acid addition salts formed with inorganic acids, such as hydrochloric acid, sulfuric acid, hydrobromic acid, and phosphoric acid; or organic acids, such as acetic acid, propionic acid, trimethylacetic acid, tert-butylacetic acid, hexanoic acid, succinic acid, malic acid, fumaric acid, tartaric acid, citric acid, benzoic acid, methanesulfonic acid, and ethanesulfonic acid. A salt can also be formed when an acidic proton present can react with an inorganic or organic base; examples of the inorganic base include, but are not limited to, sodium hydroxide, potassium hydroxide, sodium carbonate, calcium hydroxide, and lithium hydroxide, and examples of the organic base include, but are not limited to, ethanolamine, diethanolamine, triethanolamine, and triethylamine. In one embodiment, the "salt" is preferably a pharmaceutically acceptable salt.

### <Step I-1>

Step I-1 is a step of producing a compound represented by the following formula A-8: 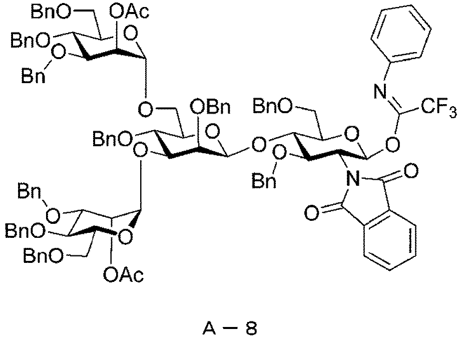 the step comprising the step of:
subjecting a compound represented by formula A-3: 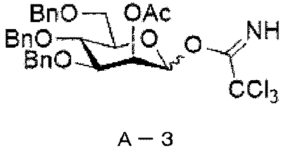
and a compound represented by the following formula A-5: 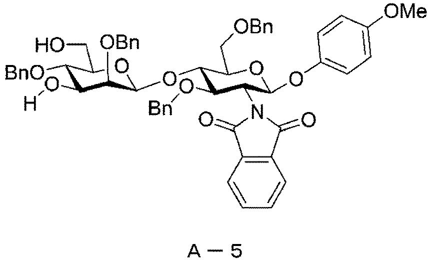
to α-1,3-glycosidic linkage and α-1,6-glycosidic linkage to give a compound represented by the following formula A-6: 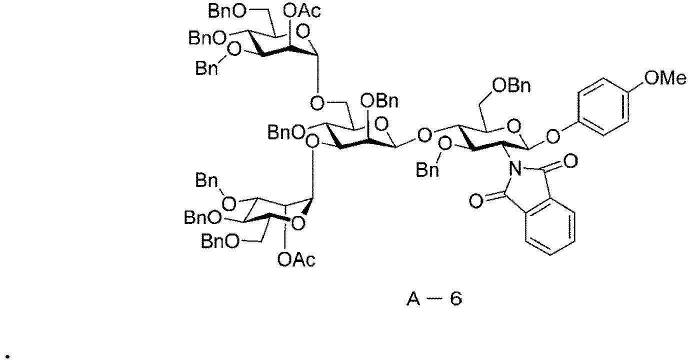
The step I-1 comprises the following steps I-1-1 to I-1-3.

### <Step I-1-1>

Step I-1-1 is a step of subjecting the compound represented by formula A-3 and the compound represented by formula A-5 to α-1,3-glycosidic linkage and α-1,6-glycosidic linkage to give a compound represented by formula A-6. This step may be performed by using or applying a known procedure. However, a procedure shown in Example 32 or 55, for example, may be preferably used. For example, by sequentially adding, in an organic solvent (e.g., toluene), Molecular Sieves 4A powder and trimethylsilyl trifluoromethanesulfonate (TMSOTf), the compound represented by formula A-3 and the compound represented by formula A-5 above may be subjected to α-1,3-glycosidic linkage and α-1,6-glycosidic linkage to produce the compound represented by formula A-6 above. Here, the starting materials, compounds represented by formula A-3 and formula A-5, may be produced as follows.

### <Production of compound represented by formula A-3>

In one embodiment of the present invention, the compound represented by formula A-3 may be produced by the following steps, but the method is not limited to this production method.

First, the compound (3,4,6-tri-O-benzyl-1,2-O-(1-methoxyethyliden)-β-D-mannopyranose) represented by the following formula A-1: 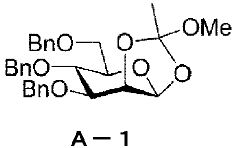 is added to, for example, water and p-TsOH·H₂O and then reacted with triethylamine to give a compound represented by the following formula A-2: 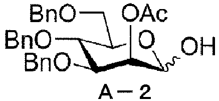 . This step may be preferably performed using, for instance, the procedure shown in Example 29 or 52.

Next, for example, trichloroacetonitrile and diazabicycloundecene (DBU) are added to the compound represented by formula A-2 to produce the compound represented by formula A-3. This step may be preferably performed using, for instance, the procedure shown in Example 30 or 53.

### <Production of compound represented by formula A-5>

The compound represented by formula A-5 is produced by first producing a compound represented by the following formula A-4, and then producing a compound represented by formula A-5 from that compound.

In one embodiment of the present invention, the compound represented by formula A-4 is produced by the following steps X-1 to X-14 or the following steps X-1 to X-8 + X-15 to X-16. The details of each step are illustrated below. Each step can be also carried out by using or applying usual procedures for producing a monosaccharide or oligosaccharide.

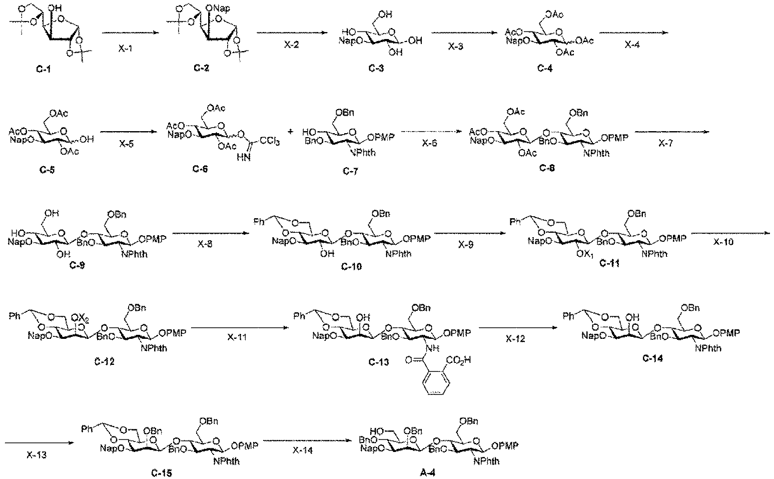

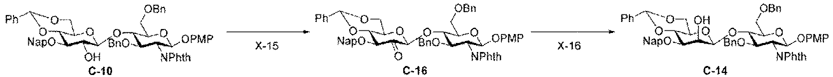

### <Step X-1>

Step X-1 is a step of protecting, by 2-naphthylmethyl (NAP) group, a hydroxyl group attached to carbon at position 3 of a compound represented by the following formula C-1: 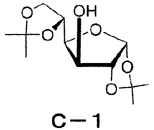 to produce a compound represented by the following formula C-2: 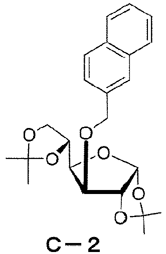 . The compound represented by formula C-1, which is the starting material for this step, may be produced by a known procedure or a commercially available product may be used. Examples of the commercially available compound represented by formula C-1 include 1,2:5,6-di-O-isopropylidene-α-D-glucofuranose manufactured by Sigma-Aldrich. This step may be performed by using or applying a known procedure. However, the procedure shown in Example 1, for example, may be preferably used.

### <Step X-2>

Step X-2 is a step of acid hydrolysis of two isopropylidene moieties of the compound represented by formula C-2 and pyranose ring formation to produce a compound represented by the following formula C-3: 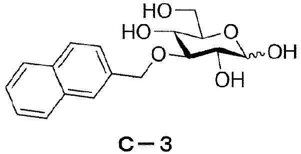 . This step may be performed by using or applying a known procedure. However, the procedure shown in Example 2, for example, may be preferably used.

### <Step X-3>

Step X-3 is a step of protecting, by an acetyl group, each hydroxyl group on the compound represented by formula C-3 to produce a compound represented by the following formula C-4: 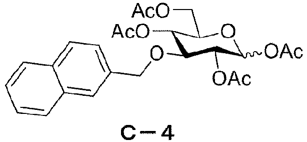 . This step may be performed by using or applying a known procedure. However, the procedure shown in Example 3, for example, may be preferably used.

### <Step X-4>

Step X-4 is a step of selectively eliminating only the acetyl group of the acetyloxy group attached to carbon at position 1 of the compound represented by formula C-4 to produce a compound represented by the following formula C-5: 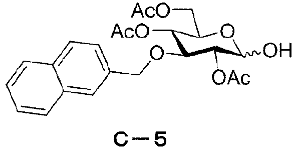 . This step may be performed by using or applying a known procedure. However, the procedure shown in Example 3, for example, may be preferably used.

The above step of producing the compound represented by formula C-5 from the compound represented by formula C-3 may be performed in a one-pot process, for example, as shown in Example 3.

### <Step X-5>

Step X-5 is a step of reacting the compound represented by formula C-5 with trichloroacetonitrile to produce a compound represented by the following formula C-6: 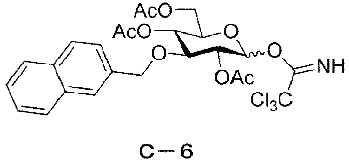 . This step may be performed by using or applying a known procedure. However, the procedure shown in Example 4, for example, may be preferably used.

### <Step X-6>

Step X-6 is a step of reacting the compound represented by formula C-6 with a compound represented by the following formula C-7: 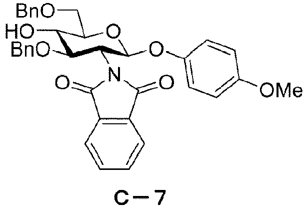 to produce a compound represented by the following formula C-8: 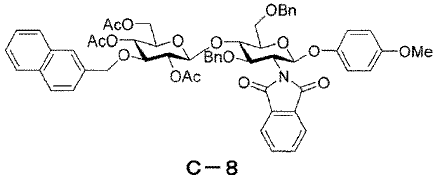 . The compound represented by formula C-7 may be produced by a known procedure or a commercially available product may be used. Examples of the commercially available compound represented by formula C-7 include 4-methoxyphenyl 3,6-di-O-benzyl-2-deoxy-2-phthalimido-β-D-glucopyranoside, manufactured by TOKYO CHEMICAL INDUSTRY CO., LTD. This step may be performed by using or applying a known procedure. However, the procedure shown in Example 5, for example, may be preferably used.

### <Step X-7>

Step X-7 is a step of eliminating each acetyl group from the compound represented by formula C-8 to produce a compound represented by the following formula C-9: 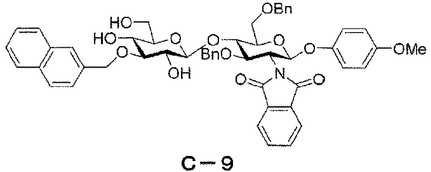 . This step may be performed by using or applying a known procedure. However, the procedure shown in Example 6, for example, may be preferably used.

In an embodiment of the present invention, step X-7 is a step of reacting the compound represented by formula C-8 with a strong base in the presence of a trifluoroacetate to eliminate each acetyl group to give the compound represented by formula C-9. A report (Org. Biomol. Chem., 2018, 16, 4720-4727) shows that the acetyl groups were eliminated using sodium methoxide in methanol. In this case, the phthalimide ring may be opened at the same time as an undesired side reaction. On the other hand, by using the technique of reaction with an alkoxide-based strong base in the presence of an alkyl ester of perfluorocarboxylic acid, the acetyl group(s) is eliminated while the ring opening of the phthalimide group is suppressed.

The "alkyl ester of perfluorocarboxylic acid" used in the above step is not limited as long as the reaction proceeds. Examples include methyl trifluoroacetate, ethyl trifluoroacetate, propyl trifluoroacetate, isopropyl trifluoroacetate, butyl trifluoroacetate, methyl pentafluoropropionate, ethyl pentafluoropropionate, propyl pentafluoropropionate, isopropyl pentafluoropropionate, butyl pentafluoropropionate, methyl heptafluorobutyrate, ethyl heptafluorobutyrate, propyl heptafluorobutyrate, isopropyl heptafluorobutyrate, butyl heptafluorobutyrate, methyl nonafluorovalerate, ethyl nonafluorovalerate, propyl nonafluorovalerate, isopropyl nonafluorovalerate, butyl nonafluorovalerate, methyl undecafluorocaproate, ethyl undecafluorocaproate, propyl undecafluorocaproate, isopropyl undecafluorocaproate, or butyl undecafluorocaproate. Methyl trifluoroacetate is preferred.

The above "strong base" is not limited as long as the reaction proceeds. For example, the strong base is selected from the group consisting of sodium, lithium, and potassium salts of metal amides; sodium, lithium, potassium, cesium, and barium salts of C1-C20 alkoxides; sodium hydride, potassium hydride, lithium hydride, butyllithium, potassium carbonate, sodium carbonate, cesium carbonate, lithium carbonate, potassium phosphate, sodium phosphate, cesium phosphate, lithium phosphate, diazabicycloundecene (DBU), diazabicyclononene (DBN), and 1,1,3,3-tetramethylguanidine (TMG); and combinations thereof. Examples of the sodium, lithium, or potassium salt of C1-C20 alkoxide include lithium methoxide, sodium methoxide, potassium methoxide, lithium ethoxide, sodium ethoxide, potassium ethoxide, lithium isopropoxide, sodium isopropoxide, potassium isopropoxide, lithium tert-butoxide, sodium tert-butoxide, potassium tert-butoxide, lithium tert-pentoxide, sodium tert-pentoxide, or potassium tert-pentoxide. Particularly preferable examples include sodium tert-butoxide, lithium tert-butoxide, potassium tert-butoxide, or lithium hexamethyldisilazide (LHMDS).

The solvent in this step is not limited as long as the reaction proceeds. For example, the reaction may be carried out in a C₁-C₁₀ alcohol solvent alone, or using a mixed solvent of C₁-C₁₀ alcohol solvent and amide-based solvent (e.g., dimethylformamide, dimethylacetamide), ether-based solvent (e.g., tetrahydrofuran, dimethoxyethane, cyclopentyl methyl ether), ester-based solvent (e.g., ethyl acetate), aromatic solvent (e.g., toluene), halogen-based solvent (e.g., dichloromethane), hydrocarbon-based solvent (e.g., hexane), or nitrile-based solvent (e.g., acetonitrile). It is possible to preferably use methanol or a mixed solvent system of methanol and tetrahydrofuran. However, the solvent is not limited to them. Note that the above "C1-C10 alcohol solvents" can be replaced by alcohols having more carbon atoms. However, C1-C5 alcohols (e.g., methanol, ethanol, propanol, butanol) may be preferably used due to their availability and convenience.

The reaction temperature in this step is not limited as long as the reaction proceeds, and may be, for example, from -20°C to 80°C, preferably from 0°C to 70°C, more preferably from 20°C to 65°C, and particularly preferably from 40°C to 60°C.

### <Step X-8>

Step X-8 is a step of selectively protecting, using benzaldehyde dimethyl acetal, the hydroxyl groups attached to the carbon atoms at positions 4 and 6 of D-glucopyranoside in the compound represented by formula C-9 to produce a compound represented by the following formula C-10: 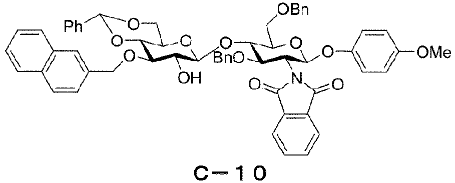 . This step may be performed by using or applying a known procedure. However, the procedure shown in Example 7, for example, may be preferably used.

### <Step X-9>

Step X-9 is a step of reacting the compound represented by formula C-10 with a compound that provides a leaving group selected from the group consisting of a trifluoromethanesulfonyloxy group, a nonafluorobutanesulfonyloxy group, a 2-nitrobenzenesulfonyloxy group, and a 4-nitrobenzenesulfonyloxy group to produce a compound represented by the following formula C-11: 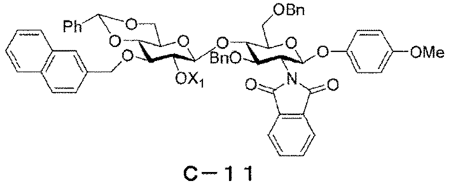 wherein X₁ represents a substituent selected from the group consisting of a trifluoromethanesulfonyl group, a nonafluorobutanesulfonyl group, a 2-nitrobenzenesulfonyl group, and a 4-nitrobenzenesulfonyl group. This step may be performed by using or applying a known procedure for providing a leaving group. However, the procedure shown in Example 8, for example, may be preferably used.

In this step, examples of the "a compound that provides a leaving group selected from the group consisting of a trifluoromethanesulfonyloxy group, a nonafluorobutanesulfonyloxy group, a 2-nitrobenzenesulfonyloxy group, and a 4-nitrobenzenesulfonyloxy group" include trifluoromethanesulfonic anhydride, nonafluoro-1-butanesulfonyl fluoride, bis(nonafluoro-1-butanesulfonic acid)anhydride, 2-nitrobenzenesulfonyl chloride, or 4-nitrobenzenesulfonyl chloride. Preferred is trifluoromethanesulfonic anhydride.

The solvent in this step is not limited as long as the reaction proceeds. Examples include ethyl acetate, toluene, dichloromethane, acetonitrile, cyclopentyl methyl ether, or tert-butyl methyl ether. Preferred is ethyl acetate, toluene, or dichloromethane.

The reaction temperature in this step is not limited as long as the reaction proceeds, and may be, for example, from -40°C to 60°C, preferably from -30°C to 40°C, and more preferably from -20°C to 10°C.

This step can suitably be performed in the presence of a base. The base used in this step is not particularly limited as long as the reaction proceeds. Examples include 1-methylimidazole, pyridine, 4-dimethylaminopyridine, picoline, lutidine, or collidine. Preferred is 1-methylimidazole.

### <Step X-10>

Step X-10 is a step of reacting the compound represented by formula C-11 with cesium acetate or tetrabutylammonium acetate to produce a compound represented by the following formula C-12: 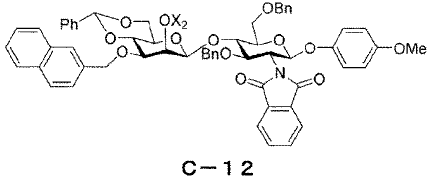 wherein X₂ is an acetyl group,
or a step of reacting the compound represented by formula C-11 with tetrabutylammonium benzoate to produce a compound represented by the following formula C-12: 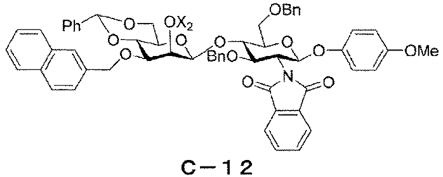 wherein X₂ is a benzoyl group. There are known conversion reactions, namely stereoinversions, from glucose → mannose. However, no conversion has been reported when a 2-naphthylmethyl (Nap) group is the protecting group of the hydroxyl group attached to the carbon at position 3 of D-glucopyranoside of a glucose-glucosamine disaccharide linked by a β-glycosidic linkage. This method may be adopted to achieve the stereoinversion from glucose → mannose, so that the mannose-glucosamine disaccharide skeleton linked by a β-glycosidic linkage can be constructed in high yield and selectivity.

The solvent in this step is not limited as long as the reaction proceeds. Examples include dimethyl sulfoxide, dimethylformamide, dimethylacetamide, N-methylpyrrolidone, N,N-dimethylimidazolidinone, sulfolane, tetrahydrofuran, or acetonitrile. Preferred is dimethylsulfoxide.

The reaction temperature in this step is not limited as long as the reaction proceeds, and may be, for example, from 20°C to 80°C, preferably from 23°C to 70°C, more preferably from 26°C to 60°C, and particularly preferably from 30°C to 50°C.

### <Step X-11>

Step X-11 is a step of eliminating the X₂ group and by opening the ring of the phthalimide group in the compound represented by formula C-12 to produce a compound represented by the following formula C-13; 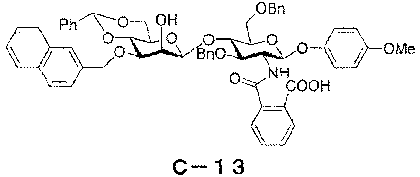 . This step may be performed by using or applying a known hydrolysis procedure. However, the procedure shown in Example 9, for example, may be preferably used.

The compound represented by formula C-13 that is produced in this step and dissolved in a solvent may be used as it is in the next step or may be isolated and purified by recrystallization. The major advantage of the compound represented by formula C-13 is that it can be isolated and purified by crystallization, which can almost completely remove impurities having similar structures that are difficult to remove by column purification. In this case, the compound represented by formula C-13 with HPLC purity of 99% or higher can be obtained.

Examples of the isolation and purification by recrystallization in this step include a process for completely removing the solvent from the dissolved state by decompression drying; or a process for using tetrahydrofuran as a good solvent and adding dropwise isopropanol as a poor solvent in the presence of a trace amount of water.

The recrystallization in this step may also be performed using seed crystals of the compound represented by formula C-13. In the case of using seed crystals, the crystallization may be conducted by, for instance, a method in which tetrahydrofuran is used as a good solvent; in the presence of a trace amount of water, some of isopropanol is added dropwise as a poor solvent; seed crystals are added; crystal precipitation is observed; and the remaining isopropanol is then added dropwise.

The above step of producing the compound represented by formula C-13 from the compound represented by formula C-11 may be performed in a one-pot process, for example, as shown in Example 9.

### <Step X-12>

Step X-12 is a step of subjecting the ring-opened phthalimide group in the compound represented by formula C-13 to dehydration condensation to close the ring to produce a compound represented by the following formula C-14; 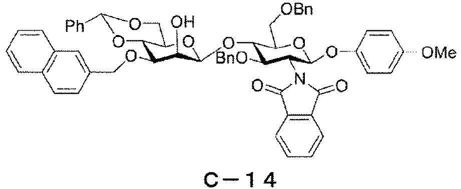 . This step may be performed by using or applying a known procedure. However, the procedure shown in Example 10, for example, may be preferably used.

### <Step X-13>

Step X-13 is a step of protecting, by a benzyl group, the hydroxyl group attached to the carbon at position 2 of D-mannopyranoside in the compound represented by formula C-14 to produce a compound represented by the following formula C-15: 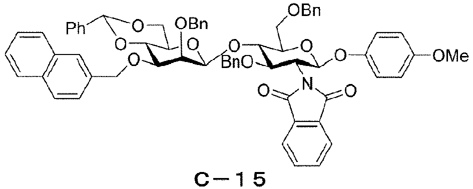 . This step may be performed by using or applying a known procedure. However, the procedure shown in Example 11, for example, may be preferably used.

In an embodiment of the present invention, step X-13 comprises a step of protecting, in the presence of lithium tert-butoxide or lithium tert-amoxide, the hydroxyl group attached to the carbon at position 2 of D-mannopyranoside in the compound represented by formula C-14 by a benzyl group to produce the compound represented by formula C-15. The ring-opening of phthalimide can be inhibited by performing step X-15 in the presence of lithium tert-butoxide or lithium tert-amoxide. In addition, it is safer to perform and easier to scale up than the general conditions using sodium hydride.

The solvent in this step is not limited as long as the reaction proceeds. Examples include dimethylacetamide, dimethylformamide, N-methylpyrrolidone, or N,N-dimethylimidazolidinone. Preferred is dimethylacetamide.

The reaction temperature in this step is not limited as long as the reaction proceeds, and may be, for example, from -20°C to 100°C, preferably from -15°C to 70°C, and particularly preferably from -10°C to 50°C.

### <Step X-14>

Step X-14 is a step of selective reducing the benzylidene protecting group in the compound represented by formula C-15 (for more details, see Angew. Chem. Int. Ed. 2005, 44, 1665-1668) to produce a compound represented by the following formula A-4: 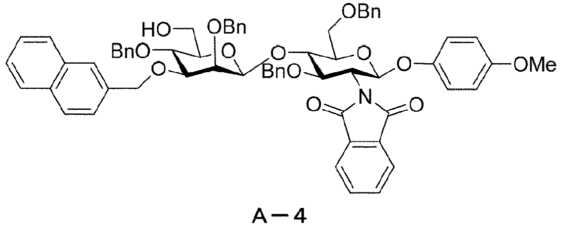 wherein only the hydroxyl group attached to the carbon at position 6 of D-mannopyranoside is deprotected. This step may be performed by using or applying a known procedure. However, the procedure shown in Example 12, for example, may be preferably used.

The compound represented by formula A-4 that is produced in this step and dissolved in a solvent may be used as it is in the next step or may be isolated and purified by, for instance, column purification.

In an embodiment of the present invention, this step comprises the following steps X-15 and X-16 for stereoinversion from glucose → mannose by using a redox reaction, instead of steps X-9 to X-12 for the stereoinversion by using an S_{N}2 reaction.

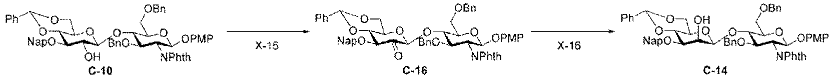

### <Step X-15>

Step X-15 is a step of oxidizing position 2 of D-glucopyranoside in the compound represented by formula C-10 to produce a compound represented by the following formula C-16: 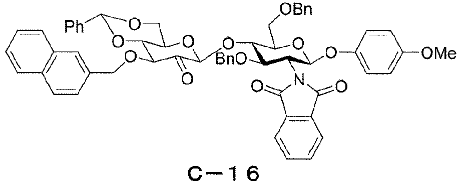 . This step may be performed by using or applying a known procedure.

### <Step X-16>

Step X-16 is a step of reducing the ketone group attached to the carbon at position 2 of 2-keto-D-glucopyranoside in the compound represented by formula C-16 to produce a compound represented by the following formula C-14: 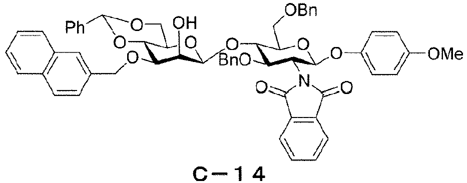 . This step may be performed by using or applying a known procedure.

The solvent in this step is not limited as long as the reaction proceeds. Examples include diethyl ether, cyclopentyl methyl ether, tert-butyl methyl ether, diisopropyl ether, dipropyl ether, dibutyl ether, or 1,4-dioxane. Preferred is tetrahydrofuran.

The reaction temperature in this step is not limited as long as the reaction proceeds, and may be, for example, from -80°C to 20°C. Note that as described below, the optimum reaction temperature varies depending on the reducing agent used.

In an embodiment of the present invention, the oxo group attached to the carbon at position 2 of 2-keto-D-glucopyranoside in the compound represented by formula C-16 is reduced in the presence of a reducing agent selected from the group consisting of L-selectride; LS-selectride; lithium diisobutyl-tert-butoxyaluminum hydride (LDBBA); a compound represented by the following formula W: 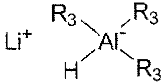 wherein R₃ is a di-tert-butylmethylphenoxide or hydride as shown in the following formula: 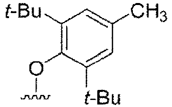 provided that at least two R₃ are each di-tert-butylmethylphenoxide; and a combination thereof. In the reduction step, NaBH₄, for example, may be used. In this case, the stereoselectivity was low (about 7:3), and it was difficult to efficiently obtain the desired stereoinversion from Glc → Man (Org. Biomol. Chem., 2018, 16, 4720-4727). On the other hand, in the case of using the above listed reducing agents, the selectivity of stereoinversion from Glc → Man is greatly improved (93.6:6.4 to 98.1:1.9) when compared to the case of using NaBH₄.

Among the compounds represented by formula W, a compound where three R₃ moieties are each di-tert-butylmethylphenoxide can be obtained, for example, by adding, to a tetrahydrofuran suspension (2 mL) containing lithium aluminum hydride (50.0 mg, 1.32 mmol), dibutylhydroxytoluene (885.41 mg, 4.02 mmol) at 0°C, followed by stirring at 25°C. Among the compounds represented by formula W, a compound where two R₃ moieties are each di-tert-butylmethylphenoxide can be obtained in a similar manner by using 2 molar equivalents of dibutylhydroxytoluene for 1 molar equivalent of lithium aluminum hydride.

As described above, the reaction temperature in this step is not limited as long as the reaction proceeds. When L-selectride, LS-selectride, or LDBBA is used as the reducing agent, the reaction temperature may be preferably from -80°C to -20°C, more preferably from -80°C to -30°C, still more preferably from -80°C to -40°C, and particularly preferably from -80°C to -50°C. When the compound represented by formula W is used as the reducing agent, the reaction temperature may be preferably from - 20°C to 20°C, more preferably from -15°C to 15°C, and particularly preferably from -10°C to 10°C. Therefore, in terms of the fact that the reaction proceeds at an easy-to-handle temperature, the particularly suitable reducing agent used for this step is a compound represented by formula W.

Next, the compound represented by formula A-5 is formed from the compound represented by formula A-4. In an embodiment of the present invention, this step comprises reacting the compound represented by formula A-4 with DDQ (2,3-dichloro-5,6-dicyano-p-benzoquinone) in a mixed solvent of fluorous alcohol and water to eliminate the 2-naphthylmethyl group in the compound represented by formula A-4 above (de-2-naphthylmethylation reaction) to give the oligosaccharide represented by formula A-5 above. Here, the procedure shown in Example 31 or 54, for example, may be preferably used.

For the above-mentioned de-2-naphthylmethylation reaction, the present inventors have found that the reaction (action) of 2,3-dichloro-5,6-dicyano-p-benzoquinone with a substrate having a 2-naphthylmethyl group attached via an oxygen atom in fluorous alcohol and water allows the reaction to be carried out under mild conditions in a favorable stirring property state; and the de-2-naphthylmethylated product can be obtained in high yield. The advantages of this de-2-naphthylmethylation reaction are explained in more detail below. In the above-mentioned de-2-naphthylmethylation reaction of the present invention, the de-2-naphthylmethylated product can be obtained in high yield under mild conditions from a substrate e.g., a sugar to which a 2-naphthylmethyl group is attached via an oxygen atom. The reaction can be carried out reproducibly without any deterioration in stirring properties or adhesion to the vessel wall due to the byproduct 2,3-dichloro-5,6-dicyano-p-benzohydroquinone. Thus, this also fits for mass synthesis of such a product. Due to the anomalous freezing point fall of HFIP-H₂O, no coagulation of the solvent was observed even when the reaction temperature was lowered to -30°C. Therefore, a wide temperature range is applicable depending on the reactivity of the reaction substrate (melting point of HFIP: -3.3°C, H₂O: 0°C). In the case of the denaphthylmethylation reaction of the compound represented by formula A-5 having a plurality of benzyl groups, DDQ is used as an oxidant and HFIP-H₂O is used as a solvent. As a result, the reaction is found to proceed with superior selectivity when compared to conventional conditions. In most of the reported cases, dichloromethane-water-based bilayer system reaction conditions are applied to this conversion reaction. In this case, debenzylation of multiple benzyl groups proceeds at a constant rate and the yield remains moderate. There are reported examples of conditions improved using β-pinene as an additive (see, for example, J. Org. Chem., 2017,82,3926). For compounds with multiple Bn groups, however, the yield remains moderate (see, for example, Angew. Chem. Int. Ed. 2021,60,19287). Further, the selectivity especially for a substrate with four or more benzyl groups, such as the compound represented by formula A-5, is not well understood. In the dichloromethane-water system, the problem is that DDQ and DDQ-derived byproducts cause deterioration of stirring properties. The reaction conditions are not suitable for mass synthesis. On the other hand, the DDQ/HFIP-H₂O system of the present method achieved a high selectivity of 95% or more for the compound represented by formula A-5 having four benzyl groups. This method does not show any deterioration of stirring properties derived from DDQ as described above. Further, due to the anomalous freezing point fall of HFIP-H₂O, no coagulation of the solvent was observed even when the reaction temperature was lowered to -30°C. Therefore, a wide temperature range is applicable depending on the reactivity of the reaction substrate (melting point of HFIP: -3.3°C, H₂O: 0°C).

Note that the above de-2-naphthylmethylation reaction is not limited to the reaction in this step I-1-1. Thus, an aspect of the present invention also provides a method for producing the oligosaccharide represented by formula A-5 above, the method comprising a step of reacting the compound represented by formula A-4 with DDQ (2,3-dichloro-5,6-dicyano-p-benzoquinone) in a mixed solvent of fluorous alcohol and water to eliminate the 2-naphthylmethyl group in the compound represented by formula A-5.

The above "fluorous alcohol" is not limited as long as the reaction proceeds, and is preferably selected from the group consisting of hexafluoro 2-propanol (HFIP), 2,2,2-trifluoroethanol (TFE), 2,2,3,3,4,4,5,5-octafluoro-1-pentanol, nonafluoro-tert-butyl alcohol, and combinations thereof.

The above de-2-naphthylmethylation reaction is not limited as long as the reaction proceeds, and is preferably carried out at -35°C to 70°C, and more preferably at -30°C to -10°C.

### <Purification of compound represented by formula A-6>

In this step I-1-1, the compound represented by formula A-6 can be obtained in a purified form by the following purification method. The purification method involves purifying the compound represented by formula A-6 above by stopping the reaction of the compound represented by formula A-3 with the compound represented by formula A-5; then adding a hydrophobic carrier and water to a water-soluble organic solvent containing the resulting compound represented by formula A-6 above and contaminants to adsorb the compound represented by formula A-6 above onto the hydrophobic carrier; then filtering the resultant and washing the hydrophobic carrier with a mixed solution of the above water-soluble organic solvent and water to remove the contaminants; and then eluting the compound represented by formula A-6 above using an organic solvent, from the hydrophobic carrier. This purification method allows for easy removal of post-glycosylation residual reagent debris and impurities derived from a glycosyl donor and a glycosyl acceptor in the liquid phase synthesis of an oligosaccharide chain by washing with a small amount of hydrophobic carrier. Since the reaction inhibition and side reactions caused by these impurities can be suppressed, it has become possible to produce a large quantity of high-quality oligosaccharide efficiently. In addition, compared to the conventionally developed methods, the present invention can reduce the number of steps for tag desorption and prevent the tag functionality from deteriorating during oligomerization by utilizing the hydrophobicity of the substrate itself, and thus can produce an oligosaccharide(s) more efficiently. In particular, in this step, the compound represented by formula A-6 can be easily separated and purified from the degradation products derived from the compound represented by formula A-4 by the above purification method.

The purification of the compound represented by formula A-6 above is not limited to the purification in this step I-1-1. Then, an embodiment of the present invention also provides a method comprising the following: adding a hydrophobic carrier and water to a water-soluble organic solvent containing the compound represented by formula A-6 above and contaminants to adsorb the compound represented by formula A-6 above onto the hydrophobic carrier; then filtering the resultant and washing the hydrophobic carrier with a mixed solution of the above water-soluble organic solvent and water to remove the contaminants; and then eluting the compound represented by formula A-6 above using an organic solvent, from the hydrophobic carrier to purify the compound represented by formula A-6 above. Further, the above purification method can be applied to the purification of organic compounds other than sugar compounds. When the organic compound to be purified is a sugar compound, it is possible to suitably purify an oligosaccharide (protected oligosaccharide) having a glycan structure consisting of 3-15 sugar residues in which one or all hydroxyl groups in the sugar are protected. Examples of the protecting groups of the glycan in this case include, but are not limited to, alkyl ethers, benzyl ethers, silyl ethers, esters, or carbonate esters.

The above term "contaminants" refers to compounds and/or reagents other than the protected oligosaccharide (in this step, the compound represented by formula A-6 above), and mainly means reagents used in a reaction of synthesizing a protected oligosaccharide and their remnants, sugars other than the protected oligosaccharide, such as mono- or disaccharide compounds used in the elongation reaction of the protected oligosaccharide, or byproducts generated by the deprotection reaction of the protected oligosaccharide.

The above term "hydrophobic carrier" refers to a hydrophobic adsorption material that adsorbs specific compounds including sugar compounds. Examples include a resin for packing in reversed-phase partition chromatography. Examples of the "resin for packing in reversed-phase partition chromatography" include, but are not limited to, poly(styrene/divinylbenzene) polymer gel resins, polystyrene-divinylbenzene resins, polyhydroxymethacrylate resins, styrene-vinylbenzene copolymer resins, polyvinyl alcohol resins, polystyrene resins, polymethacrylate resins, chemically bonded silica gel resins, and a combination thereof.

The above "chemically bonded silica gel resin" is selected from the group consisting of (1) resins obtained by reacting silica gel with a silane coupling agent, (2) resins obtained by chemically bonding a dimethyloctadecyl, octadecyl, trimethyloctadecyl, dimethyloctyl, octyl, butyl, ethyl, methyl, phenyl, cyanopropyl, or aminopropyl group to silica gel, and (3) resins obtained by chemically bonding a docosyl or triacontyl group to silica gel, and (4) combinations of the resins (1) to (3). Octadecyl group-bonded silica gel resins (ODS resins) are preferably used. The above resin is not limited to them.

The above "water-soluble organic solvent" is not particularly limited, and a water-soluble alcohol-based solvent (suitably C1-C4), water-soluble nitrile-based solvent (e.g., acetonitrile, etc.), water-soluble ether-based solvent (e.g., tetrahydrofuran, etc.), water-soluble ketone-based solvent (e.g., acetone), water-soluble amide-based solvent (e.g., dimethylformamide), or water-soluble sulfoxide-based solvent (e.g., dimethyl sulfoxide) may be used. Acetonitrile can be used as a suitable solvent.

The "organic solvent" used in the elution step of the target product from the above hydrophobic carrier is not particularly limited. However, it is possible to use a nitrile-based solvent (e.g., acetonitrile), ether-based solvent (e.g., tetrahydrofuran), ester-based solvent (e.g., ethyl acetate), ketone-based solvent (e.g., acetone), halogen-based solvent (e.g., dichloromethane), or aromatic solvent (e.g., toluene), or a mixed solvent containing at least one of the above solvent systems. Preferred is acetonitrile, ethyl acetate, tetrahydrofuran, or toluene.

The above purification process can be performed at temperatures from 0°C to 50°C, although it is not limited to any particular temperature.

After the above step I-1-1, the following steps I-1-2 to I-1-3 may be used to produce the compound represented by formula A-8 above from the compound represented by formula A-6 above. However, the procedure is not limited to these production steps.

### <Step I-1-2>

Step I-1-2 is a step of eliminating the 4-methoxyphenyl group from the compound represented by formula A-6 to give a compound represented by formula A-7:

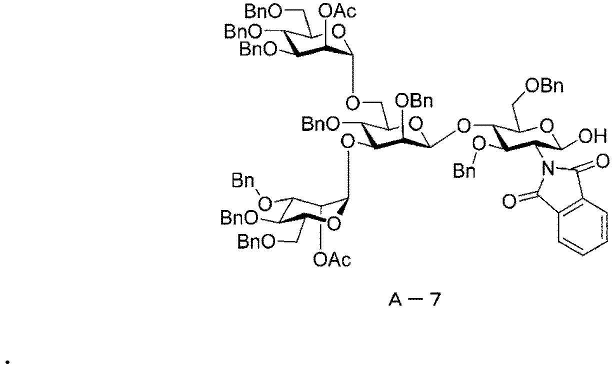

In an embodiment of the present invention, this step is a step of producing the compound represented by formula A-7 above by reacting the compound represented by formula A-6 above with λ³-iodane in fluorous alcohol and water to deprotect the 4-methoxyphenyl group. This step may be preferably performed using, for instance, the procedure shown in Example 33 or 56.

The term "λ3-iodane" described above means a trivalent/hypervalent iodine compound. In an embodiment, a compound represented by formula R^{4a}-I(OR^{4b})₂ (wherein R^{4a} is an unsubstituted or substituted phenyl group and R^{4b} is selected from the group consisting of H, acetoxy, trifluoroacetoxy, tosyloxy, methane sulfonyloxy, and combinations thereof). As defined in the above formula, R^{4a} may be a "substituted phenyl group". Examples of the substituent include a linear or branched, saturated or unsaturated hydrocarbon group, an oxygen-containing group (e.g., alkoxy, ester), a nitrogen-containing group (e.g., cyano, azide), or a halogen atom (e.g., fluorine atom, chlorine atom, bromine atom, iodine atom). More preferred is a hydrocarbon group, an oxygen-containing substituent, or a halogen atom. When the above substituent contains carbon, for example, a C1-C5 or C1-C3 substituent may be preferably used. Specific examples of the λ3-iodane include, but are not limited to, [bis(trifluoroacetoxy)iodo]benzene (PIFA), [hydroxy(tosyloxy)iodo]benzene (HTIB), (diacetoxyiodo)benzene (PIDA), [bis(trifluoroacetoxy)iodo]pentafluorobenzene, and [hydroxy(methanesulfonyloxy)iodo]benzene.

As used in the above step, the term "fluorous alcohol" means a fluorine-containing alcohol compound in which all carbon atoms except those bonded to the hydroxyl group have fluorous. The fluorous alcohol preferably has a greater number of fluorine atoms as long as fluorine substitution is allowed. Examples of the fluorous alcohol include, but are not limited to, a fluorous aliphatic alcohol. The hydrocarbon moiety in the fluorous aliphatic alcohol may be saturated or unsaturated, linear or branched, or cyclic. The fluorous aliphatic alcohol is, for example, a fluorous C₂-C₈ aliphatic alcohol, preferably a fluorous C₂-C₅ aliphatic alcohol, and more preferably a fluorous C₂-C₃ aliphatic alcohol. Specific examples of the fluorous alcohol include, but are not limited to a compound selected from the group consisting of hexafluoro2-propanol (HFIP), 2,2,2-trifluoroethanol (TFE), 2,2,3,3,4,4,5,5-octafluoro-1-pentanol, nonafluoro-tert-butyl alcohol, and combinations thereof. Preferably, hexafluoro 2-propanol (HFIP) is used.

This step is carried out in the co-presence of the above-mentioned fluorous alcohol and "water". The amount of water can be set, if appropriate, from the viewpoint of, for instance, achieving a high yield of the product. For example, the amount may be about 1.0 equivalent or more, about 1.5 equivalents or more, about 2.0 equivalents or more, or about 2.5 equivalents or more in a mole ratio based on the compound represented by formula A-6. The amount may be about 10 or less, about 8 or less, about 5 or less, or about 3 or less in a volume ratio based on the compound represented by formula A-6.

In this step, an additional "additive" may be added to the fluorous alcohol and water. The additive is preferably selected from the group consisting of sodium dihydrogen phosphate, potassium dihydrogen phosphate, disodium hydrogen phosphate, trifluoroacetic acid, and combinations thereof. The amount of additive can be set, if appropriate, and may be, for example, about 0.5 to 8 equivalents, about 1 to 6 equivalents, or about 1.5 to 5 equivalents based on the compound represented by formula A-6.

### <Step I-1-3>

Step I-1-3 is a step of producing the compound represented by formula A-8 from the compound represented by formula A-7. This step may be performed by using or applying a known procedure. However, the procedure shown in Example 34 or 57, for example, may be preferably used.

In an embodiment of the present invention, this step is a step of reacting the compound represented by formula A-7 above with 2,2,2-trifluoro-N-phenylacetimidoyl chloride (TFPC) in the presence of N-methylimidazole to give the compound represented by formula A-8 above. Instead of N-methylimidazole, DBU can also be used. By using N-methylimidazole or DBU as the base used, the equivalent amount of TFPC can be reduced when compared to the case of using, for example, potassium carbonate, and the target product can be obtained in high yield. Since TFPC is an expensive reagent, an increase in the yield of this step is very beneficial for commercial production.

The solvent in this step is not limited as long as the reaction proceeds. Examples include dichloromethane, toluene, ethyl acetate, acetonitrile, or tetrahydrofuran. Preferred is dichloromethane.

The reaction temperature in this step is not limited as long as the reaction proceeds, and may be preferably from -20°C to 40°C, more preferably from -10°C to 35°C, and particularly preferably from 0°C to 30°C.

This step is suitably performed in the presence of a dehydrating agent. The dehydrating agent in this step is not limited as long as the reaction proceeds. Examples include Molecular Sieves. Preferred is Molecular Sieves 4A powder with a powder particle size of 10 µm or less.

The compound represented by formula A-8 given in this step and dissolved in a solvent may be used as it is in the next step or may be isolated and purified by, for instance, column purification if the base used in the reaction has been removed. Examples of the isolation and purification using a column include isolation and purification using silica gel as a stationary phase and a dichloromethane or toluene-ethyl acetate mixed solvent system as a mobile phase.

### <Step I-2>

Step I-2 is a step of producing a compound represented by the following formula A-11: 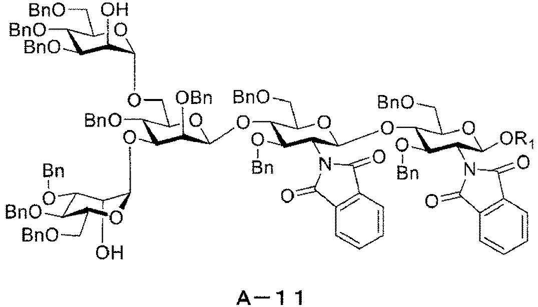 wherein R₁ is a C₁ to C₆ alkyl group or a phenyl group, and the phenyl group is optionally substituted with one to three C₁ to C₆ alkoxy groups,
the step comprising the step of:
subjecting the compound represented by formula A-8 and a compound represented by the following formula A-9: 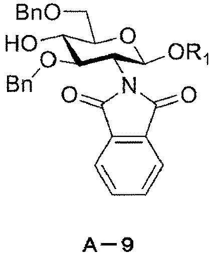
to β-1,4-glycosidic linkage to give a compound represented by the following formula A-10: 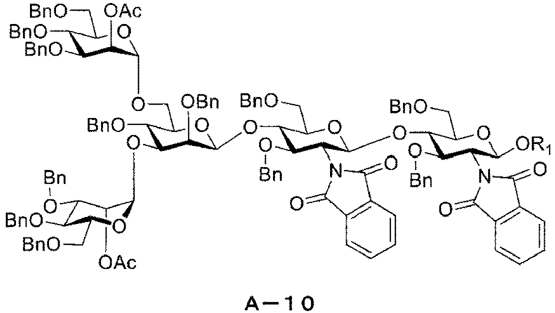
wherein R₁ is a C₁ to C₆ alkyl group or a phenyl group, and the phenyl group is optionally substituted with one to three C₁ to C₆ alkoxy groups.
The step I-2 comprises the following steps I-2-1 to I-2-2.

### <Step I-2-1>

Step I-2-1 is a step of subjecting the compound represented by formula A-8 and the compound represented by formula A-9 to β-1,4-glycosidic linkage to give a compound represented by formula A-10. The compound represented by formula A-9 may be produced by a known procedure or a commercially available product may be used. Examples of the commercially available compound represented by formula A'-9 (compound represented by formula A-9 wherein R₁ is 4-methoxyphenyl) include 4-methoxyphenyl 3,6-di-O-benzyl-2-deoxy-2-phthalimido-β-D-glucopyranoside, manufactured by TOKYO CHEMICAL INDUSTRY CO., LTD. Alternatively, the compound represented by formula A"-9 can be obtained by subjecting the compound represented by formula A-12 produced as described below to, for example, the treatment shown in Example 59 to obtain isopropyl 4-O-acetyl-3,6-di-O-benzyl-2-deoxy-2-(1,3-dioxo-1,3-dihydro-2H-isoindol-2-yl)-β-D-glucopyranoside (compound represented by formula F"-1), and further subjecting the compound represented by formula F"-1 to, for example, the treatment shown in Example 60. This step of producing the compound represented by formula A-10 may be performed by using or applying a known procedure. However, the procedure shown in Example 35 or 61, for example, may be preferably used.

### <Purification of compound represented by formula A-10>

In this step I-2-1, the compound represented by formula A-10 can be obtained in a purified form by the following purification method. The purification method involves purifying the compound represented by formula A-10 above by stopping the reaction of the compound represented by formula A-8 with the compound represented by formula A-9; then adding a hydrophobic carrier and water to a water-soluble organic solvent containing the resulting compound represented by formula A-10 above and contaminants to adsorb the compound represented by formula A-10 above onto the hydrophobic carrier; then filtering the resultant and washing the hydrophobic carrier with a mixed solution of the above water-soluble organic solvent and water to remove the contaminants; and then eluting the compound represented by formula A-10 above using an organic solvent, from the hydrophobic carrier. As described in the method of purifying the compound represented by formula A-6 above, this purification method makes it possible to produce a large amount of high-quality oligosaccharide efficiently in the liquid-phase synthesis of oligosaccharide chains by using a small amount of hydrophobic carrier. In particular, the monosaccharide compound represented by formula A-9 and the pentasaccharide compound represented by formula A-10 have very close polarity in normal-phase silica gel column chromatography. This makes it difficult to separate them, for example, under the typical column solvent system (hexane-ethyl acetate) conditions because of the same Rf value. However, the purification method in the present invention can be used to easily separate the monosaccharide from the pentasaccharide with very close polarity.

The purification of the compound represented by formula A-10 above is not limited to the purification in this step I-2-1. Then, an embodiment of the present invention also provides a method comprising the following: adding a hydrophobic carrier and water to a water-soluble organic solvent containing the compound represented by formula A-10 above and contaminants to adsorb the compound represented by formula A-10 above onto the hydrophobic carrier; then filtering the resultant and washing the hydrophobic carrier with a mixed solution of the above water-soluble organic solvent and water to remove the contaminants; and then eluting the compound represented by formula A-10 above using an organic solvent, from the hydrophobic carrier to purify the compound represented by formula A-10 above.

The above term "contaminants" refers to compounds and/or reagents other than the protected oligosaccharide (in this step, the compound represented by formula A-10), and mainly means reagents used in a reaction of synthesizing a protected oligosaccharide and their remnants, sugars other than the protected oligosaccharide, such as mono- or disaccharide compounds used in the elongation reaction of the protected oligosaccharide, or byproducts generated by the deprotection reaction of the protected oligosaccharide. Note that the "hydrophobic carrier" (e.g., a resin for packing in reversed-phase partition chromatography), "water-soluble organic solvent," "organic solvent," and the purification temperature used in this step are substantially the same as those described in the method of purifying the compound represented by formula A-6 above.

### <Step I-2-2>

Step I-2-2 is a step of producing the compound represented by formula A-11 from the compound represented by formula A-10.

In an embodiment of the present invention, this step I-2-2 is a step of reacting the compound represented by formula A-10 with a strong base in the presence of an alkyl ester of perfluorocarboxylic acid in a solvent to eliminate the acetyl group (deacetylation reaction) to give the compound represented by formula A-11. Here, the procedure shown in Example 36 or 62, for example, may be preferably used. The deacetylation reaction can be performed in the same way as in the deacetylation reaction described in step X-7, except that the substrate is different. By using the technique of reaction with a strong base in the presence of an alkyl ester of perfluorocarboxylic acid, the deacetylation reaction can be carried out while the ring opening of the phthalimide group is suppressed.

Note that the above deacetylation reaction is not limited to use in this step I-2-2. Thus, an embodiment of the present invention provides a method for producing the compound represented by formula A-11 above, the method comprising a step of reacting the compound represented by formula A-10 above with a strong base in the presence of an alkyl ester of perfluorocarboxylic acid.

The alkyl ester of perfluorocarboxylic acid, the strong base, the solvent, and the reaction temperature used in this step are as described in step X-7 above.

### <Step I-3>

Step I-3 is a step of subjecting the compound represented by formula A-11 and a compound represented by the following formula A-12: 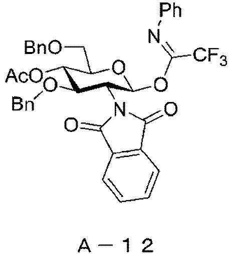 to β-1,2-glycosidic linkage to give a compound represented by the following formula A-13: 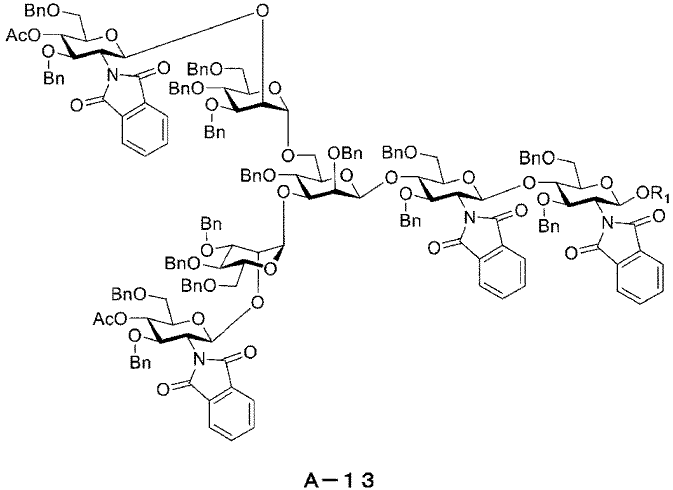
wherein R₁ is a C₁ to C₆ alkyl group or a phenyl group, and the phenyl group is optionally substituted with one to three C₁ to C₆ alkoxy groups,
then eliminating each phthaloyl group as a protecting group of an amino group and each acetyl group on a hydroxyl group on the compound represented by formula A-13 to give a compound represented by the following formula A-14: 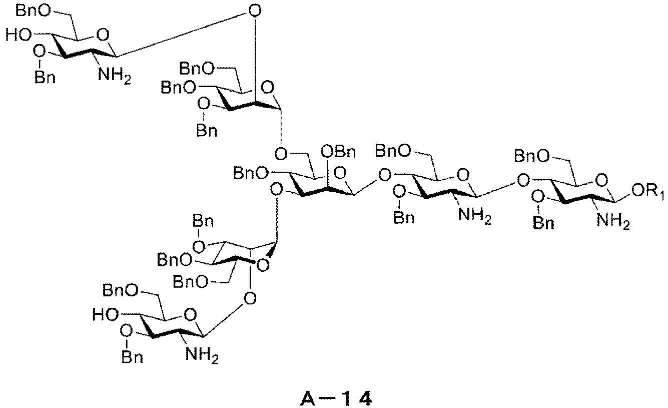
wherein R₁ is a C₁ to C₆ alkyl group or a phenyl group, and the phenyl group is optionally substituted with one to three C₁ to C₆ alkoxy groups,
and then protecting the amino group in the compound represented by formula A-14 by a protecting group selected from an aryloxycarbonyl (COOAr) group, an acetyl (Ac) group, a 2,2,2-trichloroethoxycarbonyl (Troc) group, or a phthaloyl (Phth) group to give a compound represented by the following formula A-15: 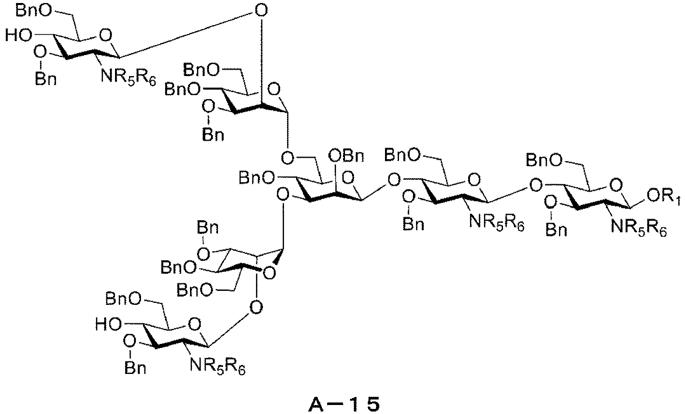
wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
or eliminating each acetyl (Ac) group on the compound represented by formula A-13) to give the compound represented by formula A-15 wherein R₅ and R₆ together with a nitrogen atom attached thereto form a phthalimide group. In an embodiment of the present invention, step I-3 comprises the following steps I-3-1 to I-3-3.

### <Step I-3-1>

Step I-3-1 is a step of subjecting the compound represented by formula A-11 above and the compound represented by formula A-12 above to β-1,2-glycosidic linkage to give the compound represented by formula A-13 above. This glycosidic linkage step may be performed by using or applying a known procedure. However, the procedure shown in Example 37 or 63, for example, may be preferably used. In addition, the compound represented by formula A-12 may be produced as follows. Further, the compound represented by formula A-13 may be purified as described below.

### <To produce compound represented by formula A-12>

The compound represented by formula A-12 above may be produced like in the following substeps Z-1 to Z-3.

### <Substep Z-1>

First, the hydroxyl group on the compound represented by formula A-9: 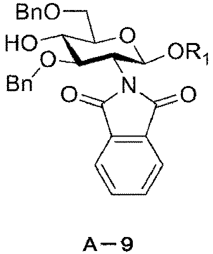 used in the above step I-2 is protected by an acetyl group to give a compound represented by the following formula F-1: 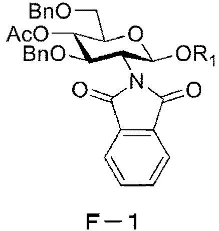 . This step may be performed by using or applying a known procedure. However, the procedure shown in Example 13, for example, may be preferably used. This step can be implemented by adding triethylamine, dimethylaminopyridine, and acetic anhydride to an ethyl acetate solution containing the compound represented by formula A-9, but is not limited to this procedure.

### <Substep Z-2>

Next, the 4-methoxyphenyl group from the compound represented by formula F-1 is eliminated to give a compound represented by the following formula F-2:

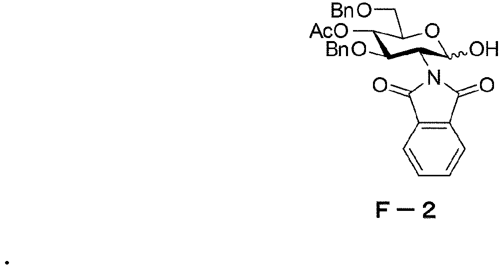

In one embodiment of the present invention, this substep Z-2 is a step of reacting the compound represented by formula F-1 with λ3-iodane in fluorous alcohol and water to eliminate the 4-methoxyphenyl group to produce the compound represented by formula F-2. Here, the procedure shown in Example 14, for example, may be preferably used. This step can be performed according to the above step I-1-2. The fluorous alcohol and λ3-iodane used in the step can be the same as those used in the above step I-1-2.

### <Substep Z-3>

Next, in this step, the compound represented by formula F-2 is reacted with 2,2,2-trifluoro-N-phenylacetimidoyl chloride (TFPC) to produce a compound represented by formula A-12: 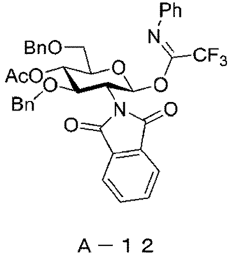 . This step may be performed by using or applying a known procedure.

In an embodiment of the present invention, substep Z-3 is a step of reacting with 2,2,2-trifluoro-N-phenylacetimidoyl chloride (TFPC) in the presence of N-methylimidazole to give the compound represented by formula A-12 above. Here, the procedure shown in Example 15 or 58, for example, may be preferably used. As described for substantially the same reaction in step I-1-3, by using the above N-methylimidazole as the base used, the equivalent amount of TFPC can be reduced when compared to the case of using, for example, potassium carbonate, and the target product can be obtained in high yield. Note that the solvent and reaction temperature used, the fact that the reaction is preferably carried out in the presence of a dehydrating agent, and the fact that the product may be isolated and purified by, for instance, column purification, are the same as those in the above step I-1-3.

### <Purification of compound represented by formula A-13>

In the step I-3-1 above, the compound represented by formula A-13 can be obtained in a purified form by the following purification method. The purification method involves purifying the compound represented by formula A-13 by stopping the reaction of the compound represented by formula A-11 with the compound represented by formula A-12; then adding a hydrophobic carrier and water to a water-soluble organic solvent containing the resulting compound represented by formula A-13 and contaminants to adsorb the compound represented by formula A-13 onto the hydrophobic carrier; then filtering the resultant and washing the hydrophobic carrier with a mixed solution of the water-soluble organic solvent and the water to remove the contaminants; and then eluting the compound represented by formula A-13 using an organic solvent, from the hydrophobic carrier. As described in the method of purifying the compound represented by formula A-6 above, the above purification method makes it possible to produce a large amount of high-quality oligosaccharide efficiently in the liquid-phase synthesis of oligosaccharide chains by using a small amount of hydrophobic carrier.

The purification of the compound represented by formula A-13 above is not limited to the purification in this step I-3-1. Then, an embodiment of the present invention also provides a method comprising the following: adding a hydrophobic carrier and water to a water-soluble organic solvent containing the compound represented by formula A-13 above and contaminants to adsorb the compound represented by formula A-13 above onto the hydrophobic carrier; then filtering the resultant and washing the hydrophobic carrier with a mixed solution of the above soluble organic solvent and water to remove the contaminants; and then eluting the compound represented by formula A-13 above using an organic solvent, from the hydrophobic carrier to purify the compound represented by formula A-13 above.

The above term "contaminants" refers to compounds and/or reagents other than the protected oligosaccharide (in this step, the compound represented by formula A-13), and mainly means reagents used in a reaction of synthesizing a protected oligosaccharide and their remnants, sugars other than the protected oligosaccharide, such as mono- or disaccharide compounds used in the elongation reaction of the protected oligosaccharide, or byproducts generated by the deprotection reaction of the protected oligosaccharide.

The "hydrophobic carrier" (e.g., a resin for packing in reversed-phase partition chromatography), "water-soluble organic solvent," "organic solvent," and the purification temperature used in the above step are substantially the same as those described in the method of purifying the compound represented by formula A-5 above.

### <Step I-3-2>

Step I-3-2 is a step of eliminating each phthaloyl group as a protecting group of an amino group and each acetyl group on a hydroxyl group on the compound represented by formula A-13 above to give the compound represented by formula A-14. This step may be preferably performed using, for instance, the procedure shown in Example 38 or 64. For example, this step can be implemented by adding n-butanol and ethylenediamine to a solution containing the compound represented by formula A-13, but is not limited to these compounds. Alternatively, an acid (e.g., fumaric acid) may be added after the addition of N-butanol and ethylenediamine, and then the mixture may be stirred, then seed crystals may be inoculated to obtain crystals of an acid salt (e.g., fumarate) of the compound represented by formula A-14 (e.g., see Example 64). Isolation of this acid salt allows removal of an analog (such as an isomer) of the compound represented by formula A-14 by filtration, and allows high purity. Fumaric acid may be preferably used as the "acid" to be added, but the acid is not limited thereto.

### <Purification of compound represented by formula A-14>

In this step I-3-2, the compound represented by formula A-14 can be obtained in a purified form by the following purification method. The purification method involves purifying the compound represented by formula A-14 by adding a hydrophobic carrier and water to a water-soluble organic solvent containing the compound represented by formula A-14 produced as described above and contaminants to adsorb the compound represented by formula A-14 onto the hydrophobic carrier; then filtering the resultant and washing the hydrophobic carrier with a mixed solution of the water-soluble organic solvent and the water to remove the contaminants; and then eluting the compound represented by formula A-14 using an organic solvent, from the hydrophobic carrier. As described in the method of purifying the compound represented by formula A-6 above in step I-1-1, this purification method makes it possible to produce a large amount of high-quality oligosaccharide efficiently in the liquid-phase synthesis of oligosaccharide chains by using a small amount of hydrophobic carrier.

The purification of the compound represented by formula A-14 above is not limited to the purification in this step. Then, an embodiment of the present invention also provides a method comprising the following: adding a hydrophobic carrier and water to a water-soluble organic solvent containing the compound represented by formula A-14 above and contaminants to adsorb the compound represented by formula A-14 above onto the hydrophobic carrier; then filtering the resultant and washing the hydrophobic carrier with a mixed solution of the above water-soluble organic solvent and water to remove the contaminants; and then eluting the compound represented by formula A-14 above using an organic solvent, from the hydrophobic carrier to purify the above compound represented by formula A-14 above.

The above term "contaminants" refers to compounds and/or reagents other than the protected oligosaccharide (in this step, the compound represented by formula A-14), and mainly means reagents used in a reaction of synthesizing a protected oligosaccharide and their remnants, sugars other than the protected oligosaccharide, such as mono- or disaccharide compounds used in the elongation reaction of the protected oligosaccharide, or byproducts generated by the deprotection reaction of the protected oligosaccharide. Note that the "hydrophobic carrier" (e.g., a resin for packing in reversed-phase partition chromatography), "water-soluble organic solvent," "organic solvent," and the purification temperature used in this step are substantially the same as those described in the method of purifying the compound represented by formula A-6 in step I-1-1 above.

### <Step I-3-3>

Step I-3-3 is a step of protecting each amino group in the compound represented by formula A-14 above by a protecting group selected from an aryloxycarbonyl (COOAr) group, an acetyl (Ac) group, a 2,2,2-trichloroethoxycarbonyl (Troc) group, or a phthaloyl (Phth) group to give the compound represented by formula A-15 above (wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with the nitrogen atom attached thereto form a phthalimide group). The above amino group-protecting group is introduced because in the glycosylation reaction using the compound represented by formula A-15 and the compound represented by formula A-16 in the next step I-4, an acetyl-protected amino group (-NHAc group), if present in the reaction substrate, interacts with Lewis acid, causing a marked decrease in the reactivity of the desired glycosylation reaction and an excess amount of glycosyl donor is often required to complete the reaction, though the use of an acetyl group as the amino group-protecting group is more efficient in producing the target compound (the compound represented by formula A-22) since it is a linear route. Therefore, during the above glycosylation reaction, glucosamine is temporarily protected by a protecting group selected from an aryloxycarbonyl (COOAr) group, a 2,2,2-trichloroethoxycarbonyl (Troc) group, or a phthaloyl (Phth) group on the nitrogen and deprotected after the glycosylation reaction to form a -NHAc group. In this way, the above disadvantages can be avoided. Here, an aryloxycarbonyl (COOAr) group is the most preferred protective group above. The "aryl (Ar) group" in the aryloxycarbonyl means a group formed by removing one hydrogen atom on an aromatic ring in an aromatic hydrocarbon. Examples include, but are not limited to, a phenyl, 2-naphthyl, 1-naphthyl, 2-pyridyl, 3-pyridyl, nitrophenyl, chlorophenyl, fluorophenyl, bromophenyl, iodophenyl, methoxyphenyl, or C1-C4 alkylphenyl group. Preferred is a phenyl group. It has been found that the glycosylation reaction proceeds better with the aryloxycarbonyl (COOAr) group than with other protecting groups, and that the subsequent deprotection reaction can be further carried out under suitable conditions e.g., at room temperature and within 1 hour under typical hydrolysis conditions for deprotection.

The above step may be preferably performed using, for instance, the procedure shown in Examples 39 or 65. For example, this can be implemented by adding an aqueous solution containing tetrahydrofuran and sodium bicarbonate, potassium bicarbonate, disodium hydrogen phosphate, or dipotassium hydrogen phosphate (preferably, sodium bicarbonate is used) dissolved in water to a tetrahydrofuran solution containing the compound represented by formula A-14, but is not limited to these solutions.

Instead of the above steps I-3-2 and I-3-3, the acetyl (Ac) group on the compound represented by formula A-13 may be selectively removed to produce the compound represented by formula A-15 above (wherein R₅ and R₆ together with the nitrogen atom attached thereto form a phthalimide group). The selective removal of this acetyl group can be performed under, but not limited to, methyl trifluoroacetate conditions. This step yields the same result as in steps I-3-2 and I-3-3 when a phthaloyl (Phth) group is selected as each amino group-protecting group in the compound represented by formula A-14 above.

### <Step I-4>

Step I-4 is a step of producing a compound represented by the following formula A-20: 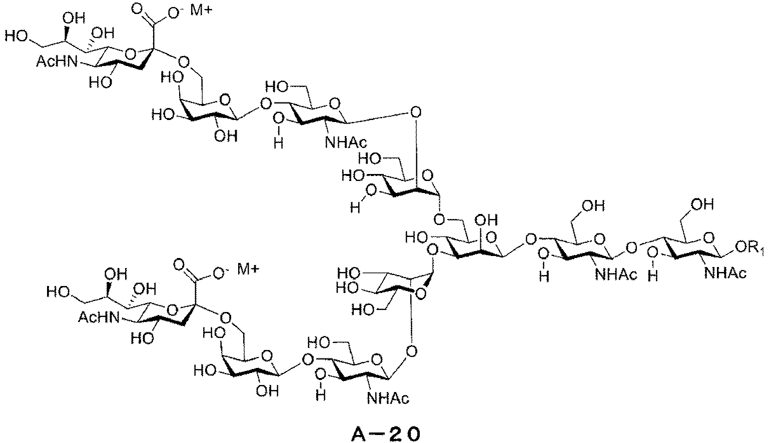 wherein R₁ is a C₁ to C₆ alkyl group or a phenyl group, the phenyl group is optionally substituted with one to three C₁ to C₆ alkoxy groups, and M⁺ is a sodium ion, lithium ion, potassium ion, or protonated triethylamine cation,
the step comprising the step of:
subjecting the compound represented by A-15 and a compound represented by the following formula 16: 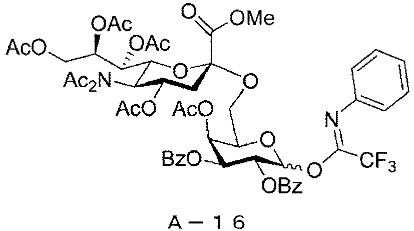
to β-1,4-glycosidic linkage to give a compound represented by the following formula A-17: 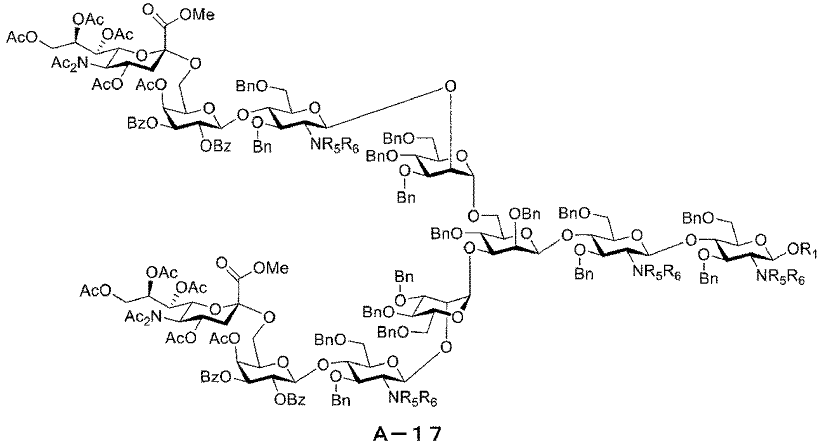
wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
and then eliminating each protecting group of an amino group in the compound represented by formula A-17 to give a compound represented by the following formula A-18: 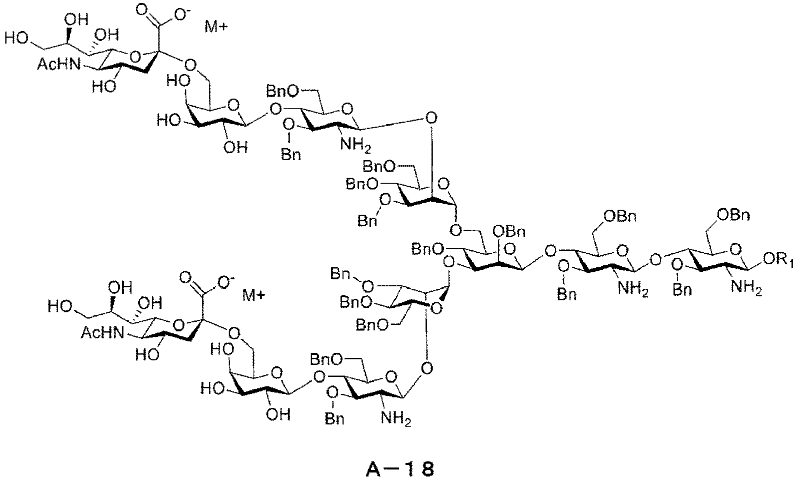
wherein R₁ is a C₁ to C₆ alkyl group or a phenyl group, the phenyl group is optionally substituted with one to three C₁ to C₆ alkoxy groups, and M⁺ is a sodium ion, lithium ion, potassium ion, or protonated triethylamine cation. In an embodiment of the present invention, step I-4 comprises the following steps I-4-1 to I-4-4.

### <Step I-4-1>

This step is a step of subjecting the compound represented by formula A-15 above and the compound represented by formula A-16 above to β-1,4-glycosidic linkage to give the compound represented by formula A-17. The above glycosidic linkage step may be performed by using or applying a known procedure. However, the procedure shown in Examples 40 or 66, for example, may be preferably used.

### <To produce compound represented by formula A-16>

In an embodiment of the present invention, the compound represented by formula A-16 above may be produced like in the following substeps V-1 to V-11. This step comprises essential substep V-7 described below, in which two molecules of monosaccharide are subjected to α-2,6-glycosidic linkage to synthesize a disaccharide block. The other substeps can be carried out by using or applying usual procedures for producing a monosaccharide or oligosaccharide.

In an embodiment of the present invention, step V comprises the following substeps:

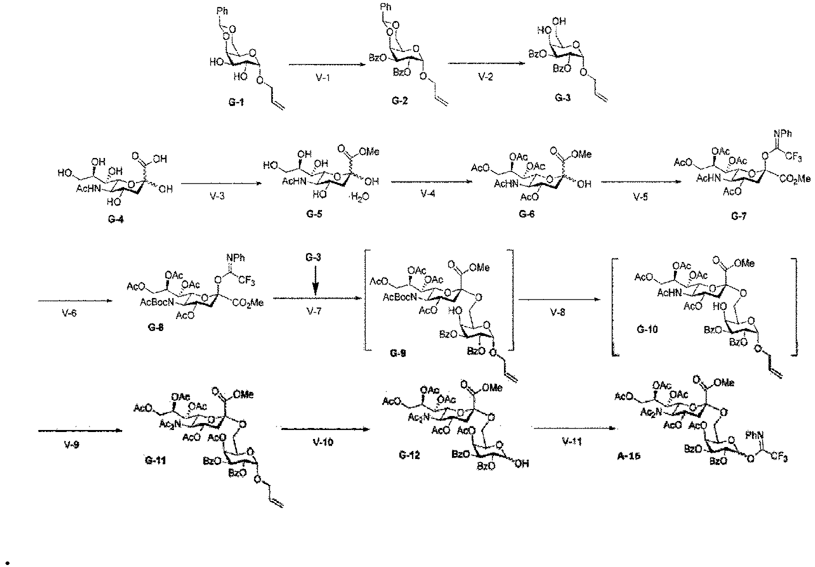

### <Substep V-1>

Substep V-1 is a step of protecting, by a benzoyl group, each hydroxyl group on the compound represented by the following formula G-1: 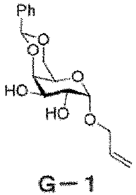 to produce a compound represented by the following formula G-2: 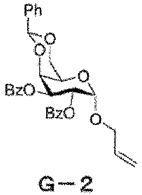 . The compound represented by formula G-1, which is the starting material for this step and is the compound specified as CAS No. 100759-10-2, may be produced by a known procedure. Here, the procedure shown in Examples 16 and 17, for example, may be used for production. This step may be performed by using or applying a known procedure. However, the procedure shown in Example 18, for example, may be preferably used.

### <Substep V-2>

Substep V-2 is a step of eliminating the benzylidene protecting group from the compound represented by formula G-2 to produce a compound represented by the following formula G-3: 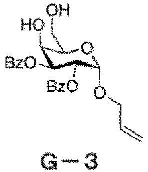 . This step may be performed by using or applying a known procedure. However, the procedure shown in Example 19, for example, may be preferably used.

In an embodiment of the present invention, this step comprises a step of bringing a solvent dissolving the resulting compound represented by formula G-3 into contact with silica gel to perform solid-phase extraction of the compound represented by formula G-3. Since an unreacted compound represented by formula G-2 and eliminated benzaldehyde are not adsorbed on silica gel, the compound represented by formula G-3 can be efficiently purified by this step.

Examples of the solvent used to dissolve the compound represented by formula G-3 include toluene, heptane, dichloromethane, chloroform, or a combination thereof. Preferable examples include toluene, dichloromethane, chloroform, or a combination thereof. Particularly preferable examples include, but are not limited to, toluene.

As silica gel in this step, silica gel in an amount 2 to 5 times the amount of the raw material, for example, may be used, silica gel in an amount 2 to 4 times the amount of the raw material may be preferably used, and silica gel in an amount about 3 times the amount of the raw material may be more preferably used.

In this step, the solvent used to elute the compound represented by formula G-3 adsorbed on silica gel is not limited as long as the solvent does not dissolve the silica gel and can elute the target compound. Examples include cyclopentyl methyl ether, ethyl acetate, or tert-butyl methyl ether.

### <Substep V-3>

Substep V-3 is a step of subjecting the carboxylic acid of the compound represented by the following formula G-4: 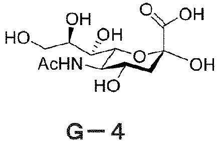 to esterification, followed by water pouring to produce a compound represented by the following formula G-5: 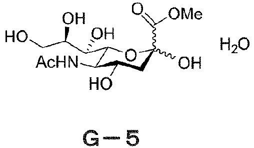 . The compound represented by formula G-4, which is the starting material for this step, may be produced by a known procedure or a commercially available product may be used. Examples of the commercially available compound represented by formula G-4 include N-acetylneuraminic acid, manufactured by TOKYO CHEMICAL INDUSTRY CO., LTD. This step may be performed by using or applying a known procedure. However, the procedure shown in Example 20, for example, may be preferably used.

### <Substep V-4>

Substep V-4 is a step of protecting, by an acetyl group, each hydroxyl group other than the hydroxyl group attached to the carbon at position 1 of the compound represented by formula G-5 to produce a compound represented by the following formula G-6: 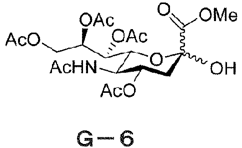 . This step may be performed by using or applying a known procedure. However, the procedure shown in Example 21, for example, may be preferably used.

### <Substep V-5>

Substep V-5 is a step of reacting the compound represented by formula G-6 with 2,2,2-trifluoro-N-phenylacetimidoyl chloride (TFPC) to produce a compound represented by the following formula G-7: 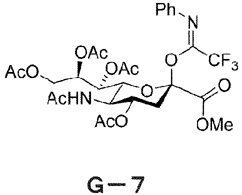 . This step may be performed by using or applying a known procedure. However, the procedure shown in Example 22, for example, may be preferably used.

In an embodiment of the present invention, this step is a step of reacting the compound represented by formula G-6 with TFPC in the presence of N-methylimidazole to produce the compound represented by formula G-7. Compared to the case of using K₂CO₃, N-methylimidazole may be used as a base in this step. In this case, the equivalent amount of TFPC can be reduced, and the target product can be obtained in high yield. Since TFPC is an expensive reagent, an increase in the yield of this step is very beneficial for commercial production.

The solvent in this step is not limited as long as the reaction proceeds. Examples include dichloromethane, toluene, ethyl acetate, acetonitrile, or tetrahydrofuran. Preferred is dichloromethane.

The reaction temperature in this step is not limited as long as the reaction proceeds, and may be preferably from 0°C to 40°C, more preferably from 0°C to 35°C, and particularly preferably from 0°C to 30°C.

This step is suitably performed in the presence of a dehydrating agent. The dehydrating agent in this step is not limited as long as the reaction proceeds. Examples include Molecular Sieves. Preferred is Molecular Sieves 4A powder with a powder particle size of 10 µm or less.

### <Substep V-6>

Substep V-6 is a step of protecting, by a tert-butoxycarbonyl group, the nitrogen atom of the acetamide group in the compound represented by formula G-7 to produce a compound represented by the following formula G-8: 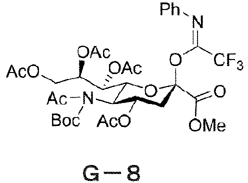 . This step may be performed by using or applying a known procedure. However, the procedure shown in Example 23, for example, may be preferably used.

The compound represented by formula G-8 that is produced in this step and dissolved in a solvent may be used as it is in the next step or may be isolated and purified by recrystallization. The major advantage of the compound represented by formula G-8 is that the compound can be isolated and purified by crystallization. The compound represented by formula G-8 with HPLC purity of 99% or higher can be obtained by recrystallization. Moreover, no impurities are included. Thus, a glycosylation reaction in the next step can be carried out stably. The isolation and purification by recrystallization may be performed by a procedure comprising adding heptane to a cyclopentyl methyl ether-containing solution for crystallization.

### <Substep V-7>

Substep V-7 is a step of subjecting the compound represented by formula G-8 and the compound represented by formula G-3 to α-2,6-glycosidic linkage to produce a compound represented by the following formula G-9: 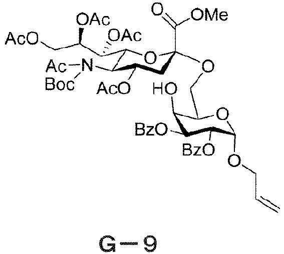 . It is difficult to selectively link an N-acetylneuraminic acid derivative and a galactose derivative by α-2,6-glycosidic linkage. For example, a method for synthesizing a disaccharide by reacting a compound represented by formula G-7 with a compound represented by formula G-3 has been reported (J. Org. Chem., 2016, 81, 10600-10616). The reaction was not easy to reproduce, and the desired yield and selectivity could not be obtained. Unfortunately, in this reaction, the selectivity decreases as the scale increases, the reaction temperature tolerance is narrow, and the effect of reaction heat is significant. The compound represented by formula G-7, which is one of the raw material compounds in this reaction, is very expensive. Therefore, the low reproducibility, yield, and selectivity in this reaction are a major problem, especially in commercial production where scale-up is required. On the other hand, if the compound represented by formula G-8 with a tert-butoxycarbonyl group is used instead of the compound represented by formula G-7 as a raw material compound, the high selectivity for α-2,6-glycosidic linkage (α : β = 93 : 7) can be well-reproduced, and the yield is also improved. In addition, the temperature tolerance is wider, and high reproducibility, yield, and selectivity can be achieved even when scaled up. This has a very beneficial effect on commercial production.

This step can suitably be performed in the presence of a Lewis acid. The Lewis acid in this step is not limited as long as the reaction proceeds. Examples include trimethylsilyl trifluoromethanesulfonate, triisopropylsilyl trifluoromethanesulfonate, or tert-butyl dimethylsilyl trifluoromethanesulfonate. Preferred is trimethylsilyl trifluoromethanesulfonate.

The solvent in this step is not limited as long as the reaction proceeds. Examples include cyclopentyl methyl ether, diisopropyl ether, tert-butyl methyl ether, diethyl ether, dibutyl ether, dipropyl ether, 1,4-dioxane, dichloromethane, 1,2-dichloroethane, toluene, chlorobenzene, trifluoromethylbenzene, propionitrile, or acetonitrile. Preferred is cyclopentyl methyl ether.

The reaction temperature in this step is not limited as long as the reaction proceeds, and may be, for example, from -78°C to 0°C, preferably from -78°C to - 20°C, more preferably from -78°C to -30°C, and particularly preferably from -78°C to -40°C.

In this step, it is preferable to use 1 to 3 equivalents of the compound represented by formula G-3 with respect to 1 equivalent of the compound represented by formula G-8. It is more preferable to use 1.4 to 2 equivalents of the compound represented by formula G-3 with respect to 1 equivalent of the compound represented by formula G-8.

This step is not limited as long as the reaction proceeds. For example, a mixed solution containing the compound represented by formula G-8 and the compound represented by formula G-3 (suitably, a cyclopentyl methyl ether mixed solution) is added dropwise over a long time to a Lewis acid-containing solution (suitably, cyclopentyl methyl ether solution). Alternatively, a solution containing the compound represented by formula G-8 (suitably, a cyclopentyl methyl ether solution) is added dropwise over a long time to a solution containing a Lewis acid and the compound represented by formula G-3 (suitably, cyclopentyl methyl ether solution). Preferably, a solution containing the compound represented by formula G-8 (suitably, a cyclopentyl methyl ether solution) is added dropwise over a long time to a solution containing a Lewis acid and the compound represented by formula G-3 (suitably, cyclopentyl methyl ether solution). The time spent for the dropwise addition is not limited as long as the reaction proceeds, and may be, for example, from 30 minutes to 5 hours, preferably from 1 to 4 hours, more preferably from 2 to 3.5 hours, and particularly preferably about 3 hours.

In an embodiment of the present invention, this step comprises a step of bringing a solvent dissolving the compound represented by formula G-9 into contact with silica gel to perform solid-phase extraction of the compound represented by formula G-9. N-phenyl trifluoroacetamide, a byproduct of the glycosylation reaction, and other trace impurities in the toluene solvent that are not adsorbed on silica gel are not adsorbed on silica gel. Therefore, the compound represented by formula G-9 can be efficiently purified by this step.

Examples of the solvent used to dissolve the compound represented by formula G-9 include toluene, heptane, dichloromethane, chloroform, or a combination thereof. Preferable examples include toluene, dichloromethane, chloroform, or a combination thereof. Particularly preferable examples include, but are not limited to, toluene.

As silica gel in this step, silica gel in an amount 2 to 5 times the amount of the raw material, for example, may be used, silica gel in an amount 2 to 4 times the amount of the raw material may be preferably used, and silica gel in an amount about 3.5 times the amount of the raw material may be more preferably used.

In this step, the solvent used to elute the compound represented by formula G-9 adsorbed on silica gel is not limited as long as the solvent does not dissolve the silica gel and can elute the target compound. Examples include ethyl acetate, cyclopentyl methyl ether, or tert-butyl methyl ether. Preferred is ethyl acetate.

This step may be performed using, for instance, the procedure shown in Example 24.

### <Substep V-8>

Substep V-8 is a step of eliminating the tert-butoxycarbonyl group from the compound represented by formula G-9 to produce a compound represented by the following formula G-10: 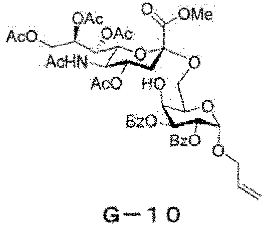 . This step may be performed by using or applying a known procedure. However, the procedure shown in Example 25, for example, may be preferably used.

### <Substep V-9>

Substep V-9 is a step of further protecting, by acetyl groups, the hydroxyl group and the nitrogen atom of the acetamide group in the compound represented by formula G-10 to produce a compound represented by the following formula G-11: 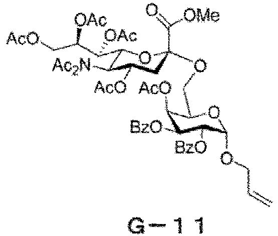 . This step may be performed by using or applying a known procedure. However, the procedure shown in Example 26, for example, may be preferably used.

In an embodiment of the present invention, this step comprises a step of bringing a solvent dissolving the compound represented by formula G-11 into contact with silica gel to perform solid-phase extraction of the compound represented by formula G-11. A byproduct, such as a diacetyl form of the compound represented by formula G-3, which is produced through acetylation of the compound represented by formula G-3 used in excess during the upstream glycosylation reaction, is not adsorbed on silica gel, and therefore this step allows the compound represented by formula G-11 to be efficiently purified.

Examples of the solvent used to dissolve the compound represented by formula G-11 include toluene, heptane, dichloromethane, chloroform, or a combination thereof. Preferable examples include toluene, dichloromethane, chloroform, or a combination thereof. Particularly preferable examples include, but are not limited to, toluene.

As the silica gel in this step, silica gel in an amount 2 to 5 times the amount of the raw material, for example, may be used, silica gel in an amount 2 to 4 times the amount of the raw material may be preferably used, and silica gel in an amount about 3.5 times the amount of the raw material may be more preferably used.

In this step, the solvent used to elute the compound represented by formula G-11 adsorbed on silica gel is not limited as long as the solvent does not dissolve the silica gel and can elute the target compound. Examples include ethyl acetate, cyclopentyl methyl ether, or tert-butyl methyl ether. Preferred is ethyl acetate.

### <Substep V-10>

Substep V-10 is a step of eliminating the allyl group attached to the carbon at position 1 of D-galactopyranoside in the compound represented by formula G-11 to produce a compound represented by the following formula G-12: 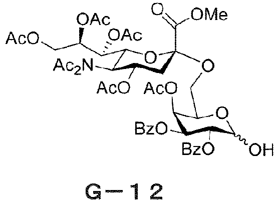 . This step may be performed by using or applying a known procedure. However, the procedure shown in Example 27-1, for example, may be preferably used. Alternatively, for example, as exemplified in Example 27-2, the compound represented by formula G-12 may be produced by stirring a solution containing the compound represented by formula G-11 and carbonylchlorohydridotris(triphenylphosphine) ruthenium(II), and then adding water and 1,3-dibromo-5,5-dimethylhydantoin, iodide, or N-bromosuccinimide to the solution and stirring the mixture to eliminate the allyl group attached via oxygen to the carbon at the 1-position of D-galactopyranoside in the compound represented by formula G-11. The solvent used in this step may be tetrahydrofuran, tetrahydropyran, cyclopentyl methyl ether, toluene, 2-methyltetrahydrofuran, 1,2-dimethoxyethane, t-butyl methyl ether, or the like, and the isomerizing temperature may be, for example, 40 to 65°C.

### <Substep V-11>

Substep V-11 is a step of reacting the compound represented by formula G-12 with 2,2,2-trifluoro-N-phenylacetimidoyl chloride (TFPC) to produce a compound represented by formula D-16 above. This step may be performed by using or applying a known procedure. However, the procedure shown in Example 28, for example, may be preferably used.

### <Purification of compound represented by formula A-17>

In this step I-4-1, the compound represented by formula A-17 can be obtained in a purified form by the following purification method. The purification method involves purifying the compound represented by formula A-17 above by stopping the reaction of the compound represented by formula A-15 above with the compound represented by formula A-16 above; then adding a hydrophobic carrier and water to a water-soluble organic solvent containing the resulting compound represented by formula A-17 above and contaminants to adsorb the compound represented by formula A-17 above onto the hydrophobic carrier; then filtering the resultant and washing the hydrophobic carrier with a mixed solution of the above water-soluble organic solvent and water to remove the contaminants; and then eluting the compound represented by formula A-17 above using an organic solvent, from the hydrophobic carrier. As described in the method of purifying the compound represented by formula A-6 above in step I-1-1, this purification method makes it possible to produce a large amount of high-quality oligosaccharide efficiently in the liquid-phase synthesis of oligosaccharide chains by using a small amount of hydrophobic carrier.

The purification of the compound represented by formula A-17 above is not limited to the purification in this step. Then, an embodiment of the present invention also provides a method comprising the following: adding a hydrophobic carrier and water to a water-soluble organic solvent containing the compound represented by formula A-17 above and contaminants to adsorb the compound represented by formula A-17 above onto the hydrophobic carrier; then filtering the resultant and washing the hydrophobic carrier with a mixed solution of the above water-soluble organic solvent and water to remove the contaminants; and then eluting the compound represented by formula A-17 above using an organic solvent, from the hydrophobic carrier to purify the above compound represented by formula A-17 above.

The above term "contaminants" refers to compounds and/or reagents other than the protected oligosaccharide (in this step, the compound represented by formula A-17), and mainly means reagents used in a reaction of synthesizing a protected oligosaccharide and their remnants, sugars other than the protected oligosaccharide, such as mono- or disaccharide compounds used in the elongation reaction of the protected oligosaccharide, or byproducts generated by the deprotection reaction of the protected oligosaccharide. Note that the "hydrophobic carrier" (e.g., a resin for packing in reversed-phase partition chromatography), "water-soluble organic solvent," "organic solvent," and the purification temperature used in this step are substantially the same as those described in the method of purifying the compound represented by formula A-6 in step I-1-1 above.

### <Step I-4-2>

This step is a step of eliminating each amino group-protecting group and each hydroxyl group acyl-based protecting group on the compound represented by formula A-17 to give the compound represented by formula A-18. The above removal (deprotection) of each amino group-protecting group may be performed by using or applying a known procedure. However, the procedure shown in Example 43 or 67, for example, may be preferably used. For example, this can be implemented by sequential addition of 1,2-dimethoxyethane and potassium hydroxide, sodium hydroxide, or lithium hydroxide aqueous solution, but is not limited to these solutions.

In the compound represented by formula A-18, "M⁺" is preferably a sodium ion, lithium ion, potassium ion, or protonated triethylamine cation, but is not limited thereto. On the other hand, the compound represented by formula A-18 obtained in the above step can be isolated as its free form (compound represented by formula A-18-FF) by a known or well-known method. The compound represented by formula A-18 is one form of "salt" of the compound represented by formula A-18-FF. As described above, a compound in which a salt is formed with a hydroxyl group, an amino group, or the like in the compound is also included in the scope of the present invention. Note that the same applies to, for example, compounds represented by formulas A-19 and A-20 described below (free forms thereof are compounds represented by formula A-19-FF and formula A-20-FF, respectively).

The following steps I-4-3 to I-4-4 are exemplary embodiments for the production of the compound represented by formula A-20 from the compound represented by formula A-18. However, the procedure is not limited to these production steps.

### <Step I-4-3>

This step is a step of protecting, by an acetyl group, each amino group on the compound represented by formula A-18 to give a compound represented by the following formula A-19: 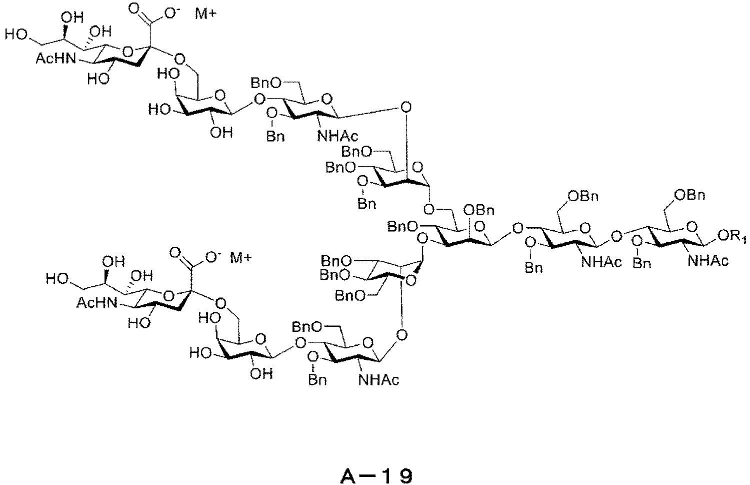 wherein R₁ is a C₁ to C₆ alkyl group or a phenyl group, the phenyl group is optionally substituted with one to three C₁ to C₆ alkoxy groups, and M⁺ is a sodium ion, lithium ion, potassium ion, or protonated triethylamine cation. The above protection of each amino group by an acetyl group may be performed by using or applying a known procedure. However, the procedure shown in Example 44 or 68, for example, may be preferably used.

### <Step I-4-4>

This step is a step of removing a benzyl group from each benzyloxy group on the compound represented by formula A-19 to give the above compound represented by formula A-20. The above benzyl group removal may be performed by using or applying a known procedure. However, the procedure shown in Example 45 or 69, for example, may be preferably used. For example, this can be implemented by adding N-methylpyrrolidone and Pd/C to the compound represented by formula A-19, then performing pressure reduction → nitrogen displacement and hydrogen pressurization → pressure release. However, the procedure is not limited to the above.

### <Step I-5>

Step I-5 is a step comprising a step of reacting the compound represented by formula A-20 above with an azido PEG linker compound (11-azido-3,6,9-trioxaundecan-1-amine) represented by the following formula A-21: 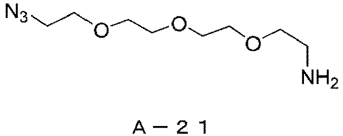 to give the oligosaccharide represented by formula A-22 above. The above compound represented by formula A-20 and the compound represented by formula A-21 may be linked by using or applying a known procedure. However, the procedure shown in Example 51 or 70, for example, may be preferably used. For example, this can be implemented by adding, to a solution containing the compound represented by formula A-20, in sequence, the compound represented by formula A-21, N-ethyl diisopropyl amine, and hexafluorophosphate (benzotriazol-1-yloxy)tripyrrolidinophosphonium, bromotripyrrolidinophosphonium=hexafluorophosphate, or 4-(4,6-dimethoxy-1,3,5-triazin-2-yl)-4-methylmorpholinium chloride, and then stirring the mixture. However, the procedure is not limited to the above.

### <Purification of compound represented by formula A-21>

In an embodiment of the present invention, the above compound represented by formula A-21 is obtained by the purification method comprising: a step of adding, to a solution containing the crude compound represented by formula A-21, a compound represented by the following formula E-1: 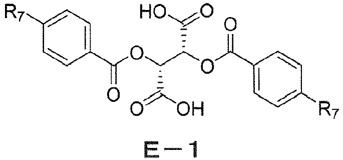 (wherein R₇ is a hydrogen atom, a methyl group, or a methoxy group) to give a crystallin compound represented by the following formula E-2: 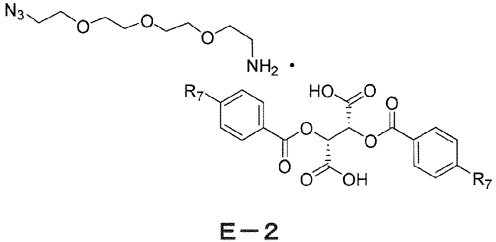 (wherein R₇ is a hydrogen atom, a methyl group, or a methoxy group); and a step of isolating the crystalline compound and then extracting the compound represented by formula A-21 from the crystalline compound isolated. This purification method makes it possible to obtain the high-purity compound represented by formula A-21. The compound represented by formula A-21 preferably has a purity (also referred to herein as "HPLC purity") of 95% or higher, more preferably 96% or higher or 97% or higher, and still more preferably 98% or higher or 99% or higher when measured by HPLC. As such, the compound represented by A-21 is purified because the compound-containing commercially available reagent is mixed with their dimers and other impurities. Moreover, in conventional technology, their purification requires either rigorous distillation purification or complicated column purification. Further, if an azide structure is included, distillation operations that require heating cannot be applied due to concerns about its explosion. This is inconvenient. The present inventors have investigated purification methods to obtain a high-purity compound represented by formula A-21 and as a result, have found that when the three different tartaric acid derivatives (wherein R₇ is a hydrogen atom, a methyl group, or a methoxy group) represented by formula E-1 above are used, the compound represented by formula A-21 forms a one-to-one salt with each tartaric acid derivative and is able to be isolated as crystals. The obtained compound represented by formula E-2 above is a new crystalline compound, and it is possible to obtain the compound represented by formula A-21, with a higher HPLC purity (suitably with a HPLC purity of 95% or higher) than before purification, by isolation and then liquid-separation with, for instance, ethyl acetate/hydrochloric acid solution, followed by freeing and extraction.

The above purification method is exemplified as follows. First, the compound represented by formula E-1 is added to a solution containing the compound represented by formula A-21 in a solvent e.g., acetonitrile and water. The mixture is stirred. After dissolution is checked, a solvent e.g., acetonitrile is added. The resulting slurry solution is concentrated under reduced pressure and stirred. Then, the precipitated crystals are filtered. The filtered crystals are washed with acetonitrile and dried under reduced pressure to obtain crystals of the compound represented by formula E-2 (crystal formation step). Then, a solution containing the obtained crystalline compound in ethyl acetate and water is admixed with concentrated hydrochloric acid. The mixture is stirred and liquid-separated. The resulting aqueous layer is washed with, for instance, ethyl acetate and adjusted to be basic with, for instance, aqueous sodium hydroxide solution. Subsequently, sodium chloride, for instance, is added and dissolved. A solvent such as dichloromethane is then added. The mixture is stirred and liquid-separated. The resulting organic layer is concentrated under reduced pressure. After a solvent such as acetonitrile is added and the mixture is concentrated under reduced pressure, the resulting solution is filtered and washed with a solvent such as acetonitrile. The obtained solution is concentrated under reduced pressure (extraction process) to obtain the compound represented by formula A-21 with high HPLC purity. More preferably, the procedure shown in Examples 46 to 50, for instance, may be performed.

The purification of the compound represented by formula A-21 above is not limited to the purification in this step. Thus, an embodiment of the present invention also provides a method of purifying the compound represented by formula A-21, the method comprising: a step of adding, to a solution containing the crude compound represented by formula A-21 above, the compound represented by formula E-1 above (wherein R₇ is a hydrogen atom, a methyl group, or a methoxy group) to give the crystallin compound represented by formula E-2 above (wherein R₇ is a hydrogen atom, a methyl group, or a methoxy group); and a step of isolating the crystalline compound and then extracting the compound represented by formula A-21 from the crystalline compound isolated.

### <Novel compounds>

The desired oligosaccharide represented by formula A-22 is obtained by the method of the present invention comprising steps I-1 to I-5 above. In addition, the intermediates for the oligosaccharide represented by formula A-22 above are useful in the production of the oligosaccharide, but are not limited to the production of the oligosaccharide. The intermediates are applicable to any usage. Thus, the present invention provides the oligosaccharide represented by formula A-22 above and intermediates thereof.

An embodiment of the present invention provides an oligosaccharide represented by the following formula A-22: 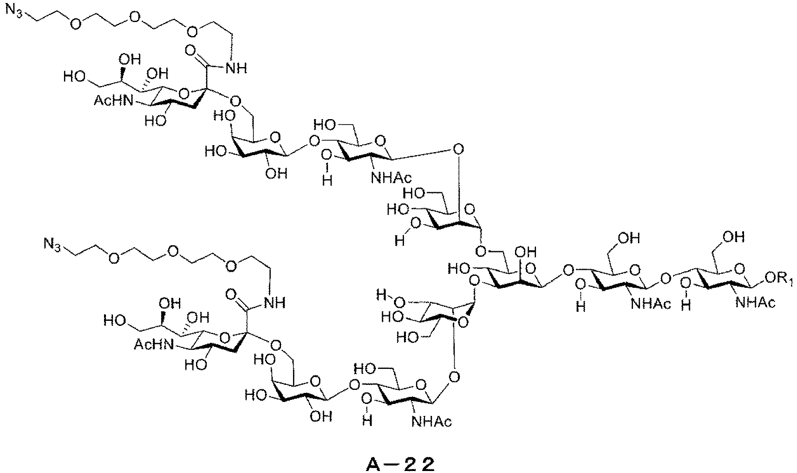 wherein R₁ is a C₁ to C₆ alkyl group or a phenyl group, and the phenyl group is optionally substituted with one to three C₁ to C₆ alkoxy groups.

An embodiment of the present invention provides an oligosaccharide represented by the following formula A'-22:

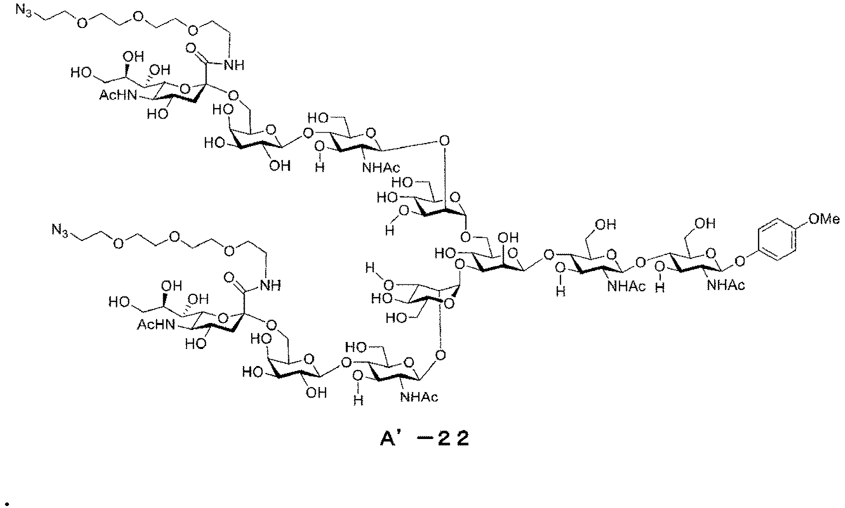

An embodiment of the present invention provides an oligosaccharide represented by the following formula A"-22:

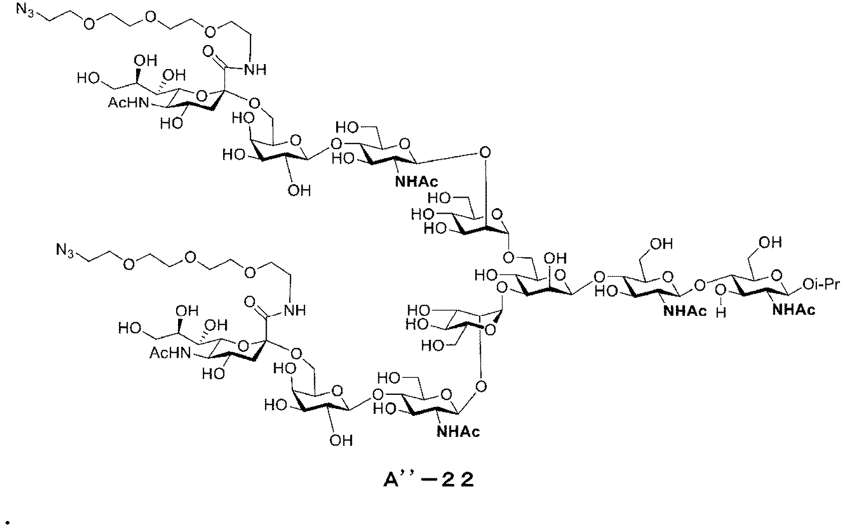

An embodiment of the present invention provides a compound represented by the following formula A-8: 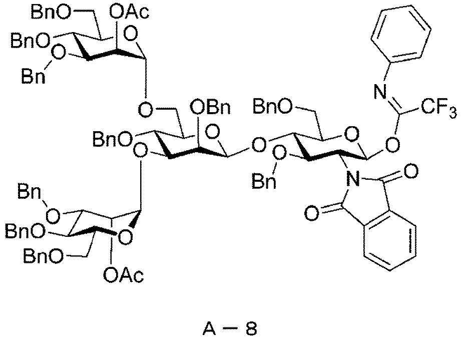 wherein R₁ is a C₁ to C₆ alkyl group or a phenyl group, and the phenyl group is optionally substituted with one to three C₁ to C₆ alkoxy groups.

An embodiment of the present invention provides a compound represented by the following formula A-10: 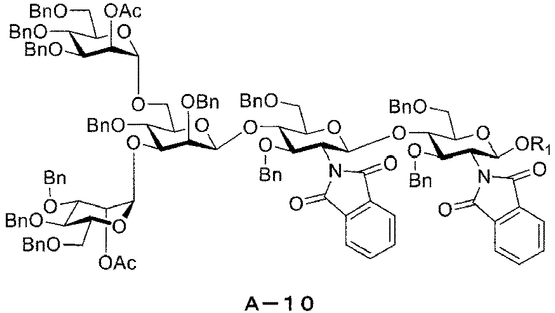 wherein R₁ is a C₁ to C₆ alkyl group or a phenyl group, and the phenyl group is optionally substituted with one to three C₁ to C₆ alkoxy groups.

An embodiment of the present invention provides a compound represented by the following formula A-11: 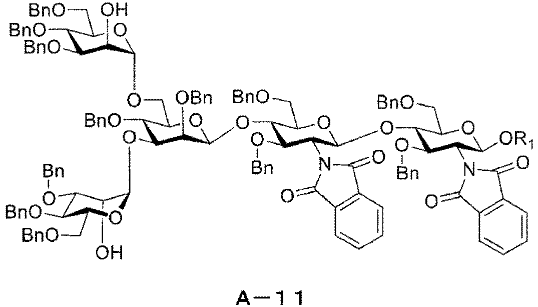 wherein R₁ is a C₁ to C₆ alkyl group or a phenyl group, and the phenyl group is optionally substituted with one to three C₁ to C₆ alkoxy groups.

An embodiment of the present invention provides a compound represented by the following formula A-13: 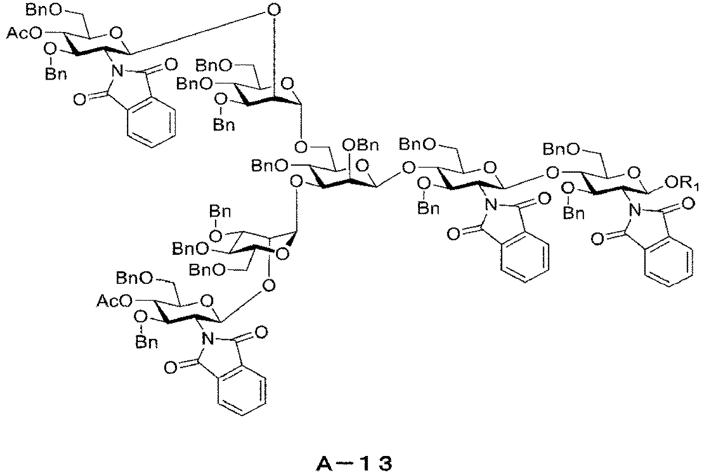 wherein R₁ is a C₁ to C₆ alkyl group or a phenyl group, and the phenyl group is optionally substituted with one to three C₁ to C₆ alkoxy groups.

There is provided a compound represented by the following formula A-14: 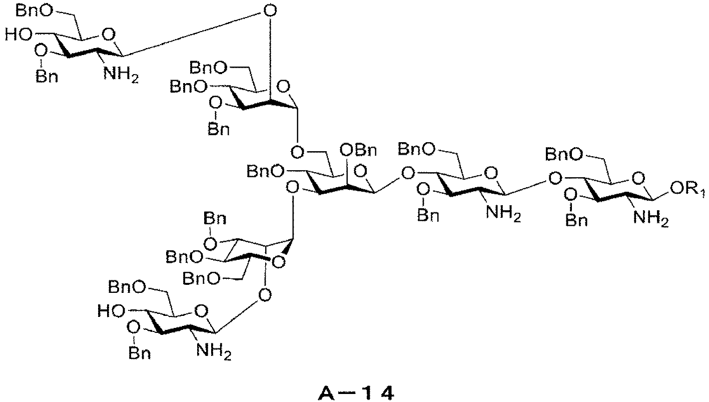 wherein R₁ is a C₁ to C₆ alkyl group or a phenyl group, and the phenyl group is optionally substituted with one to three C₁ to C₆ alkoxy groups.

An embodiment of the present invention provides a compound represented by the following formula A-14-FMA: 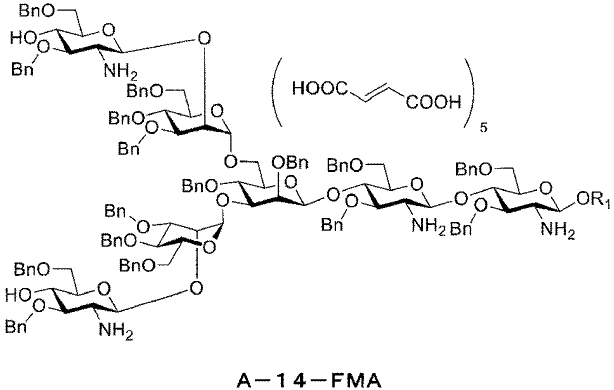 wherein R₁ is a C₁ to C₆ alkyl group or a phenyl group, and the phenyl group is optionally substituted with one to three C₁ to C₆ alkoxy groups.

An embodiment of the present invention provides a compound represented by the following formula A-15: 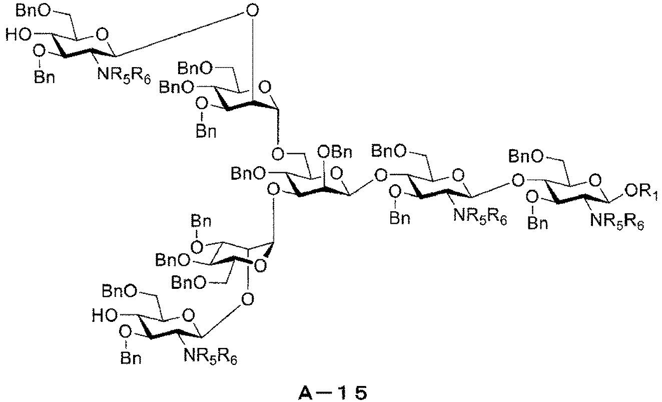 wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group.

An embodiment of the present invention provides a compound represented by the following formula A-17: 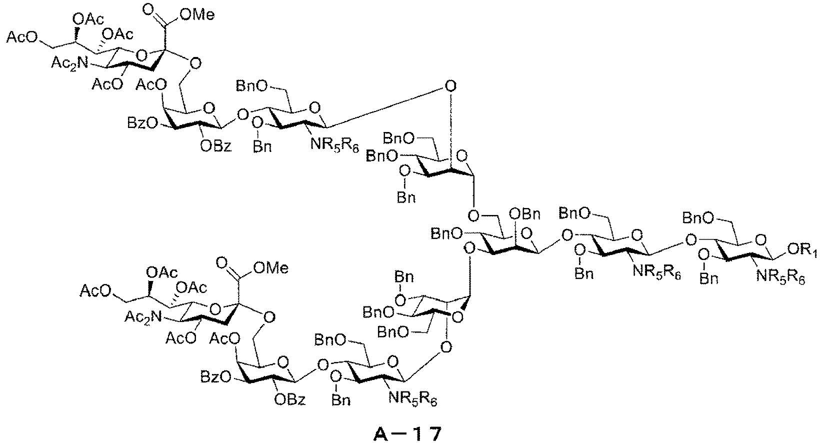 wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group.

An embodiment of the present invention provides a compound represented by the following formula A-18-FF: 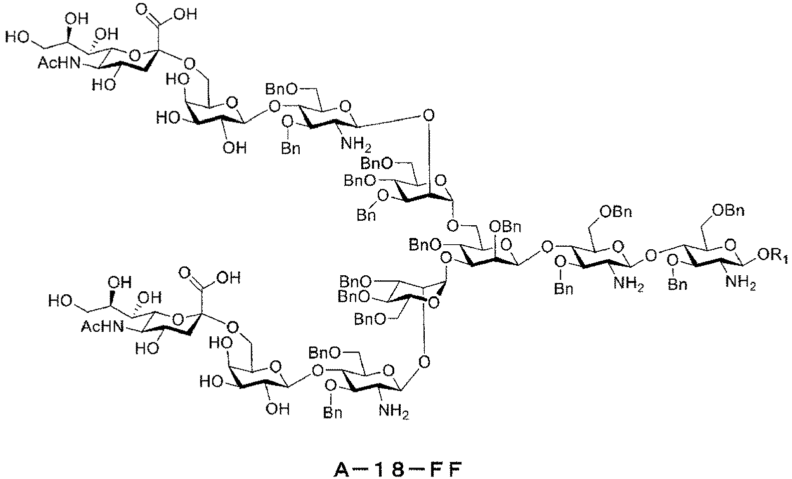 wherein R₁ is a C₁ to C₆ alkyl group or a phenyl group, and the phenyl group is optionally substituted with one to three C₁ to C₆ alkoxy groups,
or a salt thereof.

An embodiment of the present invention provides a compound represented by the following formula A-18: 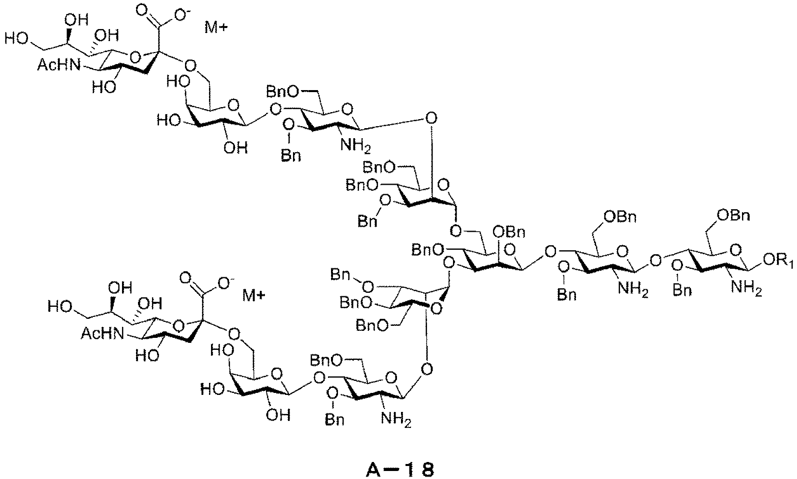 wherein R₁ is a C₁ to C₆ alkyl group or a phenyl group, the phenyl group is optionally substituted with one to three C₁ to C₆ alkoxy groups, and M⁺ is a sodium ion, lithium ion, potassium ion, or protonated triethylamine cation.

An embodiment of the present invention provides a compound represented by the following formula A-19-FF: 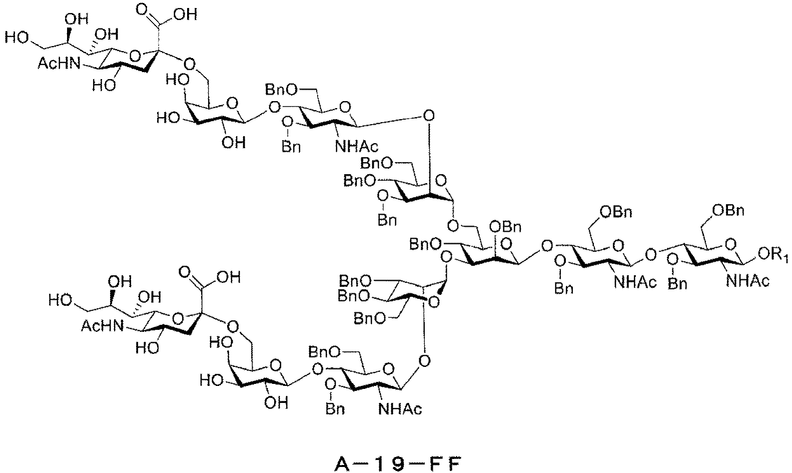 wherein R₁ is a C₁ to C₆ alkyl group or a phenyl group, and the phenyl group is optionally substituted with one to three C₁ to C₆ alkoxy groups,
or a salt thereof.

An embodiment of the present invention provides a compound represented by the following formula A-19: 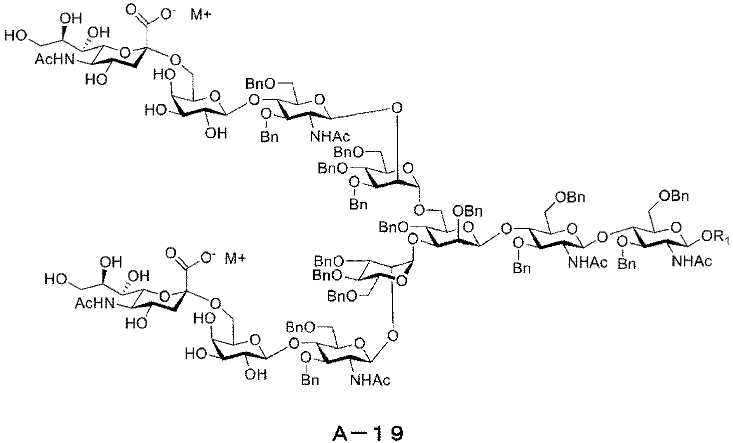 wherein R₁ is a C₁ to C₆ alkyl group or a phenyl group, the phenyl group is optionally substituted with one to three C₁ to C₆ alkoxy groups, and M⁺ is a sodium ion, lithium ion, potassium ion, or protonated triethylamine cation.

An embodiment of the present invention provides a compound represented by the following formula A-20-FF: 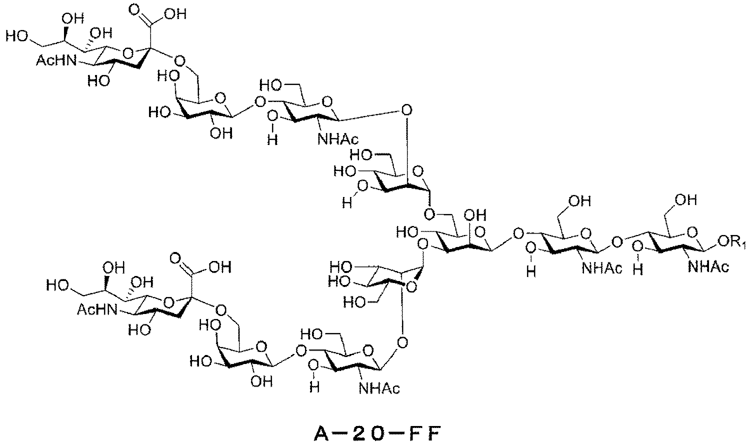 wherein R₁ is a C₁ to C₆ alkyl group or a phenyl group, and the phenyl group is optionally substituted with one to three C₁ to C₆ alkoxy groups,
or a salt thereof.

An embodiment of the present invention provides a compound represented by the following formula A'-20-FF: 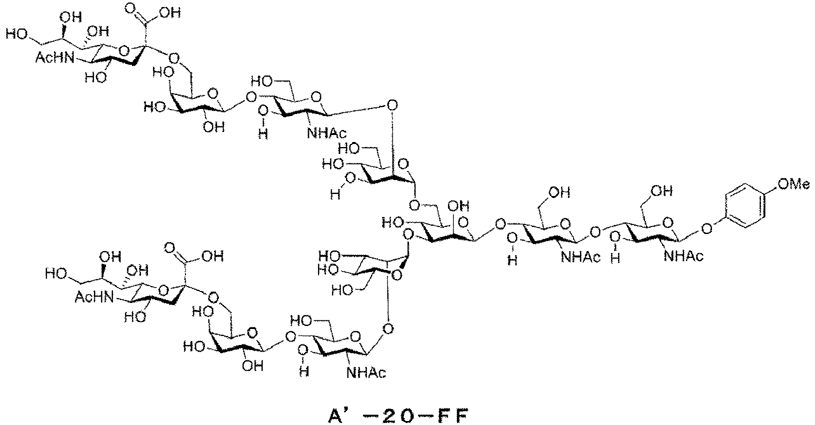 or a salt thereof.

An embodiment of the present invention provides a compound represented by the following formula A-20: 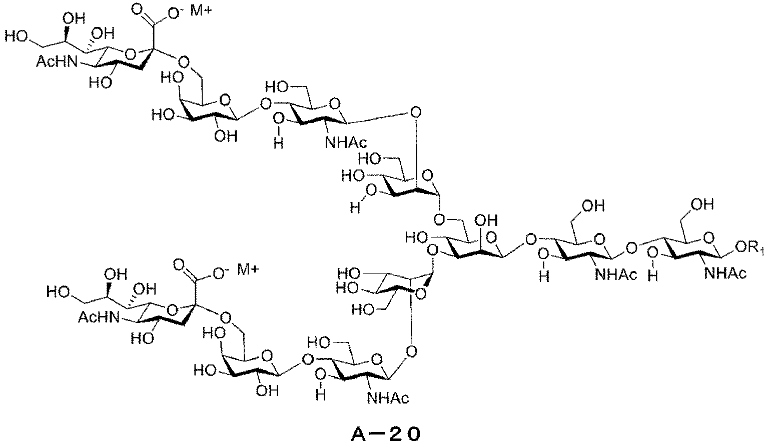 wherein R₁ is a C₁ to C₆ alkyl group or a phenyl group, the phenyl group is optionally substituted with one to three C₁ to C₆ alkoxy groups, and M⁺ is a sodium ion, lithium ion, potassium ion, or protonated triethylamine cation.

### <Glycoprotein or the like and production method therefor>

One embodiment of the present invention provides, for instance, a novel glycoprotein and its novel production method in which a biantennary glycan having an α2,6-sialic acid structure at a non-reducing end (i.e., an oligosaccharide represented by formula A-22) is used as a donor molecule in synthesizing, for example, a glycoprotein (especially, a glycan-remodeled antibody or its FC region-containing molecule, or an antibody-drug conjugate). As detailed below, the oligosaccharide represented by formula A-22 obtained by the production method of the present invention may be used for, but is not limited to, the production of a glycoprotein (in particular, a glycan-remodeled antibody or a molecule containing an Fc region thereof, or an antibody-drug conjugate) (for methods of conjugating an oligosaccharide and an antibody using "one-pot process", see WO 2018/003983, WO 2022/050300, and PLoS ONE 2018, 13, e0193534. In addition, for methods of conjugating an oligosaccharide and an antibody using the oxazolination method, see WO 2019/065964, WO 2020/050406, etc.), or may be used for other applications.

It has recently been reported that the heterogeneous glycans of antibodies can be remodeled by enzymatic reactions to introduce functionalized glycans uniformly (ACS Chem. Biol. 2012, 7, 110-122, ACS Med. Chem. Lett. 2016, 7, 1005-1008). Using this glycan remodeling technology, attempts have been made to synthesize a homogeneous antibody-drug conjugate (ADC) by introducing a drug(s) in a site-specific manner (Bioconjugate Chem. 2015, 26, 2233-2242; Angew. Chem. Int. Ed. 2016, 55, 2361-2367, US2016361436).

In the glycan remodeling, heterogeneous glycans added to a protein (e.g., an antibody) are cleaved off, using hydrolase, to leave only N-acetylglucosamine (GlcNAc) at each terminus thereby producing a homogenous protein moiety with GlcNAc (hereinafter, referred to as an "acceptor molecule"). Subsequently, a given glycan separately prepared (hereinafter, referred to as a "donor molecule") is provided, and the acceptor molecule and the donor molecule are linked together by using glycosyltransferase. Thereby, a homogeneous glycoprotein with a given glycan structure can be synthesized.

In an embodiment of the present invention, the novel production method of the present invention may be used to produce an oligosaccharide represented by formula A-22. Its terminal structure may then be activated, so that the oligosaccharide can be used as a donor molecule in the synthesis of the above homogeneous glycoprotein (in particular, a glycan-remodeled antibody or a molecule containing an Fc region thereof).

As used herein, the term "glycan" refers to a structural unit in which two or more monosaccharides are linked by a glycosidic linkage. A specific monosaccharide or glycan may be denoted by an abbreviation such as "GlcNAc-", for example. When such an abbreviation is used in a structural formula, an oxygen or nitrogen atom belonging to a glycosidic linkage with another structural unit at a reducing end is not included in the abbreviation representing the glycan, unless otherwise defined.

In the present invention, in the description of a monosaccharide serving as a basic unit of a glycan, unless otherwise defined, for convenience, in the ring structure, a carbon atom attached to an oxygen atom constituting the ring and directly attached to a hydroxy group (or an oxygen atom belonging to a glycosidic linkage) is represented as the 1-position (the 2-position only in sialic acid). The names of example compounds are each given for the entire chemical structure, and this rule does not necessarily apply thereto.

In the present invention, when a glycan is shown as a symbol (e.g., GlcNAc), unless otherwise defined, the carbon at a reducing end is included in the symbol, but N or O belonging to an N-or O-glycosidic linkage is not included in the symbol.

As used herein, the term "glycoprotein" refers to a protein in which a glycan is attached to a portion of the amino acids constituting the protein.

In an embodiment of the present invention, "glycoprotein" is a glycan-remodeled antibody or a molecule containing an Fc region thereof. Thus, an embodiment of the present invention provides a method for producing a glycan-remodeled antibody or a molecule containing an Fc region thereof, the method comprising the steps of obtaining a glycan donor molecule, the step comprising the novel method for producing an oligosaccharide of the present invention; and reacting the glycan donor molecule with an acceptor molecule that is an antibody having core GlcNAc to which fucose is optionally added as an N297-linked glycan or a molecule containing an Fc region thereof. As used herein, the term "core GlcNAc" is GlcNAc at the reducing end of the N297-linked glycan.

As used herein, the term "glycan donor molecule" refers to a molecule responsible for donating a glycan to the acceptor molecule. In an embodiment of the present invention, the glycan donor molecule is the oligosaccharide represented by formula A-22. In a preferred embodiment of the present invention, the oligosaccharide represented by formula A-22 is an oligosaccharide wherein R₁ is a 4-methoxyphenyl group or an isopropyl group, but is not limited thereto. In the present invention, an oligosaccharide wherein the 4-methoxyphenyl group of R₁ is replaced by an isopropyl group can be produced using, instead of the compound represented by formula A'-9, a compound wherein the 4-methoxyphenyl group of the above compound is replaced by an isopropyl group (compound represented by formula A"-9) (see Examples 13, 60, etc.).

As used herein, the term "acceptor molecule" refers to a molecule that receives a glycan from the glycan donor molecule. The "acceptor molecule" is not particularly limited as long as it is a molecule capable of receiving a glycan from the glycan donor molecule. Preferably, the acceptor molecule is a glycan-cleaved antibody obtained by cleaving most of the glycan from an antibody or a molecule containing an Fc region thereof, and more preferably an antibody having core GlcNAc or a molecule containing an Fc region thereof. The glycan to be partially cleaved in the glycan-cleaved antibody is an N-linked glycan or an O-linked glycan, and is preferably an N-linked glycan.

The N-linked glycan is attached to an amino acid side chain of the antibody via an N-glycosidic linkage, and the O-linked glycan is attached to an amino acid side chain of the antibody via an O-glycosidic linkage.

The antibody as the "acceptor molecule" is preferably IgG, and more preferably IgG1, IgG2 or IgG4.

IgG has a well-conserved N-linked glycan at the 297th asparagine residue (hereinafter referred to as "Asn297 or N297") in the heavy chain Fc region thereof (hereinafter referred to as "Asn297-linked glycan or N-linked glycan"), which is known to contribute to the activity, kinetics, and the like of an antibody molecule (Eon-Duval, A. et al, Biotechnol. Prog. 2012, 28, 608-622; Sanglier-Cianferani, S., Anal. Chem. 2013, 85, 715-736).

The amino acid sequence in the constant region of IgG is well conserved, and the respective amino acids are identified by EU numbering (EU INDEX) in the report by Edelman et al. (Proc. Natl. Acad. Sci. U.S.A., 63, 78-85, (1969)). For example, Asn297 to which an N-linked glycan is added in the Fc region corresponds to position 297 according to EU numbering. Even when the actual amino acid position varies due to molecular fragmentation or region deletion, the amino acid can be uniquely identified if it is represented by EU numbering.

In an embodiment of the present invention, the "acceptor molecule" is an antibody having core GlcNAc as the N297-linked glycan or a molecule containing an Fc region thereof. Another monosaccharide or glycan, for example, fucose, may be added to the core GlcNAc as the N297-linked glycan. Thus, in an embodiment of the present invention, the "acceptor molecule" is an antibody having core GlcNAc to which fucose is optionally added as an N297-linked glycan, or a molecule containing an Fc region thereof.

In the step of reacting the glycan donor molecule with an acceptor molecule that is an antibody having core GlcNAc to which fucose is optionally added as an N297-linked glycan or a molecule containing an Fc region thereof, any reaction methods and conditions may be used as long as the reaction proceeds.

In the present invention, the glycan-remodeled antibody or molecule containing an Fc region thereof may be produced by the method shown in the following scheme, according to the methods as disclosed in, for example, WO 2018/003983 and WO 2022/050300.

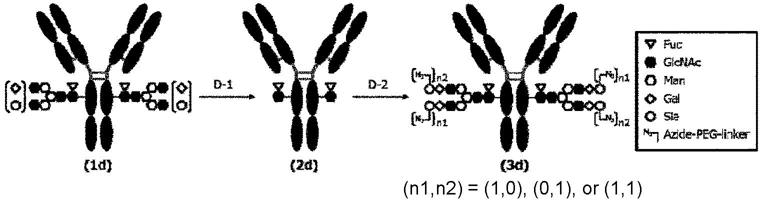

### (Step D-1)

This step is a step of subjecting a desired antibody to hydrolytic cleavage of a GlcNAcβ1-4GlcNAc glycosidic linkage of a reducing-end chitobiose structure of an N-linked glycan (N297-linked glycan) attached to the 297th asparagine in the amino acid sequence of the antibody, using a known enzymatic reaction, to produce a glycan-cleaved antibody. The desired antibody (1d) is subjected to hydrolysis reaction of the GlcNAcβ1-4GlcNAc glycosidic linkage of the reducing-end chitobiose structure with a hydrolase such as the wild-type Endo-S enzyme, at 0 to 40°C in a buffer (phosphate buffer or the like). The reaction time is 10 minutes to 72 hours, preferably 1 to 16 hours. The wild-type Endo-S enzyme or the like is used in an amount of 0.005 to 10 mg, preferably 0.01 to 3 mg, based on 100 mg of the antibody (1d). After the completion of the reaction, the reaction product is purified by a purification method suitable for the reaction scale (such as affinity chromatography, hydroxyapatite column, cation exchange chromatography, or multimodal chromatography) to obtain a (Fucα1,6)GlcNAc antibody (2d).

### (step D-2)

This step is a step of reacting the (Fucα1,6)GlcNAc antibody (2d) obtained in step D-1 with the oligosaccharide represented by formula A-22 by transglycosylation reaction with two Endo enzymes to prepare a reaction solution containing the glycan-remodeled antibody (3d), and then purifying the antibody by an appropriate method to produce the desired antibody (3d).

For the two Endo enzymes to be used, an enzyme A (Endo-S-like enzyme) and an enzyme B (Endo-M-like enzyme) may be suitably combined.

As used herein, the term "enzyme-A" refers to endo-β-N-acetylglucosaminidase acting on an N297-linked glycan of an Fc-containing molecule (such as an antibody) as a substrate. Examples of the enzyme A include Endo-S, Endo-S2 (EndoS-49), Endo-Si, Endo-Sd, Endo-Se, or Endo-Sz, and an Endo-S mutant, an Endo-S2 (Endo-S49) mutant, an Endo-Si mutant, an Endo-Sd mutant, an Endo-Se mutant, or an Endo-Sz mutant in which the hydrolysis activity of each enzyme has been reduced. Preferred as the enzyme A are Endo-S D233Q, Endo-S D233Q/Q303L, Endo-S D233Q/E350A, Endo-S D233Q/E350Q, Endo-S D233Q/E350D, Endo-S D233Q/E350N, Endo-S D233Q/D405A, Endo-S2 D184M, Endo-S2 T138Q, Endo-S2 D184Q, Endo-S2 D184Q/Q250L, Endo-S2 D184Q/E289Q, Endo-S2 D184M/Q250L, Endo-S2 D184M/E289Q, Endo-S2 D182Q, Endo-S2 D226Q, Endo-S2 T227Q, Endo-S2 T228Q, Endo-Si D241Q/Q311L, Endo-Si D241Q/E360Q, Endo-Si D241M, Endo-Si D241M/Q311L, Endo-Si D241M/E360Q, Endo-Si T190Q, Endo-Si T190/D241Q, Endo-Si T190Q/D241M, Endo-Si D241X₁ wherein X₁ represents any amino acid residue other than K, R and D, and specifically represents A, N, C, Q, E, G, H, I, L, M, F, P, S, T, W, Y or V, preferably A, Q, E, H, I, L, M, F, P, S, T, W, Y or V, Endo-Si T190X₂ wherein X₂ represents an amino acid residue of F, H, K, M, Q, R, W or Y, Endo-Si Q311X₃ wherein X₃ represents an amino acid residue of F, N or Y, Endo-Si E360K, Endo-Sd D232Q, Endo-Sd D232M, Endo-Sd D232Q/Q302L, Endo-Sd D232M/Q302L, Endo-Se D233Q, Endo-Se D233M, Endo-Se D233Q/Q303L, Endo-Se D233M/Q303L, Endo-Sz D234Q, Endo-Sz D234M, Endo-Sz D234Q/Q304L, or Endo-Sz D234M/Q304L.

As used herein, the term "enzyme-B" refers to endo-β-N-acetylglucosaminidase acting on a glycan of the glycan donor molecule as a substrate, particularly endo-β-N-acetylglucosaminidase acting on a glycan of the glycan donor molecule as a substrate but not acting on an N297-linked glycan as a substrate (in other words, having low reactivity, preferably extremely low reactivity, to the N297-linked glycan). Examples of the enzyme B include Endo-M, Endo-Om, Endo-CC, or Endo-Rp, and an Endo-M mutant, an Endo-Om mutant, an Endo-CC mutant, or an Endo-Rp mutant in which the hydrolytic activity of each enzyme has been reduced. Preferred as the enzyme B are Endo-M N175Q, Endo-M N175Q/Y217F, Endo-CC N180H, Endo-Om N194Q, Endo-Rp N172Q, Endo-Rp N172H, Endo-Rp N172A, Endo-Rp N172C, Endo-Rp N172D, Endo-Rp N172E, Endo-Rp N172G, Endo-Rp N172I, Endo-Rp N172L, Endo-Rp N172M, Endo-Rp N172P, Endo-Rp N172S, Endo-Rp N172T, Endo-Rp N172V, Endo-Rp W278F/S216V, Endo-Rp W278F/N246D, Endo-Rp W278F/D276N, Endo-Rp W278F/A310D, Endo-Rp W278F/N172D/F307Y, Endo-Rp W278F/N172D/F307H, Endo-Rp W278F/N172D/A310D, and Endo-Rp W214F/F307Y/L306I.

The transglycosylation reaction is carried out by reacting the antibody (2d) with the oligosaccharide represented by formula A-22 in a buffer (Tris buffer or the like) in the presence of glycosyltransferases that are the enzyme A (Endo-S-like enzyme) and the enzyme B (Endo-M-like enzyme). The reaction temperature may be appropriately selected according to the optimal temperature for the enzymes to be used, and is usually 15 to 50°C, and preferably 25 to 40°C. The reaction time can be appropriately selected from 2 to 48 hours. After the completion of the reaction, a purification method suitable for the reaction scale (such as affinity chromatography, hydroxyapatite column, cation exchange chromatography, or multimodal chromatography) or an ultrafiltration method (ultrafiltration membrane) may be selected to obtain the glycan-remodeled antibody (3d).

In preparing the above glycan-remodeled antibody, concentration of an aqueous solution of the antibody, measurement of concentration, and buffer exchange may be carried out according to Common Operations A to C described in WO 2020/050406.

As described above, the method for producing a glycan-remodeled antibody or a molecule containing an Fc region thereof according to the present invention may further comprise the step of reacting each of or both the two azide groups (N₃-) at the ends in the compound represented by formula A-22 with a molecule having an alkyne structure. This reaction step may be carried out either before or after the step of reacting the glycan donor molecule with the acceptor molecule. As used herein, the above term "molecule having an alkyne structure" may be any molecule with an alkyne structure. Examples include chemotherapeutic agents, molecular target drugs, immunostimulatory agents, toxins, antimicrobial agents, antiviral agents, diagnostic agents, proteins, peptides, amino acids, nucleic acids, antigens, vitamins, and hormones. In the present invention, in the "step of reacting an azide group (N₃-) with a molecule having an alkyne structure", any reaction methods and conditions may be used as long as the reaction proceeds, and the step may be implemented according to, for example, known methods (such as WO 2019/065964 and WO 2020/050406). For example, the "step of reacting an azide group (N₃-) with a molecule having an alkyne structure" can be implemented by linking the glycan-remodeled antibody (3d) obtained in step D-2 above to the molecule having an alkyne structure by the SPAAC (strain-promoted azide-alkyne cycloaddition: J. Am. Chem. Soc. 2004, 126, 15046-15047) reaction.

An embodiment of the present invention further provides a method for producing an antibody-drug conjugate, comprising the above method for producing a glycan-remodeled antibody or a molecule containing an Fc region thereof. In the present invention, the antibody and the drug included in the "antibody-drug conjugate" are not limited as long as they exhibit intended effects, and any antibody and any drug may be used according to the purpose. In the present invention, examples of the method for producing an antibody-drug conjugate include, but are not limited to, a production method comprising step D-1, step D-2, and the SPAAC reaction described above.

As used herein, the term "antibody-drug conjugate" refers to a complex in which a drug is conjugated to an antibody via a linker. By using the method of the present invention, a drug can be introduced site-specifically to synthesize a homogeneous antibody-drug conjugate.

As used herein, the "antibody-drug conjugate" is represented by the following formula (XIII): 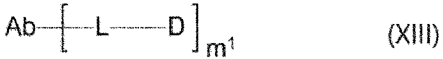 wherein m¹ is in the range of 1 to 10 and represents the number of drugs conjugated per antibody molecule in the antibody-drug conjugate; Ab represents the antibody or a functional fragment of the antibody; L represents the linker connecting Ab and D; and D represents the drug.

Examples of the antibody to be used in the present invention include a full antibody and a functional fragment of an antibody. The term "functional fragment of an antibody", which is also referred to as "antigen-binding fragment of an antibody", refers to a partial fragment of an antibody having antigen-binding activity, and includes Fab, F(ab')₂, Fv, scFv, a diabody, a linear antibody, and a multispecific antibody formed from an antibody fragment. In addition, the antigen-binding fragment of an antibody also includes Fab', a monovalent fragment of a variable region of an antibody obtained by treating F(ab')₂ under reducing conditions. However, the antigen-binding fragment of an antibody is not limited to these molecules as long as it has the ability to bind the antigen. In addition, these antigen-binding fragments include not only those obtained by treating the full-length molecule of the antibody protein with an appropriate enzyme, but also proteins produced in an appropriate host cell using a genetically engineered antibody gene.

In the present invention, the antibody may be derived from any species, but preferably, examples include human, rat, mouse, and rabbit. If the antibody is derived from a species other than human, the antibody is preferably chimerized or humanized using well-known techniques. In the present invention, the antibody may be a polyclonal antibody or a monoclonal antibody, but is preferably a monoclonal antibody. The monoclonal antibody includes monoclonal antibodies derived from non-human animals such as rat antibodies, mouse antibodies, and rabbit antibodies, chimeric antibodies, humanized antibodies, human antibodies, functional fragments thereof, or modified forms thereof.

The antibody to be used in the present invention is not limited as long as it exhibits the intended effects, and examples include anti-HER2 antibodies, anti-HER3 antibodies, anti-DLL3 antibodies, anti-FAP antibodies, anti-CDH11 antibodies, anti-CDH6 antibodies, anti-A33 antibodies, anti-CanAg antibodies, anti-CD19 antibodies, anti-CD20 antibodies, anti-CD22 antibodies, anti-CD30 antibodies, anti-CD33 antibodies, anti-CD56 antibodies, anti-CD70 antibodies, anti-CD98 antibodies, anti-TROP2 antibodies, anti-CEA antibodies, anti-Cripto antibodies, anti-EphA2 antibodies, anti-G250 antibodies, anti-MUC1 antibodies, anti-GPNMB antibodies, anti-Integrin antibodies, anti-PSMA antibodies, anti-Tenascin-C antibodies, anti-SLC44A4 antibodies, anti-Mesothelin antibodies, anti-ENPP3 antibodies, anti-CD47 antibodies, anti-EGFR antibodies, anti-GPR20 antibodies, and anti-DR5 antibodies.

The antibody to be used in producing the antibody-drug conjugate of the present invention may be obtained by immunizing an animal with an antigenic polypeptide and collecting and purifying the antibody produced in the body using a method commonly used in this field. The origin of the antigen is not limited to humans, and an animal may also be immunized with an antigen derived from an animal other than a human, such as a mouse or a rat. In this case, the cross-reactivity of the resulting antibody that binds to the heterologous antigen with the human antigen may be tested to screen for an antibody applicable to a human disease.

Alternatively, a monoclonal antibody may be obtained from a hybridoma established by fusing an antibody-producing cell that produces an antibody against the antigen with a myeloma cell in accordance with a known method (e.g., Kohler and Milstein, Nature (1975) 256, p. 495-497, Kennett, R. ed., Monoclonal Antibodies, p. 365-367, Plenum Press, N.Y. (1980)).

The antigen may be obtained through gene engineering to allow host cells to produce a gene encoding the antigen protein.

The humanized antibody to be used in producing the antibody-drug conjugate of the present invention may be obtained in accordance with a known method (e.g., Proc. Natl. Acad. Sci. U.S.A., 81, 6851-6855, (1984), Nature (1986) 321, p. 522-525, and WO 90/07861).

For example, anti-HER2 antibodies (US 5821337, WO 2004/008099, WO 2020/050406, etc.), anti-CD33 antibodies (WO 2014/057687, WO 2020/050406, etc.), anti-EphA2 antibodies (WO 2009/028639, WO 2020/050406, etc.), anti-CDH6 antibodies (WO 2018/212136, WO 2020/050406, etc.), anti-CD70 antibodies (WO 2004/073656, WO 2007/038637, etc.), anti-TROP2 antibodies (WO 2015/098099, etc.), and anti-EGFR antibodies (WO 1998/050433, WO 2002/092771, etc.) may be obtained by known means.

The drug to be used in the present invention is not limited as long as it exhibits the intended effects, and examples include pharmacologically active compounds, for example, chemotherapeutic agents, molecular target drugs, immunostimulatory agents, toxins, antimicrobial agents, antiviral agents, diagnostic agents, proteins, peptides, amino acids, nucleic acids, antigens, vitamins, and hormones.

The drug D is represented by, for example, the following formulas: 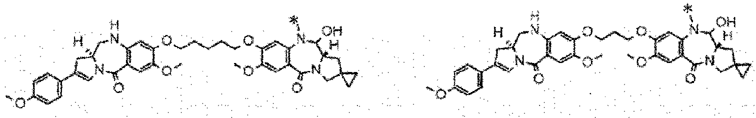 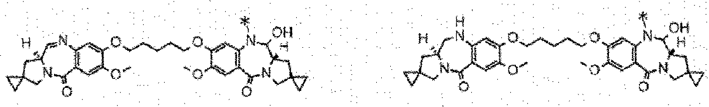 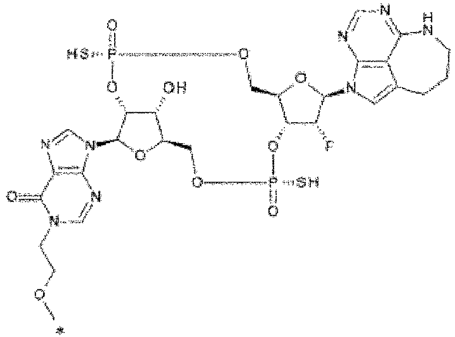 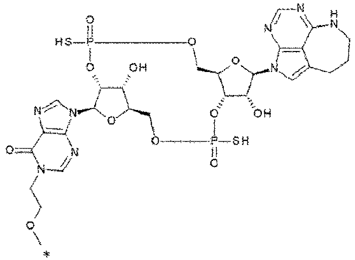 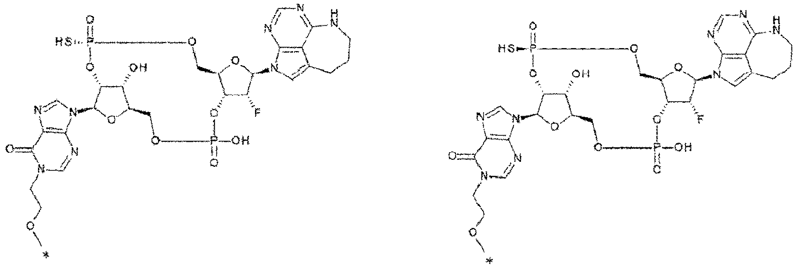 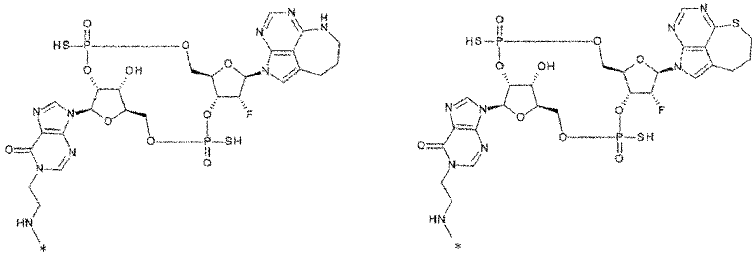 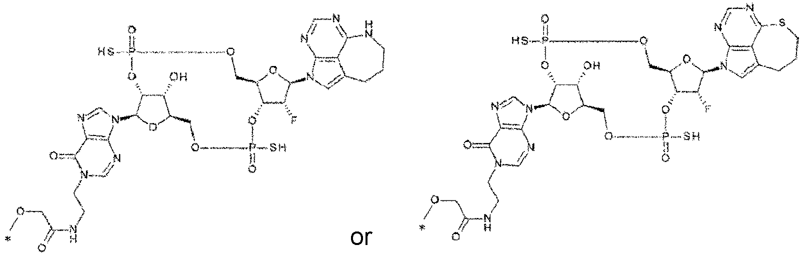 wherein each asterisk represents that the drug is linked to the linker L.

In the present invention, the linker L connecting the antibody Ab and the drug D is represented by the following formula:

-Lb-La-Lp-Lc-*

wherein each asterisk represents that the linker is linked to the drug D.

Lp represents a linker consisting of an amino acid sequence cleavable in a target cell, or is absent. Specific examples include -GGFG-, -GGPI-, -GGVA-, -GGFM-, -GGVCit-, -GGFCit-, -GGICit-, -GGPL-, -GGAQ-, or -GGPP-.

La represents any one selected from the following:

-C(=O)-(CH₂CH₂)n²-C(=O)-,

-C(=O)-(CH₂CH₂)n²-CH₂-C(=O)-,

-C(=O)-(CH₂CH₂)n²-C(=O)-NH-(CH₂CH₂)n³-C(=O)-,

-C(=O)-(CH₂CH₂)n²-C(=O)-NH-(CH₂CH₂)n³-CH₂-C(=O)

-C(=O)-(CH₂CH₂)n²-C(=O)-NH-(CH₂CH₂O)n³-CH₂-C(=O)-,

-(CH₂)n⁴-O-C(=O) -,

or

- (CH₂)n⁹-C(=O)-

wherein n² represents an integer of 1 to 3, n³ represents an integer of 1 to 5, n⁴ represents an integer of 0 to 2, and n⁹ represents an integer of 2 to 7.

Lb represents a spacer connecting La and a glycan or remodeled glycan of Ab. Specific examples include the following: 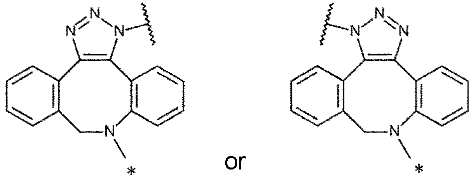 or 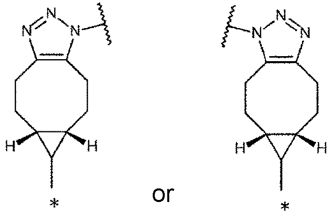 wherein each asterisk represents that Lb is linked to La, and each wavy line represents that Lb is linked to a glycan or remodeled glycan of Ab.

Lc represents -NH-CH₂- or -NH-B-CH2-O(C=O)-, or is absent, wherein B is a 1,4-phenyl group, a 2,5-pyridyl group, a 3,6-pyridyl group, a 2,5-pyrimidyl group, or a 2,5-thienyl group.

Hereinafter, the present invention will be described with reference to examples; however, the present invention is not limited to the examples. In the following, for example, "compound A-1" or "A-1" in the synthesis scheme represents that it corresponds to "compound represented by formula A-1", and the same applies to the subsequent compound numbers.

### Examples

In the following Examples, the room temperature indicated is from 15°C to 35°C. Silica gel chromatography was performed using Biotage Sfar HC D (20 µm, manufactured by Biotage), reverse-phase column chromatography was performed using Universal Column ODS Premium 30-µm L-size (manufactured by Yamazen Corporation) and Inject column ODS L-size (manufactured by Yamazen Corporation), and preparative HPLC was performed using Agilent Preparative HPLC System (manufactured by Agilent Technology). The preparative column used was XBridge Prep OBD (5 µm, C18, 130 Å, 250 × 30 mm; manufactured by Waters).

The following instruments were used to measure various spectral data. ¹H-NMR and ¹³C-NMR spectra were measured using JEOL ECZ500R and ECX400P. Mass spectra were measured using Shimadzu LCMS-2010 and LCMS-2020 (manufactured by Shimadzu Corporation), XEVO Q-Tof MS (Waters), and Q-Exactive (Thermo Fisher).

### <Synthesis of compound represented by formula A-4>

Compound represented by formula A-4 was synthesized according to the following synthesis scheme 1.

### [Synthesis Scheme 1]

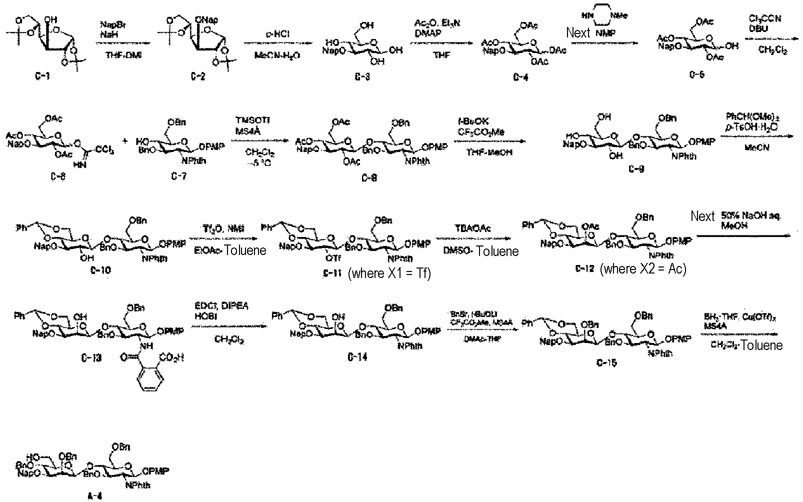

### Example 1

### 1,2:5,6-Bis-O-(1-methylethyliden)-3-O-(2-naphthylmethyl)-α-D-glucofuranose (Compound represented by formula C-2)

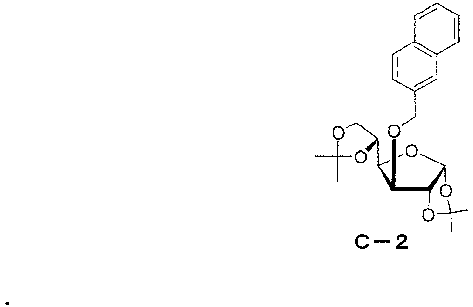

A tetrahydrofuran (900 mL) solution containing sodium hydride (55.32 g, 1.38 mol, content: 50-72%) was cooled to 0°C. Next, a tetrahydrofuran (1.05 L) solution containing 1,2:5,6-bis-O-(1-methylethyliden)-α-D-glucofuranose (compound represented by formula C-1) (300.00 g, 1.15 mol) was added dropwise over 1 hour. The temperature was then raised to 25°C and 1,3-dimethyl-2-imidazolidinone (150 mL) and 2-bromomethylnaphthalene (280.31 g, 1.27 mol) were added. After stirring at 25°C for 6 hours, the completion of the reaction was checked by HPLC. Ethylenediamine (anhydrous) (13.85 g, 230.52 mmol) was added, and the mixture was stirred for another 1 hour. The solution was cooled to 0°C and 10% aqueous citric acid solution (1.2 L) was added over 1 hour. The reaction solution was diluted with heptane (3 L) and separated into an organic layer and an aqueous layer. The organic layer was washed with water (900 mL) and concentrated under reduced pressure until the liquid volume reached 900 mL. Acetonitrile (3 L) was further added, and the mixture was concentrated again until the liquid volume reached 900 mL to prepare, as an acetonitrile solution, crude 1,2:5,6-bis-O-(1-methylethyliden)-3 -O-(2-naphthylmethyl)-α-D-glucofuranose (compound represented by formula C-2). This compound was used as it was in the next step.

### Example 2

### 3-O-(2-Naphthylmethyl)-D-glucopyranose (Compound represented by formula C-3)

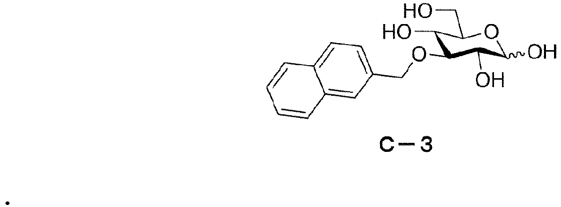

To a solution (900 mL) containing the crude compound represented by formula C-2 obtained in Example 1 were added acetonitrile (1.5 L), water (600 mL), and concentrated hydrochloric acid (17.51 g, 172.89 mmol), and the mixture was stirred at 55°C for 18.5 hours. After the completion of the reaction was checked by HPLC, the reaction solution was cooled to 0°C and the pH in the system was adjusted to 6.25 with a 4 N aqueous sodium hydroxide solution (43.22 mL). The reaction solution was diluted with heptane (900 mL) and separated into an acetonitrile layer and a heptane layer. Ethyl acetate (2.4 L) and water (600 mL) were added to the acetonitrile layer, and the mixture was liquid-separated to give an organic layer A and an aqueous layer. A mixed solution of ethyl acetate (1.5 L) and tetrahydrofuran (1.5 L) was added to the aqueous layer again, and the mixture was liquid-separated to give an organic layer B and an aqueous layer. The organic layers A and B were mixed, washed with saturated brine (600 mL), and concentrated under reduced pressure until the liquid volume reached 1.5 L (crystals were found to precipitate during the concentration step). Ethyl acetate (4.5 L) was further added, and the mixture was concentrated again until the liquid volume reached 3 L. To this suspension were added ethyl acetate (1.5 L) and cyclopentyl methyl ether (1.5 L), and the mixture was stirred at 55°C for 1 hour. Heptane (3 L) was added dropwise over 1.5 hours. After stirring for 1 hour, the mixture was cooled to 0°C. After that, the precipitated crystals were filtered and washed with an ethyl acetate (1.2 L)/heptane (600 mL) mixed solution cooled to 0°C. The resulting crystals were dried at 40°C under reduced pressure to afford 3-O-(2-naphthylmethyl)-D-glucopyranose (compound represented by formula C-3) (356.95 g, yield 96.7%).
¹H-NMR (500 MHz, DMSO-d₆) δ 7.85-7.90 (m, 4H), 7.59 (dd, J = 8.0, 1.5 Hz, 1H), 7.46-7.51 (m, 2H), 6.69 (d, J = 6.0 Hz, 1H), 5.12 (dd, J = 5.0, 3.0 Hz, 2H), 4.94-5.00 (m, 2H), 4.53 (t, J = 6.0 Hz, 1H), 4.35 (dd, J = 8.0, 6.5 Hz, 1H), 3.70 (ddd, J = 11.5, 5.0, 2.0 Hz, 1H), 3.45-3.50 (m, 1H), 3.25-3.31 (m, 2H), 3.11-3.16 (m, 2H).

¹³C-NMR (125 MHz, DMSO-d₆) δ 137.3, 132.8, 132.3, 127.6, 127.5, 127.4, 126.1, 126.0, 125.6, 125.5, 96.9, 85.4, 76.7, 74.8, 73.7, 69.9, 61.1.

HRMS (ESI⁻) [M-H]⁻ calcd for C₁₇H₁₉O₆:319.1187; found 319.1175.

### Example 3

### 2,4,6-Tri-O-acetyl-3-O-(2-naphthylmethyl)-D-glucopyranose (Compound represented by formula C-5)

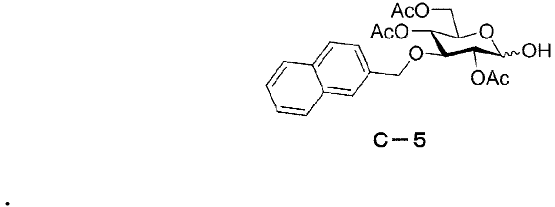

To a tetrahydrofuran (675 mL) solution containing 3-O-(2-naphthylmethyl)-D-glucopyranose (compound represented by formula C-3) (150.00 g, 468.25 mmol) were added triethylamine (236.92 g, 2.34 mol) and 4-dimethylaminopyridine (0.29 g, 2.34 mmol). After the mixture was cooled to 0°C, acetic anhydride (195.99 g, 1.92 mol) was added dropwise over 30 minutes. The temperature was then raised to 25°C, and after 3 hours of stirring, the completion of the reaction was checked by HPLC. The reaction solution was cooled to 10°C and 1-methylpiperazine (60.97 g, 608.73 mmol) was added. After stirring at 35°C for 18 hours, the completion of the reaction was checked by HPLC. The mixture was then cooled to 0°C. The pH was adjusted to 6.36 with 6 N aqueous hydrochloric acid (480 mL), and the solution was diluted with heptane (375 mL) and separated into an organic layer and an aqueous layer. The organic layer was washed with a saturated sodium bicarbonate aqueous solution (450 mL) and then water (450 mL) and concentrated under reduced pressure until the liquid volume reached 450 mL. Ethyl acetate (2.25 L) was added, the mixture was concentrated again until the liquid volume reached 450 mL, and the same operation was repeated one more time. Dichloromethane (2.25 L) was added to this solution, the mixture was concentrated until the liquid volume reached 450 mL, and the same operation was repeated one more time to prepare, as a dichloromethane solution, crude 2,4,6-tri-O-acetyl-3-O-(2-naphthylmethyl)-D-glucopyranose (compound represented by formula C-5). This compound was used as it was in the next step.

Note that the step from the compound represented by formula C-3 to the compound represented by formula C-5 was carried out in a one-pot process.

### Example 4

### 2,4,6-Tri-O-acetyl-3-O-[(naphthalen-2-yl)methyl]-1-O-(2,2,2-trichloroethanimidoyl)-D-glycero-hexopyranose (Compound represented by formula C-6)

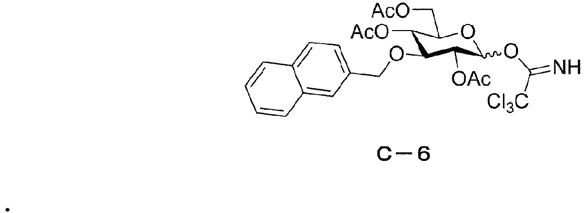

To a solution (450 ml) containing the crude 2,4,6-tri-O-acetyl-3-O-(2-naphthylmethyl)-D-glucopyranose (compound represented by formula C-5) obtained in Example 3 were added dichloromethane (450 mL) and trichloroacetonitrile (338.03 g, 2.34 mol), and the mixture was cooled to 0°C. Next, 1,8-diazabicyclo[5.4.0]-7-undecene (5.70 g, 37.46 mmol) was added dropwise. After stirring at 0°C for 14.5 hours, the completion of the reaction was checked by HPLC. Acetic acid (2.25 g, 37.46 mmol) was then added. Silica gel 60N (particle size: 40 to 50 µm; 150 g; manufactured by KANTO CHEMICAL CO., INC.) was added to the solution, and the mixture was stirred for 1.5 hours and then filtered. The silica gel was washed with dichloromethane (1.5 L) and the filtrate was concentrated under reduced pressure until the liquid volume reached 450 mL. Dichloromethane (1.5 L) was further added, and the solution was concentrated until the liquid volume reached 450 mL to prepare, as a dichloromethane solution, 2,4,6-tri-O-acetyl-3-O-[(naphthalen-2-yl)methyl]-1-O-(2,2,2-trichloroethanimidoyl)-D-glycero-hexopyranose (compound represented by formula C-6). This compound was used as it was in the next step.

### Example 5

### 4-Methoxyphenyl 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-β-D-glucopyranosyl}-β-D-glucopyranoside (Compound represented by formula C-8)

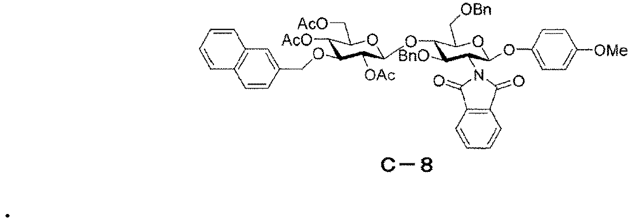

To a solution (450 mL) containing the 2,4,6-tri-O-acetyl-3-O-[(naphthalen-2-yl)methyl]-1-O-(2,2,2-trichloroethanimidoyl)-D-glycero-hexopyranose (compound represented by formula C-6) obtained in Example 4 were added 4-methoxyphenyl 3,6-di-O-benzyl-2-deoxy-2-(1,3-dioxo-1,3-dihydro-2H-isoindol-2-yl)-β-D-glucopyranoside (compound represented by C-7) (276.66 g, 468.25 mmol), dichloromethane (4.2 L), and Molecular Sieves 4A powder (10µm or less, 83.00 g), and the mixture was cooled to-5°C. Trimethylsilyl trifluoromethanesulfonate (10.41 g, 46.83 mmol) was added dropwise to this suspension over 20 minutes, and the mixture was then stirred for 3 hours. After the completion of the reaction was checked by HPLC, triethylamine (23.69 g, 234.13 mmol) was added. After the suspension was filtered, the filtrate was washed with ethyl acetate (2.8 L) and concentrated under reduced pressure until the liquid volume reached 1.4 L. Ethyl acetate (4.2 L) was further added, the mixture was concentrated until the liquid volume reached 1.4 L, and the same operation was repeated one more time. To this solution was added ethyl acetate (2.8 L), and the mixture was washed with a saturated sodium bicarbonate aqueous solution (830 mL) and water (830 mL). The resulting organic layer was then concentrated under reduced pressure to a liquid volume of 830 mL. Next, 2-propanol (4.2 L) was added, and the mixture was concentrated to a liquid volume of 1.4 L. The suspension was then warmed to 65°C. Ethyl acetate (830 mL) was added. After the mixture was stirred at 65°C for 2 hours, 2-propanol (5.53 L) was added dropwise over 2 hours. After this suspension was cooled to 0°C, the crystals were filtered and washed with 2-propanol (1.4 L) cooled to 0°C. The resulting crystals were dried under reduced pressure at 40°C to give crude 4-methoxyphenyl 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-β-D-glucopyranosyl}-β-D-glucopyranoside (compound represented by formula C-8) (355.34 g, yield 90.5%, in terms of compound represented by formula C-3).

The resulting crude compound represented by formula C-8 (350.00 g) was admixed with methyl isobutyl ketone (2.1 L). The compound was dissolved at 50°C, and ethyl cyclohexane (1.4 L) was added dropwise over 1 hour. 4-Methoxyphenyl 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-β-D-glucopyranosyl}-β-D-glucopyranoside (compound represented by formula C-8) (70.00 mg) were added, and the mixture was stirred for 1 hour. After the crystals were found to precipitate, ethylcyclohexane (4.9 L) was added dropwise over 2 hours. The suspension was cooled to room temperature and stirred for 14.5 hours. The precipitated crystals were then filtered, and washed with a mixed solution of methyl isobutyl ketone (350 mL) and ethyl cyclohexane (1.4 L). The resulting crystals were dried under reduced pressure at 40°C to give 4-methoxyphenyl 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-β-D-glucopyranosyl}-β-D-glucopyranoside (compound represented by formula C-8) (331.74 g, yield 94.8%).
¹H-NMR (500 MHz, CDCl₃) δ 7.81-7.84 (m, 4H), 7.69 (br, 1H), 7.65 (br, 3H), 7.46-7.51 (m, 2H), 7.28-7.36 (m, 6H), 7.00 (dd, J = 7.0, 1.5 Hz, 2H), 6.77-6.84 (m, 5H), 6.66-6.69 (m, 2H), 5.59, (d, J = 9.0 Hz, 1H), 5.09-5.15 (m, 2H), 4.82 (d, J = 12.5 Hz, 1H), 4.77 (d, J = 12.0 Hz, 1H), 4.71-4.77 (m, 2H), 4.60 (d, J = 8.0 Hz, 1H), 4.50 (d, J = 12.5 Hz, 1H), 4.45 (d, J = 13.0 Hz, 1H), 4.36 (dd, J = 11.0, 8.5 Hz, 1H), 4.28 (dd, J = 11.0, 8.5 Hz, 1H), 4.20 (dd, J = 12.5, 5.0 Hz, 1H), 4.10 (dd, J = 10.0, 8.5 Hz, 1H), 3.99 (dd, J = 12.0, 2.0 Hz, 1H), 3.80 (br, 2H), 3.69 (s, 3H), 3.58-3.62 (m, 2H), 3.44 (ddd, J = 10.0, 4.5, 2.5 Hz, 1H), 1.98 (s, 3H), 1.938 (s, 3H), 1.937 (s, 3H).
¹³C-NMR (125 MHz, CDCl₃) δ 171.0, 169.5, 169.1, 155.6, 151.0, 138.7, 138.2, 135.5, 133.9, 133.4, 133.2, 128.7, 128.4, 128.23, 128.20, 128.06, 128.05, 127.9, 127.2, 126.5, 126.2, 125.7, 123.5, 118.9, 114.5, 100.7, 97.8, 80.6, 78.5, 76.8, 75.2, 74.8, 74.1, 73.8, 73.1, 72.1, 69.9, 67.8, 62.2, 55.78, 55.75, 21.1, 20.94, 20.85.

HRMS (ESI⁺) [M+H]⁺ calcd for C₅₈H₅₈NO₁₆: 1024.3750; found 1024.3706.

The spectrum was found to correspond to the following literature:
Literature 1) Org. Biomol. Chem., 2018, 16, 4720-4727.

### Example 6

### 4-Methoxyphenyl 3,6-di-O-benzyl-2-deoxy-2-(1,3-dioxo-1,3-dihydro-2H-isoindol-2-yl)-4-O-{3-O-[(naphthalen-2-yl)methyl]-β-D-glucopyranosyl}-β-D-glucopyranoside (Compound represented by formula C-9)

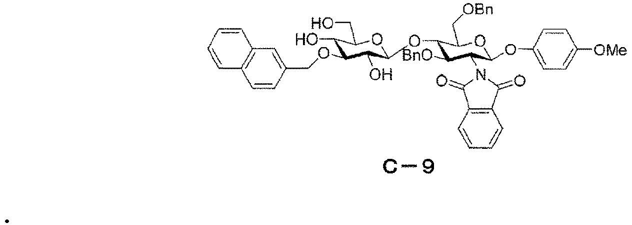

To a tetrahydrofuran (150 mL) solution containing 4-methoxyphenyl 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-β-D-glucopyranosyl}-β-D-glucopyranoside (compound represented by formula C-8) (30.00 g, 29.29 mmol) were added methanol (90 mL) and methyl trifluoroacetate (3.75 g, 29.29 mmol). The mixture was stirred at 25°C for 10 min, and potassium tert-butoxide (1 mol/L tetrahydrofuran solution) (14.7 mL, 14.65 mmol) was then added. Next, the temperature was raised to 55°C. After stirring for 2 hours, the completion of the reaction was checked by HPLC. The reaction solution was cooled to 25°C and acetic acid (1.76 g, 29.29 mmol) and ethyl acetate (300 mL) were added in this order. This solution was washed twice with 1% sodium chloride aqueous solution (300 mL) and concentrated under reduced pressure to a liquid volume of 90 mL. Ethyl acetate (450 mL) was added, and the mixture was concentrated again until the liquid volume reached 90 mL. Acetonitrile (450 mL) was further added, and the mixture was concentrated until the liquid volume reached 90 mL to prepare, as an acetonitrile solution, 4-methoxyphenyl 3,6-di-O-benzyl-2-deoxy-2-(1,3-dioxo-1,3-dihydro-2H-isoindol-2-yl)-4-O-{3-O-[(naphthalen-2-yl)methyl]-β-D-glucopyranosyl}-β-D-glucopyranoside (compound represented by formula C-9). This compound was used as it was in the next step.

### Example 7

### 4-Methoxyphenyl 3,6-di-O-benzyl-4-O-{4,6-O-benzyliden-3-O-[(naphthalen-2-yl)methyl]-β-D-glucopyranosyl}-2-deoxy-2-(1,3-dioxo-1,3-dihydro-2H-isoindol-2-yl)-β-D-glucopyranoside (Compound represented by formula C-10)

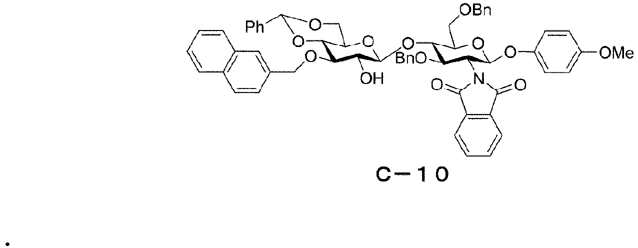

To a solution (90 mL) containing the 4-methoxyphenyl 3,6-di-O-benzyl-2-deoxy-2-(1,3-dioxo-1,3-dihydro-2H-isoindol-2-yl)-4-O-{3-O-[(naphthalen-2-yl)methyl]-β-D-glucopyranosyl}-β-D-glucopyranoside (compound represented by formula C-9) were added acetonitrile (210 mL), benzaldehyde dimethyl acetal (5.13 g, 33.69 mmol), and p-toluenesulfonic acid monohydrate (0.17 g, 0.88 mmol), and the mixture was stirred at 25°C for 30 minutes. Toluene (600 mL) was added to this solution, and the solution was concentrated until the liquid volume reached 300 mL. At this time point, the completion of the reaction was checked by HPLC. Further, 1-methylimidazole (12.03 g, 146.47 mmol) was added, and the mixture was concentrated until the liquid volume reached 90 mL to prepare, as a 1-methylimidazole-containing toluene solution, 4-methoxyphenyl 3,6-di-O-benzyl-4-O-{4,6-O-benzyliden-3-O-[(naphthalen-2-yl)methyl]-β-D-glucopyranosyl}-2-deoxy-2-(1,3-dioxo-1,3-dihydro-2H-isoindol-2-yl)-β-D-glucopyranoside (compound represented by formula C-10). This compound was used as it was in the next step.

### Example 8

### 4-Methoxyphenyl 3,6-di-O-benzyl-4-O-{4,6-O-benzyliden-3-O-[(naphthalen-2-yl)methyl]-2-O-(trifluoromethanesulfonyl)-β-D-glucopyranosyl}-2-deoxy-2-(1,3-dioxo-1,3-dihydro-2H-isoindol-2-yl)-β-D-glucopyranoside (Compound represented by formula C-11, wherein X₁ is a Tf group)

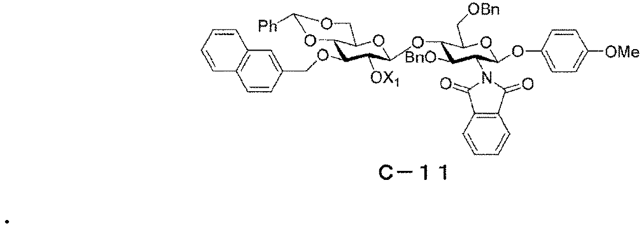

To a (90 mL, 1-methylimidazole-containing) solution containing the 4-methoxyphenyl 3,6-di-O-benzyl-4-O-{4,6-O-benzyliden-3-O-[(naphthalen-2-yl)methyl]-β-D-glucopyranosyl}-2-deoxy-2-(1,3-dioxo-1,3-dihydro-2H-isoindol-2-yl)-β-D-glucopyranoside (compound represented by formula C-10) obtained in Example 7 was added ethyl acetate (210 mL), and the mixture was cooled to 0°C. Ttrifluoromethanesulfonic anhydride (16.53 g, 58.59 mmol) was added dropwise to this solution over 1 hour, followed by stirring for 30 minutes. After the completion of the reaction was checked by HPLC, water (300 mL) was added and organic and aqueous layers were separated. The organic layer was washed twice with water (300 mL) and once with a saturated sodium chloride aqueous solution (150 mL) and then concentrated under reduced pressure to a liquid volume of 90 mL. Ethyl acetate (300 mL) was added, and the mixture was concentrated again until the liquid volume reached 90 mL to prepare, as an ethyl acetate solution, 4-methoxyphenyl 3,6-di-O-benzyl-4-O-{4,6-O-benzyliden-3-O-[(naphthalen-2-yl)methyl]-2-O-(trifluoromethanesulfonyl)-β-D-glucopyranosyl}-2-deoxy-2-(1,3-dioxo-1,3-dihydro-2H-isoindol-2-yl)-β-D-glucopyranoside (Compound represented by formula C-11, wherein X₁ is a Tf group). This compound was used as it was in the next step.

### Example 9

### 4-Methoxyphenyl 3,6-di-O-benzyl-4-O-{4,6-O-benzyliden-3-O-[(naphthalen-2-yl)methyl]-β-D-mannopyranosyl}-2-(2-carboxybenzamido)-2-deoxy-β-D-glucopyranoside (Compound represented by formula C-13)

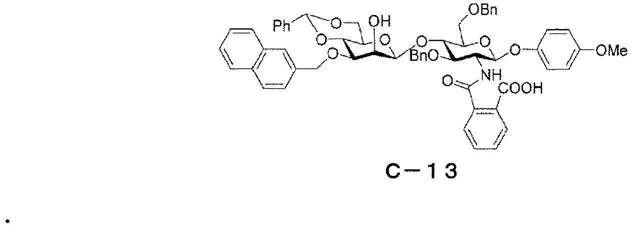

To a solution (90 mL) containing 4-methoxyphenyl 3,6-di-O-benzyl-4-O-{4,6-O-benzyliden-3-O-[(naphthalen-2-yl)methyl]-2-O-(trifluoromethanesulfonyl)-β-D-glucopyranosyl}-2-deoxy-2-(1,3-dioxo-1,3-dihydro-2H-isoindol-2-yl)-β-D-glucopyranoside (compound represented by formula C-11 (wherein X₁ is a Tf group)) obtained in Example 8 were added dimethyl sulfoxide (150 mL) and tetrabutylammonium acetate (17.67 g, 58.59 mmol). Next, the temperature was raised to 30°C. After 17 hours of stirring, the completion of the reaction was checked by HPLC. Toluene (150 mL) was added to this reaction solution, and the mixture was concentrated under reduced pressure until the liquid volume reached 165 mL. Methanol (45 mL) and 50% aqueous sodium hydroxide solution (3.52 g, 87.88 mmol) were added, and the mixture was stirred at 25°C for 1.5 hours. After the completion of the reaction was checked by HPLC, ethyl acetate (450 mL) and water (300 mL) were added for liquid separation. Water (300 mL) was added to the resulting organic layer. The mixture was cooled to 0°C, and adjusted to pH 2.73 with 6 N hydrochloric acid under strong stirring. Tetrahydrofuran (300 mL) was added to the separated organic layer, and the mixture was concentrated under reduced pressure until the liquid volume reached 150 mL. Tetrahydrofuran (300 mL) was added, the mixture was concentrated again until the liquid volume reached 90 mL, and the internal temperature was then adjusted to 45°C. Tetrahydrofuran (60 mL) was added, and the mixture was then cooled to 25°C. Next, 2-propanol (150 mL) and water (15 mL) were added. 4-Methoxyphenyl 3,6-di-O-benzyl-4-O-{4,6-O-benzyliden-3-O-[(naphthalen-2-yl)methyl]-β-D-mannopyranosyl}-2-(2-carboxybenzamido)-2-deoxy-β-D-glucopyranoside (compound represented by formula C-13) (30 mg) were then added. The mixture was stirred at 25°C for 14 hours, and after the crystals were found to precipitate, 2-propanol (210 mL) was added dropwise over 1 hour. The mixture was then cooled to 0°C. After stirring for 2 hours, the precipitated crystals were filtered and washed with 2-propanol (150 mL) cooled to 0°C. The resulting crystals were dried under reduced pressure at 40°C to give 4-methoxyphenyl 3,6-di-O-benzyl-4-O-{4,6-O-benzyliden-3-O-[(naphthalen-2-yl)methyl]-β-D-mannopyranosyl}-2-(2-carboxybenzamido)-2-deoxy-β-D-glucopyranoside (compound represented by formula C-13) (27.74 g, yield 94.3%, in terms of compound represented by formula C-8).
*The step from the compound represented by formula C-12 to the compound represented by formula C-13 was carried out in a one-pot process.
*For the seed crystals, a part of the reaction solution was divided into small portions and concentrated to precipitate solids for use.
*This reaction proceeds well with tetrabutylammonium acetate manufactured by TOKYO CHEMICAL INDUSTRY CO., LTD. (product code: T2694, purity: >90.0%) or Sigma-Aldrich (product code: 86849, purity: >90%). Use of tetrabutylammonium acetate from other manufacturers slowed down the reaction in the past. In this case, cesium acetate is preferably used as an alternative (see details below).
*The conversion of the compound represented by formula C-11 to the compound represented by formula C-12 was also feasible under the conditions using cesium acetate (3 equivalents) and dimethyl sulfoxide at 50°C for 24 hours. Subsequently, substantially the same reaction (compound represented by formula C-12 → compound represented by formula C-13) was followed by post-treatment to prepare the compound represented by formula C-13.
¹H-NMR (500 MHz, CDCl₃) δ 8.02 (dd, J = 6.0, 2.0 Hz, 1H), 7.69-7.83 (m, 4H), 7.38-7.49 (m, 12H), 7.32-7.34 (m, 2H), 7.16-7.29 (m, 8H), 6.97 (ddd, J = 9.0, 4.0, 2.5 Hz, 2H), 6.76 (ddd, J = 9.5, 3.5, 2.5 Hz, 2H), 5.51 (s, 1H), 5.40 (d, J = 6.0 Hz, 1H), 4.83-4.91 (m, 3H), 4.76 (d, J = 11.5 Hz, 1H), 4.55 (d, J = 0.5 Hz, 1H), 4.49 (d, J = 12.0 Hz, 1H), 4.36 (d, J = 12.0 Hz, 1H), 4.26-4.30 (m, 1H), 4.16 (t, J = 6.5 Hz, 1H), 4.05-4.09 (m, 2H), 3.99 (dd, J = 3.0, 0.5 Hz, 1H), 3.93 (t, J = 9.5 Hz, 1H), 3.80-3.86 (m, 2H), 3.71 (s, 3H), 3.65-3.69 (m, 1H), 3.56 (t, J = 10.0 Hz, 1H), 3.51 (dd, J = 10.0, 3.5 Hz, 1H), 3.13 (td, J = 9.5, 5.0 Hz, 1H).
¹³C-NMR (125 MHz, CDCl₃) δ 170.9, 168.4, 155.3, 151.4, 138.7, 138.0, 137.6, 136.2, 135.4, 133.4, 133.3., 132.2, 132.1, 130.7, 130.3, 129.2, 128.6, 128.49, 128.45, 128.11, 128.07, 128.0, 127.89, 127.87, 127.8, 126.8, 126.4, 126.3, 126.2, 125.8, 118.6, 114.7, 101.7, 100.4, 99.1, 78.3, 76.7, 76.4, 75.1, 73.7, 73.3, 72.5, 69.9, 69.4, 68.5, 67.0, 55.8, 54.4.
HRMS (ESI⁺) [M+H]⁺ calcd for C₅₉H₅₈NO₁₄: 1004.3852; found 1004.3873.

### Example 10

### 4-Methoxyphenyl 3,6-di-O-benzyl-4-O-{4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (compound represented by formula C-14).

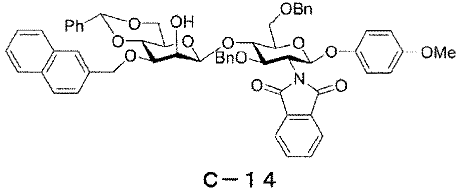

To a dichloromethane (30 mL) solution containing 4-methoxyphenyl 3,6-di-O-benzyl-4-O-{4,6-O-benzyliden-3-O-[(naphthalen-2-yl)methyl]-β-D-mannopyranosyl}-2-(2-carboxybenzamido)-2-deoxy-β-D-glucopyranoside (compound represented by formula C-13) (6.00 g, 5.98 mmol) were added 1-hydroxybenzotriazole monohydrate (0.18 g, 1.20 mmol), N,N-diisopropylethylamine (0.85 g, 6.57 mmol), and 1-(3-dimethylaminopropyl)-3-ethylcarbodiimide hydrochloride (1.26 g, 6.57 mmol). The temperature was raised to 40°C, and the mixture was stirred for 31 hours. After the completion of the reaction was checked by HPLC, the reaction solution was cooled to 0°C. Ethyl acetate (90 mL) and water (60 mL) were then added, and under strong stirring, 6 N hydrochloric acid was added until pH 7 was reached. An organic layer and an aqueous layer were separated. The resulting organic layer was washed with water (60 mL) and then saturated brine (30 mL). The solution was concentrated under reduced pressure until the liquid volume reached 12 mL. Tetrahydrofuran (60 mL) was added, and the mixture was concentrated again until the liquid volume reached 9 mL to prepare a tetrahydrofuran solution containing crude 4-methoxyphenyl 3,6-di-O-benzyl-4-O-{4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (compound represented by formula C-14). This compound was used as it was in the next step.

### Example 11

### 4-Methoxyphenyl 3,6-di-O-benzyl-4-O-{2-O-benzyl-4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (Compound represented by formula C-15)

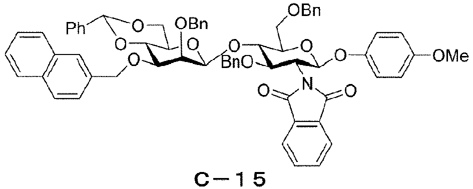

To a solution containing the crude 4-methoxyphenyl 3,6-di-O-benzyl-4-O-{4,6-O-benzyliden-3-O-[(naphthalen-2-yl)methyl]-β-D-mannnopyranosyl}-2-deoxy-2-(1,3-dioxo-1,3-dihydro-2H-isoindol-2-yl)-β-D-glucopyranoside (compound represented by formula C-14) were added N,N-dimethylacetamide (60 mL), benzyl bromide (1.53 g, 8.96 mmol), methyl trifluoroacetate (0.15 g, 1.20 mmol), and Molecular Sieves 4A (1.8 g). The mixture was cooled to 0°C. A lithium tert-butoxide-containing tetrahydrofuran solution (note that this solution was used and prepared such that a mixed solution of 2-methyl-2-propanol (0.66 g, 8.96 mmol) and tetrahydrofuran (2.4 mL) was cooled to 0°C, and a hexane solution containing normal butyl lithium (1.55 mol/L) (5.78 mL, 8.96 mmol) was added, and the mixture was stirred for 30 minutes) was added, and the mixture was stirred for 3 hours. After the completion of the reaction was checked by HPLC, ethylenediamine (anhydrous) (0.18 g, 2.99 mmol) was added. The mixture was stirred for another 1 hour. Acetic acid (0.72 g. 11.95 mmol) was added to this solution, and the mixture was filtered. After the Molecular Sieves 4A was washed with ethyl acetate (90 mL), water (60 mL) was added for liquid separation. The organic layer was washed twice with water (60 mL) and then concentrated under reduced pressure until the liquid volume reached 12 mL. Toluene (30 mL) was added, and the mixture was concentrated again until the liquid volume reached 12 mL. Toluene (18 mL) and silica gel 60N (spherical; particle size: 40 to 50 µm; manufactured by KANTO CHEMICAL CO., INC.) (9 g) were then added, and the mixture was stirred at 25°C for 30 minutes. The suspension was filtered, and the silica gel was washed with a mixed solution of toluene (191 mL) and ethyl acetate (19 mL). The filtrate was concentrated under reduced pressure until the liquid volume reached 9 mL to prepare a toluene solution containing crude 4-methoxyphenyl 3,6-di-O-benzyl-4-O-{2-O-benzyl-4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (compound represented by formula C-15).

### Example 12

### 4-Methoxyphenyl 3,6-di-O-benzyl-2-deoxy-4-O-{2,4-di-O-benzyl-3-O-[(naphthalen-2-yl)methyl]-β-D-mannopyranosyl}-2-(1,3-dioxo-1,3-dihydro-2H-isoindol-2-yl)-β-D-glucopyranoside (Compound represented by formula A-4)

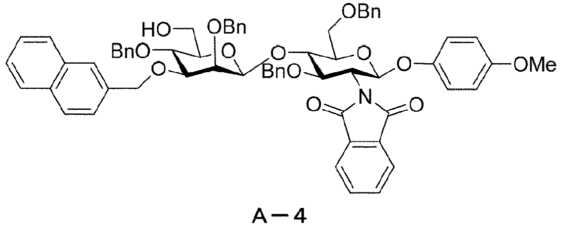

To a solution containing the crude 4-methoxyphenyl 3,6-di-O-benzyl-4-O-{2-O-benzyl-4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (compound represented by formula C-15) obtained (5.50 g (5.48 mmol) equivalent in terms of the compound represented by formula C-14 was used) were added dichloromethane (16.5 mL) and Molecular Sieves 4A (550 mg), and the mixture was cooled to 0°C. A boranetetrahydrofuran complex (0.91 mol/L tetrahydrofuran solution) (18.06 mL, 16.43 mmol) and copper(II) trifluoroacetate (0.59 g, 1.64 mmol) were added, and the mixture was then stirred for 3 hours. After the completion of the reaction was checked by HPLC, methanol (5.5 mL) was added and the mixture was stirred for another 30 minutes. The solution is filtered. After the Molecular Sieves 4A was washed with ethyl acetate (110 mL), 0.5 N hydrochloric acid (55 mL) was added, and the mixture was then stirred for 30 minutes. An organic layer and an aqueous layer were separated. The organic layer was washed with 0.5 N hydrochloric acid (55 mL) and saturated brine (27.5 mL), and the resulting solution was then concentrated to dryness under reduced pressure. The resulting material was purified by silica gel column chromatography (silica gel: 300 g; hexane:ethyl acetate = 55:45 → 30:70) to give 4-methoxyphenyl 3,6-di-O-benzyl-2-deoxy-4-O-{2,4-di-O-benzyl-3-O-[(naphthalen-2-yl)methyl]-β-D-mannopyranosyl}-2-(1,3-dioxo-1,3-dihydro-2H-isoindol-2-yl)-β-D-glucopyranoside (compound represented by formula A-4) (4.94 g, yield 83.6%, HPLC area: 98.54%).
¹H-NMR (500 MHz, CDCl₃) δ 7.67-7.84 (m, 8H), 7.44-7.49 (m, 4H), 7.40 (dd, J = 8.0, 1.5 Hz, 1H), 7.21-7.33 (m, 13H), 6.87-6.93 (m, 5H), 6.82 (ddd, J = 9.5, 4.0, 2.5 Hz, 2H), 6.70 (ddd, J = 9.0, 4.0, 2.0 Hz, 2H), 5.64 (d, J = 8.5 Hz, 1H), 4.94 (d, J = 12.5 Hz, 1H), 4.91 (d, J = 10.0 Hz, 1H), 4.90 (s, 2H), 4.66 (s, 2H), 4.60 (d, J = 11.0 Hz, 1H), 4.59 (d, J = 12.0 Hz, 1H), 4.55 (s, 1H), 4.40-4.46 (m, 3H), 4.33 (dd, J = 11.0, 9.0 Hz, 1H), 4.06 (dd, J = 9.5, 8.5 Hz, 1H), 3.80-3.85 (m, 2H), 3.70-3.76 (m, 2H), 3.71 (s, 3H), 3.61-3.68 (m, 2H), 3.45-3.48 (m, 1H), 3.44 (dd, J = 9.5, 3.0 Hz, 1H), 3.23 (ddd, J = 9.5, 5.5, 2.5 Hz, 1H), 1.97 (br-t, 1H).
¹³C-NMR (125 MHz, CDCl₃) δ 155.6, 151.1, 138.9, 138.64, 138.56, 138.0, 135.9, 134.0, 133.5, 133.2, 131.8, 128.7, 128.6, 128.4, 128.3, 128.20, 128.19, 128.15, 128.1, 120.04, 128.01, 127.9, 127.7, 127.6, 127.3, 126.4, 126.3, 126.1, 125.8, 123.6, 119.0, 114.6, 101.2, 98.0, 82.6, 79.0, 77.2, 75.9, 75.4, 75.3, 75.2, 75.1, 74.8, 74.7, 73.8, 72.1, 68.7, 62.6, 55.82, 55.78, 34.4, 30.5.
HRMS (ESI⁻) [M+HCO₂]⁻ calcd for C₆₇H₆₄NO₁₅: 1122.4281; found 1122.4285.

### <Synthesis of compound represented by formula A-12>

Compound represented by formula A-12 was synthesized according to the following synthesis scheme 2.

### [Synthesis Scheme 2]

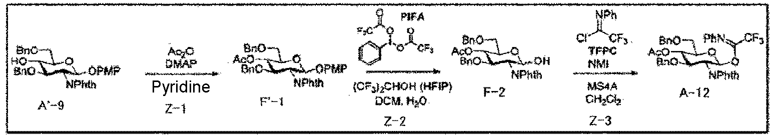

### Example 13

### 4-Methoxyphenyl 4-O-acetyl-3,6-di-O-benzyl-2-deoxy-2-(1,3-dioxo-1,3-dihydro-2H-isoindol-2-yl)-β-D-glucopyranoside (Compound represented by formula F'-1)

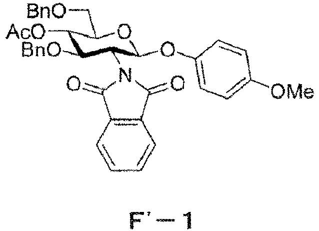

### (Substep Z-1)

A solution of 4-methoxyphenyl 3,6-di-O-benzyl-2-deoxy-2-(1,3-dioxo-1,3-dihydro-2H-isoindol-2-yl)-β-D-glucopyranoside (compound represented by formula A'-9) (50.0 g, 83.94 mmol) in ethyl acetate (200 mL) was admixed with triethylamine (11.04 g, 109.12 mmol), dimethylaminopyridine (0.31 g, 2.52 mmol), and acetic anhydride (11.10 g, 109.12 mmol), and the mixture was stirred at 20°C for 4 hours. After the completion of the reaction was checked by HPLC, ethanol (500 mL) and water (150 mL) were added dropwise. The slurry solution was stirred for 1 hour, and the precipitated crystals were filtered. The filtered crystals were washed with a mixture of ethanol and water (150/50 mL) and dried under reduced pressure at 40°C to give 4-methoxyphenyl 4-O-acetyl-3,6-di-O-benzyl-2-deoxy-2-(1,3-dioxo-1,3-dihydro-2H-isoindol-2-yl)-β-D-glucopyranoside (compound represented by formula F'-1) (49.0 g, yield 91%) as white crystals.

¹H-NMR (500 MHz, CDCl₃) δ 7.85-7.60 (m, 4H), 7.24-7.34 (m, 5H), 7.04-7.00 (m, 2H), 6.96-6.87 (m, 3H), 6.84 (dt, J = 9.0, 3.0 Hz, 2H), 6.67 (dt, J = 8.5, 2.5 Hz, 2H), 5.66 (t, J = 4.0 Hz, 1H), 5.22-5.18 (m, 1H), 4.64 (d, J = 12.0 Hz, 1H), 4.55-4.48 (m, 4H), 4.36 (d, J = 12.0 Hz, 1H), 3.90-3.84 (m, 1H), 3.68 (s, 3H), 3.68-3.64 (m, 2H), 1.98 (s, 3H).

¹³C-NMR (125 MHz, CDCl₃) δ 169.6, 155.3, 150.6, 137.8, 137.5, 133.9, 128.2, 128.0, 127.7, 127.7, 127.5, 127.4, 123.3, 118.4, 114.3, 97.4, 76.8, 73.9, 73.7, 73.5, 72.2, 69.4, 55.4, 55.3, 20.8.

HRMS (ESI⁺) [M+H]⁺ calcd for C₃₇H₃₆NO₉: 638.2385; found 638.2401.

### Example 14

### 4-O-Acetyl-3,6-di-O-benzyl-2-deoxy-2-(1,3-dioxo-1,3-dihydro-2H-isoindol-2-yl)-D-glucopyranoside (Compound represented by formula F-2)

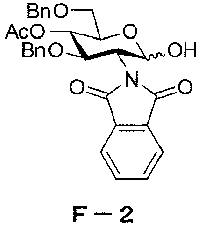

### (Substep Z-2)

To a solution of 4-methoxyphenyl 4-O-acetyl-3,6-di-O-benzyl-2-deoxy-2-(1,3-dioxo-1,3-dihydro-2H-isoindol-2-yl)-β-D-glucopyranoside (compound represented by formula F'-1) (49.0 g, 76.84 mmol) in dichloromethane (392 mL), hexafluoro 2-propanol (245 mL), and water (25 mL) was added [bis(trifluoroacetoxy)iodo]benzene (46.3 g, 107.58 mmol) at 25°C or below, and the mixture was stirred at the same temperature for 4 hours. After the completion of the reaction was checked by HPLC, ethyl acetate (1225 mL) was added. After the mixture was cooled on ice, water (490 mL) dissolving sodium hydrogen carbonate (24.5 g) and sodium sulfite (24.5 g) was poured, and the mixture was then liquid-separated to obtain an organic layer. The organic layer obtained was washed again with water (490 mL) dissolving sodium hydrogen carbonate (24.5 g) and sodium sulfite (24.5 g), and further washed with 20% brine (245 g). The resulting organic layer was concentrated under reduced pressure to 490 mL (crystals were found to precipitate during concentration), and heptane (735 mL) was added dropwise. The resulting slurry solution was cooled to 0°C to -5°C, and stirred at the same temperature for 1 hour. The precipitated crystals were then filtered. The filtered crystals were washed with a mixture of ethyl acetate and heptane (39/118 mL) at 0°C to 5°C and dried under reduced pressure at 40°C to give 4-O-acetyl-3,6-di-O-benzyl-2-deoxy-2-(1,3-dioxo-1,3-dihydro-2H-isoindol-2-yl)-D-glucopyranoside (compound represented by formula F-2) (37.5 g, yield 92%) as white crystals.

¹H-NMR (400 MHz, CDCl₃) δ 7.71-7.65 (m, 4H), 7.34-7.26 (m, 5H), 7.01-6.87 (m, 5H), 5.36 (dd, J = 8.0, 8.0 Hz, 1H), 5.13 (dd, J = 8.4, 10.0 Hz, 1H), 4.59 (d, J = 12.4 Hz, 1H), 4.54 (s, 2H), 4.50 (dd, J = 8.4, 10.4 Hz, 1H), 4.33 (d, J = 12.4 Hz, 1H), 4.17 (dd, J = 8.4, 10.4 Hz, 1H), 3.79 (ddd, J = 8.4, 5.2, 4.8 Hz, 1H), 3.61-3.53 (m, 2H), 3.41 (d, J = 8.0 Hz, 1H), 1.93 (s, 3H).

¹³C-NMR (100 MHz, CDCl₃) δ 169.8, 168.1, 137.7, 137.7, 134.0, 131.6, 128.4, 128.2, 128.0, 127.8. 127.7, 127.5, 123.4, 116.2, 92.9, 73.9, 73.7, 73.5, 72.2, 69.3, 57.1, 20.9.

HRMS (ESI⁻) [M-H]⁻ calcd for C₃₀H₂₈NO₈: 530.1820; found 530.1841.

### Example 15

### 4-O-Acetyl-3,6-di-O-benzyl-2-deoxy-2-(1,3-dioxo-1,3-dihydro-2H-isoindol-2-yl)-O-[2,2,2-trifluoro-N-phenylethanimidoyl]-β-D-glucopyranoside (Compound represented by formula A-12)

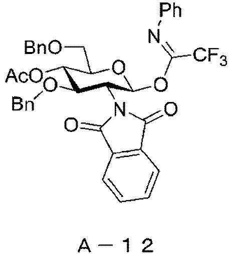

### (Substep Z-3)

First, 4-O-acetyl-3,6-di-O-benzyl-2-deoxy-2-(1,3-dioxo-1,3-dihydro-2H-isoindol-2-yl)-D-glucopyranoside (compound represented by formula F-2) (20.0 g, 37.63 mmol) was added to a 500-mL recovery flask, and was admixed with dichloromethane (200 mL) and Molecular Sieves 4A powder (10 µm or less, 10.0 g). N-Methylimidazole (3.40 g, 41.39 mmol) and 2,2,2-trifluoro-N-phenylacetimidoyl chloride (8.20 g, 39.51 mmol) were sequentially added at 0°C under nitrogen, and the mixture was stirred at the same temperature for 18 hours. After the completion of the reaction was checked by HPLC, the reaction solution was filtered and washed with dichloromethane (100 mL). The filtrate was filtered through a neutral silica gel pad (silica gel 60N; particle size: 40 to 50 µm; 60 g; manufactured by KANTO CHEMICAL CO., INC.) packed with dichloromethane, and collected every 100 mL. The silica gel pad was washed with dichloromethane (400 mL, 100 mL each collected) and ethyl acetate/dichloromethane (1:4; 400 mL, 100 mL each collected). The selected fractions were concentrated until the liquid volume reached 40 mL. Toluene (200 mL) was added, and the mixture was concentrated again until the liquid volume reached 40 mL. Toluene (200 mL) was further added, and the mixture was concentrated until the liquid volume reached 40 mL to give a toluene solution containing crude 4-O-acetyl-3,6-di-O-benzyl-2-deoxy-2-(1,3-dioxo-1,3-dihydro-2H-isoindol-2-yl)-O-[2,2,2-trifluoro-N-phenylethanimidoyl]-β-D-glucopyranoside (compound represented by formula A-12). This was used as it was in the next step.

### <Synthesis of compound represented by formula A-16>

Compound represented by formula A-16 was synthesized according to the following synthesis scheme 3.

### [Synthesis Scheme 3]

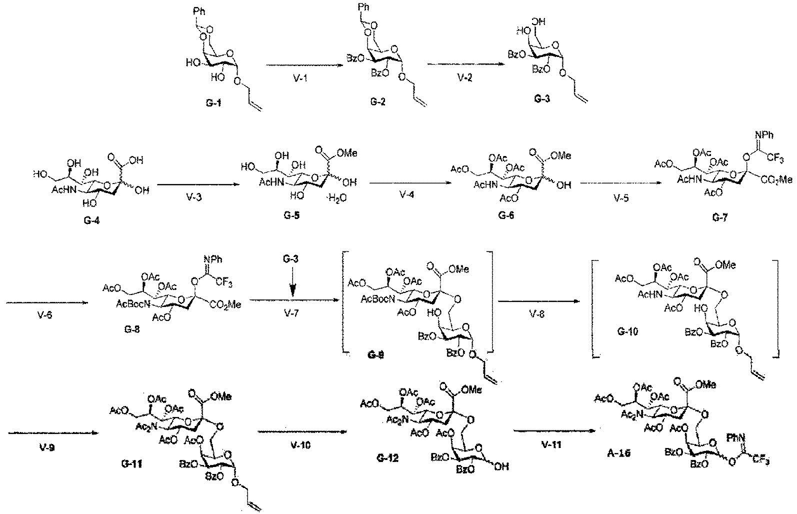

First, the compound represented by formula G-1 was synthesized according to the following synthesis scheme.

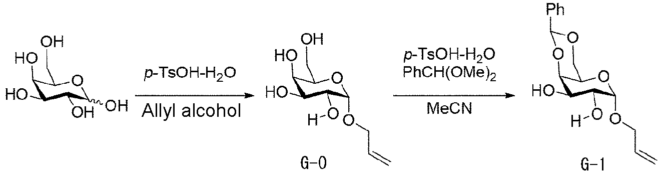

### Example 16

### Allyl α-D-galactopyranoside (Compound represented by G-0)

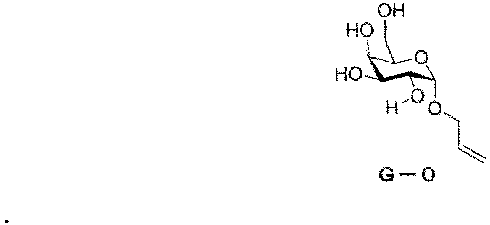

D-galactopyranose (20.00 g, 111.01 mmol) was added to a 500-mL four-neck flask, and allyl alcohol (200.0 mL) was then added. Under a nitrogen atmosphere, p-TsOH·H₂O (2.11 g, 11.10 mmol) was added at room temperature. The temperature was raised to 70°C, and the mixture was stirred for 24 hours. The reaction solution was cooled to 40°C, and admixed with triethylamine (1.69 g, 16.65 mmol), stirred for 5 min, and the reaction solution was then concentrated under reduced pressure to a liquid volume of 100 mL. Next, nBuOH (200 mL) was added dropwise to this concentrated liquid over 30 minutes, and the mixture was stirred at room temperature for 1 hour. The reaction solution was then concentrated under reduced pressure to a liquid volume of 80 mL and stirred at room temperature overnight. The suspension was filtered. The resulting crystals were washed with nBuOH (40 mL) at 0°C and dried under reduced pressure at 40°C to give allyl α-D-galactopyranoside (compound represented by G-0) (8.41 g, yield: 34.4%) as white crystals.

¹H-NMR (500 MHz, METHANOL-D4) δ 5.97 (qd, J = 11.2, 5.7 Hz, 1H), 5.33 (dd, J = 17.2, 1.7 Hz, 1H), 5.17 (dd, J = 10.6, 1.4 Hz, 1H), 4.22 (dd, J = 13.2, 5.2 Hz, 1H), 4.04 (dd, J = 13.0, 6.0 Hz, 1H), 3.88 (d, J = 1.7 Hz, 1H), 3.82-3.66 (m, 5H).

¹³C-NMR (125 MHz, METHANOL-D4) δ 62.74, 69.36, 70.21, 71.08, 71.51, 72.49, 99.46, 117.47, 135.69.

MS (ESI) m/z: 221 (M+H)⁺, 219 (M-H)⁻.

### Example 17

### Prop-2-en-1-yl 4.6-O-benzylidene-α-D-galactopyranoside (Compound represented by formula G-1)

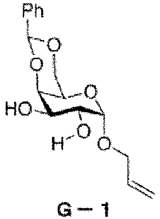

Under a nitrogen atmosphere, acetonitrile (5.0 mL), benzaldehyde dimethyl acetal (5.18 g, 34.1 mmol), and p-TsOH·H₂O (431.9 mg, 2.27 mmol) were added to a 100-mL recovery flask, followed by addition of allyl α-D-galactopyranoside (5.00 g, 22.7 mmol) little by little. The temperature was then raised to 40°C, and the solution was stirred for 30 minutes. Subsequently, triethylamine (344.6 mg, 3.41 mmol) was added, and the mixture was stirred for 5 min, followed by dropwise addition of isopropyl alcohol (75 mL) at 40°C. The reaction solution was concentrated under reduced pressure until the liquid volume reached 25 mL and stirred at 0°C overnight. Subsequently the suspension was filtered. The resulting crystals were then washed with isopropyl alcohol (5 mL) chilled to 0°C and dried under reduced pressure at 40°C to give prop-2-en-1-yl 4.6-O-benzyliden-α-D-galactopyranoside (Compound represented by formula G-1) (5.35 g, yield: 76.3%) as white crystals.

¹H-NMR (500 MHz, METHANOL-D4) δ 7.53-7.52 (dd, J = 7.5, 2.0 Hz, 2H), 7.34 (m, 3H), 5.98 (qd, J = 11.1, 5.4 Hz, 1H), 5.59 (s, 1H), 5.35 (d, J = 18.9 Hz, 1H), 5.19 (d, J = 10.3 Hz, 1H), 4.95 (d, J = 4.0 Hz, 1H), 4.26 (d, J = 3.4 Hz, 1H), 4.22 (dd, J = 13.2, 5.2 Hz, 1H), 4.13 (s, 2H), 4.08 (q, J = 6.5 Hz, 1H), 3.92 (ddd, J = 24.1, 10.3, 3.4 Hz, 2H), 3.74 (s, 1H). ¹³C-NMR (125 MHz, METHANOL-D4) δ 64.60, 69.70, 70.03, 70.08, 70.34, 78.07, 100.22, 102.28, 117.58, 127.54, 129.02, 129.86, 135.59, 139.77.

MS (ESI) m/z: 309 (M+H)⁺, 307 (M-H)⁻.

### Example 18

### Prop-2-en-1-yl 2,3-di-O-benzoyl-4,6-O-benzyliden-α-D-galactopyranoside (Compound represented by formula G-2)

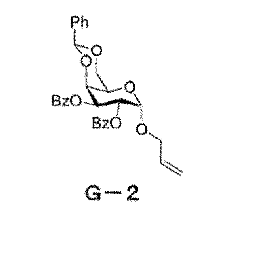

### (Substep V-1)

To a solution of prop-2-en-1-yl 4,6-O-benzylidene-α-D-galactopyranoside (compound represented by formula G-1) (30.0 g, 97.30 mmol) in pyridine (150 mL) was added dropwise at 40°C or below benzoyl chloride (47.87 g, 340.54 mmol). The mixture was stirred at the same temperature for 2 hours. After the completion of the reaction was checked by HPLC, the mixture was cooled to 20°C to 30°C. After ethanol (450 mL) was poured, water (300 mL) was added dropwise over 30 minutes. The resulting slurry solution was stirred at 20°C to 30°C for 1 hour. The precipitated crystals were then filtered. The filtered crystals were washed with a mixture of ethanol and water (75/75 mL) and dried under reduced pressure at 40°C to give prop-2-en-1-yl 2,3-di-O-benzoyl-4,6-O-benzyliden-α-D-galactopyranoside (compound represented by formula G-2) (47.9 g, yield 95%) as white crystals.

¹H-NMR (500 MHz, CDCl₃) δ 8.03-7.98 (m, 4H), 7.55-7.46 (m, 4H), 7.40-7.30 (m, 7H), 5.90-5.78 (m, 2H), 5.82 (s, 1H), 5.57 (s, 1H), 5.42 (d, J = 1.7 Hz, 1H), 5.31 (dd, J = 17.2, 1.7 Hz, 1H), 5.15 (dd, J = 1.7, 10.5 Hz, 1H), 4.66 (s, 2H), 4.33 (d, J = 12.5 Hz, 1H), 4.26 (dd, J = 12.5, 4.5 Hz, 1H), 4.12 (dd, J = 12.5, 1.0 Hz, 1H), 4.08 (dd, J = 6.5, 13.5 Hz, 1H), 3.95 (s, 1H).

¹³C-NMR (125 MHz, CDCl₃) δ 166.1, 165.8, 137.5, 133.4, 133.2, 133.1, 129.8, 129.7, 129.5, 129.4, 128.8, 128.3, 128.1, 126.1, 117.5, 100.6, 96.2, 74.2, 69.3, 69.1, 68.7, 68.6, 62.4.

HRMS (ESI⁺) [M+H]⁺ calcd for C₃₀H₂₉O₈: 517.1857; found 517.1880.

### Example 19

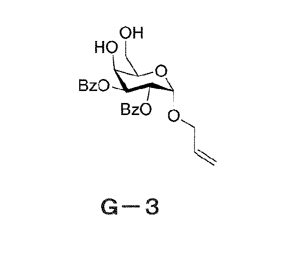

### Prop-2-en-1-yl 2,3-di-O-benzoyl-α-D-galactopyranoside (Compound represented by formula G-3)

### (Substep V-2)

A solution of prop-2-en-1-yl 2,3-di-O-benzoyl-4,6-O-benzyliden-α-D-galactopyranoside (Compound represented by formula G-2) (47.1 g, 91.18 mmol) in acetonitrile (377 mL) was heated to 45°C. Water (24 mL) and concentrated hydrochloric acid (9.2 g, 91.18 mmol) were added, and the mixture was stirred at the same temperature for 30 minutes. Water (353 mL) was added dropwise at 45°C to 50°C over 3 hours, and the mixture was stirred for another 30 minutes. After the completion of the reaction was checked by HPLC, sodium acetate (11.22 g, 136.78 mmol) was added and ethyl acetate (942 mL) and water (471 mL) were poured. After cooling to 25°C or below, the mixture was liquid-separated to obtain an organic layer. The organic layer obtained was washed twice with water (471 mL) and further washed with 20% brine (236 mL). The organic layer was concentrated under reduced pressure to 141 mL, and toluene (707 mL) was added. The mixture was concentrated again under reduced pressure to 141 mL. Toluene (236 mL) was added to the concentrated solution obtained, and the mixture was concentrated under reduced pressure to 141 mL. The concentrated liquid was cooled to 0°C to 5°C and a slurry solution of neutral silica gel (silica gel 60N; particle size: 40 to 50 µm; 141 g; manufactured by KANTO CHEMICAL CO., INC.) cooled to 0 to 5°C in toluene (330 mL) was poured. The mixture was stirred at the same temperature for 15 minutes, and the product was adsorbed on silica gel, followed by filtration. The silica gel solid phase containing the product was washed with toluene (942 mL) at 0°C to 5°C (the filtrate at the time of the toluene wash was discarded). The target substance was then desorbed from silica gel by using cyclopentyl methyl ether (707 mL) to give a cyclopentyl methyl ether solution containing prop-2-en-1-yl 2,3-di-O-benzoyl-α-D-galactopyranoside (compound represented by formula G-3) (quantitative value 36.2 g, quantitative yield 93%). This solution was used in the next step.

### Example 20

### Methyl 5-acetamido-3,5-dideoxy-D-glycero-D-galacto-non-2-ulopyranosonate monohydrate (Compound represented by formula G-5)

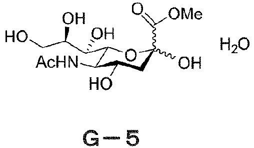

### (Substep V-3)

To a solution of 5-acetamido-3,5-dideoxy-D-glycero-D-galacto-non-2-ulopyranosonic acid (compound represented by formula G-4) (40.1 g, 129.66 mmol) and methyl orthoformate (15.60 mL, 142.59 mmol) in methanol (321 mL) was added sulfuric acid (1.0 g, 10.20 mmol), and the mixture was heated to 40°C and stirred for 3 hours. After the completion of the reaction was checked by HPLC, the mixture was cooled to 25°C. Dimethylacetamide (40 mL) was added, and the mixture was concentrated under reduced pressure to 160 mL. The temperature of the resulting concentrated solution was adjusted to 15°C. Water (20 mL) and ethyl acetate (722 mL) were poured. The mixture was then stirred at 25°C for 1 hour. The slurry solution was cooled to 0°C to 5°C, and then stirred at the same temperature for 2 hours. The precipitated crystals were filtered. The filtered crystals were washed with ethyl acetate (80 mL) at 0°C to 5°C and dried under reduced pressure at 40°C to give methyl 5-acetamido-3,5-dideoxy-D-glycero-D-galacto-non-2-ulopyranosonate monohydrate (compound represented by formula G-5) (41.1 g, yield 93%) as white crystals.

¹H-NMR (500 MHz, CD₃OD) δ 4.07-3.98 (m, 2H), 3.85-3.77 (m, 2H), 3.78 (s, 3H), 3.72-3.68 (m, 1H), 3.62 (dd, J = 10.9, 5.7 Hz, 1H), 3.48 (dd, J = 9.2, 1.1 Hz, 1H), 2.22 (dd, J = 12.9, 4.9 Hz, 1H), 2.02 (s, 3H), 1.89 (dd, J = 12.6, 11.5 Hz, 1H).

¹³C-NMR (125 MHz, CD₃OD) δ 175.2, 175.1, 171.8, 96.6, 72.1, 72.0, 71.6, 70.1, 67.9, 64.8, 54.4, 54.3, 53.2, 40.7, 22.7, 22.7.

HRMS (ESI⁺) [M+H]⁺ calcd for C₁₂H₂₂NO₉: 324.1289; found 324.1288.

### Example 21

### Methyl 5-acetamido-4,7,8,9-tetra-O-acetyl-3,5-dideoxy-D-glycero-D-galacto-non-2-ulopyranosonate (Compound represented by formula G-6)

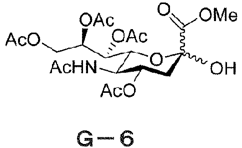

### (Substep V-4)

The temperature of a slurry solution of methyl 5-acetamido-3,5-dideoxy-D-glycero-D-galacto-non-2-ulopyranosonate monohydrate (compound represented by formula G-5) (40.3 g, 118.07 mmol) in acetonitrile (403 mL) was adjusted to 25°C. Acetic anhydride (60.27 g, 590.36 mmol) and paratoluenesulfonic acid monohydrate (1.12 g, 5.89 mmol) were added, and the mixture was stirred at 25°C for 24 hours. The reaction solution was cooled to 15°C, acetic anhydride (12.05 g, 118.03 mmol) was added, and the mixture was then stirred at the same temperature for 47 hours. After the completion of the reaction was checked by HPLC, methanol (40 mL) was added. The temperature was adjusted to 25°C, and the mixture was stirred at the same temperature for 2 hours. Next, sodium acetate (0.97 g, 11.82 mmol) was added, and the mixture was stirred at the same temperature for another 1 hour. The reaction solution was concentrated under reduced pressure to 120 mL, and cooled to 0°C to 5°C. Ethyl acetate (403 mL) and water (161 mL) were then poured, and under stirring at 0°C to 5°C, triethylamine was added to adjust the pH to 7.0. The liquid-separated organic layer was washed twice with 10% brine (121 mL), and concentrated under reduced pressure to 200 mL. Ethyl acetate (605 mL) was added to the concentrated solution, and the mixture was concentrated again under reduced pressure to 200 mL. Ethyl acetate (40 mL) was added to the concentrated solution. Seed crystals were inoculated, and the mixture was stirred at 25°C for 4 hours. Heptane (302 mL) was then added dropwise over 30 minutes. The resulting slurry solution was stirred at 25°C for 2 hours, and the precipitated crystals were then filtered. The filtered crystals were washed with a mixture of ethyl acetate and heptane (67/135 mL) and dried under reduced pressure at 35°C to give methyl 5-acetamido-4,7,8,9-tetra-O-acetyl-3,5-dideoxy-D-glycero-D-galacto-non-2-ulopyranosonate (compound represented by formula G-6) (44.1 g, yield 76%) as white crystals.

¹H-NMR (500 MHz, CDCl₃) δ 6.28 (d, J = 10.3 Hz, 1H), 5.41 (dd, J = 4.6, 2.3 Hz, 1H), 5.28-5.23 (m, 1H), 5.21-5.14 (m, 1H), 5.09 (s, 1H), 4.62 (dd, J = 12.3, 2.6 Hz, 1H), 4.28 (dd, J = 10.3, 2.3 Hz, 1H), 4.21-4.11 (m, 1H), 4.04 (dd, J = 12.3, 8.3 Hz, 1H), 3.85 (s, 3H), 2.24-2.20 (m, 2H), 2.16 (s, 3H), 2.12 (s, 3H), 2.03 (s, 3H), 2.01 (s, 3H), 1.91 (s, 3H).

¹³C-NMR (125 MHz, CDCl₃) δ 171.5, 171.1, 170.8, 170.3, 170.2, 168.9, 94.9, 72.1, 71.4, 69.1, 68.3, 62.5, 53.2, 49.1, 36.1, 23.0, 21.0, 20.8, 20.7, 20.7.

HRMS (ESI⁺) [M+H]⁺ calcd for C₂₃H₃₀NO₁₃: 492.1712; found 492.1712.

### Example 22

### Methyl 5-acetamido-4,7,8,9-tetra-O-acetyl-3,5-dideoxy-2-O-(2,2,2-trifluoro-N-phenylethanimidoyl)-D-glycero-β-D-galacto-non-2-ulopyranosonate (Compound represented by formula G-7)

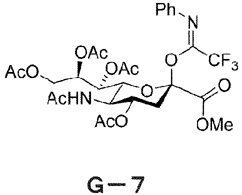

### (Substep V-5)

The temperature of a slurry solution of methyl 5-acetamido-4,7,8,9-tetra-O-acetyl-3,5-dideoxy-D-glycero-D-galacto-non-2-ulopyranosonate (compound represented by formula G-6) (44.0 g, 89.53 mmol) and Molecular Sieves 4A powder (powder particle size: 10 µm or less) (22 g) in dichloromethane (352 mL) was adjusted to 20°C. After stirring at the same temperature for 30 minutes, 2,2,2-trifluoro-N-phenylacetimidoyl chloride (26.02 g, 125.35 mmol) was added. Next, N-methylimidazole (11.03 g, 134.33 mmol) was added dropwise, and the mixture was stirred at 20°C for 7.5 hours. The completion of the reaction was checked by HPLC. The reaction solution was then filtered and washed with dichloromethane (88 mL) to obtain a filtrate. The filtrate obtained was cooled to 0°C and admixed with cold water (440 mL). Under stirring at 0°C to 5°C, triethylamine was added to adjust the pH to 7.5. The mixture was stirred at 0°C to 5°C for 30 minutes, and then liquid-separated. The resulting organic layer was washed twice with cold water (440 mL) and then cooled 20% brine (220 mL), and concentrated under reduced pressure to 88 mL. Ethyl acetate (440 mL) was added to the concentrated solution, and the mixture was concentrated again under reduced pressure to 88 mL. The concentrated solution was admixed with t-butyl methyl ether (308 mL). Seed crystals were then inoculated, and the mixture was stirred at 20°C for 4 hours. Heptane (264 mL) was added dropwise to the resulting slurry solution over 1 hour. The mixture was stirred at the same temperature for 2 hours. The precipitated crystals were then filtered. The filtered crystals were washed with a mixture of t-butyl methyl ether and heptane (132/88 mL) and dried under reduced pressure at 35°C to give methyl 5-acetamido-4,7,8,9-tetra-O-acetyl-3,5-dideoxy-2-O-(2,2,2-trifluoro-N-phenylethanimidoyl)-D-glycero-β-D-galacto-non-2-ulopyranosonate (compound represented by formula G-7) (39.5 g, yield 67%) as white crystals.

¹H-NMR (500 MHz, CDCl₃) δ 7.30-7.24 (m, 2H), 7.09 (t, J = 7.4 Hz, 1H), 6.72 (d, J = 8.0 Hz, 2H), 5.76 (d, J = 9.7 Hz, 1H), 5.48-5.45 (m, 1H), 5.30 (td, J = 10.9, 4.8 Hz, 1H), 5.15-5.10 (m, 1H), 4.60 (dd, J = 12.6, 2.3 Hz, 1H), 4.30 (q, J = 10.3 Hz, 1H), 4.23 (dd, J = 10.3, 2.3 Hz, 1H), 4.11 (dd, J = 12.3 Hz, 7.7 Hz, 1H), 3.81 (s, 3H), 2.79 (dd, J = 13.5, 4.9 Hz, 1H), 2.21-2.15 (m, 1H), 2.16 (s, 3H), 2.10 (s, 3H), 2.07 (s, 3H), 1.90 (s, 3H), 1.75 (s, 3H).

¹³C-NMR (125 MHz, CDCl₃) δ 171.0, 170.7, 170.4, 170.2, 170.1, 165.3, 142.6, 141.0 (q, ²J_{C-F}, = 36.0 Hz), 128.8, 124.6, 119.0, 115.1 (q, ¹J_{C-F} = 284.4 Hz), 99.7, 73.6, 71.9, 68.3, 68.0, 62.4, 53.1, 48.6, 35.6, 23.0, 20.8, 20.8, 20.7, 20.3.

HRMS (ESI⁺) [M+NH₄]⁺ calcd for C₂₈H₃₇F₃N₃O₁₃: 680.2273; found 680.2314.

### Example 23

### Methyl 4,7,8,9-tetra-O-acetyl-5-[acetyl(tert-butoxycarbonyl)amino]-3,5-dideoxy-2-O-(2,2,2-trifluoro-N-phenylethanimidoyl)-D-glycero-β-D-galacto-non-2-ulopyranosonate (Compound represented by formula G-8)

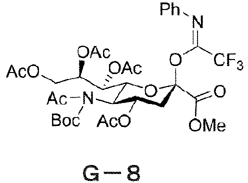

### (Substep V-6)

To a solution of methyl 5-acetamido-4,7,8,9-tetra-O-acetyl-3,5-dideoxy-2-O-(2,2,2-trifluoro-N-phenylethanimidoyl)-D-glycero-β-D-galacto-non-2-ulopyranosonate (compound represented by formula G-7) (39.0 g, 58.86 mmol) in tetrahydrofuran (390 mL) were added di-tert-butyl bicarbonate (27.05 g, 123.94 mmol) and dimethylaminopyridine (1.80 g, 14.73 mmol), and the mixture was heated to reflux temperature. The mixture was stirred under reflux for 30 minutes. After the completion of the reaction was checked by HPLC, the reaction solution was concentrated under reduced pressure to 117 mL. Toluene (195 mL) was added to the concentrated solution, and the mixture was concentrated again under reduced pressure to 117 mL. The concentrated solution was filtered using a silica gel-filled funnel (silica gel 60N; particle size: 40 to 50 µm; 117 g; toluene wet packed; manufactured by KANTO CHEMICAL CO., INC.) and washed with a toluene-ethyl acetate mixture (8/2) (975 mL) to obtain a filtrate. The filtrate obtained was concentrated under reduced pressure (to a weight of 59 g), and admixed with cyclopentyl methyl ether (23 mL). The temperature of the solution was adjusted to 20°C, heptane (156 mL) was added dropwise over 15 minutes, and the mixture was then stirred at the same temperature for 1 hour. After the crystals were found to precipitate, heptane (312 mL) was added dropwise over 1 hour. The precipitated crystals were then filtered. The filtered crystals were washed with heptane (78 mL) and dried under reduced pressure at 35°C to give methyl 4,7,8,9-tetra-O-acetyl-5-[acetyl(tert-butoxycarbonyl)amino]-3,5-dideoxy-2-O-(2,2,2-trifluoro-N-phenylethanimidoyl)-D-glycero-β-D-galacto-non-2-ulopyranosonate (compound represented by formula G-8) (37.0 g, yield 82%) as white crystals.

### ¹H-NMR (500 MHz, CDCl₃)

Note: Detected as a ca. 1/4 isomer mixture. Major isomer: δ 7.27 (t, J = 8.9 Hz, 2H), 7.09 (t, J = 7.2 Hz, 1H), 6.73 (d, J = 8.0 Hz, 2H), 5.75-5.65 (m, 1H), 5.31 (d, J = 4.6 Hz, 1H), 5.18-5.14 (m, 1H), 5.15 (d, J = 6.0 Hz, 2H), 4.54 (dd, J = 12.0, 2.0 Hz, 1H), 4.08 (dd, J = 12.6, 6.9 Hz, 1H), 3.84 (s, 3H), 2.90 (dd, J = 13.7, 5.2 Hz, 1H), 2.39 (s, 3H), 2.25 (dd, J = 13.7, 11.2 Hz, 1H), 2.09 (s, 3H), 2.07 (s, 3H), 1.99 (s, 3H), 1.77 (s, 3H), 1.62 (s, 9H). Minor isomer: δ inter alia 6.76 (d, J = 8.0 Hz, 2H), 5.85-5.80 (m, 1H), 5.29-5.25 (m, 1H), 5.22-5.19 (m, 1H), 4.44 (d, J = 11.0 Hz, 1H), 4.15-4.11 (m, 1H), 3.03 (dd, J = 14.0, 5.0 Hz, 1H), 2.41 (s, 3H), 2.12 (s, 3H), 2.00 (s, 3H), 1.88 (s, 3H), 1.74 (s, 3H), 1.54 (s, 9H) .¹³C-NMR (125 MHz, CDCl₃) Mixture: δ 173.7, 170.4, 170.2, 170.0, 169.9, 165.4, 151.7, 142.8, 128.7, 124.5, 119.1, 100.6, 85.2, 72.8, 71.3, 67.7, 65.9, 62.0, 53.1, 52.0, 36.7, 27.9, 27.7, 26.6, 20.8, 20.7, 20.6, 20.3. HRMS (ESI⁺) [M+NH₄]⁺ calcd for C₃₃H₄₅F₃N₃O₁₅: 780.2797; found 780.2801.

### Example 24

### Prop-2-en-1-yl 2,3-di-O-benzoyl-6-O-{4,7,8,9-tetra-O-acetyl-5-[acetyl(tert-butoxycarbonyl)amino]-3,5-dideoxy-1-methyl-D-glycero-α-D-galacto-non-2-ulopyranosyl}-α-D-galactopyranoside (Compound represented by formula G-9)

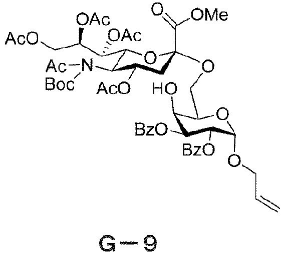

### (Substep V-7)

A cyclopentyl methyl ether solution containing prop-2-en-1-yl 2,3-di-O-benzoyl-α-D-galactopyranoside (compound represented by formula G-3) (quantitative value 31.46 g, 73.43 mmol) was concentrated under reduced pressure to 105 mL. This solution was added to a solution of methyl 4,7,8,9-tetra-O-acetyl-5-[acetyl(tert-butoxycarbonyl)amino]-3,5-dideoxy-2-O-(2,2,2-trifluoro-N-phenylethanimidoyl)-D-glycero-β-D-galacto-non-2-ulopyranosonate (compound represented by formula G-8) (35.0 g, 45.89 mmol) in cyclopentyl methyl ether (175 mL). Next, cyclopentyl methyl ether was added to the resulting mixed solution, and the total volume was adjusted to 350 mL (cyclopentyl methyl ether mixed solution containing compound represented by formula G-3 and compound represented by formula G-8). Cyclopentyl methyl ether (525 mL) and Molecular Sieves 4A powder (powder particle size: 10 µm or less) (17.5 g) were added to another vessel. After the mixed solution was cooled to -60°C, trimethylsilyl trifluoromethanesulfonate (4.2 mL, 23.24 mmol) was added. To this solution, the cyclopentyl methyl ether mixed solution containing compound represented by formula G-3 and compound represented by formula G-8 was added dropwise over 4.5 hours at -60°C under strong stirring, and the mixture was stirred at the same temperature for 2 hours. After the completion of the reaction was checked by HPLC, triethylamine (4.5 mL, 32.12 mmol) was added. The reaction solution was heated to 0°C. After that, Celite 545 (35.00 g) was added, and the reaction solution was filtered and washed with cyclopentyl methyl ether (175 mL). Water (350 mL) was added to the filtrate and the liquid was separated. Next, 0.5 N hydrochloric acid water (350 mL) was added to the organic layer, and the mixture was stirred at 20°C for 2 hours. After the decomposition of byproducts was checked by HPLC, the liquid was separated to obtain an organic layer. The organic layer was washed with water (350 mL) and then 20% brine (175 mL), and concentrated under reduced pressure to 70 mL. Toluene (700 mL) was added to the concentrated solution, and the mixture was concentrated under reduced pressure to 70 mL. The concentrated solution was admixed again with toluene (700 mL) and neutral silica gel (silica gel 60N; particle size: 40 to 50 µm; 158 g; manufactured by KANTO CHEMICAL CO., INC.) and stirred at 20°C for 30 minutes. The product was adsorbed on silica gel, and then filtered. The silica gel solid phase containing the product was washed with toluene (1575 mL) (the filtrate at the time of the toluene wash was discarded). The target substance was desorbed from silica gel by using ethyl acetate (875 mL). The resulting ethyl acetate solution was concentrated under reduced pressure to 70 mL. Toluene (175 mL) was added, and the mixture was concentrated again under reduced pressure to 70 mL to give a toluene solution containing prop-2-en-1-yl 2,3-di-O-benzoyl-6-O-{4,7,8,9-tetra-O-acetyl-5-[acetyl(tert-butoxycarbonyl)amino]-3,5-dideoxy-1-methyl-D-glycero-α-D-galacto-non-2-ulopyranosyl}-α-D-galactopyranoside (compound represented by formula G-9). This solution was used in the next step.

### Example 25

### Prop-2-en-1-yl 6-O-{5-acetamido-4,7,8,9-tetra-O-acetyl-3,5-dideoxy-1-methyl-D-glycero-α-D-galacto-non-2-ulopyranosyl}-2,3-di-O-benzoyl-α-D-galactopyranoside (Compound represented by formula G-10)

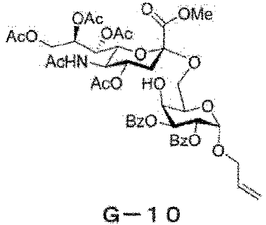

### (Substep V-8)

To the toluene solution containing prop-2-en-1-yl 2,3-di-O-benzoyl-6-O-{4,7,8,9-tetra-O-acetyl-5-[acetyl(tert-butoxycarbonyl)amino]-3,5-dideoxy-1-methyl-D-glycero-α-D-galacto-non-2-ulopyranosyl}-α-D-galactopyranoside (compound represented by formula G-9) obtained in Example 24 were added dichloromethane (525 mL) and copper(II) trifluoromethanesulfonate (8.30 g, 22.95 mmol). The temperature was raised to 40°C and the mixture was stirred at the same temperature for 2 hours. After the completion of the reaction was then checked by HPLC, the mixture was cooled to 25°C and concentrated under reduced pressure to 70 mL. The concentrated solution was admixed with ethyl acetate (525 mL), and washed three times with 5% brine (350 mL). Heptane (263 mL) was then added to the organic layer, and the mixture was washed four times with 20% methanol water (525 mL). HPLC was used to check whether impurities derived from the β-eliminated form of compound 8, a byproduct of the glycosylation reaction, were removed into the aqueous layer. The organic layer was then concentrated under reduced pressure to 70 mL. Isopropenyl acetate (525 mL) was added to the concentrated solution, and the mixture was concentrated under reduced pressure to 350 mL to give an isopropenyl acetate solution containing prop-2-en-1-yl 6-O-{5-acetamido-4,7,8,9-tetra-O-acetyl-3,5-dideoxy-1-methyl-D-glycero-α-D-galacto-non-2-ulopyranosyl}-2,3-di-O-benzoyl-α-D-galactopyranoside (compound represented by formula G-10). This solution was used in the next step.

### Example 26

### Prop-2-en-1-yl 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-α-D-galactopyranoside (Compound represented by formula G-11)

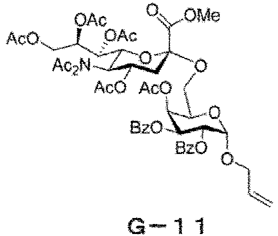

### (Substep V-9)

To the isopropenyl acetate solution containing prop-2-en-1-yl 6-O-{5-acetamide-4,7,8,9-tetra-O-acetyl-3,5-dideoxy-1-methyl-D-glycero-α-D-galacto-non-2-ulopyranosyl}-2,3-di-O-benzoyl-α-D-galactopyranoside (compound represented by formula G-10) obtained in Example 25 was added paratoluenesulfonic acid monohydrate (0.88 g, 4.62 mmol). The temperature was raised to reflux temperature (internal temperature at or near 90°C) and the mixture was stirred at the same temperature for 3 hours. After the completion of the reaction was checked by HPLC, the mixture was cooled to 25°C. Triethylamine (0.95 mL, 6.85 mmol) was added, and the mixture was concentrated under reduced pressure to 70 mL. Toluene (350 mL) was added to the concentrated solution, and the mixture was concentrated again under reduced pressure to 70 mL. The concentrated solution was admixed with toluene (630 mL) and then neutral silica gel (silica gel 60N; particle size: 40 to 50 µm; 123 g; manufactured by KANTO CHEMICAL CO., INC.). The mixture was stirred at the same temperature for 30 minutes. The product was adsorbed on the silica gel, and then filtered. The silica gel solid phase containing the product was washed with toluene (1925 mL) and then a toluene/ethyl acetate mixture (97/3, 1400 mL) (the filtrate at the time of the wash was discarded). The target substance was desorbed from the silica gel solid phase containing the product by using ethyl acetate (1050 mL). The resulting ethyl acetate solution was concentrated under reduced pressure to give prop-2-en-1-yl 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-α-D-galactopyranoside (compound represented by formula G-11) (30.1 g, yield 67% (in terms of compound represented by formula G-8)) as a white foamy solid (containing 0.42 equivalents of toluene (about 4% by weight)).

¹H-NMR (500 MHz, CDCl₃) δ 7.99 (d, J = 8.4 Hz, 2H), 7.88 (dd, J = 8.4, 1.4 Hz, 2H), 7.53-7.47 (m, 2H), 7.37 (dt, J = 14.7, 6.9 Hz, 4H), 5.90-5.82 (m, 1H), 5.82 (dd, J = 10.9, 3.4 Hz, 1H), 5.73 (d, J = 2.9 Hz, 1H), 5.58 (dd, J = 10.9, 4.0 Hz, 1H), 5.51 (td, J = 10.6, 5.0 Hz, 1H), 5.35-5.30 (m, 3H), 5.17-5.15 (m, 2H), 4.94 (dd, J = 10.3, 1.7 Hz, 1H), 4.38-4.27 (m, 3H), 4.20-4.13 (m, 2H), 4.08 (dd, J = 13.2, 5.7 Hz, 1H), 3.94 (dd, J = 10.3, 6.3 Hz, 1H), 3.82 (s, 3H), 3.50 (dd, J = 9.7, 7.4 Hz, 1H), 2.73 (dd, J = 13.2, 5.2 Hz, 1H), 2.37 (s, 3H), 2.31 (s, 3H), 2.19 (s, 3H), 2.15 (s, 3H), 2.14 (s, 3H), 2.03 (s, 3H), 1.97 (s, 3H), 1.86 (dd, J = 13.2, 10.9 Hz, 1H).

¹³C-NMR (125 MHz, CDCl₃) δ 174.5, 173.6, 170.5, 170.1, 169.9, 169.8, 169.6, 167.3, 166.0, 165.5, 133.5, 133.3, 133.1, 129.8, 129.5, 129.4, 128.4, 128.3, 117.5, 98.6, 95.5, 77.2, 69.8, 68.9, 68.6, 68.6, 68.3, 68.3, 67.5, 67.0, 66.7, 62.4, 61.8, 57.0, 52.9, 38.7, 27.9, 25.9, 21.0, 20.9, 20.7, 20.7, 20.6.

HRMS (ESI⁺) [M+H]⁺ calcd for C₄₇H₅₆NO₂₂: 986.3288; found 986.3277.
The obtained compound was found to correspond to the spectrum in the following literature: Literature 4) J. Org. Chem. 2016, 81, 10600-10616.

### Example 27-1

### 4-O-Acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-D-galactopyranose (Compound represented by formula G-12)

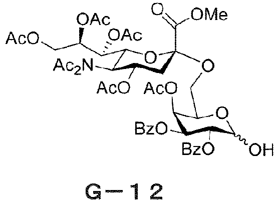

### (Substep V-10)

The pressure of a solution of prop-2-en-1-yl 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-α-D-galactopyranoside (compound represented by formula G-11) (29.00 g, 29.41 mmol), 1,3-dimethylbarbituric acid (9.19 g, 58.86 mmol), and triphenylphosphine (2.31 g, 8.81 mmol) in methanol (290 mL) was reduced. The solution was subjected to nitrogen substitution. This operation was repeated five times for deaeration. Palladium(II) acetate (0.66 g, 2.94 mmol) was then added, and the mixture was stirred at 40°C for 12 hours. After the completion of the reaction was checked by HPLC, toluene (580 mL) and water (1015 mL) were added. The resulting liquid was separated to obtain an organic layer. The organic layer was washed four times with 20% methanol water (580 mL) to remove 1,3-dimethylbarbituric acid into the aqueous layer. The organic layer was concentrated under reduced pressure to 58 mL, and toluene (435 mL) was added. The mixture was concentrated again under reduced pressure to 58 mL. The concentrated solution was admixed with toluene (383 mL), chloroform (197 mL), and neutral silica gel (silica gel 60N; particle size: 40 to 50 µm; 145 g; manufactured by KANTO CHEMICAL CO., INC.). The mixture was stirred for 30 minutes. The product was adsorbed on the silica gel, and then filtered. The silica gel solid phase containing the product was washed with toluene/chloroform mixture (2/1, 4350 mL) (the filtrate at the time of the wash was discarded). The target substance was desorbed from the silica gel solid phase containing the product by using ethyl acetate (870 mL). SH silica gel (29.00 g) was added to the resulting ethyl acetate solution. The mixture was stirred for 30 minutes, filtered, and then washed with ethyl acetate (145 mL) to obtain an ethyl acetate solution containing the target product. The resulting solution was concentrated under reduced pressure to 58 mL, and toluene (145 mL) was added. The mixture was concentrated again under reduced pressure to 58 mL. The concentrated solution was purified by silica gel column purification (silica gel 60N, 40-50 µm, 290 g; mobile phase hexane/ethyl acetate 50/50 to 30/70; manufactured by KANTO CHEMICAL CO., INC.) and the selected fractions were concentrated under reduced pressure to 29 mL. The concentrated solution was admixed with ethyl acetate (290 mL) and activated carbon (Shirasagi A, 14.5 g), stirred for 30 minutes, filtered, and then washed with ethyl acetate (87 mL) to obtain a purified ethyl acetate solution containing the target product. The resulting solution was concentrated under reduced pressure to give 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-D-galactopyranose (compound represented by formula G-12) (18.70 g, 67% yield) as a white foamy solid.

¹H-NMR (500 MHz, CDCl₃) Major isomer: δ 7.98-8.03 (m, 2H), 7.89 (t, 2H, J = 8.9 Hz), 7.52-7.48 (m, 2H), 7.39-7.34 (m, 4H), 5.92 (dd, 1H, J = 10.3, 2.9 Hz), 5.82 (d, 1H, J = 3.4 Hz), 5.68 (t, 1H, J = 2.3 Hz), 5.59-5.48 (m, 2H), 5.37 (td, 1H, J = 7.5, 2.5 Hz), 5.17 (d, 1H, J = 7.5 Hz), 5.02 (d, 1H, J = 10.5 Hz), 4.74-4.71 (m, 1H), 4.44-4.38 (m, 1H), 4.16-4.08 (m, 2H), 3.85 (s, 3H), 3.80 (m, 1H), 3.60-3.54 (m, 1H), 2.76 (dd, 1H, J = 13.0, 6.0 Hz), 2.38 (s, 3H), 2.33 (s, 3H), 2.31 (s, 3H), 2.15 (s, 3H), 2.12 (s, 3H), 2.04 (s, 3H), 1.98 (s, 3H), 1.93-1.87 (m, 1H).

¹³C-NMR (125 MHz, CDCl₃) α/β mixture: 174.5, 173.8, 173.6, 171.7, 171.0, 170.5, 170.3, 170.3, 169.9, 169.8, 169.8, 169.6, 167.6, 167.3, 166.3, 166.0, 165.6, 165.4, 133.3, 133.2, 133.2, 133.1, 129.8, 129.7, 129.5, 129.4, 129.4, 129.3, 129.0, 128.4, 128.3, 99.1, 98.8, 95.9, 91.0, 72.2, 71.6, 71.5, 69.9, 69.9, 69.3, 69.3, 68.8, 68.5, 68.1, 67.5, 67.5, 67.3, 67.0, 66.6, 62.9, 62.6, 62.4, 60.3, 57.2, 56.8, 53.0, 52.9, 38.6, 38.3, 27.9, 27.8, 26.1, 25.7, 21.0, 20.9, 20.8, 20.7, 20.7, 20.6, 20.5.

HRMS (ESI⁻) [M+HCOO]⁻ calcd for C₄₅H₅₂NO₂₄: 990.2885; found 990.2873.

### Example 27-2

### 4-O-Acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-D-galactopyranose (Compound represented by formula G-12)

Instead of Example 27-1, the compound represented by formula G-12 was synthesized as in this example. The pressure of a solution of prop-2-en-1-yl 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-α-D-galactopyranoside (compound represented by formula G-11) (5.0 g, 5.07 mmol) in tetrahydrofuran (25 mL) was reduced. The solution was subjected to nitrogen substitution. This operation was repeated three times for deaeration. Carbonylchlorohydridotris(triphenylphosphine) ruthenium(II) (24.1 mg, 0.03 mmol) was added and the mixture was stirred at 55 to 60°C for 3 hours. After the completion of the isomerization reaction was checked by HPLC, the mixture was cooled to 20 to 25°C. Water (5 mL) and 1,3-dibromo-5,5-dimethylhydantoin (870 mg, 3.04 mmol) were added (washed in with 5 mL of tetrahydrofuran), and the mixture was stirred at 20 to 30°C for 1 hour. After the completion of the reaction was checked by HPLC, 1% brine (25 mL) containing sodium sulfite (255 mg) and ethyl acetate (50 mL) were added, and the mixture was stirred and then liquid-separated. The resulting organic layer was washed with 5% brine (25 mL), and the organic layer was admixed with purified Shirasagi (2.0 g). The mixture was stirred for 1 hour and then filtered and washed with ethyl acetate (50 mL). The resulting filtrate was concentrated under reduced pressure to 6.5 mL, admixed with ethyl acetate (25 mL), and concentrated again under reduced pressure to 6.5 mL for the purpose of dehydration. 2-Propanol (100 mL) was added to the resultant, seed crystals were inoculated, and the mixture was stirred for 16 hours. After the crystals were found to precipitate, the resulting slurry solution was concentrated under reduced pressure to 30 mL. The slurry solution was cooled to 0 to 5°C and filtered, and the resulting solid was washed with 2-propanol (25 mL) at 0 to 5°C and dried under reduced pressure at 35°C to give 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-D-galactopyranose (compound represented by formula G-12) (3.76 g, yield 78%) as white crystals (α/β mixture).

¹H-NMR (500 MHz, CDCl₃) Major isomer: δ 7.98-8.03 (m, 2H), 7.89 (t, 2H, J = 8.9 Hz), 7.52-7.48 (m, 2H), 7.39-7.34 (m, 4H), 5.92 (dd, 1H, J = 10.3, 2.9 Hz), 5.82 (d, 1H, J = 3.4 Hz), 5.68 (t, 1H, J = 2.3 Hz), 5.59-5.48 (m, 2H), 5.37 (td, 1H, J = 7.5, 2.5 Hz), 5.17 (d, 1H, J = 7.5 Hz), 5.02 (d, 1H, J = 10.5 Hz), 4.74-4.71 (m, 1H), 4.44-4.38 (m, 1H), 4.16-4.08 (m, 2H), 3.85 (s, 3H), 3.80 (m, 1H), 3.60-3.54 (m, 1H), 2.76 (dd, 1H, J = 13.0, 6.0 Hz), 2.38 (s, 3H), 2.33 (s, 3H), 2.31 (s, 3H), 2.15 (s, 3H), 2.12 (s, 3H), 2.04 (s, 3H), 1.98 (s, 3H), 1.93-1.87 (m, 1H).
¹³C-NMR (125 MHz, CDCl₃) α/β mixture: 174.5, 173.8, 173.6, 171.7, 171.0, 170.5, 170.3, 170.3, 169.9, 169.8, 169.8, 169.6, 167.6, 167.3, 166.3, 166.0, 165.6, 165.4, 133.3, 133.2, 133.2, 133.1, 129.8, 129.7, 129.5, 129.4, 129.4, 129.3, 129.0, 128.4, 128.3, 99.1, 98.8, 95.9, 91.0, 72.2, 71.6, 71.5, 69.9, 69.9, 69.3, 69.3, 68.8, 68.5, 68.1, 67.5, 67.5, 67.3, 67.0, 66.6, 62.9, 62.6, 62.4, 60.3, 57.2, 56.8, 53.0, 52.9, 38.6, 38.3, 27.9, 27.8, 26.1, 25.7, 21.0, 20.9, 20.8, 20.7, 20.7, 20.6, 20.5.
HRMS (ESI⁻) [M+HCOO]⁻ calcd for C₄₅H₅₃NO₂₄: 991.2958; found 990.2789.

### Process for purifying 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-D-galactopyranose (compound represented by formula G-12) by crystallization

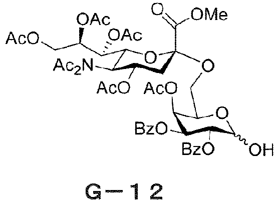

First, 4-O-Acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-D-galactopyranose (compound represented by formula G-12) (3.00 g, 3.17 mmol; sialyl moiety α/β ratio = 95.7/4.3) was dissolved in ethyl acetate (4 mL). Next, 2-propanol (60 mL) was added, and the mixture was stirred at 25°C and then concentrated under reduced pressure to 18 mL. The slurry solution was stirred at 0°C for 3 hours, and the precipitated crystals were then filtered. The filtered crystals were washed with cold 2-propanol (9 mL) and dried under reduced pressure at 40°C to give 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-D-galactopyranose (compound represented by formula G-12) (2.66 g, yield 88.7%; sialyl moiety α/β ratio => 99.9/N.D.) as white crystals.

### [Analytical conditions]

Column: CAPCELL PAK ADME φ4.6 × 150 mm, film thickness 3 µm
Wavelength: 220 nm
Oven: 40°C
Eluent: (A) 0.1% trifluoroacetic acid solution, (B) acetonitrile
Gradient: 0 to 150 min (B) concentration 40%
   150.1 min (B) concentration 95%
   155 min (B) concentration 95%
   155.1 min (B) concentration 40%
   160 min (B) concentration 40%
Flow rate: 1 mL/min
Injection: 5 µL

### Example 28

### 4-O-Acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-1-O-(2,2,2-trifluoro-N-phenylethanimidoyl)-D-galactopyranose (Compound represented by formula A-16)

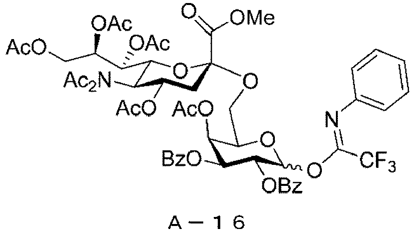

### (Substep V-11)

First, 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-D-galactopyranose (compound represented by formula G-12) (20.0 g, 21.1 mmol) was added to a 500-mL recovery flask. Dichloromethane (200 mL) and Molecular Sieves 4A powder (10 µm or less, 10.0 g) were added, and the mixture was cooled to 0°C. N-Methylimidazole (1.91 g, 23.3 mmol) and 2,2,2-trifluoro-N-phenylacetimidoyl chloride (4.39 g, 21.1 mmol) were added at the same temperature under nitrogen. The temperature was raised to room temperature, and the mixture was stirred for 24 hours. After the completion of the reaction was checked by HPLC, the reaction solution was filtered and washed with dichloromethane (100 mL). The filtrate was filtered through a neutral silica gel pad (silica gel 60N; particle size: 40 to 50 µm; 60 g; manufactured by KANTO CHEMICAL CO., INC.) packed with dichloromethane, and collected every 100 mL. The silica gel pad was washed with ethyl acetate/dichloromethane (1:9; 1000 mL, 200 mL each collected), and the selected fractions were concentrated under reduced pressure to give 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-1-O-(2,2,2-trifluoro-N-phenylethanimidoyl)-D-galactopyranose (compound represented by formula A-16) (20.1 g, yield: 85%) as a white amorphous material.

¹H-NMR (500 MHz, CDCl₃) δ 7.99 (dd, 2H, J = 8.3, 1.4 Hz), 7.91-7.88 (m, 2H), 7.59-7.56 (m, 1H), 7.51 (tt, 1H, J = 9.7, 2.4 Hz), 7.44-7.34 (m, 4H), 7.12 (t, 2H, J = 7.7 Hz), 7.01 (t, 1H, J = 7.4 Hz), 6.82 (brs, 1H), 6.43 (brs, 2H), 5.87-5.77 (m, 3H), 5.52 (td, 1H, J = 11.0, 5.0 Hz), 5.38-5.34 (m, 1H), 5.18 (dd, 1H, J = 8.6, 1.7 Hz), 4.95 (dd, 1H, J = 10.0, 2.0 Hz), 4.53 (brs, 1H), 4.29 (dd, 1H, J = 12.6, 2.9 Hz), 4.21-4.09 (m, 3H), 4.04 (dd, 1H, J = 10.0, 6.0 Hz), 3.84 (s, 3H), 3.53 (dd, 1H, J = 10.3, 7.4 Hz), 2.76 (dd, 1H, J = 12.9, 5.4 Hz), 2.39 (s, 3H), 2.31 (s, 3H), 2.18 (s, 3H), 2.17 (s, 3H), 2.14 (s, 3H), 2.02 (s, 3H), 1.98 (s, 3H), 1.86 (dd, 1H, J = 13.0, 11.0 Hz). ¹³C-NMR (125 MHz, CDCl₃) δ 174.5, 173.5, 170.5, 170.1, 169.8, 169.7, 169.6, 167.2, 165.5, 165.4, 165.3, 142.9, 133.6, 133.3, 129.8, 129.7, 129.6, 129.5, 129.0, 128.7, 128.6, 128.5, 128.4, 124.2, 119.0, 98.7, 98.5, 70.4, 69.8, 68.3, 68.2, 67.5, 67.5, 66.9, 66.7, 62.0, 61.7, 60.3, 56.9, 53.7, 52.9, 38.7, 31.7, 29.2, 27.9, 26.0, 25.8, 21.0, 21.0, 20.9, 20.8, 20.7, 20.6, 20.5.

HRMS (ESI⁺) [M+NH₄]⁺ calcd for C₅₂H₅₉F₃N₃O₂₂: 1134.3537; found 1134.3564.
The spectrum was found to correspond to the following literature: Literature 4) J. Org. Chem. 2016, 81, 10600-10616.

### <Synthesis of compound represented by formula A'-22>

A compound represented by formula A-22 wherein R₁ is methoxyphenyl (i.e., the compound represented by formula A'-22) was synthesized according to the following synthesis scheme 4.

### [Synthesis Scheme 4]

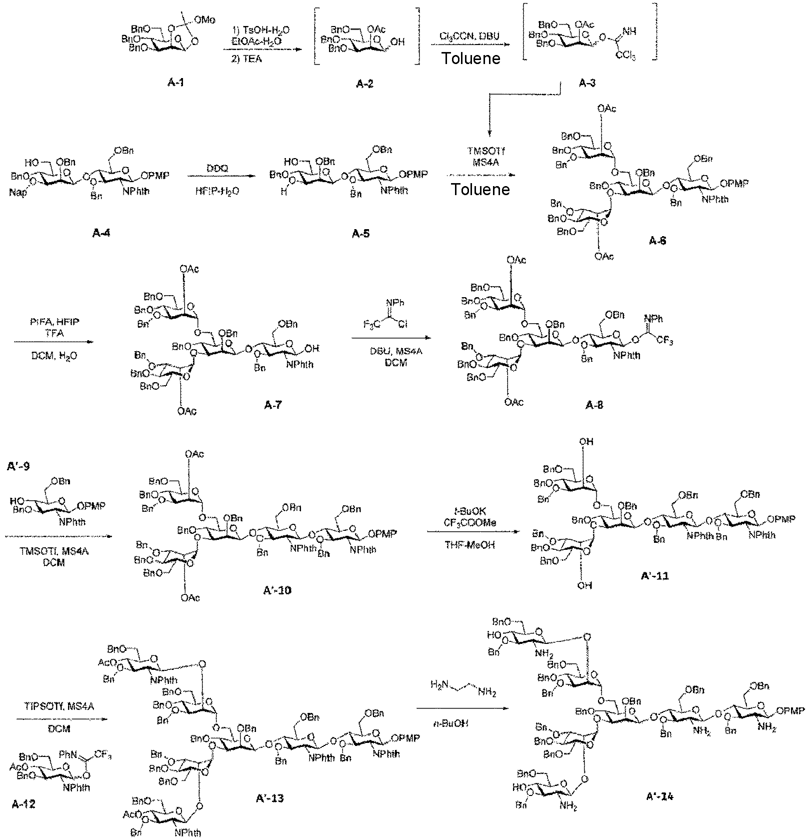

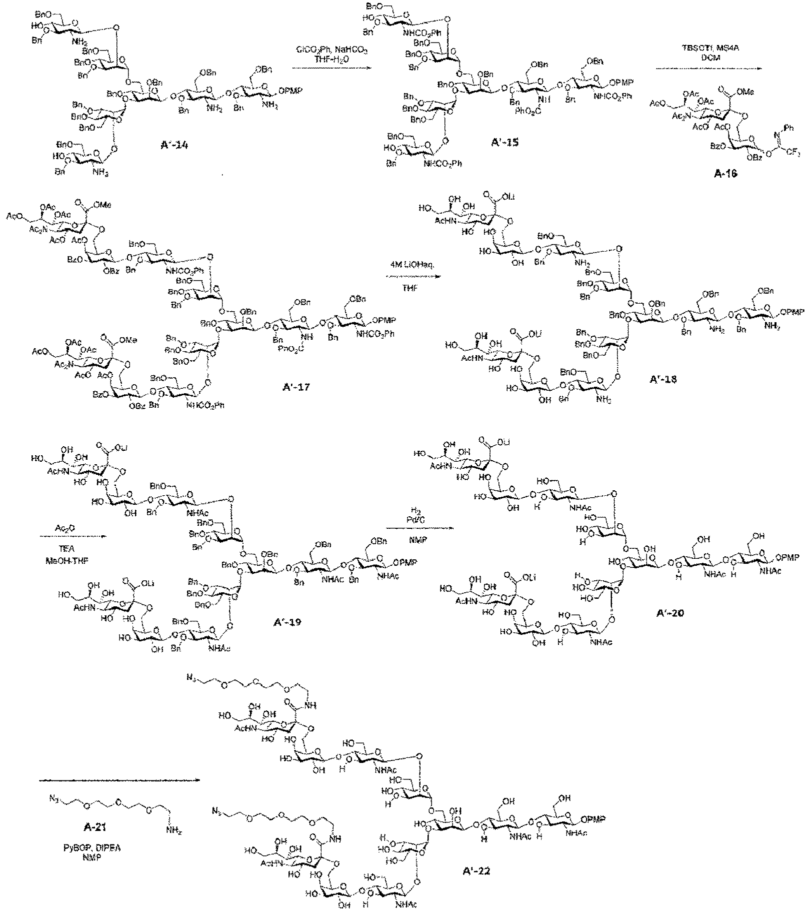

### Example 29

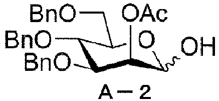

First, 3,4,6-tri-O-benzyl-1,2-O-(1-methoxyethyliden)-β-D-mannopyranose (compound represented by formula A-1) (40.0 g, 78.9 mmol) was added to a 1-L four-neck flask, and ethyl acetate (400 mL) was then added. Under a nitrogen atmosphere, water (2 mL) and p-TsOH·H₂O (45 mg, 0.237 mmol) were added at room temperature, and the mixture was stirred at the same temperature for 6 hours. After the completion of the reaction was checked by HPLC, triethylamine (7.99 g, 78.9 mmol) was added. The mixture was stirred overnight at the same temperature. After the completion of the acetyl group transfer was checked by HPLC, the reaction solution was admixed with 5% sodium bicarbonate water (400 mL) for liquid-separation. Then, 20% brine (200 mL) was added to the organic layer for liquid-separation. The resulting organic layer was concentrated under reduced pressure to 80 mL, and toluene (400 mL) was added. The mixture was concentrated under reduced pressure to a liquid volume of 80 mL. Toluene (400 mL) was added again, and the mixture was concentrated again under reduced pressure to a liquid volume of 80 mL. Dehydrated toluene (120 mL) was added to prepare, as a colorless solution, a toluene solution containing 2-O-acetyl-3,4,6-tri-O-benzyl-D-mannopyranose (compound represented by formula A-2).

### Example 30

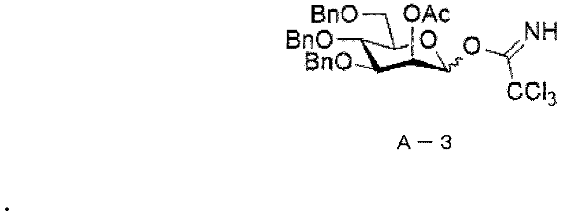

A toluene solution containing 2-O-acetyl-3,4,6-triO-benzyl-D-mannopyranose (compound represented by formula A-2) (78.9 mmol) was added to a 1-L flask, and trichloroacetonitrile (12 mL, 118 mmol) and DBU (119 µL, 0.789 mmol) were added. The mixture was stirred under nitrogen at 0°C for 2 hours. After the completion of the reaction was checked by HPLC, acetic acid (45 µL, 0.789 mmol) was added to the reaction solution at 0°C to prepare, as a brown solution, a toluene solution containing 2-O-acetyl-3,4,6-tri-O-benzyl-1-O-(2,2,2-trichloroethanimidoyl)-D-mannopyranose (compound represented by formula A-3) (78.9 mmol). This solution was used as it was in the next step.

### Example 31

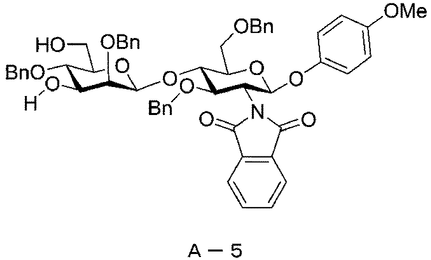

4-Methoxyphenyl 3,6-di-O-benzyl-2-deoxy-4-O-{2,4-di-O-benzyl-3-O-[(naphthalen-2-yl)methyl]-β-D-mannopyranosyl}-2-(1,3-dioxo-1,3-dihydro-2H-isoindol-2-yl)-β-D-glucopyranoside (compound represented by formula A-4) (20.0 g, 18.5 mmol) was added to a 1-L four-neck flask, dissolved in 1,1,1,3,3,3-hexafluoro-2-propanol (160 mL), and admixed with water (16 mL). 2,3-Dichloro-5,6-dicyano-p-benzoquinone (4.63 g, 20.4 mmol) was added at -20°C under nitrogen, and the mixture was stirred at the same temperature for 6 hours. After the completion of the reaction was checked by HPLC, an aqueous solution having sodium sulfite (1.17 g, 9.25 mmol) dissolved in water (10 mL) was added. The mixture was stirred overnight. Dichloromethane (300 mL) was added, and the mixture was liquid-separated and washed with an aqueous solution having sodium sulfite (4 g) and sodium bicarbonate (4 g) dissolved in water (200 mL). Then, the organic layer was concentrated under reduced pressure to 60 mL, and ethyl acetate (300 mL) was added. The resulting organic layer was washed with 5% sodium bicarbonate water (200 mL) and 20% brine (100 mL) and then concentrated under reduced pressure to 40 mL. Toluene (400 mL) was added, and the mixture was concentrated under reduced pressure to 40 mL. Toluene (100 mL) was added, and silica gel 60N (particle size: 40 to 50 µm; 60 g; manufactured by KANTO CHEMICAL CO., INC.) was added. Under stirring, heptane (40 mL) was added dropwise over 30 minutes to adsorb the target substance onto the silica gel, and the mixture was then filtered. The silica gel was washed with toluene/heptane (7/2, 400 mL) (the filtrate was discarded), and then the target substance was desorbed with ethyl acetate/toluene (1/1, 700 mL). The filtrate was concentrated under reduced pressure to give 4-methoxyphenyl 3,6-di-O-benzyl-2-deoxy-4-O-(2,4-di-O-benzyl-β-D-mannopyranosyl)-2-(1,3-dioxo-1,3-dihydro-2H-isoindol-2-yl)-β-D-glucopyranoside (compound represented by formula A-5) (17.8 g, yield: >99%) as a white amorphous material.

¹H-NMR (500 MHz, CDCl₃) δ 7.67 (br, 4H), 7.27-7.37 (m, 15H), 6.89-6.96 (m, 5H), 6.83 (ddd, J = 9.5, 4.0, 2.5 Hz, 2H), 6.70 (ddd, J = 9.0, 4.0, 2.5 Hz, 2H), 5.64 (d, J = 9.0 Hz, 1H), 5.02 (d, J = 11.5 Hz, 1H), 4.94 (d, J = 12.0 Hz, 1H), 4.82 (d, J = 11.5 Hz, 1H), 4.70 (d, J = 12.0 Hz, 1H), 4.66 (d, J = 12.0 Hz, 1H), 4.62 (s, 1H), 4.57 (d, J = 11.0 Hz, 1H), 4.51 (d, J = 12.0 Hz, 1H), 4.42-4.46 (m, 2H), 4.33 (dd, J = 10.5, 8.5 Hz, 1H), 4.09 (t, J = 9.0 Hz, 1H), 3.69-3.79 (m, 4H), 3.71 (s, 3H), 3.65 (d, J = 3.5 Hz, 1H), 3.51 (td, J = 9.0, 3.5 Hz, 1H), 3.45 (t, J = 9.0 Hz, 1H), 3.44-3.48 (m, 1H), 3.17 (m, 1H), 2.29 (d, J = 9.0 Hz, 1H), 1.92 (t, J = 6.0 Hz, 1H).

¹³C-NMR (125 MHz, CDCl₃) δ 155.6, 151.0, 138.54, 138.46, 138.3, 137.8, 134.1, 131.8, 128.8, 128.6, 128.3, 128.21, 128.19, 128.15, 128.1, 128.0, 127.5, 127.4, 123.6, 118.9, 114.6, 101.5, 97.9, 79.1, 78.5, 77.3, 76.7, 75.6, 75.4, 75.3, 74.9, 74.8, 74.4, 73.9, 68.5, 62.5, 55.8.

HRMS (ESI⁻) [M-H]⁻calcdforC₅₅H₅₄NO₁₃: 936.3601; found 936.3592.
The spectrum was found to correspond to the following literature:
Literature 1) Org. Biomol. Chem., 2018, 16, 4720-4727.

Further, the reaction conditions were changed, and the compound represented by formula A-5 was synthesized from the compound represented by formula A-4. The table below shows the respective reaction conditions and the yields of the compound represented by formula A-5 (as measured by HPLC).

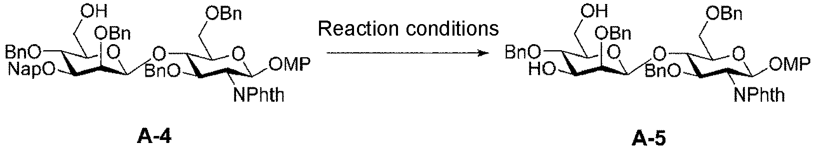

**[Table 1]**

| Entry | Reaction Conditions | HPLC PAR (Compound of formula A-5) (%) |
|---|---|---|
| 1 | DDQ, KH₂PO₄, CH₂Cl₂, H₂O, Room Temperature | 85.5 |
| 2 | DDQ, HFIP, H₂O, 0°C | 94.0 |
| 3 | 4M HCl/EtOAc, HFIP, CH₂Cl₂, Room Temperature | 19.2 |
| 4 | H₂, Pd/C, EtOH, THF, 50°C | 43.7 |

| | | |
|---|---|---|
| - Entry 1: the reaction proceeded in moderate yield under the conventional method conditions. - Entry 2: the reaction proceeded in high yield by using HFIP. - Entries 3 and 4: acidic and hydrogenolytic deprotection resulted in low yields. | | |

### Example 32

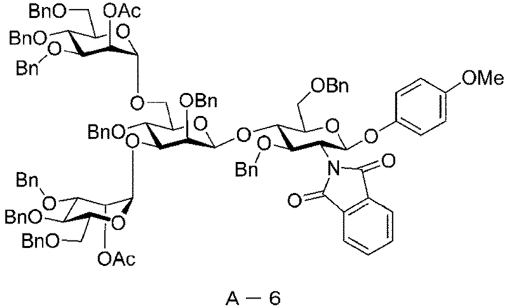

4-Methoxyphenyl 3,6-di-O-benzyl-2-deoxy-4-O-(2,4-di-O-benzyl-β-D-mannopyranosyl)-2-(1,3-dioxo-1,3-dihydro-2H-isoindol-2-yl)-β-D-glucopyranoside (compound represented by formula A-5 (17.8 g, 18.9 mmol) and a toluene solution containing 2-O-acetyl-3,4,6-tri-O-benzyl-1-O-(2,2,2-trichloroethanimidoyl)-D-mannopyranose (compound represented by formula A-3) (66.1 mmol equivalents) were added to a 1-L four-neck flask, and were admixed with toluene (178 mL) and Molecular Sieves 4A powder (10 µm or less, 2.7 g). Trimethylsilyl trifluoromethanesulfonate (0.683 mL, 3.78 mmol) was added dropwise at -20°C under nitrogen over 1 hour, and the mixture was stirred at the same temperature for 1 hour. After the completion of the reaction was checked by HPLC, triethylamine (1.06 mL, 7.56 mmol) was added, and the mixture was stirred at room temperature for 30 minutes. The reaction solution was filtered through Celite and then washed with acetonitrile (178 mL). The filtrate was concentrated under reduced pressure to give concentrated residue (the concentrated residue was divided into two portions, i.e., 12.8-g and 5.0-g raw material portions). The 12.8-g raw material portion of the residue was admixed with acetonitrile (256 mL (the 5.0-g raw material portion was similarly treated) and then admixed with silica gel 120RP-18 (particle size: 40 to 50 µm; 38.4 g; manufactured by KANTO CHEMICAL CO., INC.) for reversed phase. Water (128 mL) was added dropwise over 30 minutes to adsorb the target substance onto the solid phase, and the mixture was then filtered. The solid phase was sequentially washed with acetonitrile/water (2/1, 1.53 L) and acetonitrile/water (3/1, 512 mL) (the filtrate was discarded), and then the target substance was desorbed with acetonitrile (1.02 L). The filtrate was concentrated under reduced pressure to give, as a white amorphous material, 4-methoxyphenyl 2-O-acetyl-3,4,6-tri-O-benzyl-α-D-mannopyranosyl-(1→3)-[2-O-acetyl-3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranoside (compound represented by formula A-6) (19.6 g (separately synthesized product 8.1 g), isolation yield 77%).

¹H-NMR (500 MHz, CDCl₃) δ 7.63-6.60 (m, 58H), 5.54 (d, J = 8.4 Hz, 1H), 5.49 (dd, J = 3.2, 2.0 Hz, 1H), 5.34 (brt, J = 3.2 Hz, 1H), 5.13 (brd, J = 1.6 Hz, 1H), 5.05 (d, J = 12.0 Hz, 1H), 4.91-4.78 (m, 6H), 4.69 (d, J = 11.2 Hz, 1H), 4.63-4.55 (m, 6H), 4.48-4.32 (m, 9H), 4.21 (t, J = 11.2 Hz, 2H), 4.07 (t, J = 9.6 Hz, 1H), 4.00-3.73 (m, 9H), 3.70 (s, 3H), 3.68-3.51 (m, 8H), 3.44 (brd, J = 8.0 Hz, 1H), 3.21 (brd, J = 9.6 Hz, 1H), 2.10 (s, 3H), 1.86 (s, 3H).

¹³C-NMR (125 MHz, CDCl₃) δ 170.2, 170.0, 155.4, 151.0, 138.9, 138.7, 138.6, 138.5, 138.2, 138.1, 138.0, 137.9, 133.7, 131.7, 128.7, 128.5, 128.4, 128.3, 128.2, 127.9, 127.9, 127.8, 127.7, 127.6, 127.5, 127.5, 127.1, 123.4, 118.8, 114.4, 101.9, 99.8, 98.5, 97.7, 81.3, 79.5, 78.2, 78.0, 77.9, 76.9, 76.6, 75.2, 75.2, 75.1, 74.9, 74.7, 74.5, 74.4, 74.2, 74.2, 73.6, 73.5, 73.4, 72.4, 72.0, 71.9, 71.4, 69.4, 69.4, 68.8, 68.8, 68.3, 66.7, 55.7, 21.2, 20.9.

HRMS (ESI⁺) [M+NH₄]⁺calcd for C₁₁₃H₁₁₉N₂O₂₅: 1903.8096; found 1903.8075.

### Example 33

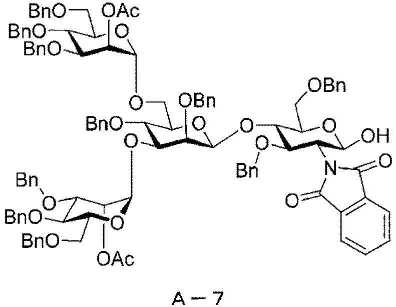

4-Methoxyphenyl 2-O-acetyl-3,4,6-tri-O-benzyl-α-D-mannopyranosyl-(1→3)-[2-O-acetyl-3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranoside (compound represented by formula A-6) (27.7 g, 14.6 mmol) was added to a 1-L four-neck flask, and was admixed with dichloromethane (222 mL), 1,1,1,3,3,3-hexafluoro-2-propanol (139 mL), and water (14 mL). [Bis(trifluoroacetoxy)iodo]benzene (12.6 g, 29.2 mmol) and trifluoroacetic acid (3.36 mL, 43.8 mmol) were added at -2°C under nitrogen, and the mixture was stirred at the same temperature for 21 hours. After the completion of the reaction was checked by HPLC, the mixture was washed twice with an aqueous solution having sodium bicarbonate (13.9 g) and sodium sulfite (13.9 g) dissolved in water (277 mL), and then 20% brine (138 mL) was added for washing the mixture. The resulting organic layer was concentrated under reduced pressure to 55 mL and then admixed with toluene (693 mL), and the mixture was concentrated again under reduced pressure to 55 mL. Toluene (97 mL) and dichloromethane (42 mL) were added, and silica gel 60N (particle size: 40 to 50 µm; 83 g; manufactured by KANTO CHEMICAL CO., INC) was added. Heptane (166 mL) was added dropwise over 30 minutes to adsorb the target substance onto the silica gel, and the mixture was then filtered. Dichloromethane/heptane (1/3, 277 mL) was used for washing (the filtrate was discarded), and the target substance was desorbed from the solid phase with ethyl acetate/dichloromethane (1/4, 970 mL). The filtrate was concentrated under reduced pressure to give, as a white amorphous material, 2-O-acetyl-3,4,6-tri-O-benzyl-α-D-mannopyranosyl-(1→3)-[2-O-acetyl-3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranose (compound represented by formula A-7) (28.1 g, yield >99%).

¹H-NMR (500 MHz, CDCl₃) σ 7.73-7.44 (m, 4H), 7.40-7.07 (m, 44H), 6.84-6.60 (m, 6H), 5.48 (dd, J = 3.2, 2.0 Hz, 1H), 5.36 (brt, J = 2.8 Hz, 1H), 5.21 (brs, 1H), 5.13 (d, J = 1.6 Hz, 1H), 5.00 (d, J = 11.6 Hz, 1H), 4.88-4.76 (m, 5H), 4.70 (d, J = 11.6 Hz, 1H), 4.63-4.36 (m, 14H), 4.26-4.19 (m, 2H), 4.10-3.43 (m, 20H), 3.26-3.19 (m, 2H), 2.09 (s, 3H), 1.89 (s, 3H).

¹³C-NMR (125 MHz, CDCl₃) σ 170.1, 167.9, 149.5, 138.6, 138.6, 138.5, 138.4, 138.3, 137.9, 137.9, 137.7, 133.6, 131.5, 128.6, 128.3, 128.2, 128.2, 128.0, 127.9, 127.8, 127.7, 127.6, 127.4, 127.4, 127.3, 126.9, 123.1, 116.1, 101.0, 99.5, 97.9, 92.8, 80.9, 78.3, 78.0, 77.8, 76.0, 74.9, 74.9, 74.8, 74.5, 74.2, 74.1, 74.0, 73.9, 73.5, 73.4, 73.2, 72.2, 71.8, 71.6, 71.2, 69.1, 68.6, 68.3, 68.2, 66.5, 57.5, 21.0, 20.8.

HRMS (ESI⁺) [M+NH₄]⁺calcd for C₁₀₆H₁₁₃N₂O₂₄⁺: 1798.7711; found 1798.7690.

### Example 34

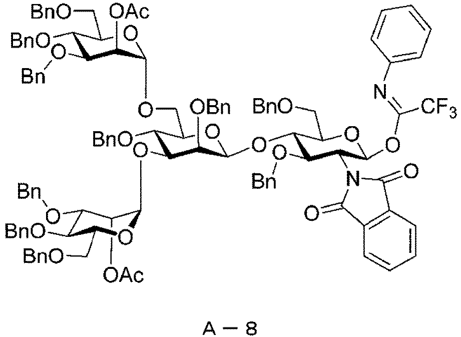

2-O-Acetyl-3,4,6-tri-O-benzyl-α-D-mannopyranosyl-(1→3)-[2-O-acetyl-3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranose (compound represented by formula A-7) (28.1 g, 15.7 mmol) was added to a 1-L four-neck flask, admixed with dichloromethane (422 mL) and Molecular Sieves 4A powder (10 µm or less, 14.1 g), and cooled to 0°C. N-Methylimidazole (1.51 mL, 18.8 mmol) and 2,2,2-trifluoro-N-phenylacetimidoylchloride (2.74 mL, 17.2 mmol) were added at the same temperature under nitrogen, and the mixture was stirred for 24 hours. Since the completion of the reaction could not be checked by HPLC, N-methylimidazole (125 µL, 1.57 mmol) and 2,2,2-trifluoro-N-phenylacetimidoylchloride (250 µL, 1.57 mmol) were added, and the mixture was stirred at 0°C for 2 hours. The reaction solution was filtered through a neutral silica gel pad (silica gel 60N; particle size: 40 to 50 µm; 84 g; manufactured by KANTO CHEMICAL CO., INC.) packed with dichloromethane. The silica gel pad was washed with 10% ethyl acetate/dichloromethane (840 mL, 140 mL each collected), and the main residue was concentrated under reduced pressure to give, as a white amorphous material, 2-O-acetyl-3,4,6-tri-O-benzyl-α-D-mannopyranosyl-(1→3)-[2-O-acetyl-3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-1-O-(2,2,2-trifluoro-N-phenylethanimidoyl)-β-D-glucopyranose (compound represented by formula A-8) (28.2 g, yield 91%).

¹H-NMR (500 MHz, CDCl₃) σ 7.64 (m, 4H), 7.41-7.09 (m, 47H), 7.01 (t, J = 8.0 Hz, 1H), 6.83 (d, J = 6.8 Hz, 2H), 6.69-6.59 (m, 5H), 5.49 (dd, J = 3.2, 2.0 Hz, 1H), 5.35 (brt, J = 2.0 Hz, 1H), 5.13 (brd, J = 1.6 Hz, 1H), 5.02 (d, J = 8.0 Hz, 1H), 4.91-4.78 (m, 5H), 4.69 (d, J = 11.6 Hz, 1H), 4.63-4.53 (m, 6H), 4.48-4.36 (m, 8H), 4.36-4.06 (m, 5H), 3.98 (dd, J = 9.2, 3.2 Hz, 1H), 3.95-3.61 (m, 13H), 3.57-3.44 (m, 4H), 3.16 (brd, J = 9.6 Hz, 1H), 2.10 (s, 3H), 1.84 (s, 3H).

¹³C-NMR (125 MHz, CDCl₃) σ 171.1, 170.0, 169.8, 138.6, 138.5, 138.4, 138.3, 137.9, 137.9, 137.8, 137.8, 137.7, 137.6, 133.6, 131.4, 129.0, 128.6, 128.5, 128.4, 128.3, 128.3, 128.2, 128.1, 128.0, 127.9, 127.8, 128.8, 127.7, 127.7, 127.7, 127.6, 127.5, 127.5, 127.4, 127.3, 127.3, 126.9, 125.2, 123.3, 101.6, 99.6, 98.2, 81.1, 78.6, 78.0, 77.7, 77.2, 76.0, 75.5, 75.0, 74.9, 74.9, 74.8, 74.7, 74.5, 74.3, 74.2, 74.0, 73.5, 73.3, 73.3, 72.2, 71.8, 71.8, 71.2, 69.3, 68.6, 68.6, 68.1, 66.4, 60.3, 54.6, 21.4, 21.0, 21.0, 20.7, 14.1.

HRMS (ESI⁺) [M+NH₄]⁺calcd for C₁₁₄H₁₁₇F₃N₃O₂₄⁺: 1969.8007; found 1969.7983.

### Example 35

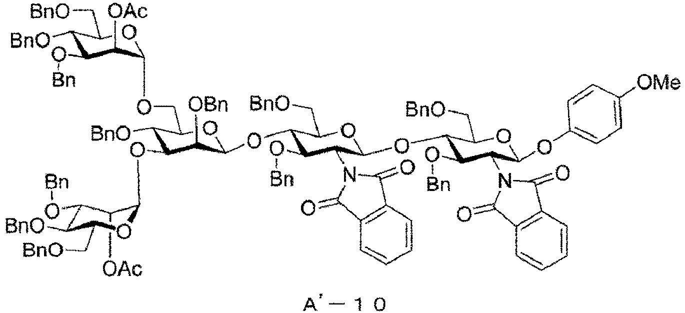

2-O-Acetyl-3,4,6-tri-O-benzyl-α-D-mannopyranosyl-(1->3)-[2-O-acetyl-3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-1-O-(2,2,2-trifluoro-N-phenylethanimidoyl)-β-D-glucopyranose (compound represented by formula A-8) (28.2 g, 14.4 mmol) and 4-methoxyphenyl 3,6-di-O-benzyl-2-deoxy-2-(1,3-dioxo-1,3-dihydro-2H-isoindol-2-yl)-β-D-glucopyranoside (compound represented by formula A'-9) (10.3 g, 17.3 mmol) were added to a 1-L four-neck flask, and were admixed with dichloromethane (424 mL) and Molecular Sieves 4A powder (8.50 g). Trimethylsilyl trifluoromethanesulfonate (392 µL, 2.16 mmol) was added dropwise at -7°C under nitrogen over 5 minutes, and the mixture was stirred at the same temperature for 1 hour. After the completion of the reaction was checked by HPLC, triethylamine (802 µL, 5.76 mmol) was added, and the mixture was stirred at room temperature for 30 minutes. The reaction solution was filtered through Celite and washed with acetonitrile (283 mL). The filtrate was concentrated under reduced pressure to 56 mL, and was admixed with acetonitrile (283 mL) and silica gel 120RP-18 (particle size: 40 to 50 µm; 85 g; manufactured by KANTO CHEMICAL CO., INC.) for reversed phase. Water (254 mL) was added dropwise over 30 minutes to adsorb the target substance onto the solid phase, and the mixture was then filtered. The solid phase was sequentially washed with acetonitrile/water (4/3, 283 mL) and acetonitrile/water (3/1, 848 mL) (the filtrate was discarded), and the target substance was desorbed with acetonitrile (1.13 L). The filtrate was concentrated under reduced pressure and azeotroped twice with toluene (283 mL) to give, as a white amorphous material, 4-methoxyphenyl 2-O-acetyl-3,4,6-tri-O-benzyl-α-D-mannopyranosyl-(1→3)-[2-O-acetyl-3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranosyl-(1→4)-3,6-di-O-benzyl-2-deoxy-(1,3-dioxo-1,3-dihydro-2H-isoindol-2-yl)-β-D-glucopyranoside (compound represented by formula A'-10) (31.0 g, 91%).

¹H-NMR (500 MHz, CDCl₃) σ 7.78-7.48 (m, 8H), 7.42 (d, J = 7.6 Hz, 2H), 7.35-7.01 (m, 49H), 6.98-6.84 (m, 4H), 6.77-6.53 (m, 9H), 5.50 (dd, J = 3.2, 1.6 Hz, 1H), 5.42 (d, J = 8.4 Hz, 1H), 5.32 (dd, J = 2.8, 2.0 Hz, 1H), 5.21 (dt, J = 8.4, 4.4 Hz, 1H), 5.14 (d, J = 1.2 Hz, 1H), 5.04 (d, J = 12.0 Hz, 1H), 4.88-4.76 (m, 6H), 4.68 (d, J = 6.8 Hz, 1H), 4.63-4.60 (m, 3H), 4.57-4.51 (m, 4H), 4.48-4.32 (m, 11H), 4.25-4.13 (m, 4H), 4.06 (dt, J = 10.0, 4.0 Hz, 1H), 4.00-3.57 (m, 19H), 3.55-3.34 (m, 5H), 3.18 (brt, J = 9.2 Hz, 2H), 2.09 (s, 3H), 1.80 (s, 3H)

¹³C-NMR (CDCl₃) σ 170.2, 169.9, 168.2, 167.5, 155.3, 150.9, 138.9, 138.8, 138.7, 138.6, 138.4, 138.1, 138.1, 138.0, 138.0, 137.9, 133.9, 133.7, 133.6, 131.9, 131.7, 131.6, 128.7, 128.5, 128.5, 128.5, 128.4, 128.3, 128.2, 127.9, 127.9, 127.8, 127.8, 127.7, 127.6, 127.5, 127.4, 127.0, 123.6, 123.3, 123.2, 118.7, 114.3, 102.1, 99.7, 98.4, 97.5, 97.2, 81.2, 79.8, 78.2, 78.0, 77.8, 77.4, 76.7, 76.1, 75.1, 75.1, 74.9, 74.7, 74.7, 74.6, 74.3, 74.1, 73.6, 73.4, 73.2, 72.7, 72.4, 72.0, 71.9, 71.3, 69.1, 68.8, 68.2, 68.1, 67.7, 66.6, 56.6, 55.7, 55.6, 31.1, 21.1, 20.8.

HRMS (ESI⁺) [M+HNEt₃]⁺ calcd for C₁₄₇H₁₅₆N₃O₃₁⁺: 2459.0717; found 2459.0720.

### Example 36

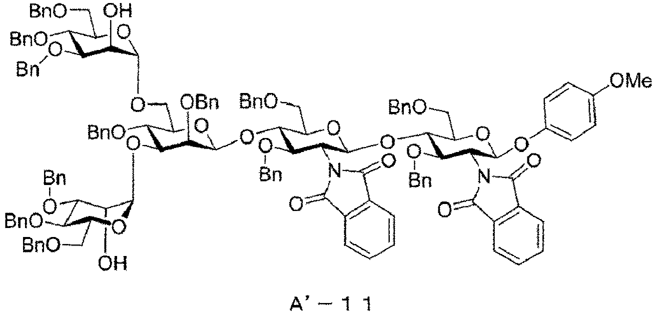

4-Methoxyphenyl 2-O-acetyl-3,4,6-tri-O-benzyl-α-D-mannopyranosyl-(1→3)-[2-O-acetyl-3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranosyl-(1→4)-3,6-di-O-benzyl-2-deoxy-(1,3-dioxo-1,3-dihydro-2H-isoindol-2-yl)-β-D-glucopyranoside (compound represented by formula A'-10) 31.0 g, 13.1 mmol) was added to a 1-L four-neck flask, and was admixed with tetrahydrofuran (124 mL), methanol (62 mL) and methyl trifluoroacetate (1.31 mL, 13.1 mmol). After stirring at room temperature under nitrogen for 5 minutes, a tetrahydrofuran solution containing 1 M potassium t-butoxide (6.6 mL, 6.6 mmol) was added. The mixture was stirred at 50°C for 2 hours. After the completion of the reaction was checked by HPLC, the mixture was cooled to room temperature. Acetic acid (754 µL, 13.1 mmol) was added, and ethyl acetate (311 mL) was then added. The resulting organic layer was washed twice with 5% brine (311 mL). The organic layer was concentrated under reduced pressure to 62 mL. Toluene (466 mL) was added, and the mixture was concentrated under reduced pressure to 62 mL. Toluene (466 mL) was added, and the mixture was concentrated again under reduced pressure to 62 mL. Toluene (155 mL) was added, and silica gel 60N (particle size: 40 to 50 µm; 93.3 g; manufactured by KANTO CHEMICAL CO., INC.) was added. Heptane (93 mL) was added dropwise over 30 minutes to adsorb the target substance onto the solid phase, and the mixture was then filtered. Toluene/heptane (7/3, 622 mL) was used for washing (the filtrate was discarded), and then the target substance was desorbed from the solid phase with ethyl acetate/toluene (1/2, 1.1 L). The resulting desorption solution was concentrated under reduced pressure to give, as a white amorphous material, 4-methoxyphenyl 3,4,6-tri-O-benzyl-α-D-mannopyranosyl-(1→3)-[3,4,6-tri-O-benzyl-O-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranosyl-(1→4)-3,6-di-O-benzyl-2-deoxy-(1,3-dioxo-1,3-dihydro-2H-isoindol-2-yl)-β-D-glucopyranoside (compound represented by formula A'-11) (27.7 g, 92%).

¹H-NMR (CDCl₃) σ 7.78-7.41 (m, 10H), 7.35-7.08 (m, 48H), 6.97-6.95 (m, 2H), 6.85-6.83 (m, 2H), 6.77-6.56 (m, 10H), 5.44 (d, J = 8.8 Hz, 1H), 5.24 (dd, J = 5.6, 2.8 Hz, 1H), 5.16 (d, J = 0.8 Hz, 1H), 5.03 (d, J = 11.6 Hz, 1H), 4.96 (d, J = 13.2 Hz, 1H), 4.93 (d, J = 1.2 Hz, 1H), 4.87 (d, J = 12.8 Hz, 1H), 4.83 (d, J = 10.8 Hz, 1H), 4.80 (d, J = 12.0 Hz, 1H), 4.73 (d, J = 11.2 Hz, 1H), 4.63-4.30 (m, 20H), 4.22-4.16 (m, 4H), 4.07 (ddd, J = 2.4, 5.6, 6.8 Hz, 1H), 3.97-3.36 (m, 25H), 3.19 (brt, J = 10 Hz, 2H), 2.38 (brs, 1H), 2.13 (d, J = 2.4 Hz, 1H)

¹³C-NMR (CDCl₃) σ 168.2, 167.4, 155.1, 150.8, 138.9, 138.8, 138.4, 138.4, 138.2, 138.2, 138.0, 137.9, 137.8, 137.7, 133.8, 133.5, 131.7, 131.4, 128.5, 128.4, 128.4, 128.2, 128.2, 128.1, 128.1, 127.8, 127.7, 127.7, 127.6, 127.5, 127.5, 127.4, 127.3, 127.2, 126.8, 123.5, 123.2, 123.0, 118.5, 114.1, 101.7, 101.4, 99.7, 97.3, 97.1, 81.6, 79.9, 79.6, 79.2, 78.2, 77.2, 76.8, 76.0, 74.9, 74.8, 74.7, 74.6, 74.5, 74.2, 74.0, 73.4, 73.2, 73.1, 72.6, 72.0, 71.9, 71.4, 71.1, 69.1, 68.7, 68.5, 67.9, 67.7, 67.6, 66.3, 56.4, 55.5, 55.4.

HRMS (ESI⁺) [M+HNEt₃]⁺ calcd for C₁₄₃H₁₅₂N₃O₂₉⁺: 2375.0506; found 2375.0514.

### Example 37

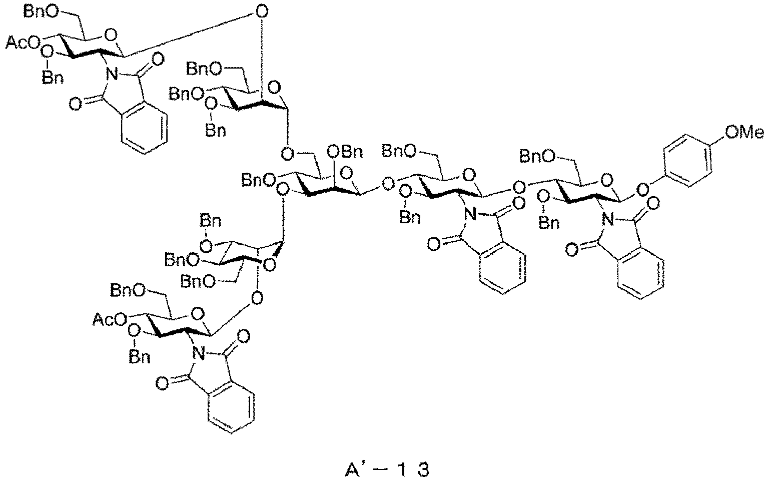

4-Methoxyphenyl 3,4,6-tri-O-benzyl-α-D-mannopyranosyl-(1→3)-[3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranosyl-(1→4)-3,6-di-O-benzyl-2-deoxy-(1,3-dioxo-1,3-dihydro-2H-isoindol-2-yl)-β-D-glucopyranoside (compound represented by formula A'-11) (27.7 g, 12.1 mmol) and 4-O-acetyl-3,6-di-O-benzyl-2-deoxy-2-(1,3-dioxo-1,3-dihydro-2H-isoindol-2-yl)-O-[2,2,2-trifluoro-N-phenylethanimidoyl]-β-D-glucopyranoside (compound represented by formula A-12) (25.7 g, 36.3 mmol) were added to a 1-L four-neck flask, and were admixed with dichloromethane (277 mL) and Molecular Sieves 4A powder (10 µm or less, 8.3 g). Triisopropylsilyl trifluoromethanesulfonate (973 µL, 3.63 mmol) was added dropwise at -62°C under nitrogen over 5 minutes, and the mixture was stirred at the same temperature for 8 hours. Since the completion of the reaction could not be checked by HPLC, triisopropylsilyl trifluoromethanesulfonate (486 µL, 1.81 mmol) was added dropwise over 5 minutes, and the mixture was stirred at the same temperature for 3 hours. Since the completion of the reaction could not be checked by HPLC, triisopropylsilyl trifluoromethanesulfonate (486 µL, 1.81 mmol) was added dropwise over 5 minutes, and the mixture was stirred at -20°C for 11 hours. Triethylamine (1.35 mL, 9.68 mmol) was added, and the mixture was stirred at room temperature for 30 minutes. The reaction solution was filtered through Celite and washed with acetonitrile (277 mL). The filtrate was concentrated under reduced pressure to 55 mL, and was admixed with acetonitrile (277 mL) and silica gel 120RP-18 (particle size: 40 to 50 µm; 83.1 g; manufactured by KANTO CHEMICAL CO., INC.) for reversed phase. Water (249 mL) was added dropwise over 30 minutes to adsorb the target substance onto the solid phase, and the mixture was then filtered. The solid phase was sequentially washed with acetonitrile/water (4/3, 277 mL) and acetonitrile/water (3/1, 831 mL) (the washing solution was discarded), and then the target substance was desorbed from the solid phase with acetonitrile (1.1 L). The desorption solution was concentrated under reduced pressure, ethyl acetate (554 mL) was added, and the mixture was concentrated again under reduced pressure to give, as a white amorphous material, 4-methoxyphenyl 4-O-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→3)-[4-O-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4) -3,6-di-O-benzyl-2-deoxy-(1,3-dioxo-1,3-dihydro-2H-isoindol-2-yl)-β-D-glucopyranosyl-(1→4)-3,6-di-O-benzyl-2-deoxy-(1,3-dioxo-1,3-dihydro-2H-isoindol-2-yl)-β-D-glucopyranoside (compound represented by formula A'-13) (39.1 g, 97%).
Note: When using t-butyl dimethylsilyl trifluoromethanesulfonate as a Lewis acid under a -78°C condition, the reaction rate was found to be improved. The reaction temperature was not raised (maintained at - 78°C), and the reaction proceeded with good reproducibility by adding 0.15 equivalents of the Lewis acid, until the completion of the reaction was checked.

¹H-NMR (CDCl₃) σ 7.85-7.41 (m, 17H), 7.41-6.6.86 (m, 73H), 6.81-6.75 (m, 3H), 6.73-6.56 (m, 7H), 5.43 (d, J = 8.0 Hz, 1H), 5.18 (d, J = 7.6 Hz, 1H), 5.05 (dt, J = 12.8, 9.6 Hz, 2H), 4.96-4.73 (m, 9H), 4.68-3.84 (m, 39H), 3.80-3.13 (m, 27H), 2.82 (dd, J = 11.2, 6.0 Hz, 1H), 2.77 (brd, J = 9.2 Hz, 1H), 2.70 (dt, J = 10.4, 4.0 Hz, 1H), 2.64 (dd, J = 10. 4, 7.2 Hz, 1H), 2.47 (dt, J = 9.6, 4.4 Hz, 1H), 1.91 (s, 3H), 1.86 (s, 3H)

¹³C-NMR (CDCl₃) σ 169.5, 169.4, 169.3, 168.4, 168.0, 167.4, 167.1, 155.1, 150.7, 138.8, 138.7, 138.6, 138.4, 138.3, 138.2, 138.0, 138.0, 137.9, 137.6, 133.5, 131.8, 131.7, 131.5, 131.4, 129.0, 128.8, 128.7, 128.6, 128.3, 128.2, 128.1, 128.0, 128.0, 127.9, 127.8, 127.8, 127.6, 127.6, 127.5, 127.4, 127.4, 127.2, 127.2, 127.1, 127.0, 126.8, 126.0, 125.2, 123.4, 123.2, 118.5, 114.2, 101.7, 98.4, 97.8, 97.3, 96.9, 96.3, 95.8, 80.5, 79.6, 78.0, 77.6, 77.2, 76.5, 76.4, 75.9, 75.0, 74.8, 74.5, 74.1, 73.9, 73.6, 73.5, 73.5, 73.4, 73.3, 73.2, 73.1, 72.8, 72.5, 72.4, 72.2, 72.1, 72.0, 71.9, 70.8, 70.3, 69.7, 69.5, 69.3, 67.9, 67.4, 66.8, 60.3, 56.5, 55.5, 55.4, 55.0, 54.9, 21.4, 21.0, 20.8, 20.8, 14.1.
HRMS (ESI⁺) [M+HNEt₃]⁺ calcd for C₂₀₃H₂₀₆N₅O₄₃⁺: 3401.4081; found 3401.4070.

### Example 38

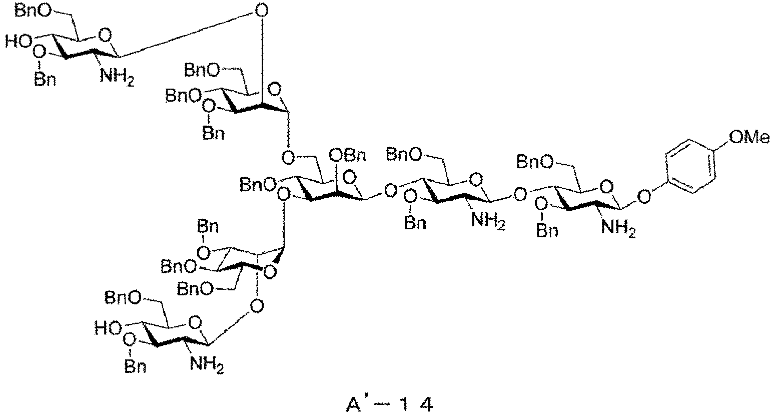

A toluene solution (72 mL, 2 v) containing 4-methoxyphenyl 4-0-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→3)-[4-O-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranosyl-(1→4)-3,6-di-O-benzyl-2-deoxy-(1,3-dioxo-1,3-dihydro-2H-isoindol-2-yl)-β-D-glucopyranoside (compound represented by formula A'-13) (36.1 g, 10.9 mmol) was added to a 1-L four-neck flask, admixed with 1-butanol (108 mL), and heated to 40°C under nitrogen. Ethylenediamine (108 mL) was added. Then, the temperature was raised to 90°C, and the mixture was stirred for 19 hours. After the completion of the reaction was checked by HPLC, the mixture was cooled to room temperature. The reaction solution was concentrated under reduced pressure to 72 mL, and was admixed with acetonitrile (361 mL) and silica gel 120RP-18 (particle size: 40 to 50 µm; 108 g; manufactured by KANTO CHEMICAL CO., INC.) for reversed phase. Water (361 mL) was added dropwise over 30 minutes to adsorb the target substance onto the solid phase, and the mixture was then filtered. Acetonitrile/water (1/1, 903 mL) was used for washing (the washing solution was discarded), and then the target substance was desorbed from the solid phase with acetonitrile/tetrahydrofuran (9/1, 1.4 L). The desorption solution was concentrated under reduced pressure to 72 mL, tetrahydrofuran (542 mL) was added, and the mixture was concentrated under reduced pressure to give, as a white amorphous material, 4-methoxyphenyl 2-amino-3,6-di-O-benzyl-2-deoxy-β-D-glucopyranosyl-(1→2)-3,4,6-tri-O-benzyl-α-D-mannopyranosyl-(1→3)-[2-amino-3,6-di-O-benzyl-2-deoxy-3-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranosyl-(1→4)-2-amino-3,6-di-O-benzyl-2-deoxy-β-D-glucopyranoside (compound represented by formula A'-14) (28.8 g, 97%).

¹H-NMR (CDCl₃) σ7.41-7.04 (m, 80H), 6.96 (d, J = 9.0 Hz, 2H), 6.78 (d, J = 9.0 Hz, 2H), 5.12-5.09 (m, 3H), 4.94-4.34 (m, 33H), 4.07-3.33 (m, 36H), 3.28-2.98 (m, 9H), 2.84-2.75 (m, 5H).

### Example 39

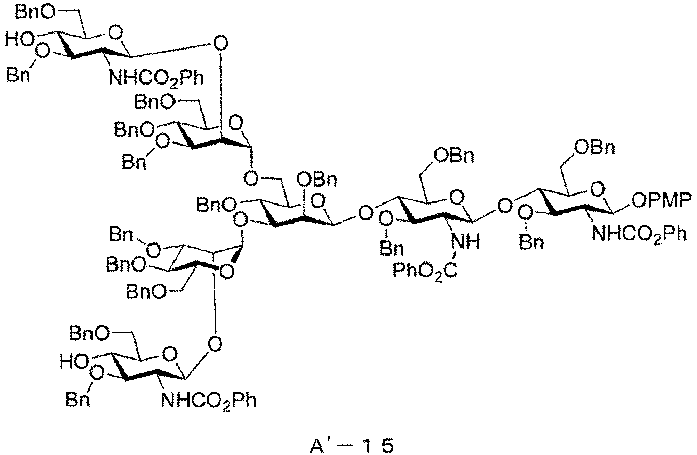

A solution of 4-methoxyphenyl 2-amino-3,6-di-O-benzyl-2-deoxy-β-D-glucopyranosyl-(1→2)-3,4,6-tri-O-benzyl-α-D-mannopyranosyl-(1→3)-[2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranosyl-(1→4)-2-amino-3,6-di-O-benzyl-2-deoxy-β-D-glucopyranoside (compound represented by formula A'-14) (31.3 g, 11.6 mmol) in tetrahydrofuran (63 mL) was added to a 1-L four-neck flask, and was admixed with an aqueous solution having tetrahydrofuran (313 mL) and sodium bicarbonate (4.78 g, 69.6 mmol) dissolved in water (157 mL). Phenyl chloroformate (5.26 mL, 51.0 mmol) was added, and the mixture was then stirred for 1 hour. After the completion of the reaction was checked by HPLC, ethyl acetate (313 mL) was added, and the resulting organic layer was washed twice with water (313 mL). The organic layer was washed with 20% brine (157 mL). The resulting organic layer was concentrated under reduced pressure to 62 mL, and admixed with toluene (313 mL), and the mixture was concentrated under reduced pressure to 62 mL. Toluene (157 mL) was added, and silica gel 60N (particle size: 40 to 50 µm; 93.3 g; manufactured by KANTO CHEMICAL CO., INC.) was added. Heptane (94 mL) was added dropwise over 30 minutes to adsorb the target substance onto the solid phase, and the mixture was then filtered. The solid phase was washed with toluene/heptane (7/3, 626 mL) (the washing solution was discarded), and then the target substance was desorbed from the solid phase with ethyl acetate/toluene (1/2, 1.1 L). The desorption solution was concentrated under reduced pressure to give, as a white amorphous material, 4-methoxyphenyl 3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→3)-[3.6-di-O-benzyl-2-deoxy-2-[(phenoxycarbonyl)amino]-β-D-glucopyranosyl-(1→2)-3,4,6-tri-O-benzyl-α-D-mannopyranosyl-(1→6)]-2, 4-di-O-benzyl-β-D-mannopyranosyl-(1→4)-3,6-di-O-benzyl-2-deoxy-[(phenoxycarbonyl)amino]-β-D-glucopyranosyl-(1→4)-3,6-di-O-benzyl-2-deoxy-[(phenoxycarbonyl)amino]-β-D-glucopyranoside (compound represented by formula A'-15) (34.8 g, 94%).

¹H-NMR (CDCl₃) σ 7.53-6.90 (m, 104H), 6.76 (d, J = 8.8 Hz, 2H), 6.31-5.72 (m, 2H), 5.19-4.19 (m, 42H), 4.19-3.11 (m, 41H), 2.89-2.69 (m, 3H).

¹³C-NMR (CDCl₃) interalia σ 155.1, 155.0, 154.8, 154.8, 154.7, 151.5, 151.2, 151.1, 151.0, 139.0, 138.9, 138.8, 138.7, 138.7, 138.6, 138.4, 138.3, 138.2, 138.1, 138.0, 137.6129.4, 129.3, 129.2, 128.7, 128.6, 128.6, 128.5, 128.4, 128.3, 128.3, 128.2, 128.1, 128.0, 127.9, 127.8, 127.8, 127.7, 127.6, 127.5, 127.5, 126.2, 125.7, 125.4, 125.3, 122.3, 121.9, 118.0, 114.6, 102.0, 99.4, 80.4, 79.8, 78.3, 77.8, 77.4, 75.3, 75.1, 74.9, 74.8, 74.7, 74.7, 74.6, 74.1, 73.8, 73.6, 73.4, 73.2, 72.7, 72.2, 71.3, 71.1, 70.6, 69.9, 69.5, 69.1, 57.8, 56.8, 55.8.

HRMS (ESI⁺) [M+HNEt₃]⁺ calcd for C₁₉₅H₂₁₀N₅O₄₁⁺: 3277.4496; found 3277.4492.

### Example 40

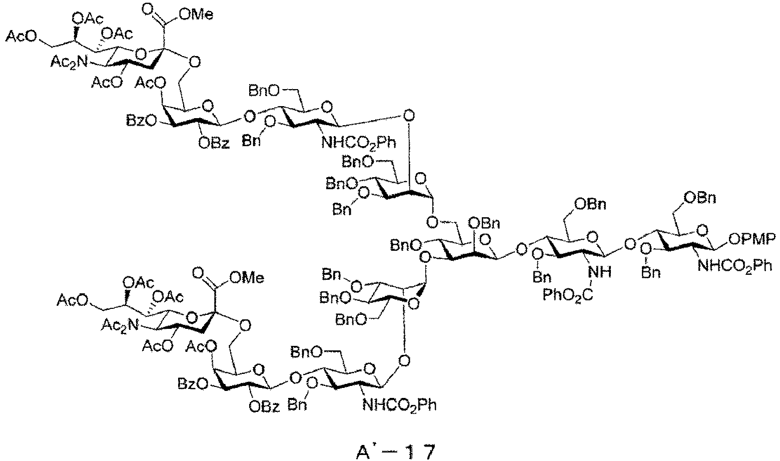

4-Methoxyphenyl 3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→3)-[3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-[(phenoxycarbonyl)amino]-β-D-glucopyranosyl-(1→4)-3,6-di-O-benzyl-2-deoxy-[(phenoxycarbonyl)amino]-β-D-glucopyranoside (compound represented by formula A'-15) (34.8 g, 10.9 mmol) and 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-1-O-(2,2,2-trifluoro-N-phenylethanimidoyl)-D-galactopyranose (compound represented by formula A-16) (34.3 g, 32.7 mmol) were added to a 1-L four-neck flask, and were admixed with dichloromethane (522 mL) and Molecular Sieves 4A powder (10 µm or less, 5.2 g). t-Butyl dimethylsilyl trifluoromethanesulfonate (706 µL, 3.27 mmol) was added dropwise at -20°C under nitrogen over 5 minutes, and the mixture was stirred at -20 to -16°C for 4 hours. After the completion of the reaction was checked by HPLC, triethylamine (777 µL, 4.36 mmol) was added, and the mixture was stirred at room temperature for 1 hour. The reaction solution was filtered through Celite and washed with acetonitrile (174 mL). The filtrate was concentrated under reduced pressure to 70 mL, and admixed with acetonitrile (348 mL), and the mixture was concentrated again under reduced pressure to 70 mL. Acetonitrile (348 mL) and silica gel 120RP-18 (particle size: 40 to 50 µm; 104 g; manufactured by KANTO CHEMICAL CO., INC.) for reversed phase were added. Water (313 mL) was added dropwise over 30 minutes to adsorb the target substance onto the solid phase, and the mixture was then filtered. The solid phase was sequentially washed with acetonitrile/water (4/3, 348 mL) and acetonitrile/water (3/1, 1.1 L) (the washing solution was discarded), and then the target substance was desorbed from the solid phase with acetonitrile (2.4 L). The desorption solution was concentrated under reduced pressure to give, as a white amorphous material, 4-methoxyphenyl 4,7,8,9-tetra-O-acetyl-3,5-dideoxy-5-(diacetylamino)-1-O-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1-+3)-[4,7,8,9-tetra-O-acetyl-3,5-dideoxy-5-(diacetylamino)-1-O-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-[(phenoxycarbonyl)amino]-β-D-glucopyranosyl-(1→4)-3,6-di-O-benzyl-2-deoxy-2-[(phenoxycarbonyl)amino]-β-D-glucopyranoside (compound represented by formula A'-17) (42.0 g, 76%).

¹H-NMR (CDCl₃) σ 8.01-7.85 (m, 18H), 7.50-6.80 (m, 104H), 6.78 (d, J = 9.2 Hz, 2H), 5.92 (dd, J = 10.8, 3.6 Hz, 1H), 5.82 (brd, J = 2.8 Hz, 1H), 5.76-5.26 (m, 21H), 5.19-5.07 (m, 6H), 5.04-4.08 (m, 65H), 4.00-3.14 (m, 67H), 3.85-3.76 (3H), 2.79-2.50 (m, 8H), 2.38-1.96 (m, 42H) .

¹³C-NMR (CDCl₃) σ 174.7, 174.6, 173.8, 171.9, 170.7, 170.5, 170.4, 170.3, 170.1, 170.0, 170.0, 169.9, 169.8, 167.8, 167.2, 166.2, 165.8, 165.5, 165.3, 165.2, 165.0, 155.3, 154.3, 151.5, 151.3, 151.1, 151.0, 139.1, 138.7, 138.4, 138.3, 138.1, 133.3, 133.3, 129.9, 129.8, 129.8, 129.7, 129.6, 129.5, 129.4, 129.3, 129.2, 128.7, 128.5, 128.4, 128.3, 128.2, 128.1, 128.0, 127.9, 127.7, 127.5, 127.4, 125.7, 125.4, 125.3, 121.9, 121.8, 121.7, 118.5, 114.6, 100.00, 99.4, 99.3, 99.1, 99.0, 95.5, 91.2, 77.4, 74.5, 74.2, 73.6, 73.5, 73.2, 73.0, 72.2, 72.0, 71.8, 71.7, 70.5, 70.1, 69.9, 69.5, 69.5, 68.6, 68.5, 68.3, 68.0, 67.7, 67.5, 67.2, 66.7, 66.6, 63.1, 62.6, 62.1, 57.4, 57.1, 57.0, 55.7, 55.0, 53.1, 53.1, 53.0, 38.9, 38.8, 32.3, 29.8, 28.126.7, 26.0, 25.9, 25.7, 21.1, 21.0, 20.7.

HRMS (ESI⁺) [M+HNEt₃+HNEt₃]²⁺ calcd for C₂₈₉H₃₂₄N₈O₈₃²⁺: 2618.5734; found 2618.5552.

### Example 41

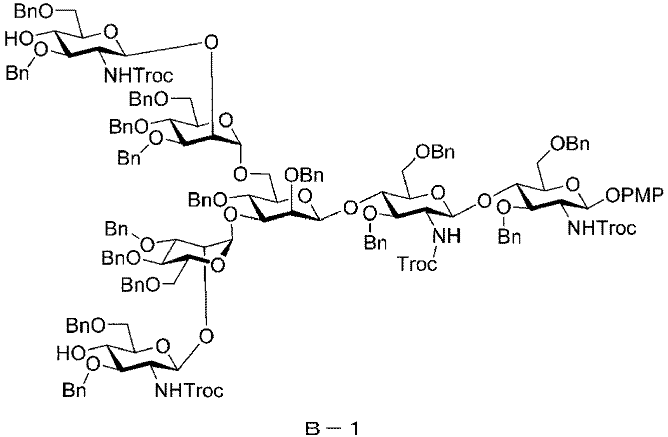

A solution of 4-methoxyphenyl 2-amino-3,6-di-O-benzyl-2-deoxy-β-D-glucopyranosyl-(1→2)-3,4,6-tri-O-benzyl-α-D-mannopyranosyl-(1→3)-[2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1-+4)-2-amino-3,6-di-O-benzyl-2-deoxy-β-D-glucopyranosyl-(1→4)-2-amino-3,6-di-O-benzyl-2-deoxy-β-D-glucopyranoside (compound represented by formula A'-14) (200 mg, 0.127 mmol) in cyclopentyl methyl ether (400 µL) was added to a 30-mL recovery flask, and admixed with tetrahydrofuran (2 mL), water (1 mL), and sodium bicarbonate (42 mg, 0.497 mmol). 2,2,2-Trichloroethyl chloroformate (44 µL, 0.325 mmol) was added dropwise over 10 minutes, and the mixture was then stirred for 3 hours. After the completion of the reaction was checked by HPLC, ethyl acetate (2 mL) was added, and the mixture was liquid-separated and washed twice with water (2 mL). The organic layer was washed with 20% brine (1 mL), dried over sodium sulfate, and then filtered and concentrated. The concentrated residue was purified by column chromatography (amount of silica: 20 g, developing solvent: toluene/ethyl acetate = 9/1 to 8/2), and the main residue was concentrated under reduced pressure to give, as a white amorphous material, 4-methoxyphenyl 3,6-di-O-benzyl-2-deoxy-2-{[(2,2,2-trichloroethoxy)carbonyl]amino}-β-D-glucopyranosyl-(1→2) -3, 4, 6-tri-O-benzyl-α-D-mannopyranosyl- (1→3) - [3, 6-di-O-benzyl-2-deoxy-2-{[(2,2,2-trichloroethoxy)carbonyl]amino}-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-{[(2,2,2-trichloroethoxy)carbonyl]amino}-β-D-glucopyranosyl-(1- 3)-3,6-di-O-benzyl-2-deoxy-{[(2,2,2-trichloroethoxy)carbonyl]amino}-β-D-glucopyranoside (compound represented by formula B-1) (147 mg, 58%).

¹H-NMR (CDCl₃) σ 7.38-7.05 (m, 84H), 6.90 (d, J = 9.2 Hz, 2H), 6.75 (d, J = 9.2 Hz, 2H), 5.48 (s, 1H), 5.27 (s, 1H), 5.03-4.22 (m, 47H), 4.04-3.03 (m, 43H), 2.88 (m, 1H), 2.71 (d, J = 2.4 Hz, 1H).

¹³C-NMR (CDCl₃) σ 155.2, 154.1, 153.8, 170.4, 151.4, 138.8, 138.7, 138.6, 138.5, 138.4, 138.2, 138.0, 137.9, 137.8, 137.6, 137.4, 128.6, 128.5, 128.5, 128.5, 128.3, 128.2, 128.2, 128.0, 128.0, 127.9, 127.9, 127.9, 127.8, 127.7, 127.7, 127.7, 127.6, 127.6, 127.5, 127.5, 127.4, 125.9, 118.2, 114.4, 101.3, 100.2, 98.8, 99.2, 95.7, 95.6, 95.5, 80.1, 79.3, 78.9, 78.2, 77.9, 77.2, 75.0, 74.8, 74.6, 74.5, 74.4, 73.8, 73.7, 73.6, 73.5, 73.2, 73.1, 73.0, 72.7, 72.2, 71.1, 70.9, 70.7, 69.9, 69.1, 68.6, 68.3, 68.3, 66.7, 57.3, 56.8, 56.3, 55.6, 55.0, 29.7.

HRMS (ESI⁺) [M+HNEt₃]⁺ calcd for C₁₇₉H₁₉₈Cl₁₂N₅O₄₁⁺: 3500.9801; found 3500.9820.

### Example 42

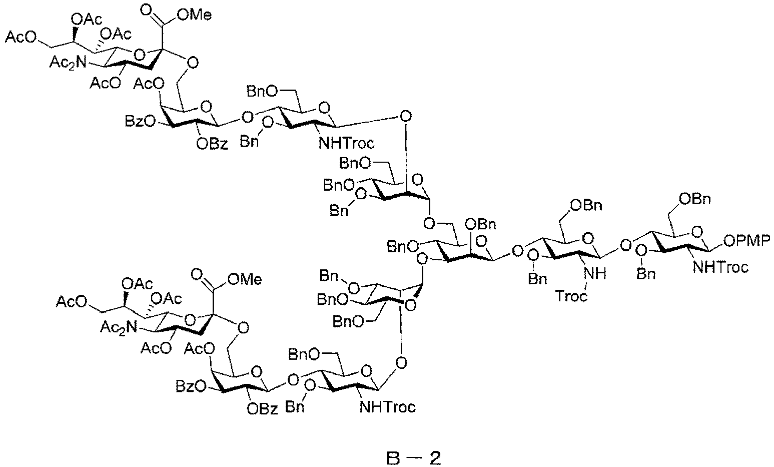

4-Methoxyphenyl 3,6-di-O-benzyl-2-deoxy-2-{[(2,2,2-trichloroethoxy)carbonyl]amino}-β-D-glucopyranosyl-(1→2) -3, 4, 6-tri-O-benzyl-α-D-mannopyranosyl- (1→3)-[3,6-di-O-benzyl-2-deoxy-2-{[(2,2,2-trichloroethoxy)carbonyl]amino}-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-{[(2,2,2-trichloroethoxy)carbonyl]amino}-β-D-glucopyranosyl-(1- 3)-3,6-di-O-benzyl-2-deoxy-{[(2,2,2-trichloroethoxy)carbonyl]amino}-β-D-glucopyranoside (compound represented by formula B-1) (147 mg, 43.0 µmol) and 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-1-O-(2,2,2-trifluoro-N-phenylethanimidoyl)-D-galactopyranose (compound represented by formula A-16) (145 mg, 0.130 mmol, 3.0 eq) were added to a 30-mL recovery flask, and were admixed with dichloromethane (2.2 mL) and Molecular Sieves 4A powder (10 µm or less, 22 mg). t-Butyl dimethylsilyl trifluoromethanesulfonate (2 µL, 8.7 µmol) was added dropwise at -20°C under nitrogen over 5 minutes, and the mixture was stirred overnight at -20°C to 0°C. After the completion of the reaction was checked by HPLC, triethylamine (2.4 µL, 44 µmol) was added, and the mixture was stirred at room temperature for 1 hour. The reaction solution was filtered through Celite and washed with dichloromethane (0.7 mL). The filtrate was liquid-separated twice with water (1.5 mL). The organic layer was dried over sodium sulfate, and then filtered and concentrated. The concentrated residue was purified by column chromatography (amount of silica: 20 g, developing solvent: heptane/ethyl acetate = 1/1 to 1/9), and the main residue was concentrated under reduced pressure to give, as a white amorphous material, 4-methoxyphenyl 4,7,8,9-tetra-O-acetyl-3,5-dideoxy-5-(diacetylamino)-1-O-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-{[(2,2,2-trichloroethoxy)carbonyl]amino}-β-D-glucopyranosyl-(1→2) -3, 4, 6-tri-O-benzyl-α-D-mannopyranosyl- (1→3) - [4,7,8,9-tetra-O-acetyl-3,5-dideoxy-5-(diacetylamino)-1-O-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-{[(2,2,2-trichloroethoxy)carbonyl]amino}-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-{[(2,2,2-trichloroethoxy)carbonyl]amino}-β-D-glucopyranosyl-(1- 3)-3,6-di-O-benzyl-2-deoxy-2-{[(2,2,2-trichloroethoxy)carbonyl] amino}-β-D-glucopyranoside (compound represented by formula B-2) (129 mg, 56.1%).

¹H-NMR (CDCl₃) σ 8.12 (d, J = 7.2 Hz, 1H), 8.01-7.86 (m, 12H), 7.53-6.95 (m, 90H), 6.76 (d, J = 9.2 Hz, 1H), 5.90 (dd, J = 3.2, 11.2 Hz, 1H), 5.82 (d, J = 2.8 Hz, 1H), 5.74 (dd, J = 4.0, 8.4 Hz, 1H), 5.67-5.32 (m, 15H), 5.25-4.09 (m, 63H), 3.96-3.02 (m, 48H), 2.79-2.66 (m, 4H), 2.23-1.96 (m, 42H).

¹³C-NMR (CDCl₃) σ 174.5, 174.5, 174.4, 173.6, 173.6, 173.5, 171.7, 170.6, 170.5, 170.3, 170.2, 170.0, 169.9, 169.9, 169.8, 169.8, 169.8, 169.7, 169.7, 169.7, 169.6, 169.6, 169.6, 169.36, 155.1, 153.9, 153.7, 151.3, 138.8, 138.7, 138.5, 138.4, 138.0, 138.0, 137.8, 133.7, 133.4, 133.2, 133.1, 133.1, 130.8, 129.8, 129.7, 129.7, 129.6, 129.5, 129.4, 129.4, 129.3, 129.2, 129.1, 129.1, 129.1, 129.0, 128.9, 128.7, 128.6, 128.5, 128.5, 128.4, 128.3, 128.2, 128.1, 128.1, 128.0, 128.0, 127.8, 127.7, 127.7, 127.6, 127.5, 127.4, 127.1, 125.4, 118.2, 114.3, 99.7, 99.2, 99.1, 98.9, 98.7, 98.3, 95.7, 95.3, 92.6, 77.4, 77.2, 74.6, 74.2, 73.7, 73.3, 73.0, 72.7, 72.0, 71.5, 71.5, 71.4, 70.0, 69.8, 69.8, 69.7, 69.2, 62.9, 62.9, 62.4, 62.3, 61.8, 61.6, 57.2, 56.9, 56.8, 55.5, 54.8, 52.9, 52.9, 52.8, 52.8, 32.1, 31.8, 30.2, 29.6, 28.8, 27.9, 27.8, 26.4, 25.9, 25.8, 25.8, 25.5, 23.6, 22.9, 22.6, 21.0, 21.0, 20.9, 20.9, 20.9, 20.9, 20.8, 20.8, 20.7, 20.7, 20.7, 20.6, 20.6, 20.5, 20.6, 14.0, 14.0, 10.9.

HRMS (ESI⁺) [M+HNEt₃+HNEt₃]²⁺ calcd for C₂₇₃H₃₁₂Cl₁₂N₈O₈₃²⁺: 2729.8355; found 2729.8382.

### Example 43

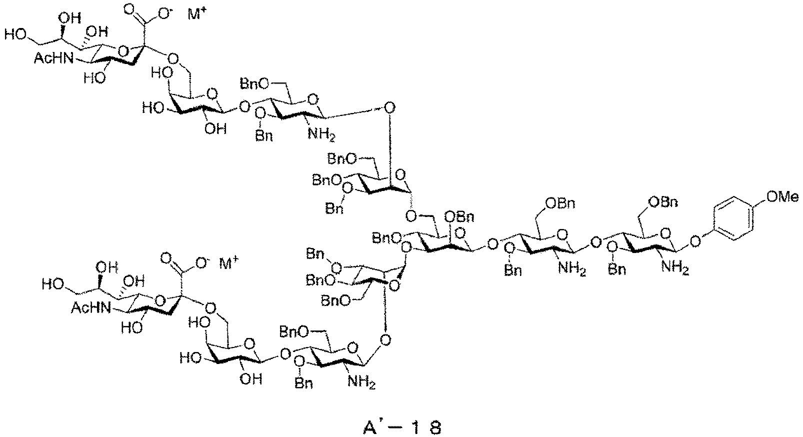

4-Methoxyphenyl 4,7,8,9-tetra-O-acetyl-3,5-dideoxy-5-(diacetylamino)-1-O-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→3)-[4,7,8,9-tetra-O-acetyl-3,5-dideoxy-5-(diacetylamino)-1-O-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-[(phenoxycarbonyl)amino]-O-D-glucopyranosyl-(1→4)-3,6-di-O-benzyl-2-deoxy-2-[(phenoxycarbonyl)amino]-β-D-glucopyranoside (compound represented by formula A'-17) (3.10 g, 0.616 mmol) was added to a 100-mL recovery flask, and admixed with tetrahydrofuran (47 mL). A 3 M aqueous lithium hydroxide solution (7.9 mL) was added at room temperature under argon, and the mixture was stirred at the same temperature for 1 hour. After the completion of the reaction was checked by HPLC, 25% brine (31 mL) was added, and the mixture was liquid-separated. Here, a white insoluble matter was left in the organic layer. Further, 25% brine (31 mL) was added to the organic layer, and the mixture was liquid-separated. The resulting tetrahydrofuran solution containing 4-methoxyphenyl 4,7,8,9-tetra-O-acetyl-3,5-dideoxy-5-(diacetylamino)-1-O-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→3)-[4,7,8,9-tetra-O-acetyl-3,5-dideoxy-5-(diacetylamino)-1-O-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-[(phenoxycarbonyl)amino]-O-D-glucopyranosyl-(1→4)-3,6-di-O-benzyl-2-deoxy-2-[(phenoxycarbonyl)amino]-β-D-glucopyranoside (compound represented by formula A'-18) was used in the next step without purification. HRMS (ESI⁺) [M+4H] ⁴⁺ calcd for C₁₉₅H₂₃₆N₆O₅₉⁴⁺: 901.8924; found 901.8889

Note) The compound represented by formula A'-18 can also be synthesized under the same conditions, using the compound represented by formula B-2; however, a reaction time of 22 hours is required, and a plurality of impurities are found in the reaction.

**[Table 2]**

| Raw material (Protecting group on nitrogen) | Reaction time | HPLC peak area ratio of compound of formula A'-18 after reaction |
|---|---|---|
| Compound of formula B-2 (Troc) | 22 hr | 66% |
| Compound of formuls A'-17 (CO₂Ph) | 1 hr | 93% |

### Example 44

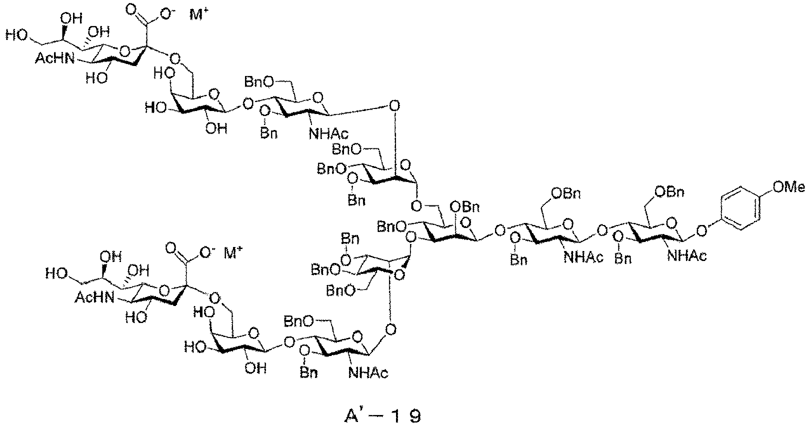

The tetrahydrofuran solution containing 4-methoxyphenyl 4,7,8,9-tetra-O-acetyl-3,5-dideoxy-5-(diacetylamino)-1-O-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→3)-[4,7,8,9-tetra-O-acetyl-3,5-dideoxy-5-(diacetylamino)-1-O-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→6)]-2,4-di-O-benzyl-β-D-mannopyranosyl-(1→4) -3,6-di-O-benzyl-2-deoxy-2-[(phenoxycarbonyl)amino]-O-D-glucopyranosyl-(1→4)-3,6-di-O-benzyl-2-deoxy-2-[(phenoxycarbonyl)amino]-β-D-glucopyranoside (compound represented by formula A'-18) (47 mL, synthesized from 3.1 g of the compound represented by formula A'-17) was added to a 200-mL recovery flask, and was admixed with methanol (31 mL) and triethylamine (2.5 mL). Acetic anhydride (1.1 mL) was added at an outside temperature of 4°C under argon, and the mixture was stirred at the same temperature for 30 minutes. After the completion of the reaction was checked by HPLC, the reaction solution was concentrated under reduced pressure. Tetrahydrofuran (62 mL), 25% brine (31 mL), and water (16 mL) were added to the concentrated residue, and the mixture was liquid-separated. Here, a white insoluble matter was left in the organic layer. Further, 25% brine (31 mL) and water (16 mL) were added to the organic layer, and the mixture was liquid-separated. The organic layer was concentrated under reduced pressure to 10 mL. Tetrahydrofuran (78 mL) and ethyl acetate (78 mL) were added to the residue, and the mixture was concentrated under reduced pressure. Ethyl acetate (78.0 mL, 25 v) was further added, and the mixture was concentrated under reduced pressure. Ethyl acetate (78 mL) was added to the resulting white solid, the mixture was stirred at room temperature for 30 minutes, and the solid was collected by filtration and washed with ethyl acetate (78 mL) to give 4-methoxyphenyl 5-acetamido-3,5-dideoxy-D-glycero-α-D-galacto-non-2-ulopyranosyl-(2→6)-β-D-galactopyranosyl-(1→4)-2-acetamido-3,6-di-O-benzyl-2-deoxy-β-D-glucopyranosyl-(1→2) -3, 4, 6-tri-O-benzyl-α-D-mannopyranosyl- (1→3) - [5-acetamido-3,5-dideoxy-D-glycero-a-D-galacto-non-2-ulopyranosyl- (2→6) -β-D-galactopyranosyl- (1→4) -2-acetamido-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-acetamido-3,6-di-O-benzyl-2-deoxy-β-D-glucopyranosyl-(1→4)-2-acetamido-3,6-di-O-benzyl-2-deoxy-β-D-glucopyranoside (compound represented by formula A'-19) (2.60 g, containing a salt) .
HRMS (ESI⁺) [M+3H]³⁺ calcd for C₂₀₃H₂₄₃N₆O₆₃³⁺: 1258.2015; found 1258.1944

### Example 45

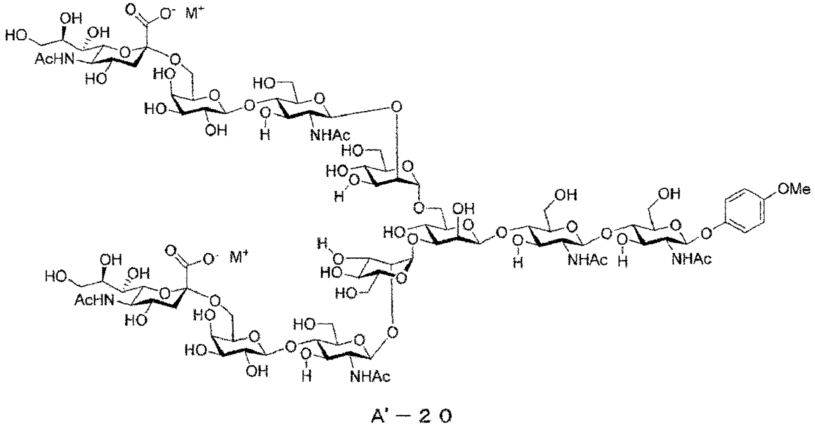

4-Methoxyphenyl 5-acetamido-3,5-dideoxy-D-glycero-α-D-galacto-non-2-ulopyranosyl-(2→6)-β-D-galactopyranosyl-(1→4)-2-acetamido-3,6-di-O-benzyl-2-deoxy-β-D-glucopyranosyl-(1→2)-3,4,6-tri-O-benzyl-α-D-mannopyranosyl- (1-3) -[5-acetamido-3, 5-dideoxy-D-glycero-α-D-galacto-non-2-ulopyranosyl-(2→6)-β-D-galactopyranosyl-(1→4)-2-acetamido-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-acetamido-3,6-di-O-benzyl-2-deoxy-β-D-glucopyranosyl-(1→4)-2-acetamido-3,6-di-O-benzyl-2-deoxy-β-D-glucopyranoside (compound represented by formula A'-19) (3.76 g) was added to a 100-mL autoclave, and N-methylpyrrolidone (38 mL) was added. Pressure reduction and then argon replacement were repeated five times, and the mixture was stirred for 30 minutes under the argon atmosphere. 10% Pd/C (PE type; water content: 55.8%; 7.5 g; manufactured by N.E. CHEMCAT CORPORATION) was added. Pressure reduction and then argon replacement were repeated five times. After that, hydrogen pressurization and then pressure release were repeated five times. The mixture was stirred for 14 hours at an outside temperature of 50°C under a hydrogen pressure of 0.5 MPa. After the completion of the reaction was checked by HPLC, the reaction solution was filtered under reduced pressure in a glove box with argon replacement. The filtered material was washed with deaerated N-methylpyrrolidone (75 mL). The filtrate was admixed with cyclopentyl methyl ether (38 mL), and concentrated to 110 mL. This operation was repeated three times, namely the dehydration operation was performed to give an N-methylpyrrolidone solution containing 4-methoxyphenyl 5-acetamido-3,5-dideoxy-D-glycero-α-D-galacto-non-2-ulopyranosyl-(2→6)-β-D-galactopyranosyl-(1→4)-2-acetamido-2-deoxy-β-D-glucopyranosyl-(1→2)-α-D-mannopyranosyl-(1→3)-[5-acetamido-3,5-dideoxy-D-glycero-α-D-galacto-non-2-ulopyranosyl-(2→6)-β-D-galactopyranosyl-(1→4)-2-acetamido-2-deoxy-β-D-glucopyranosyl-(1→2)-α-D-mannopyranosyl-(1→6)]-β-D-mannopyranosyl-(1→4)-2-acetamido-2-deoxy-β-D-glucopyranosyl-(1-+4)-2-acetamido-2-deoxy-β-D-glucopyranoside (compound represented by formula A'-20). This solution was used as it was in the next step.
Note) Sufficient care needs to be taken against air inclusion during palladium filtration. Under the influence of oxygen, a phenomenon occurs in which the residual palladium fluctuates greatly and the peak shape on HPLC changes accordingly. For the purpose of preventing the above phenomenon, the operation is carried out under an inert atmosphere, from the reaction to the completion of palladium filtration.

### Example 46

The compound represented by formula A-21 was purified according to the following synthesis scheme 5. The compound shown in the lower left below is an example of a compound wherein "R₇" is methyl in the compound represented by formula E-1: 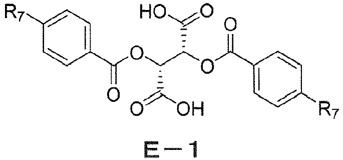 wherein R₇ is a hydrogen atom, a methyl group, or a methoxy group.

### [Synthesis Scheme 5]

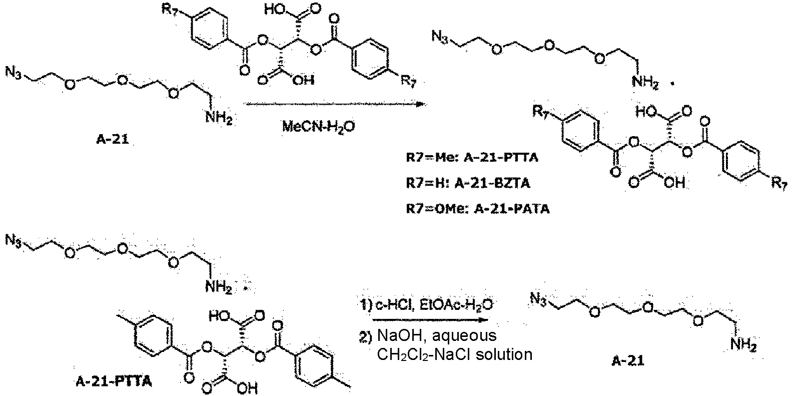

### Example 47

### 11-Azido-3,6,9-trioxaundecan-1-amine(-)-di-p-toluoyl-L-tartrate (compound represented by formula E-2 (R₇ = Me))

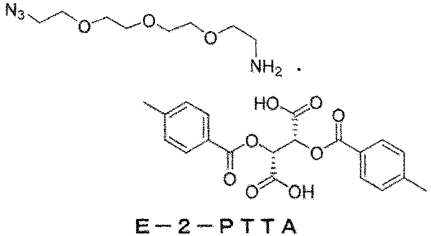

To a solution of 11-azido-3,6,9-trioxaundecan-1-amine (compound represented by formula A-21) (10.0 g, 45.82 mmol, 94.3% HPLC purity) in acetonitrile (24 mL) and water (6 mL) was added (-)-di-p-toluoyl-L-tartaric acid (17.70 g, 45.82 mmol). After the mixture was stirred at 35°C and the dissolution was checked, acetonitrile (300 mL) was added dropwise at the same temperature over 1 hour. The resulting slurry solution was concentrated under reduced pressure to 200 mL, and stirred at 25°C for 30 minutes. Then, the precipitated crystals were filtered. The filtered crystals were washed with acetonitrile (30 mL) and dried under reduced pressure at 40°C to give, as white crystals, 11-azido-3,6,9-trioxaundecan-1-amine (-)-di-p-toluoyl-L-tartrate (compound represented by formula E-2-PTTA) (24.40 g, yield: 88.0%, HPLC purity: 99.2%) (melting point: 152°C).

¹H-NMR (500 MHz, DMSO) δ 7.82 (d, J = 9.0 Hz, 4H), 7.30 (d, J = 9.0 Hz, 4H), 5.61 (s, 2H), 3.59 (t, J = 5.0 Hz, 2H), 3.56-3.47 (m, 10H), 3.39 (t, J = 5.5 Hz, 2H), 2.91 (t, J = 6.0 Hz, 2H), 2.36 (s, 6H). ¹³C-NMR (125 MHz, DMSO) δ 168.06, 164.80, 143.63, 129.25, 129.18, 126.93, 71.68, 69.72, 69.63, 69.59, 69.57, 69.20, 66.68, 49.96, 38.40, 21.14.

Substantially the same procedure can be performed using (+)-di-p-toluoyl-D-tartaric acid instead of (-)-dip-toluoyl-L-tartaric acid.

### Example 48

### 11-Azido-3,6,9-trioxaundecan-1-amine (-)-dibenzoyl-L-tartrate (Compound represented by formula E-2-BZTA)

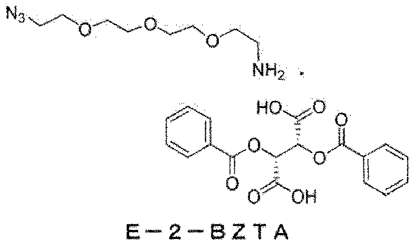

To a solution of 11-azido-3,6,9-trioxaundecan-1-amine (compound represented by formula A-21) (5.0 g, 22.91 mmol) in acetonitrile (12 mL) and water (3 mL) was added (-)-dibenzoyl-L-tartaric acid (8.21 g, 22.91 mmol). After the mixture was stirred at 35°C and the dissolution was checked, acetonitrile (150 mL) was added dropwise at the same temperature over 1 hour. The resulting slurry solution was concentrated under reduced pressure to 125 mL, and acetonitrile (100 mL) was added. The mixture was concentrated again under reduced pressure to 125 mL. The slurry solution was stirred at 25°C for 30 minutes, and the precipitated crystals were then filtered. The filtered crystals were washed with acetonitrile (15 mL) and dried under reduced pressure at 40°C to give, as white crystals, 11-azido-3,6,9-trioxaundecan-1-amine (-)-dibenzoyl-L-tartrate (compound represented by formula E-2-BZTA) (10.6 g, yield: 80%) (melting point: 131°C).

¹H-NMR (500 MHz, DMSO) δ 7.94 (d, J = 8.5 Hz, 4H), 7.64 (t, J = 7.5 Hz, 2H), 7.51 (t, J = 7.5 Hz, 4H), 5.65 (s, 2H), 3.59 (t, J = 5.0 Hz, 2H), 3.56-3.47 (m, 10H), 3.39 (t, J = 5.5 Hz, 2H), 2.90 (t, J = 5.0 Hz, 2H).

¹³C-NMR (125 MHz, DMSO) δ 167.98, 164.86, 133.38, 129.63, 129.22, 128.66, 72.10, 69.73, 69.63, 69.59, 69.57, 69.21, 66.69, 49.97, 38.38.

Substantially the same procedure can be performed using (+)-dibenzoyl-D-tartaric acid instead of (-)-dibenzoyl-L-tartaric acid.

### Example 49

### 11-Azido-3,6,9-trioxaundecan-1-amine(-)-di-p-anisoyl-L-tartrate (compound represented by formula E-2 (R₇ = OMe))

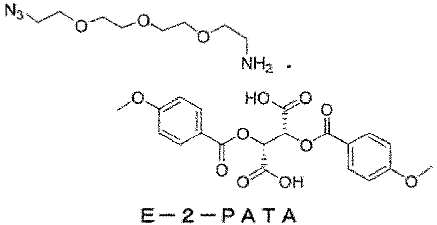

To the solution of 11-azido-3,6,9-trioxaundecan-1-amine (compound represented by formula A-21) (5.0 g, 22.91 mmol) in acetonitrile (12 mL) and water (3 mL) was added (-)-di-p-anisoyl-L-tartaric acid (9.58 g, 22.91 mmol). After the mixture was stirred at 35°C and the dissolution was checked, acetonitrile (150 mL) was added dropwise at the same temperature over 1 hour. The resulting slurry solution was concentrated under reduced pressure to 100 mL, and stirred at 25°C for 30 minutes. Then, the precipitated crystals were filtered. The filtered crystals were washed with acetonitrile (15 mL) and dried under reduced pressure at 40°C to give, as white crystals, 11-azido-3,6,9-trioxaundecan-1-amine (-)-di-p-anisoyl-L-tartrate (compound represented by formula E-2-PATA (R₇ = OMe)) (11.4 g, yield: 78%) (melting point: 153°C) .

¹H-NMR (500 MHz, DMSO) δ 7.89 (d, J = 9.0 Hz, 4H), 7.02 (d, J = 9.0 Hz, 4H), 5.59 (s, 2H), 3.82 (s, 6H), 3.59 (t, J = 5.0 Hz, 2H), 3.56-3.47 (m, 10H), 3.39 (t, J = 5.5 Hz, 2H), 2.91 (t, J = 5.5 Hz, 2H). ¹³C-NMR (125 MHz, DMSO) δ 168.25, 164.50, 163.14, 131.34, 121.88, 113.92, 71.64, 69.73, 69.63, 69.59, 69.21, 66.70, 55.48, 49.97, 38.38.

Substantially the same procedure can be performed using (+)-di-p-anisoyl-D-tartaric acid instead of (-)-dip-anisoyl-L-tartaric acid.

### Example 50

### 11-Azido-3,6,9-trioxaundecan-1-amine (compound represented by formula A-21)

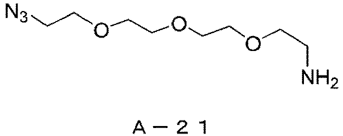

To a solution of 11-azido-3,6,9-trioxaundecan-1-amine (-)-di-p-toluoyl-L-tartrate (compound represented by formula E-2-PTTA) (12.0 g, 19.85 mmol) obtained in Example 47 in ethyl acetate (120 mL) and water (18 mL) was added concentrated hydrochloric acid (2.41 g, 23.82 mmol). The mixture was stirred at 25°C, and then liquid-separated. The resulting aqueous layer was washed twice with ethyl acetate (120 mL), adjusted to pH 11 with 10 N aqueous sodium hydroxide solution (2.15 mL, 21.50 mmol). Sodium chloride (0.6 g) was added, and then dissolved. Dichloromethane (120 mL) was added, stirred, and then liquid-separated to give the organic layer. Further, the aqueous layer was admixed with dichloromethane (120 mL), stirred, and then liquid-separated to give the organic layer. These organic layers were combined. The combined organic layer was concentrated under reduced pressure to 12 mL, and ethyl acetate (120 mL) was added. The mixture was concentrated under reduced pressure to 12 mL. The resulting solution was filtered to remove inorganic salts and washed with acetonitrile (6 mL). The resulting solution was concentrated under reduced pressure to give 11-azido-3,6,9-trioxaundecan-1-amine (compound represented by formula A-21) with HPLC purity of 99.2% (3.7 g, yield: 85%) as a pale yellow oily compound.

¹H-NMR (500 MHz, CDCl₃) δ 3.69-3.62 (m, 10H), 3.51 (t, J = 5.5 Hz, 2H), 3.39 (t, J = 5.5 Hz, 2H), 2.87 (t, J = 5.5 Hz, 2H).

### <Conditions for analyzing purity of compound represented by formula A-21>

The purity of the compound represented by formula A-21 was determined under the following HPLC analysis conditions. The sample solution was measured, a blank solution was then measured, and the blank peak was removed to calculate the HPLC purity of the compound represented by formula A-21.

### <Preparation of sample solution>

In a 10-mL measuring flask, 50 mg of the compound represented by formula A-21 was weighed, and 2 mL of acetonitrile and 100 µL of triethylamine were added and mixed. Next, 50 µL of acetic anhydride was added, mixed, and then allowed to stand for 15 minutes to acetyl-derivatize the compound represented by formula A-21. The sample solution was prepared by adding 150 µL of 4 N aqueous sodium hydroxide solution, mixing, and then filling the flask with 50% acetonitrile water.

### <Preparation of blank solution>

In a 10-mL measuring flask, 2 mL of acetonitrile and 100 µL of triethylamine were added and mixed. Next, 50 µL of acetic anhydride was added, mixed, and then allowed to stand for 15 minutes. The sample solution was prepared by adding 150 µL of 4 N aqueous sodium hydroxide solution, mixing, and then filling the flask with 50% acetonitrile water.

### <HPLC analysis conditions>

Instrument used: SHIMADZU HPLC (LC-20AD)
Column: Xbridge C18 3.5 µm, 4.6 × 150 mm (Waters)
Mobile phase A: 10 mM AcONH₄ aqueous solution
Mobile phase B: CH₃CN

**[Table 3]**

| Gradient Conditions: | | |
|---|---|---|
| Time/Solvent | 10 mM aqueous AcONH₄ solution | CH₃CN |
| 0 min | 90% | 10% |
| 13 min | 35% | 65% |
| 15 min | 35% | 65% |

Flow rate: 1 mL/min
Detection wavelength: 210 nm
Column temperature: 40°C
Injection volume: 5 µL
Retention time: 7.3 min (acetylated compound represented by formula A-21), 9.0 min (dimer of acetylated compound represented by formula A-21)

### Example 51

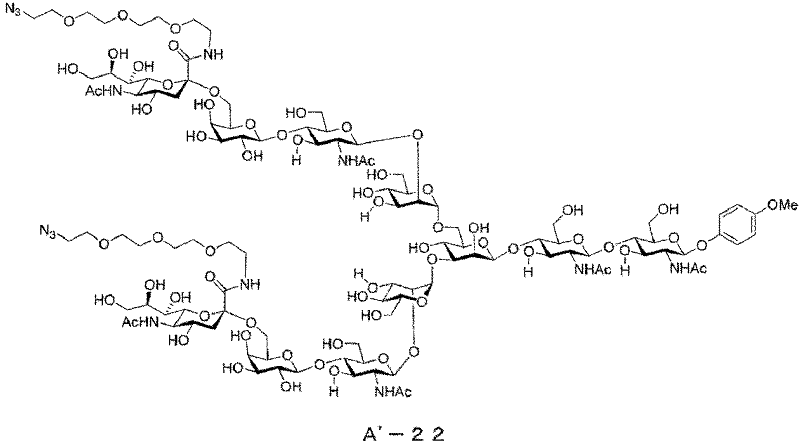

The N-methylpyrrolidone solution containing 4-methoxyphenyl 5-acetamido-3,5-dideoxy-D-glycero-α-D-galacto-non-2-ulopyranosyl-(2→6)-β-D-galactopyranosyl-(1→4)-2-acetamido-2-deoxy-β-D-glucopyranosyl-(1→2)-α-D-mannopyranosyl-(1→3)-[5-acetamido-3,5-dideoxy-D-glycero-α-D-galacto-non-2-ulopyranosyl-(2→6) -β-D-galactopyranosyl-(1→4)-2-acetamido-2-deoxy-β-D-glucopyranosyl-(1→2)-α-D-mannopyranosyl-(1→6)]-β-D-mannopyranosyl-(1→4)-2-acetamido-2-deoxy-β-D-glucopyranosyl-(1→4)-2-acetamido-2-deoxy-β-D-glucopyranoside (compound represented by formula A'-20) (110 mL, solution derived from 3.76 g of the compound represented by formula A'-19) was added to a recovery flask. 11-Azido-3,6,9-trioxaundecan-1-amine (compound represented by formula A-21) (1.01 g, 3.97 mmol), N-ethyldiisopropylamine (0.51 g, 3.97 mmol), and Molecular Sieves 4A powder (10 µm or less, 3.76 g) were added under an argon stream. After stirring, hexafluorophosphate (benzotriazol-1-yloxy)tripyrrolidinophosphonium (PyBop) (2.07 g, 3.97 mmol) was added, and the mixture was stirred at room temperature for 1.5 hours. After the completion of the reaction was checked by HPLC, the reaction solution was filtered, and the MS4A was washed with N-methylpyrrolidone (7.5 mL). The filtrate and the washing solution were combined and added dropwise to acetonitrile (555 mL). The suspension was then centrifuged. The pale yellow solid was washed twice with acetonitrile (113 mL and 165 mL), admixed with acetonitrile (165 mL) to form a suspension, and the suspension was concentrated under reduced pressure. The resulting pale yellow solid was admixed with water (7.6 mL) and filtered through a membrane filter. The filtrate was purified preparatively by HPLC (2 mL per charge). The target substance-containing fractions were concentrated under reduced pressure and lyophilized to prepare, as white solids, 4-methoxyphenyl N-(2-{2-[2-(2-azidoethoxy)ethoxy]ethoxy}ethyl)-5-acetyl-neuramamido-(2→6)-β-D-galactopyranosyl-(1→4)-2-acetamido-2-deoxy-β-D-glucopyranosyl- (1→2) -α-D-mannopyranosyl- (1→3) - [N- (2-{2-[2-(2-azidoethoxy)ethoxy]ethoxy}ethyl)-5-acetyl-neuramamido-(2→6)-β-D-galactopyranosyl-(1→4)-2-acetamido-2-deoxy-β-D-glucopyranosyl-(1→2)-α-D-mannopyranosyl-(1→6)]-β-D-mannopyranosyl-(1→4)-2-acetamido-2-deoxy-β-D-glucopyranosyl-(1→4)-2-acetamido-2-deoxy-β-D-glucopyranoside (compound represented by formula A'-22) (1.35 g, yield: 50% (total yield from the compound represented by A'-17)) (HPLC purity: 96.8% (detection wavelength 220 nm)).

¹H-NMR (D₂O) σ 7.04 (dd, J = 6.8, 2.4 Hz, 2H), 6.96 (d, J = 6.8, 2.4 Hz, 1H), 5.14 (s, 1H), 5.03 (d, J = 8.8 Hz, 1H), 4.95 (s, 1H), 4.62 (m, 3H), 4.46 (s, 1H), 4.41 (s, 1H), 4.27 (s, 1H), 4.21 (d, J = 2.8 Hz, 1H), 4.13 (d, J = 2.8 Hz, 1H), 4.01-3.50 (m, 105H), 2.71 (dd, J = 12.4, 4.4 Hz, 2H), 2.10-2.04 (m, 18H), 1.85 (t, J = 12.4, 4.4 Hz, 2H)

¹³C-NMR (D₂O) σ 185.0, 184.8, 184.7, 180.3, 168.9, 165.9, 139.7, 137.1, 128.0, 126.2, 125.4, 124.7, 124.6, 124.5, 124.5, 122.6, 109.7, 109.6, 109.4, 108.7, 108.2, 106.2, 106.0, 104.8, 104.5, 103.8, 103.3, 103.0, 102.8, 102.7, 102.6, 101.9, 101.6, 101.2, 100.8, 100.7, 100.4, 100.4, 99.8, 99.7, 99.3, 98.9, 98.6, 97.7, 97.6, 95.5, 95.1, 94.4, 94.3, 93.2, 93.0, 89.7, 89.0, 89.0, 88.7, 86.4, 85.2, 76.0, 75.6, 63.0, 62.9, 62.7, 62.7.

MS (ESI⁺) [M+2NH₄]²⁺ calcd for C₁₀₇H₁₈₄N₁₆O₆₇²⁺: 1383.0 (m/z); found 1383.0.

### <Synthesis of compound represented by formula A"-22>

Compound represented by formula A-22 wherein R₁ is isopropyl (i.e., the compound represented by formula A"-22) was synthesized according to the following synthesis scheme 6.

### [Synthesis Scheme 6]

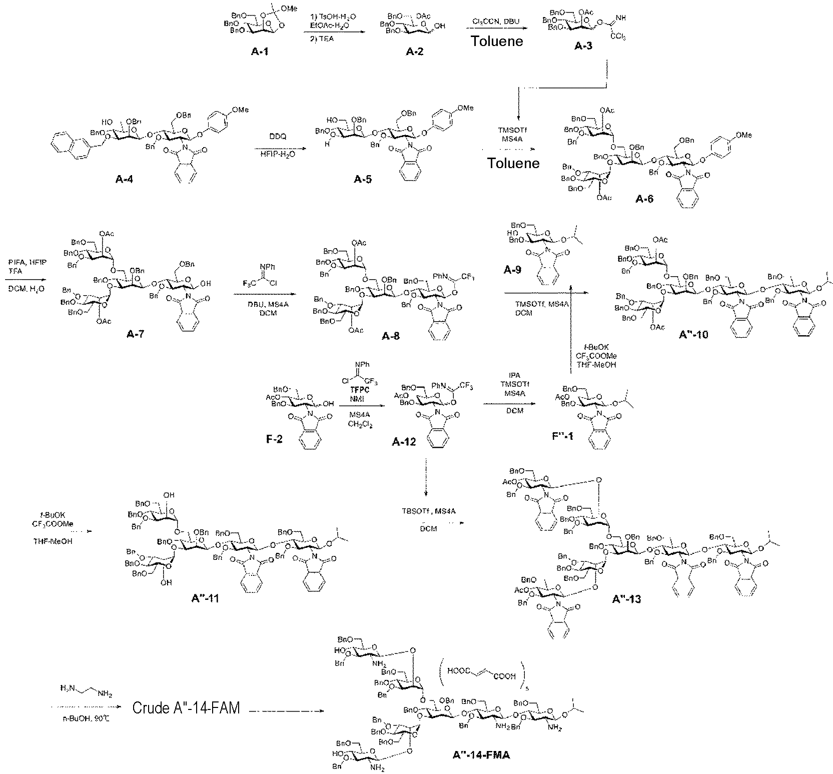

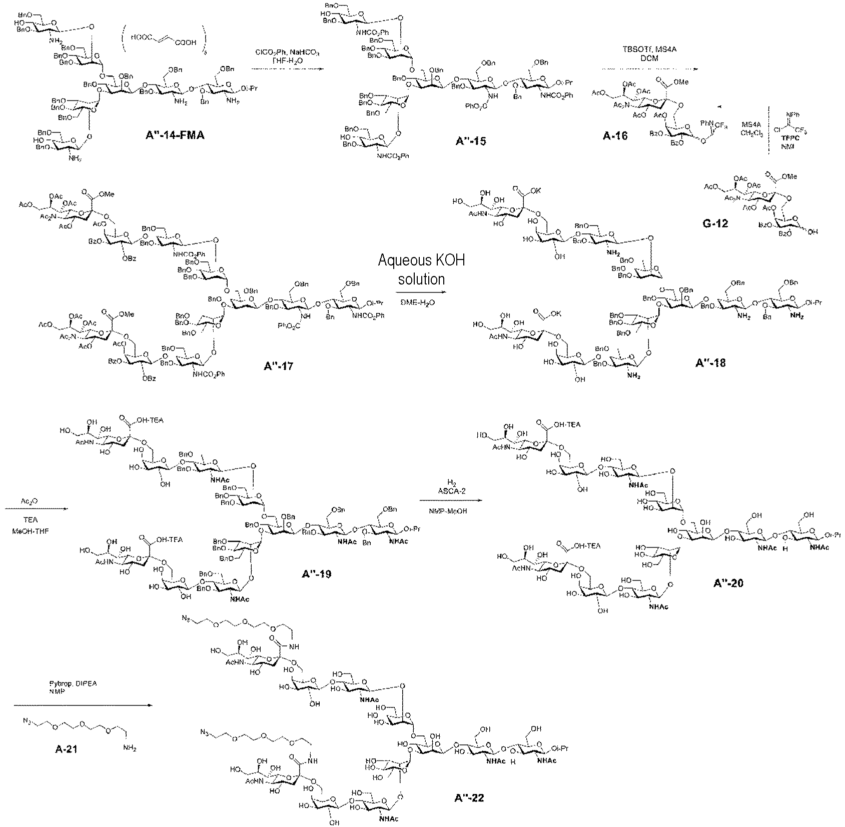

### Example 52

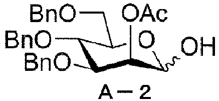

First, 3,4,6-tri-O-benzyl-1,2-O-(1-methoxyethyliden)-β-D-mannopyranose (compound represented by formula A-1) (60.0 g, 118.44 mmol) was added to a 1-L four-neck flask, and ethyl acetate (600 mL) was then added. Under the argon atmosphere, water (3 mL) and p-TsOH·H₂O (70 mg, 0.355 mmol) were added at room temperature, and the mixture was stirred at the same temperature for 6 hours. After the completion of the reaction was checked by HPLC, triethylamine (12 g, 118.44 mmol) was added. The mixture was stirred overnight at the same temperature. After the completion of the acetyl group transfer was checked by HPLC, the reaction solution was admixed with 5% sodium bicarbonate water (600 mL) for liquid-separation. Then, 20% brine (300 mL) was added to the organic layer for liquid-separation. The resulting organic layer was concentrated under reduced pressure to 120 mL, and toluene (600 mL) was added. The mixture was concentrated under reduced pressure to a liquid volume of 120 mL. Toluene (600 mL) was added again, and the mixture was concentrated again under reduced pressure to a liquid volume of 120 mL. Dehydrated toluene (180 mL) was added to prepare, as a colorless solution, a toluene solution containing 2-O-acetyl-3,4,6-tri-O-benzyl-D-mannopyranose (compound represented by formula A-2).

### Example 53

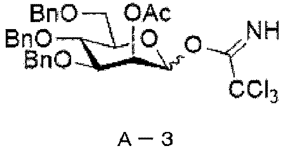

The toluene solution containing 2-O-acetyl-3,4,6-tri-O-benzyl-D-mannopyranose (compound represented by formula A-2) (118.44 mmol) was added to a 1-L flask, and trichloroacetonitrile (25.6 g, 178 mmol) and DBU (180 µL, 1.18 mmol) were added. The mixture was stirred under argon at 0°C for 6 hours. After the completion of the reaction was checked by HPLC, acetic acid (68 µL, 1.18 mmol) was added to the reaction solution at 0°C to prepare, as a brown solution, a toluene solution containing 2-O-acetyl-3,4,6-tri-O-benzyl-1-O-(2,2,2-trichloroethanimidoyl)-D-mannopyranose (compound represented by formula A-3) (118.44 mmol). This solution was used as it was in the next step.

### Example 54

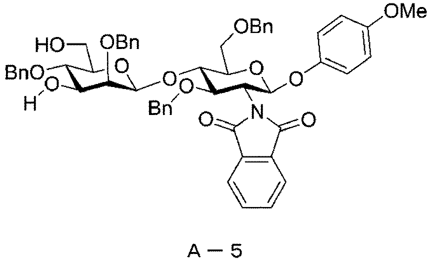

4-Methoxyphenyl 3,6-di-O-benzyl-2-deoxy-4-O-{2,4-di-O-benzyl-3-O-[(naphthalen-2-yl)methyl]-β-D-mannopyranosyl}-2-(1,3-dioxo-1,3-dihydro-2H-isoindol-2-yl)-β-D-glucopyranoside (chromatography-purified product of the compound represented by formula A-4) (30.0 g, 27.8 mmol) was added to a 1-L four-neck flask, dissolved in 1,1,1,3,3,3-hexafluoro-2-propanol (240 mL), and admixed with water (24 mL). 2,3-Dichloro-5,6-dicyano-p-benzoquinone (6.95 g, 31.6 mmol) was added at -20°C under argon, and the mixture was stirred at the same temperature for 19 hours. After the completion of the reaction was checked by HPLC, an aqueous solution having sodium sulfite (1.75 g) dissolved in water (15 mL) was added. The mixture was stirred at 0°C for 3 hours. Dichloromethane (450 mL) was added, and the mixture was liquid-separated and washed with an aqueous solution having sodium sulfite (6 g) and sodium bicarbonate (6 g) dissolved in water (300 mL). Then, the organic layer (since three layers separated, the middle and lower layers were combined) was concentrated under reduced pressure to 90 mL, and ethyl acetate (450 mL) was added. The resulting organic layer was washed with 5% sodium bicarbonate water (300 mL) and 20% brine (120 mL) and then concentrated under reduced pressure to 90 mL. Toluene (450 mL) was added, and the mixture was concentrated under reduced pressure to 90 mL. Toluene (120 mL) was added, and silica gel 60N (90 g) was added. Under stirring, heptane (60 mL) was added dropwise over 1 hour to adsorb the target substance onto the silica gel, and the mixture was then filtered. The silica gel was washed with toluene/heptane (7/2, 600 mL) (the filtrate was discarded), and then the target substance was desorbed with ethyl acetate/toluene (1/1, 1350 mL). The filtrate was concentrated under reduced pressure to 60 mL to give a toluene solution containing 4-methoxyphenyl 3,6-di-O-benzyl-2-deoxy-4-O-(2,4-di-O-benzyl-β-D-mannopyranosyl)-2-(1,3-dioxo-1,3-dihydro-2H-isoindol-2-yl)-β-D-glucopyranoside (compound represented by formula A-5). This solution was used as it was in the next step.

### Example 55

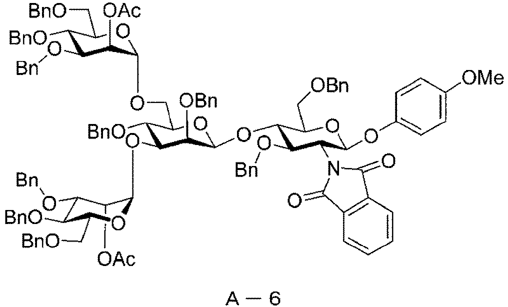

The toluene solution containing 4-methoxyphenyl 3,6-di-O-benzyl-2-deoxy-4-O-(2,4-di-O-benzyl-β-D-mannopyranosyl)-2-(1,3-dioxo-1,3-dihydro-2H-isoindol-2-yl)-β-D-glucopyranoside (compound represented by formula A-5) (27.8 mmol equivalents) and the toluene solution containing 2-O-acetyl-3,4,6-tri-O-benzyl-1-O-(2,2,2-trichloroethanimidoyl)-D-mannopyranose (compound represented by formula A-3) (83.4 mmol equivalents) were added to a 1-L four-neck flask, and were admixed with toluene (270 mL) and Molecular Sieves 4A powder (10 µm or less, 4.5 g). A solution of trimethylsilyl trifluoromethanesulfonate (1.01 mL, 5.56 mmol) in toluene (30 mL) was added dropwise at -20°C under argon over 2 hours, and the mixture was stirred at the same temperature for 2 hours. After the completion of the reaction was checked by HPLC, triethylamine (1.55 mL, 13.9 mmol) was added, and the mixture was stirred overnight at room temperature. The reaction solution was filtered and washed with acetonitrile (300 mL). The filtrate was concentrated under reduced pressure to 90 mL, and admixed with acetonitrile (600 mL), and the mixture was concentrated again under reduced pressure to 90 mL. Acetonitrile (510 mL) was added, and silica gel 120RP-18 (particle size: 40 to 50 µm; 180 g; manufactured by KANTO CHEMICAL CO., INC.) for reversed phase was then added. Under stirring, water (240 mL) was added dropwise over 1 hour to adsorb the target substance onto the solid phase, and the mixture was then filtered. The solid phase was washed with acetonitrile/water (20/8, 1.68 L) (the filtrate was discarded), and then the target substance was desorbed with ethyl acetate/acetonitrile (1/1, 1.2 L). The filtrate was concentrated under reduced pressure to 90 mL, and admixed with toluene (360 mL), and the mixture was concentrated again under reduced pressure to 90 mL to give a toluene solution containing 4-methoxyphenyl 2-O-acetyl-3,4,6-tri-O-benzyl-α-D-mannopyranosyl-(1→3)-[2-O-acetyl-3,4,6-tri-O-benzyl-α-D-mannopyranosyl-(1→6)]-2,4-di-0-benzyl-β-D-mannopyranosyl-(1→4)-3,6-di-O-benzyl-2-deoxy-(1,3-dioxo-1,3-dihydro-2H-isoindol-2-yl)-β-D-glucopyranoside (compound represented by formula A-6). This solution was used as it was in the next step.

### Example 56

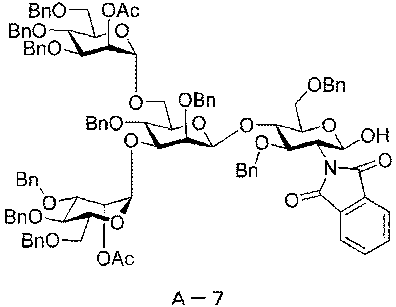

The toluene solution containing 4-methoxyphenyl 2-O-acetyl-3,4,6-tri-O-benzyl-O-D-mannopyranosyl-(1→3)-[2-O-acetyl-3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranoside (compound represented by formula A-6) (27.8 mmol equivalents) was added to a 2-L four-neck flask, and was admixed with dichloromethane (210 mL), 1,1,1,3,3,3-hexafluoro-2-propanol (150 mL), and water (12 mL). [Bis(trifluoroacetoxy)iodo]benzene (23.9 g, 55.6 mmol), dichloromethane (75 mL), and trifluoroacetic acid (9.5 g, 83.4 mmol) were added at 0°C under argon, and the mixture was stirred at the same temperature for 12 hours. After the completion of the reaction was checked by HPLC, a solution having sodium bicarbonate (18 g) dissolved in water (240 mL) was added dropwise over 20 minutes. After 15 minutes of stirring, a solution having sodium sulfite (12 g) dissolved in water (210 mL) was added dropwise. The mixture was liquid-separated, and the middle layer of the three layers separated was collected, and was concentrated under reduced pressure to 90 mL. Ethyl acetate (300 mL) was added, and brine (sodium chloride 21 g, water 195 mL) was added for washing the mixture. The resulting organic layer was concentrated under reduced pressure to 90 mL and then admixed with toluene (360 mL), and the mixture was concentrated again under reduced pressure to 90 mL. Toluene (150 mL) and dichloromethane (60 mL) were added, and silica gel 60N (particle size: 40 to 50 µm; 120 g; manufactured by KANTO CHEMICAL CO., INC) was added. Toluene (60 mL) was added dropwise to adsorb the target substance onto the silica gel, and the mixture was then filtered. Dichloromethane/toluene (4/20, 720 mL) was used for washing (the filtrate was discarded), and the target substance was desorbed from the solid phase with ethyl acetate/dichloromethane (1/2, 900 mL). The filtrate was concentrated under reduced pressure to 90 mL, and admixed with toluene (210 mL), and the mixture was concentrated again under reduced pressure to 90 mL to give a toluene solution containing 2-O-acetyl-3,4,6-triO-benzyl-α-D-mannopyranosyl-(1→3)-[2-O-acetyl-3,4,6-tri-O-benzyl-α-D-mannopyranosyl-(1→6)]-2,4-di-0-benzyl-β-D-mannopyranosyl-(1→4)-3,6-di-O-benzyl-2-deoxy-(1,3-dioxo-1,3-dihydro-2H-isoindol-2-yl)-β-D-glucopyranose (compound represented by formula A-7). This solution was used as it was in the next step.

### Example 57

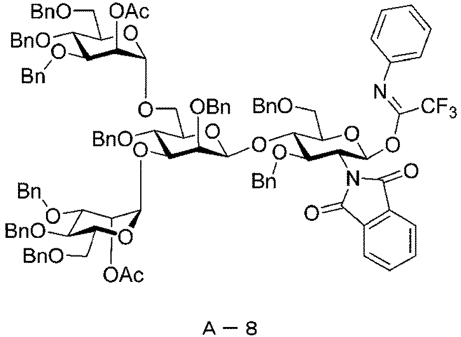

The toluene solution containing 2-0-acetyl-3,4,6-tri-O-benzyl-α-D-mannopyranosyl-(1→3)-[2-O-acetyl-3,4,6-tri-O-benzyl-α-D-mannopyranosyl-(1→6) ]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranose (compound represented by formula A-7) (27.8 mmol equivalents) was added to a 1-L four-neck flask, admixed with dichloromethane (240 mL) and Molecular Sieves 4A powder (10 µm or less, 21 g), and cooled to 0°C. DBU (5.1 g, 33.4 mmol) and 2,2,2-trifluoro-N-phenylacetimidoylchloride (6.4 g, 30.6 mmol) were added at the same temperature under argon, and the mixture was stirred for 3 hours. After the completion of the reaction was checked by HPLC, the mixture was filtered and washed with cooled dichloromethane (120 mL). The filtrate was filtered through a neutral silica gel pad (silica gel 60N; particle size: 40 to 50 µm; 120 g; manufactured by KANTO CHEMICAL CO., INC.) packed with dichloromethane (the filtrate was collected). The silica gel pad was washed with 10% ethyl acetate/dichloromethane (840 mL, 180 mL each collected), and the main residue was concentrated under reduced pressure to 90 mL. Toluene (150 mL) was added, and the mixture was concentrated again under reduced pressure to 90 mL to give a toluene solution containing 2-O-acetyl-3,4,6-tri-O-benzyl-α-D-mannopyranosyl-(1→3)-[2-O-acetyl-3,4,6-tri-O-benzyl-α-D-mannopyranosyl-(1→6)]-2,4-di-0-benzyl-β-D-mannopyranosyl-(1→4)-3,6-di-O-benzyl-2-deoxy-(1,3-dioxo-1,3-dihydro-2H-isoindol-2-yl)-1-0-(2,2,2-trifluoro-N-phenylethanimidoyl)-β-D-glucopyranose (compound represented by formula A-8). This solution was used as it was in the next step.

### Example 58

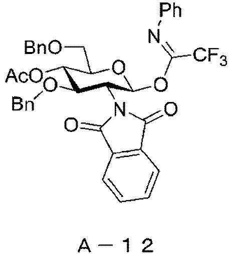

A solution of 4-0-acetyl-3,6-di-0-benzyl-2-deoxy-2-(1,3-dioxo-1,3-dihydro-2H-isoindol-2-yl)-D-glucopyranoside (compound represented by formula F-2) (20.0 g, 37.63 mmol) and Molecular Sieves 4A powder (powder particle size: 10 µm or less) (10 g) in toluene (80 mL) and dichloromethane (160 mL) was cooled to 0°C, and 2,2,2-trifluoro-N-phenylacetimidoylchloride (6.86 mL, 43.27 mmol) and N-methylimidazole (3.6 mL, 45.16 mmol) were added dropwise. The mixture was stirred at the same temperature for 22 hours. After the completion of the reaction was checked by HPLC, the reaction solution was filtered and washed with toluene (80 mL). The filtrate at 0 to 5°C was made to pass and filtered through a neutral silica gel (silica gel 60N; particle size: 40 to 50 µm; 70 g) packed with dichloromethane, and washed with dichloromethane (400 mL) at 0 to 5°C (the filtrate was divided into eight fractions). The silica pad was washed with 5% ethyl acetate/dichloromethane (400 mL) at 0 to 5°C (the filtrate was divided into four fractions). Selected fractions were mixed and concentrated to dryness to 40 mL. Toluene (100 mL) was added, and the mixture was concentrated again under reduced pressure to 40 mL to give a toluene solution containing 4-O-acetyl-3,6-di-O-benzyl-2-deoxy-2-(1,3-dioxo-1,3-dihydro-2H-isoindol-2-yl)-O-[2,2,2-trifluoro-N-phenylethanimidoy1]-β-D-glucopyranoside (compound represented by formula A-12). This solution was used as it was in the next step.

### Example 59

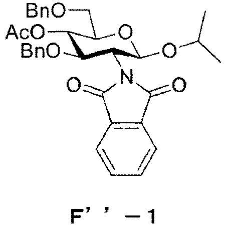

The toluene solution containing 4-0-acetyl-3,6-di-0-benzyl-2-deoxy-2-(1,3-dioxo-1,3-dihydro-2H-isoindol-2-yl)-O-[2,2,2-trifluoro-N-phenylethanimidoy1]-β-D-glucopyranoside (compound represented by formula A-12) (Gln-9, 21.4 g, 30.45 mmol equivalents) was admixed with dichloromethane (171 mL), isopropanol (4.7 mL, 60.90 mmol), and Molecular Sieves 4A powder (powder particle size 10 µm or less) (6.4 g), and the mixture was cooled to -70°C. A solution containing t-butyl dimethylsilyl trifluoromethanesulfonate (2.1 mL, 9.14 mmol) in dichloromethane (21 mL) was added dropwise at -70 to - 80°C over 2 hours, and the mixture was stirred at the same temperature for 1 hour. Triethylamine (2.55 mL, 18.27 mmol) was added, and the mixture was stirred overnight at room temperature and filtered. The Molecular Sieves 4A powder was removed and washed with dichloromethane (107 mL). The filtrates were mixed and concentrated under reduced pressure. A part of the resulting product after concentration to dryness was divided into small portions (19.2 g equivalents of the compound represented by formula A-12), and isopropanol (288 mL) was added. Seed crystals were inoculated, and the mixture was stirred overnight. Water (384 mL) was added dropwise to the slurry solution over 3 hours. The mixture was stirred overnight at 0 to 5°C and then filtered. The crystals were washed with isopropanol/water (3/4, 96 mL) and dried under reduced pressure at 40°C to give isopropyl 4-0-acetyl-3,6-di-0-benzyl-2-deoxy-2-(1,3-dioxo-1,3-dihydro-2H-isoindol-2-yl)-β-D-glucopyranoside (compound represented by formula F"-1) (14.1 g, yield 90%) as white crystals.

¹H-NMR (CDCl3) σ 7.79-7.69 (m, 4H), 7.35-7.26 (m, 5H), 7.01-6.88 (m, 5H), 5.21 (d, J = 8.8 Hz, 1H), 5.10 (t, J = 9.4 Hz, 1H), 4.60 (d, J = 12.0 Hz, 1H), 4.56 (s, 2H), 4.42 (dd, J = 10.6, 9.0 Hz, 1H), 4.33 (d, J = 12.4 Hz, 1H), 4.22 (dd, J = 10.8, 8.4 Hz, 1H), 3.87 (sept, J = 6.2 Hz, 1H), 3.78-3.73 (m, 1H), 3.62 (d, J = 4.8 Hz, 2H), 1.95 (s, 3H), 1.14 (d, J = 6.2 Hz, 3H), 0.87 (d, J = 6.2 Hz, 3H).

¹³C-NMR (CDCl3) inter alia σ 169.7, 138.0, 137.7, 133.8, 131.5, 128.4, 128.1, 127.8, 127.7, 127.4, 123.5, 96.9, 73.7, 73.6, 73.4, 72.7, 72.1, 70.0, 55.7, 23.3, 21.8, 20.9.
MS (ESI) (m/z) : 596 ([M+Na]⁺).

### Example 60

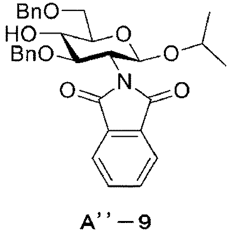

Isopropyl 3,6-di-0-benzyl-2-deoxy-2-(1,3-dioxo-1,3-dihydro-2H-isoindol-2-yl)-β-D-glucopyranoside (compound represented by formula F"-1) (1.74 g, 3.03 mmol) was admixed with tetrahydrofuran (9.45 mL), methanol (5.3 mL), and methyl trifluoroacetate (0.31 mL, 3.03 mmol), and the mixture was stirred at 20 to 30°C for 15 minutes. A 1 M tetrahydrofuran solution containing t-butoxy potassium (1.52 mL, 1.52 mmol) was added dropwise, and the mixture was stirred at 35 to 45°C for 2.5 hours. After the completion of the reaction was checked, the mixture was cooled to 20 to 30°C and admixed with acetic acid (174 µL, 3.03 mmol). Ethyl acetate (21 mL) and water (18.9 mL) were added, and the pH was adjusted to 6.0 to 7.0 with triethylamine. Sodium chloride (0.21 g) was added, and the mixture was stirred for 5 minutes. The mixture was then liquid-separated, and the resulting organic layer was washed twice with water (18.9 mL). The organic layer was concentrated under reduced pressure to 6 mL, and admixed with toluene (21 mL), and the mixture was concentrated again under reduced pressure to 6 mL. Toluene (21 mL) was further added, and the mixture was concentrated under reduced pressure to 6 mL to prepare a toluene solution containing isopropyl 3,6-di-O-benzyl-2-deoxy-2-(1,3-dioxo-1,3-dihydro-2H-isoindol-2-yl)-β-D-glucopyranoside (compound represented by formula A"-9). This solution was used as it was in the next step.

### Example 61

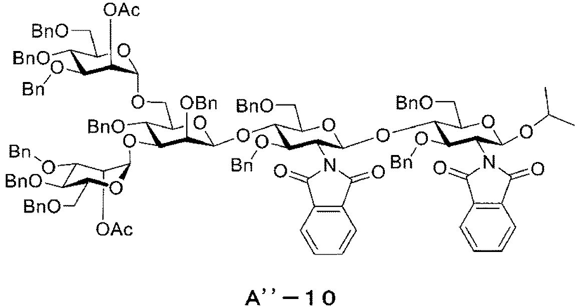

The solution of 2-O-acetyl-3,4,6-tri-O-benzyl-α-D-mannopyranosyl-(1→3)-[2-O-acetyl-3,4,6-tri-O-benzyl-α-D-mannopyranosyl-(1→6)]-2,4-di-0-benzyl-β-D-mannopyranosyl-(1→4)-3,6-di-O-benzyl-2-deoxy-(1,3-dioxo-1,3-dihydro-2H-isoindol-2-yl)-1-0-(2,2,2-trifluoro-N-phenylethanimidoyl)-β-D-glucopyranose (compound represented by formula A-8) (13.91 mmol) in toluene (45 mL) and isopropyl 3,6-di-0-benzyl-2-deoxy-2-(1,3-dioxo-1,3-dihydro-2H-isoindol-2-yl)-β-D-glucopyranoside (compound represented by formula A"-9) (8.86 g, 16.67 mmol) were added to a 1-L four-neck flask, and were admixed with toluene (225 mL) and Molecular Sieves 4A powder (6.75 g). A solution of trimethylsilyl trifluoromethanesulfonate (0.4 mL, 2.09 mmol) in toluene(15 mL) was added dropwise at -20 to -15°C under argon over 3 hours, and the mixture was stirred at the same temperature for 2 hours. After the completion of the reaction was checked by HPLC, triethylamine (0.77 mL, 5.56 mmol) was added, and the mixture was stirred overnight at room temperature. The reaction solution was filtered and washed with acetonitrile (75 mL). The filtrate was concentrated under reduced pressure to 45 mL, and admixed with acetonitrile (300 mL), and the mixture was concentrated again under reduced pressure to 45 mL. Acetonitrile (255 mL) and silica gel 120RP-18 (particle size: 40 to 50 µm; 90 g; manufactured by KANTO CHEMICAL CO., INC.) for reversed phase were added. Under stirring, water (90 mL) was added dropwise over 1 hour to adsorb the target substance onto the solid phase, and the mixture was then filtered. Acetonitrile/water (20/6, 390 mL) was used for washing (the filtrate was discarded), and the target substance was desorbed with ethyl acetate/acetonitrile (1/2, 675 mL). The filtrate was concentrated under reduced pressure and azeotroped twice with toluene (150 mL) to give a toluene solution containing isopropyl 2-O-acetyl-3,4,6-tri-O-benzyl-a-D-mannopyranosyl-(1→3)-[2-O-acetyl-3,4,6-tri-O-benzyl-α-D-mannopyranosyl-(1→6)]-2,4-di-0-benzyl-β-D-mannopyranosyl-(1→3)-3,6-di-O-benzyl-2-deoxy-(1,3-dioxo-1,3-dihydro-2H-isoindol-2-yl)-β-D-glucopyranosyl-(1→4)-3,6-di-0-benzyl-2-deoxy-(1,3-dioxo-1,3-dihydro-2H-isoindol-2-yl)-β-D-glucopyranoside (compound represented by formula A"-10). This solution was used as it was in the next step.

### Example 62

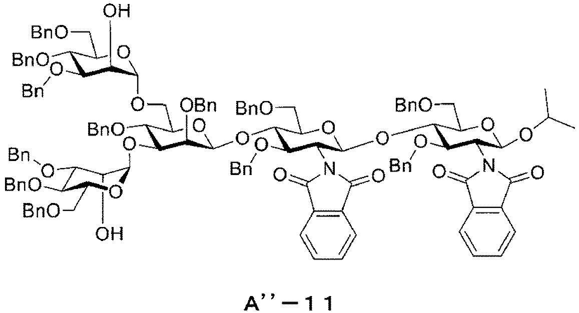

The solution of isopropyl 2-0-acetyl-3,4,6-tri-0-benzyl-α-D-mannopyranosyl-(1→3)-[2-O-acetyl-3,4,6-tri-O-benzyl-α-D-mannopyranosyl-(1→6)]-2,4-di-0-benzyl-β-D-mannopyranosyl-(1→4)-3,6-di-O-benzyl-2-deoxy-(1,3-dioxo-1,3-dihydro-2H-isoindol-2-yl)-β-D-glucopyranosyl-(1→4)-3,6-di-O-benzyl-2-deoxy-(1,3-dioxo-1,3-dihydro-2H-isoindol-2-yl)-β-D-glucopyranoside (compound represented by formula A"-10) (13.91 mmol) in toluene (45 mL) was added to a 500-mL four-neck flask, and was admixed with tetrahydrofuran (82 mL), methanol (38 mL), and methyl trifluoroacetate (1.38 mL, 13.91 mmol). After stirring at room temperature under argon for 15 minutes, a tetrahydrofuran solution containing 1 M potassium t-butoxide (6.9 mL, 6.9 mmol) was added. The mixture was stirred at 40°C for 6 hours. After the completion of the reaction was checked by HPLC, the mixture was cooled to room temperature. Acetic acid (0.56 mL, 9.74 mmol) was added, and ethyl acetate (165 mL) was then added. Water (135 mL), triethylamine (0.58 mL, 4.17 mmol), and sodium chloride (1.5 g) were added, and the mixture was stirred for 15 minutes. The mixture was then liquid-separated, and the organic layer was washed twice with water (135 mL). The organic layer was concentrated under reduced pressure to 45 mL, and admixed with toluene (150 mL), the mixture was concentrated under reduced pressure to 45 mL, and admixed with toluene (150 mL), and the mixture was concentrated again under reduced pressure to 45 mL to give a toluene solution containing isopropyl 3,4,6-tri-O-benzyl-α-D-mannopyranosyl-(1→3)-[3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranosyl-(1→4)-3,6-di-O-benzyl-2-deoxy-(1,3-dioxo-1,3-dihydro-2H-isoindol-2-yl)-β-D-glucopyranoside (compound represented by formula A"-11). This solution was used as it was in the next step.

### Example 63

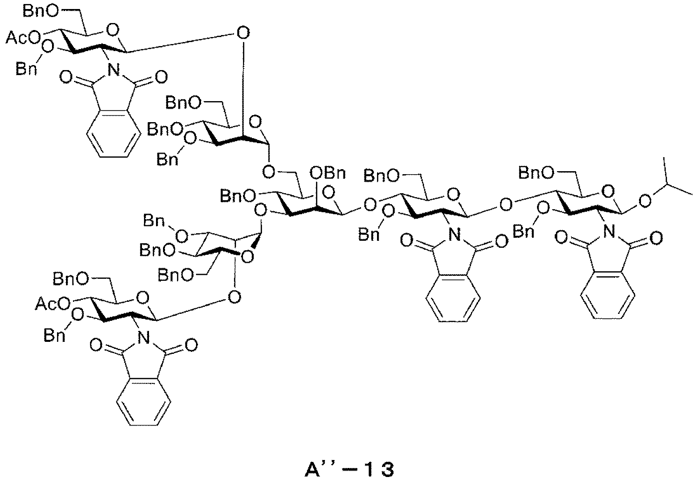

The solution of isopropyl 3,4,6-tri-O-benzyl-α-D-mannopyranosyl-(1→3)-[3,4,6-tri-O-benzyl-α-D-mannopyranosyl-(1→6)]-2,4-di-0-benzyl-β-D-mannopyranosyl-(1→4)-3,6-di-O-benzyl-2-deoxy-(1,3-dioxo-1,3-dihydro-2H-isoindol-2-yl)-β-D-glucopyranosyl-(1→4)-3,6-di-0-benzyl-2-deoxy-(1,3-dioxo-1,3-dihydro-2H-isoindol-2-yl)-β-D-glucopyranoside (compound represented by formula A"-11) (13.91 mmol) in toluene (45 mL) and the toluene solution containing 4-0-acetyl-3,6-di-0-benzyl-2-deoxy-2-(1,3-dioxo-1,3-dihydro-2H-isoindol-2-yl)-0-[2,2,2-trifluoro-N-phenylethanimidoyl]-β-D-glucopyranoside (compound represented by formula A"-12) (24.4 g, 34.78 mmol) were added to a 1-L four-neck flask, and were admixed with dichloromethane (300 mL) and Molecular Sieves 4A powder (10 µm or less, 6.0 g). A solution of t-butyl dimethylsilyl trifluoromethanesulfonate (2.55 mL, 11.13 mmol) in dichloromethane (15 mL) was added dropwise at -62°C under argon over 2 hours, and the mixture was stirred at the same temperature for 2 hours. A solution of t-butyl dimethylsilyl trifluoromethanesulfonate (1.27 mL, 5.55 mmol) in dichloromethane (7.5 mL) was added dropwise over 1 hour, and the mixture was stirred at the same temperature for 2 hours. After the completion of the reaction was checked by HPLC, triethylamine (3.86 mL, 27.82 mmol) was added, and the mixture was stirred overnight at room temperature. The reaction solution was filtered and washed with toluene (150 mL). The filtrate was concentrated under reduced pressure to 60 mL to give a toluene solution containing isopropyl 4-0-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→3)-[4-O-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranosyl-(1→4)-3,6-di-0-benzyl-2-deoxy-(1,3-dioxo-1,3-dihydro-2H-isoindol-2-yl)-β-D-glucopyranoside (compound represented by formula A"-13). This solution was used as it was in the next step.

### Example 64

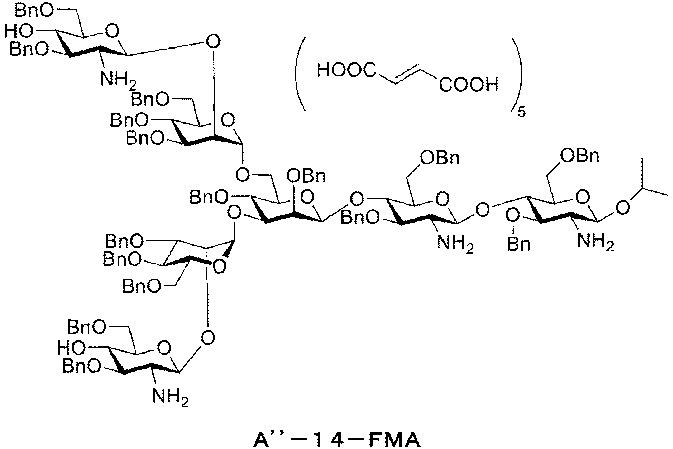

The solution of isopropyl 4-0-acetyl-3,6-di-0-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→3)-[4-O-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranosyl-(1→4)-3,6-di-O-benzyl-2-deoxy-(1,3-dioxo-1,3-dihydro-2H-isoindol-2-yl)-β-D-glucopyranoside (compound represented by formula A"-13) (13.91 mmol) in toluene (60 mL) was added to a 1-L four-neck flask, and admixed with 1-butanol (90 mL) and ethylenediamine (90 mL). The temperature was then raised to 90°C, and the mixture was stirred for 21 hours. After the completion of the reaction was checked by HPLC, the mixture was cooled to room temperature. The reaction solution was concentrated under reduced pressure to 60 mL, and was admixed with acetonitrile (390 mL) and silica gel 120RP-18 (particle size: 40 to 50 µm; 90 g; manufactured by KANTO CHEMICAL CO., INC.) for reversed phase. Under stirring, water (300 mL) was added dropwise over 1 hour to adsorb the target substance onto the solid phase, and the mixture was then filtered. Acetonitrile/water (26/20, 690 mL) was used for washing (the washing solution was discarded), and then the target substance was desorbed from the solid phase with acetonitrile/ethyl acetate (2/1, 1013 mL). The desorption solution was concentrated under reduced pressure to 60 mL, isopropyl acetate (327 mL) was added, and the mixture was concentrated under reduced pressure to 65 mL. Tetrahydrofuran (131 mL) and fumaric acid (7.21 g, 62.11 mmol) were added. After complete dissolution was checked, isopropyl acetate (654 mL) was added, and the mixture was stirred at an internal temperature of about 21°C for 2 hours. Seed crystals were inoculated, and the mixture was stirred overnight at the same temperature. The slurry solution was cooled to 3°C and stirred for 3 hours. Heptane (131 mL) was then added dropwise over 4 hours, and the mixture was stirred overnight. The slurry solution was filtered and dried under reduced pressure at room temperature to give isopropyl 2-amino-3,6-di-O-benzyl-2-deoxy-β-D-glucopyranosyl-(1→2)-3,4,6-tri-O-benzyl-α-D-mannopyranosyl-(1→3)-[2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranosyl-(1→4)-2-amino-3,6-di-O-benzyl-2-deoxy-β-D-glucopyranoside pentafumarate (compound represented by formula A"-14-FMA) (25.9 g, total yield of 64% from the compound represented by formula A-4) as white crystals.
¹HNMR (CD₃OD) σ 7.44-7.06 (m, 88H), 6.73 (s, 10H, fumaric acid), 5.32 (s, 1H), 5.16-5.00 (m, 5H), 4.99-4.18 (m, 43H), 4.08-3.32 (m, 38H), 3.23-3.21 (m, 1H), 3.07-3.04 (m, 2H), 2.98-2.92 (m, 2H), 2.85-2.80 (m, 2H), 1.98 (s, 4H, residual isopropyl acetate), 1.28-1.21 (m, 15H, containing residual isopropyl acetate)
¹³CNMR (CD₃OD) inter alia σ 170.1, 140.02, 140.00, 139.9, 139.8, 139.7, 139.64, 139.62, 139.56, 139.54, 139.51, 139.5, 139.3, 139.1, 138.8, 138.7, 135.9, 129.8, 129.78, 129.71, 129.64, 129.62, 129.59, 129.57, 129.53, 129.46, 129.44, 129.42, 129.4, 129.3, 129.28, 129.23, 129.21, 129.0, 128.9, 128.89, 128.87, 128.83, 128.80, 128.78, 128.7, 128.5, 128.4, 128.2, 99.1, 81.0, 79.6, 79.0, 76.0, 75.8, 75.6, 74.5, 74.4, 74.1, 73.4, 72.1, 70.3, 69.1, 56.3, 23.8, 22.0, 21.9.
   HRMS (ESI⁺) [M+2H]²⁺calcd for C₁₅₇H₁₈₀N₄O₃₂²⁺: 1317.13016; found 1317.13128.

### Example 65

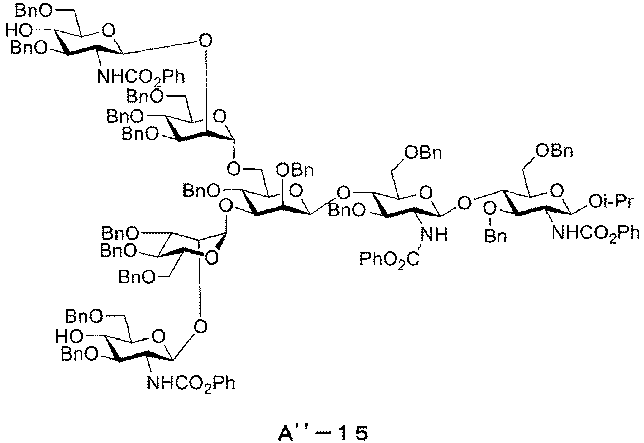

Isopropyl 2-amino-3,6-di-O-benzyl-2-deoxy-β-D-glucopyranosyl-(1→2)-3,4,6-tri-O-benzyl-α-D-mannopyranosyl-(1→3)-[2-amino-3,6-di-O-benzyl-2-deoxy-β-D-glucopyranosyl-(1→2)-3,4,6-tri-O-benzyl-α-D-mannopyranosyl-(1→6)]-2,4-di-0-benzyl-β-D-mannopyranosyl-(1→4)-2-amino-3,6-di-O-benzyl-2-deoxy-β-D-glucopyranosyl-(1→4)-2-amino-3,6-di-O-benzyl-2-deoxy-β-D-glucopyranoside pentafumarate (compound represented by formula A"-14-FMA) (27 g, 8.4 mmol) was added to a 1-L four-neck flask, and was admixed with an aqueous solution having tetrahydrofuran (216 mL) and sodium bicarbonate (14.1 g) dissolved in water (203 mL). Phenyl chloroformate (4.75 mL, 37.8 mmol) was added, and the mixture was then stirred for 1 hour. After the completion of the reaction was checked by HPLC, phenyl chloroformate (0.21 mL) was added. After checking that there was no variation in the peak, acetic acid (8.6 mL) was added to adjust the pH to 5.29. Ethyl acetate (270 mL) and water (135 mL) were added, and the mixture was stirred at room temperature for 30 minutes and then liquid-separated. The resulting organic layer was concentrated under reduced pressure to 54 mL, and was admixed with acetonitrile (216 mL) and silica gel 120RP-18 (particle size: 40 to 50 µm; 81 g; manufactured by KANTO CHEMICAL CO., INC.) for reversed phase. Under stirring, water (95 mL) was added dropwise over 1 hour to adsorb the target substance onto the solid phase, and the mixture was then filtered. Acetonitrile/water (10/3. 5,364 mL) was used for washing (the washing solution was discarded), and then the target substance was desorbed from the solid phase with acetonitrile/ethyl acetate (2/1, 1215 mL). The desorption solution was concentrated under reduced pressure to 54 mL, toluene (270 mL) was added, and the mixture was concentrated under reduced pressure to 54 mL to give a toluene solution containing isopropyl 3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→3)-[3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-[(phenoxycarbonyl)amino]-β-D-glucopyranosyl-(1→4)-3,6-di-O-benzyl-2-deoxy-[(phenoxycarbonyl)amino]-β-D-glucopyranoside (compound represented by formula A"-15). This solution was used as it was in the next step.

### Example 66

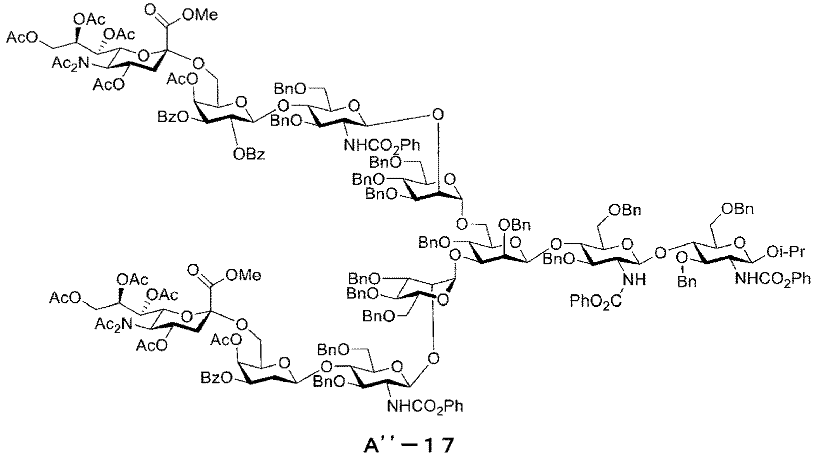

The toluene solution containing isopropyl 3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→3)-[3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-[(phenoxycarbonyl)amino]-β-D-glucopyranosyl-(1→4)-3,6-di-O-benzyl-2-deoxy-[(phenoxycarbonyl)amino]-β-D-glucopyranoside (compound represented by formula A"-15) (8.4 mmol equivalents) and a toluene solution containing 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-ulopyranosyl]-1-0-(2,2,2-trifluoro-N-phenylethanimidoyl)-D-galactopyranose (compound represented by formula A-16) (23.52 mmol) were added to a 1-L four-neck flask, and were admixed with dichloromethane (270 mL) and Molecular Sieves 4A powder (10 µm or less, 8.1 g). A solution of t-butyl dimethylsilyl trifluoromethanesulfonate (0.58 mL, 2.52 mmol) in dichloromethane (27 mL) was added dropwise at - 20°C under argon over 3 hours, and the mixture was stirred at the same temperature for 1 hour. After the completion of the reaction was checked by HPLC, triethylamine (465 µL) was added, and the mixture was stirred overnight while the temperature was allowed to rise naturally. The reaction solution was filtered and washed with 1,2-dimethoxyethane (270 mL). The filtrate was concentrated under reduced pressure to 81 mL to give a 1,2-dimethoxyethane solution containing isopropyl 4,7,8,9-tetra-0-acetyl-3,5-dideoxy-5-(diacetylamino)-1-0-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→3)-[4,7,8,9-tetra-O-acetyl-3,5-dideoxy-5-(diacetylamino)-1-O-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-[(phenoxycarbonyl)amino]-β-D-glucopyranosyl-(1→4)-3,6-di-O-benzyl-2-deoxy-2-[(phenoxycarbonyl)amino]-β-D-glucopyranoside (compound represented by formula A"-17). This solution was used as it was in the next step.

### Example 67

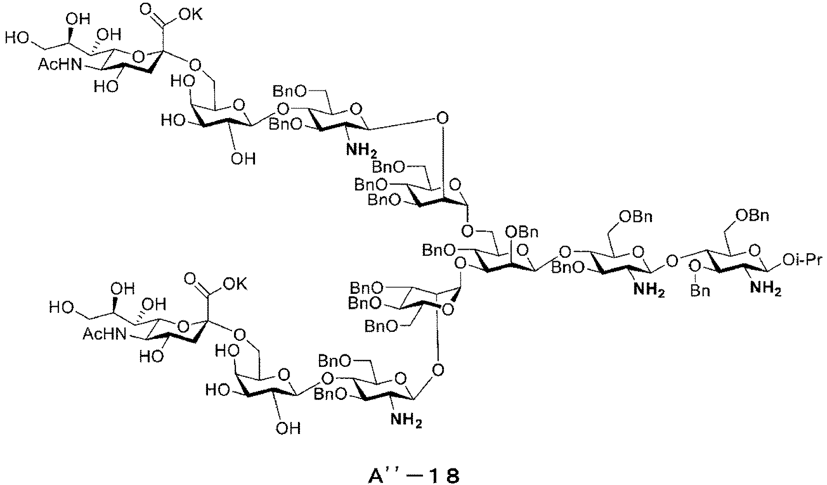

The 1,2-dimethoxyethane solution containing isopropyl 4,7,8,9-tetra-0-acetyl-3,5-dideoxy-5-(diacetylamino)-1-O-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→3)-[4,7,8,9-tetra-O-acetyl-3,5-dideoxy-5-(diacetylamino)-1-0-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→6)]-2,4-di-0-benzyl-β-D-mannopyranosyl-(1→4)-3,6-di-O-benzyl-2-deoxy-2-[(phenoxycarbonyl)amino]-β-D-glucopyranosyl-(1→4)-3,6-di-O-benzyl-2-deoxy-2-[(phenoxycarbonyl)amino]-β-D-glucopyranoside (compound represented by formula A"-17) (8.4 mmol equivalents) was added to a 1-L four-neck recovery flask, and admixed with 1,2-dimethoxyethane (243 mL) and distilled water (108 mL). An 8 M aqueous potassium hydroxide solution (41.9 mL) was added dropwise at 0 to 5°C under argon. The temperature was raised to room temperature, and the mixture was then stirred for 1 hour. After the completion of the reaction was checked by HPLC, an 8 M aqueous potassium hydroxide solution (4.2 mL) was added. After checking that there was no variation in the peak, acetic acid (6.5 mL) was added to adjust the pH to 9.47. The aqueous layer was discarded, and the organic layer was concentrated under reduced pressure to 81 mL to give a 1,2-dimethoxyethane solution containing isopropyl 4,7,8,9-tetra-0-acetyl-3,5-dideoxy-5-(diacetylamino)-1-O-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→3)-[4,7,8,9-tetra-O-acetyl-3,5-dideoxy-5-(diacetylamino)-1-O-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-[(phenoxycarbonyl)amino]-β-D-glucopyranosyl-(1→4)-3,6-di-O-benzyl-2-deoxy-2-[(phenoxycarbonyl)amino]-β-D-glucopyranoside (compound represented by formula A"-18). This solution was used as it was in the next step.

### Example 68

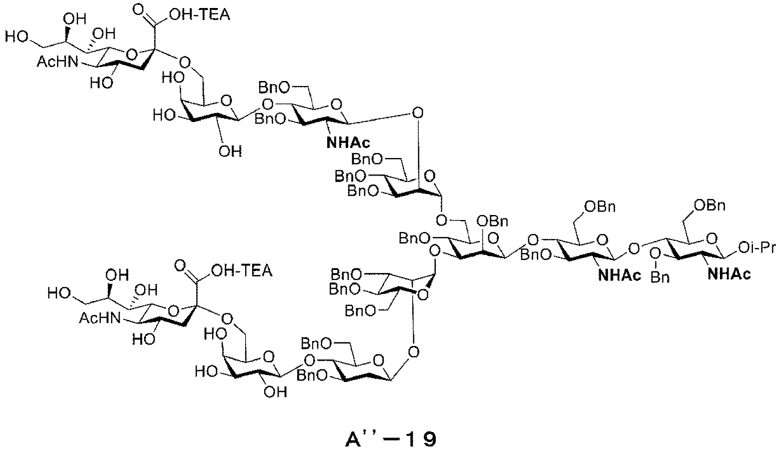

The 1,2-dimethoxyethane solution containing isopropyl 4,7,8,9-tetra-0-acetyl-3,5-dideoxy-5-(diacetylamino)-1-O-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→3)-[4,7,8,9-tetra-O-acetyl-3,5-dideoxy-5-(diacetylamino)-1-0-methyl-D-glycero-α-D-galacto-non-2-ulopyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-[(phenoxycarbonyl)amino]-β-D-glucopyranosyl-(1→4)-3,6-di-O-benzyl-2-deoxy-2-[(phenoxycarbonyl)amino]-β-D-glucopyranoside (compound represented by formula A"-18) (7.56 mmol equivalents) was added to a 500-mL four-neck flask, and was admixed with tetrahydrofuran (219 mL), methanol (97 mL) and triethylamine (19.1 g). Acetic anhydride (12.8 mL) was added dropwise over 1 hour at an outside temperature of 0 to 5°C under argon, and the mixture was stirred at the same temperature for 1 hour. Then, triethylamine (9.44 mL) and acetic anhydride (4.25 mL) were added. After the completion of the reaction was checked by HPLC, methanol (122 mL) was added, and the mixture was stirred overnight at 0 to 5°C. DMAP (9.2 mg) was added, and the mixture was stirred at the same temperature for 1 hour. The reaction solution was then concentrated under reduced pressure to 72 mL. Acetonitrile (243 mL) was added to the concentrated residue, and the mixture was concentrated under reduced pressure to 72 mL. Acetonitrile (291 mL) and water (121 mL) were added, and silica gel 120RP-18 (particle size: 40 to 50 µm; 120 g; manufactured by KANTO CHEMICAL CO., INC.) for reversed phase was added. Under stirring, water (360 mL) was added dropwise over 1 hour to adsorb the target substance onto the solid phase, and the mixture was then filtered. Acetonitrile/water (1/4, 920 mL) was used for washing (the washing solution was discarded), and then the target substance was desorbed from the solid phase with acetonitrile/methanol (1/1, 1440 mL) (the desorption solution was mixed with 0.84 mmol equivalents of the separately synthesized solution; a total of 8.4 mmol equivalents). The desorption solution was concentrated under reduced pressure to 81 mL, and tetrahydrofuran/water (8/2, 270 mL) was added, and purified Shirasagi (8.1 g) was added. The mixture was then stirred at 15 to 25°C for 1 hour, filtered, and then washed with tetrahydrofuran/water (8/2, 1350 mL). The filtrate was concentrated under reduced pressure to 270 mL. Tetrahydrofuran was added to the filtrate to adjust the composition to tetrahydrofuran/water (8/2). Purified Shirasagi (8.1 g) was added, and the mixture was then stirred at 15 to 25°C for 1 hour, filtered, and then washed with tetrahydrofuran/water (8/2, 1350 mL). The filtrate was admixed with N-methylpyrrolidone (81 mL), and the mixture was concentrated under reduced pressure to 95 mL to prepare an N-methylpyrrolidone solution containing isopropyl 5-acetamido-3,5-dideoxy-D-glycero-α-D-galacto-non-2-ulopyranosyl-(2→6)-β-D-galactopyranosyl-(1→4)-2-acetamido-3,6-di-O-benzyl-2-deoxy-β-D-glucopyranosyl-(1→2)-3,4,6-tri-O-benzyl-α-D-mannopyranosyl-(1→3)-[5-acetamido-3,5-dideoxy-D-glycero-α-D-galacto-non-2-ulopyranosyl-(2→6)-β-D-galactopyranosyl-(1→4)-2-acetamido-3,6-di-O-benzyl-2-deoxy-β-D-glucopyranosyl-(1→2)-3,4,6-tri-O-benzyl-α-D-mannopyranosyl-(1→6)]-2, 4-di-O-benzyl-β-D-mannopyranosyl-(1→4)-2-acetamido-3,6-di-O-benzyl-2-deoxy-β-D-glucopyranosyl-(1→4)-2-acetamido-3,6-di-O-benzyl-2-deoxy-β-D-glucopyranoside (compound represented by formula A"-19). This solution was used as it was in the next step.

### Example 69

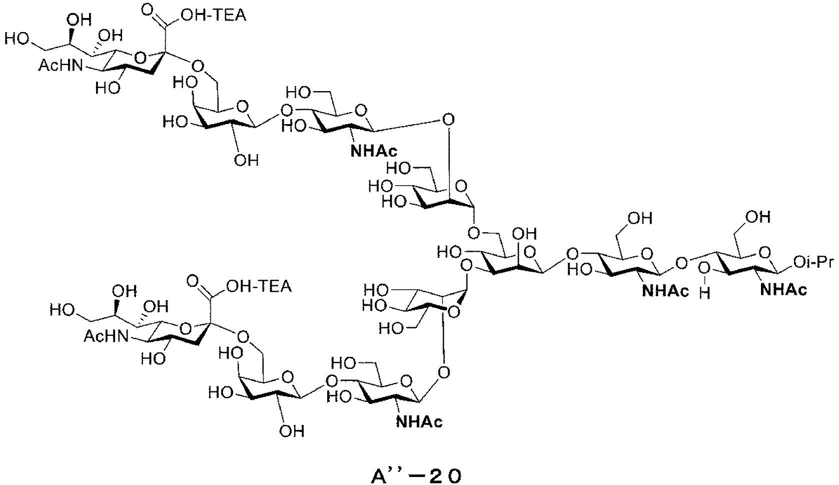

The N-methylpyrrolidone solution containing isopropyl 5-acetamido-3,5-dideoxy-D-glycero-α-D-galacto-non-2-ulopyranosyl-(2→6)-β-D-galactopyranosyl-(1→4)-2-acetamido-3,6-di-O-benzyl-2-deoxy-β-D-glucopyranosyl-(1→2)-3, 4, 6-tri-O-benzyl-α-D-mannopyranosyl- (1→3)- [5-acetamido-3,5-dideoxy-D-glycero-α-D-galacto-non-2-ulopyranosyl-(2→6)-β-D-galactopyranosyl-(1→4)-2-acetamido-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-acetamido-3,6-di-O-benzyl-2-deoxy-β-D-glucopyranosyl-(1→4)-2-acetamido-3,6-di-O-benzyl-2-deoxy-β-D-glucopyranoside (compound represented by formula A"-19) (2.8 mmol equivalents) was added to an autoclave, and methanol (45 mL) was added. Pressure reduction and then argon replacement were repeated three times. Pre-treated ASCA-2 (manufactured by N.E. CHEMCAT CORPORATION, 13.5 g) was added. Pressure reduction and then argon replacement were repeated three times. After that, hydrogen pressurization and then pressure release were repeated three times. The mixture was strongly stirred for 4 hours at an outside temperature of 40°C under a hydrogen pressure of 0.5 MPa. After the completion of the reaction was checked by HPLC, the reaction solution was filtered under reduced pressure in a glove box with argon replacement. The filtered material was washed with deaerated methanol (180 mL). The filtrate was admixed with diisopropylethylamine (2.4 mL), and concentrated under reduced pressure to 32 mL. The above operations on a 2.8 mmol scale were repeated three times, and the resulting solutions were combined to obtain a 96-mL (8.4 mmol equivalents) solution. Acetonitrile (270 mL) was added to the resulting solution, and the mixture was dehydrated and concentrated under reduced pressure to 96 mL. Acetonitrile (270 mL) was added again, and the mixture was dehydrated and concentrated under reduced pressure to 96 mL to give an N-methylpyrrolidone solution containing isopropyl 5-acetamido-3,5-dideoxy-D-glycero-α-D-galacto-non-2-ulopyranosyl-(2→6)-β-D-galactopyranosyl-(1→4)-2-acetamido-2-deoxy-β-D-glucopyranosyl-(1→2)-α-D-mannopyranosyl-(1→3)-[5-acetamido-3,5-dideoxy-D-glycero-α-D-galacto-non-2-ulopyranosyl-(2→6)-β-D-galactopyranosyl-(1→4)-2-acetamido-2-deoxy-β-D-glucopyranosyl-(1→2)-α-D-mannopyranosyl-(1→6)]-β-D-mannopyranosyl-(1→4)-2-acetamido-2-deoxy-β-D-glucopyranosyl-(1→4)-2-acetamido-2-deoxy-β-D-glucopyranoside (compound represented by formula A"-20). This solution was used as it was in the next step. Note) ASCA-2 was pre-treated according to the following method.
A slurry solution of ASCA-2 (31.2 g) in DMF (54 mL) was cooled to 0 to 5°C under argon, and then purified water (13.5 mL) and concentrated hydrochloric acid (13.5 mL) were added dropwise. After stirring at 20 to 30°C for 1 hour, the mixture was filtered under argon and washed with purified water (594 mL) to give pre-treated ASCA-2 (40.5 g).

### Example 70

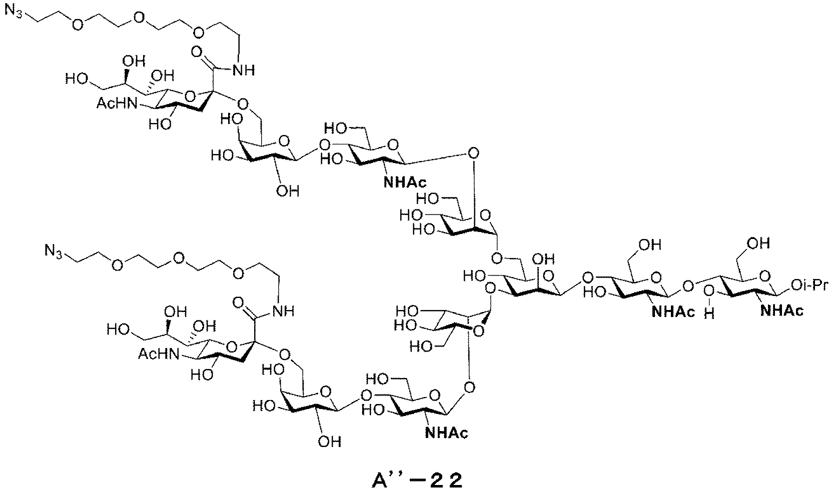

The N-methylpyrrolidone solution containing isopropyl 5-acetamido-3,5-dideoxy-D-glycero-α-D-galacto-non-2-ulopyranosyl-(2→6)-β-D-galactopyranosyl-(1→4)-2-acetamido-2-deoxy-β-D-glucopyranosyl-(1→2)-α-D-mannopyranosyl-(1→3)-[5-acetamido-3,5-dideoxy-D-glycero-α-D-galacto-non-2-ulopyranosyl-(2→6)-β-D-galactopyranosyl-(1→4)-2-acetamido-2-deoxy-β-D-glucopyranosyl-(1→2)-α-D-mannopyranosyl-(1→6)]-β-D-mannopyranosyl-(1→4)-2-acetamido-2-deoxy-β-D-glucopyranosyl-(1→4)-2-acetamido-2-deoxy-β-D-glucopyranoside (compound represented by formula A"-20) (7.56 mmol equivalents) was added to a recovery flask. 11-Azido-3,6,9-trioxaundecan-1-amine (compound represented by formula A-21) (4.95 g, 22.68 mmol) and N-ethyldiisopropylamine (3.96 g, 22.68 mmol) were added under an argon stream, and the mixture was cooled to 0 to 5°C. Bromotripyrrolidinophosphonium=hexafluorophosphate (PyBrop) (10.53 g, 22.68 mmol) was added at the same temperature, and the mixture was stirred at room temperature for 30 minutes. After the completion of the reaction was checked by HPLC, the reaction solution was added dropwise to a suspension containing Celpure 65 (36.9 g) in acetonitrile (729 mL), and the resulting slurry solution was filtered (the target substance was solidified and filtered). The filtered material was washed with acetonitrile (1827 mL) to remove residual reagent debris, and then washed with methanol/water (1/1, 1458 mL) to elute the target substance. The filtrate was concentrated under reduced pressure to 243 mL, and admixed with a 1 M aqueous sodium hydroxide solution (37.8 mL), and the mixture was stirred at room temperature for 30 minutes. After checking the conversion of the acetyl form to the target substance by HPLC, the compound was adjusted to pH 6.5 with acetic acid and lyophilized to give white powder of crude isopropyl N-(2-{2-[2-(2-azidoethoxy)ethoxy]ethoxy}ethyl)-5-acetyl-neuramamido-(2→6)-β-D-galactopyranosyl-(1→4)-2-acetamido-2-deoxy-β-D-glucopyranosyl-(1→2)-α-D-mannopyranosyl-(1→3)-[N-(2-{2-[2-(2-azidoethoxy)ethoxy]ethoxy}ethyl)-5-acetyl-neuramamido-(2→6)-β-D-galactopyranosyl-(1→4)-2-acetamido-2-deoxy-β-D-glucopyranosyl-(1→2)-α-D-mannopyranosyl-(1→6)]-β-D-mannopyranosyl-(1→4)-2-acetamido-2-deoxy-β-D-glucopyranosyl-(1→4)-2-acetamido-2-deoxy-β-D-glucopyranoside (compound represented by formula A"-22) (18.4 g, content 64.5%, HPLC purity 83.2%, total net yield from the heptasaccharide intermediate through 6 steps 59.1%). The crude material was used in an amount equivalent to 2.67 g of the charged heptasaccharide fumarate, and adjusted to a concentration of 160 mg/mL, and then subjected to HPLC preparative purification under the following conditions. The purification operation was repeated, and fractions were analyzed by HPLC. Selected fractions were mixed, concentrated under reduced pressure, and lyophilized to give white powder of isopropyl N-(2-{2-[2-(2-azidoethoxy)ethoxy]ethoxy}ethyl)-5-acetyl-neuramamido-(2→6)-β-D-galactopyranosyl-(1→4)-2-acetamido-2-deoxy-β-D-glucopyranosyl-(1→2)-α-D-mannopyranosyl-(1→3)-[N-(2-{2-[2-(2-azidoethoxy)ethoxy]ethoxy}ethyl)-5-acetyl-neuramamido-(2→6)-β-D-galactopyranosyl-(1→4)-2-acetamido-2-deoxy-β-D-glucopyranosyl-(1→2)-α-D-mannopyranosyl-(1→6)]-β-D-mannopyranosyl-(1→4)-2-acetamido-2-deoxy-β-D-glucopyranosyl-(1→4)-2-acetamido-2-deoxy-β-D-glucopyranoside (compound represented by formula A"-22) (750 mg, total yield from the heptasaccharide fumarate 33.9%, HPLC purity 97.8%).

### [Purification Conditions]

HPLC column: YMC-Triart Prep Bio200 C8 (20 nm, 10 µm), 30 mm × 250 mm
Mobile phase: methanol/water (25/75)
Flow rate: 30 mL/min
Target substance elution position: 35 to 55 minutes
Injection volume: 2 mL (160 mg/mL solution)
Fractions: 1 minute for each

¹H-NMR (D₂O) σ 5.11 (s, 1H), 4.94-4.92 (m, 1H), 4.83 (s, 1H), 4.76 (s, 1H), 4.59-4.55 (m, 4H), 4.43 (d, J = 7.2 Hz, 2H), 4.24 (s, 1H), 4.18 (d, J = 2.8 Hz, 1H), 4.10 (d, J = 2.8 Hz, 1H), 4.00-3.43 (m, 103H), 2.68 (dd, J = 13.2 Hz, 5.2 Hz, 2H), 2.07-2.00 (m, 18H), 1.83 (t, J = 12.6 Hz, 2H), 1.17 (d, J = 6.2 Hz, 3H), 1.10 (d, J = 6.2 Hz, 3H).

¹³C-NMR (D₂O) interalia σ 175.1, 174.7, 174.6, 169.2, 103.8, 99.7, 99.6, 99.5, 99.47, 99.44, 99.40, 99.36, 99.33, 99.31, 80.9, 80.8, 80.5, 79.7, 79.4, 77.5, 76.5, 76.2, 74.6, 74.4, 73.9, 73.87, 73.80, 73.60, 73.57, 73.52, 72.95, 72.90, 72.7, 72.6, 72.56, 72.5, 72.26, 72.23, 72.18, 72.17, 72.14, 72.11, 72.10, 71.22, 71.15, 71.10, 70.9, 70.7, 70.6, 70.4, 70.3, 70.26, 70.25, 70.21, 69.7, 69.6, 69.5, 69.3, 69.2, 68.6, 68.4, 67.8, 67.4, 67.1, 64.2, 63.0, 62.8, 62.75, 62.72, 62.7, 62.0, 61.8, 61.74, 61.68, 61.65, 60.2, 59.9, 55.4, 55.3, 54.9, 54.7, 54.64, 54.62, 51.8, 51.7, 50.2, 38.8, 38.3, 38.2, 22.5, 22.3, 22.2, 22.1, 21.2.

HRMS (ESI⁺) [M+H]⁺calcd for C₁₀₃H₁₇₇N₁₄O₆₆⁺: 2667.09503; found 2667.09661.

## Claims

1. A method for producing an oligosaccharide represented by the following formula A-22: 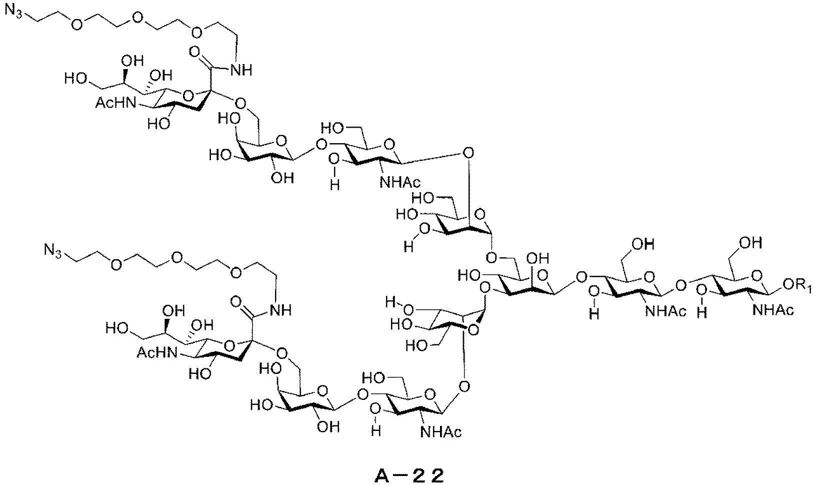
wherein R₁ is a C₁ to C₆ alkyl group or a phenyl group, and the phenyl group is optionally substituted with one to three C₁ to C₆ alkoxy groups,
the method comprising the steps of:
(step I-1)
producing a compound represented by the following formula A-8: 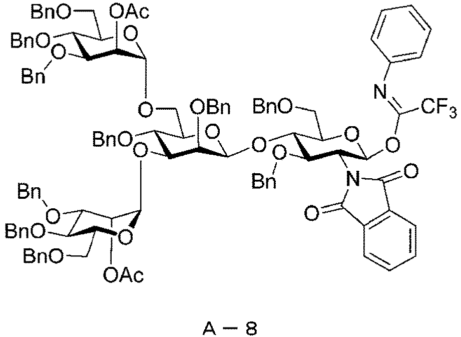 the step comprising the step of:
subjecting a compound represented by formula A-3: 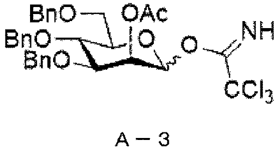
and a compound represented by the following formula A-5: 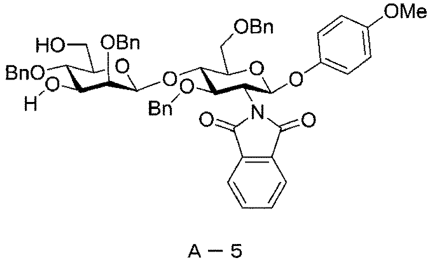
to α-1,3-glycosidic linkage and α-1,6-glycosidic linkage to give a compound represented by the following formula A-6: 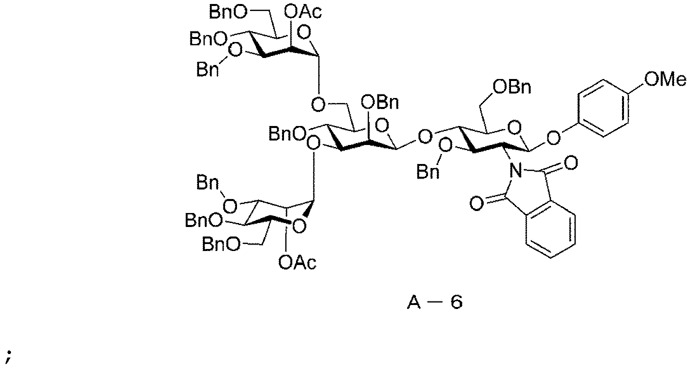
(step I-2) producing a compound represented by the following formula A-11: 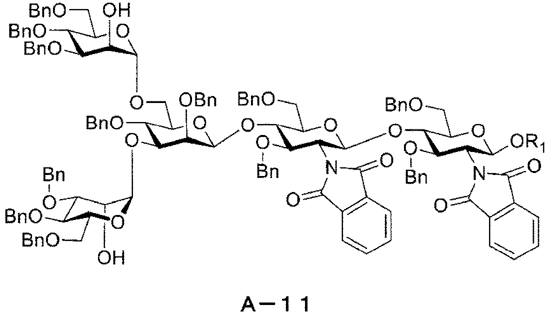 wherein R₁ is a C₁ to C₆ alkyl group or a phenyl group, and the phenyl group is optionally substituted with one to three C₁ to C₆ alkoxy groups,
the step comprising the step of:
subjecting the compound represented by formula A-8 and a compound represented by the following formula A-9: 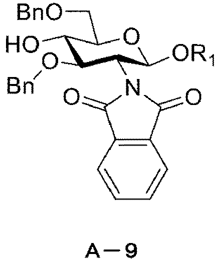
wherein R₁ is a C₁ to C₆ alkyl group or a phenyl group, and the phenyl group is optionally substituted with one to three C₁ to C₆ alkoxy groups,
to β-1,4-glycosidic linkage to give a compound represented by the following formula A-10: 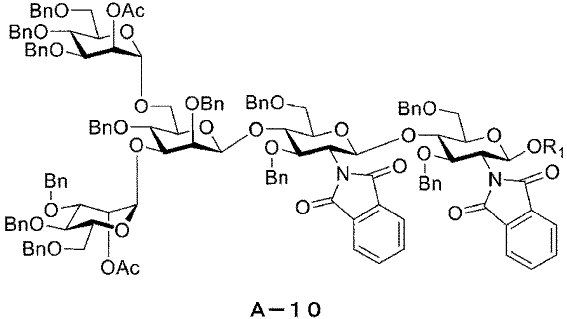
wherein R₁ is a C₁ to C₆ alkyl group or a phenyl group, and the phenyl group is optionally substituted with one to three C₁ to C₆ alkoxy groups;
(step I-3) subjecting the compound represented by formula A-11 and a compound represented by the following formula A-12: 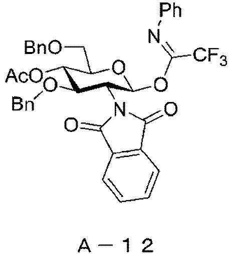 to β-1,2-glycosidic linkage to give a compound represented by the following formula A-13: 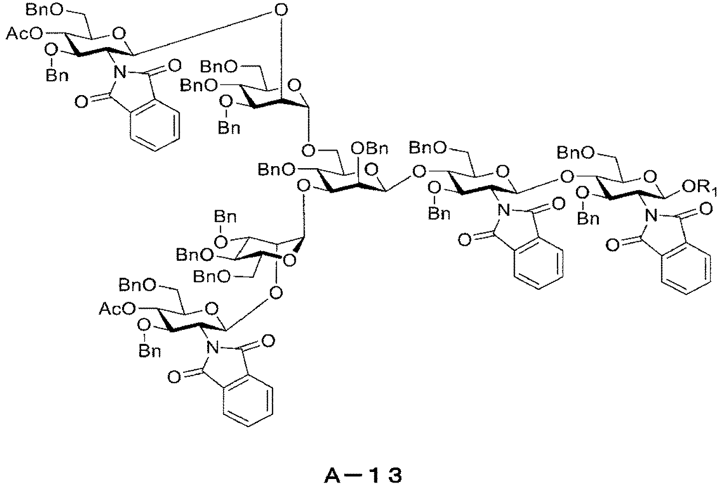
wherein R₁ is a C₁ to C₆ alkyl group or a phenyl group, and the phenyl group is optionally substituted with one to three C₁ to C₆ alkoxy groups,
then eliminating each phthaloyl group as a protecting group of an amino group and each acetyl group on a hydroxyl group on the compound represented by formula A-13 to give a compound represented by the following formula A-14: 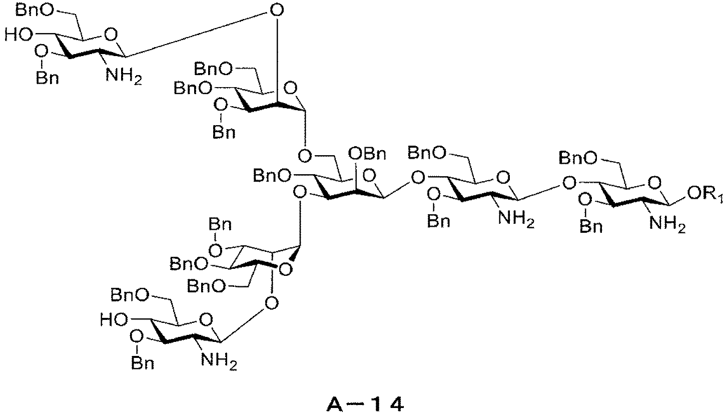
wherein R₁ is a C₁ to C₆ alkyl group or a phenyl group, and the phenyl group is optionally substituted with one to three C₁ to C₆ alkoxy groups,
and then protecting the amino group in the compound represented by formula A-14 by a protecting group selected from an aryloxycarbonyl (COOAr) group, an acetyl (Ac) group, a 2,2,2-trichloroethoxycarbonyl (Troc) group, or a phthaloyl (Phth) group to give a compound represented by the following formula A-15: 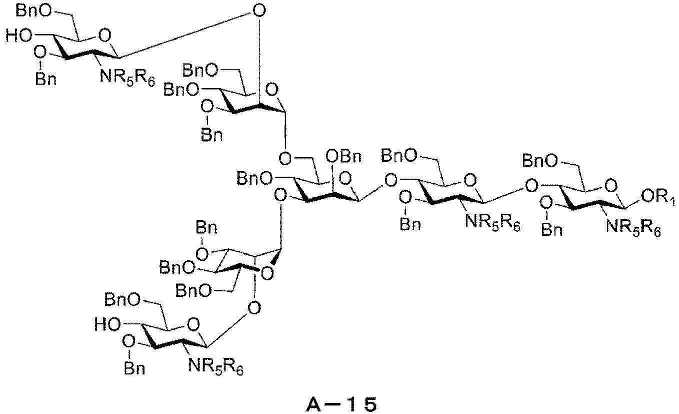
wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
or eliminating each acetyl (Ac) group on the compound represented by formula A-13 to give the compound represented by formula A-15 wherein R₅ and R₆ together with a nitrogen atom attached thereto form a phthalimide group;
(step I-4) producing a compound represented by the following formula A-20: 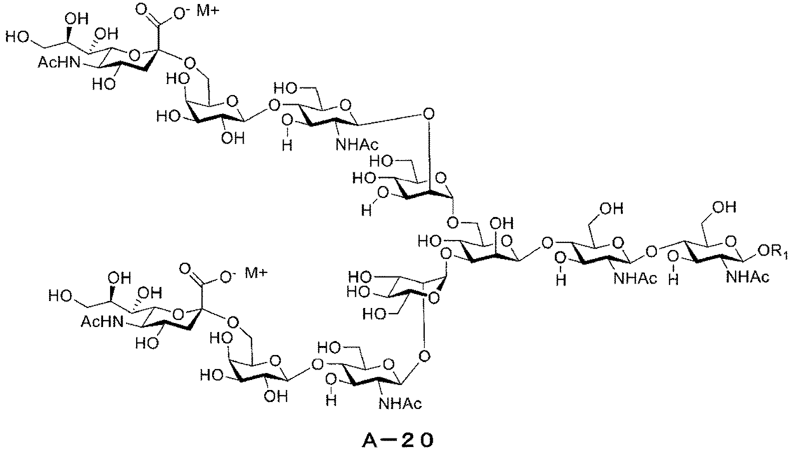 wherein R₁ is a C₁ to C₆ alkyl group or a phenyl group, the phenyl group is optionally substituted with one to three C₁ to C₆ alkoxy groups, and M⁺ is a sodium ion, lithium ion, potassium ion, or protonated triethylamine cation,
the step comprising:
subjecting the compound represented by A-15 and a compound represented by the following formula A-16: 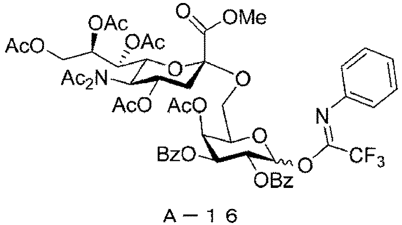
to β-1,4-glycosidic linkage to give a compound represented by the following formula A-17: 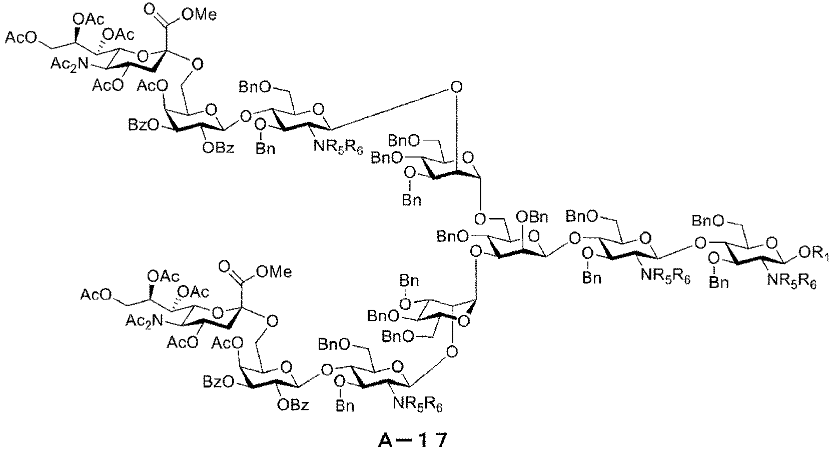
wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
and then eliminating each protecting group of an amino group and each acyl-based protecting group of a hydroxyl group in the compound represented by formula A-17 to give a compound represented by formula A-18: 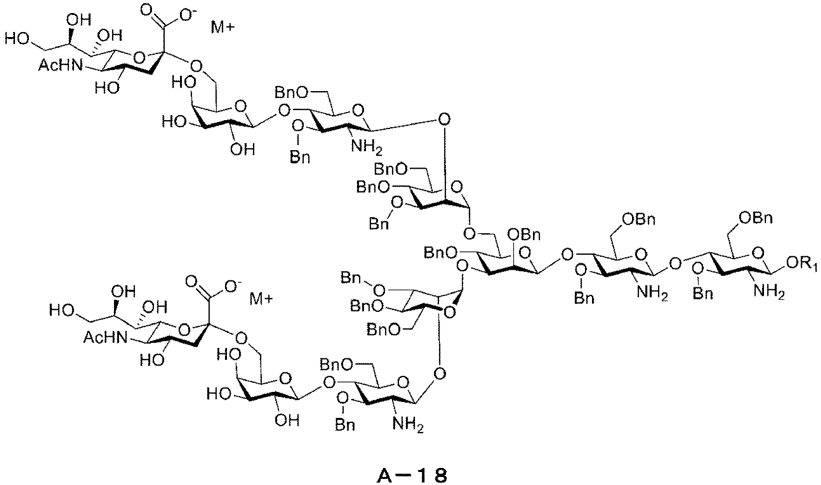
wherein R₁ is a C₁ to C₆ alkyl group or a phenyl group, the phenyl group is optionally substituted with one to three C₁ to C₆ alkoxy groups, and M⁺ is a sodium ion, lithium ion, potassium ion, or protonated triethylamine cation; and
(step I-5) producing the oligosaccharide represented by formula A-22 by reacting the compound represented by formula A-20 with a compound represented by the following formula A-21:

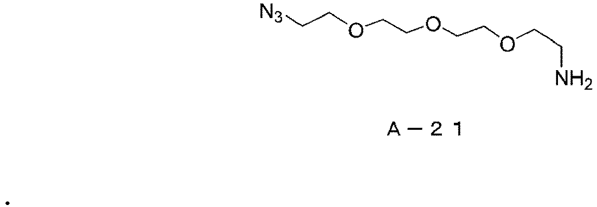

2. The method according to claim 1, wherein the method comprises reacting a compound represented by the following formula A-4: 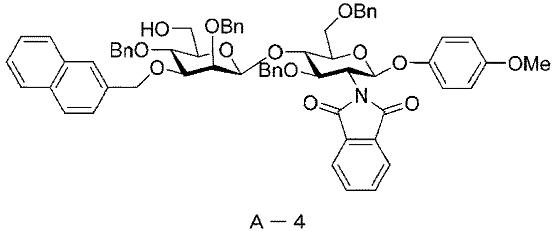 with 2,3-dichloro-5,6-dicyano-p-benzoquinone (DDQ) in a mixed solvent of fluorous alcohol and water to eliminate a 2-naphthylmethyl group in the compound represented by formula A-4 to give the compound represented by formula A-5.

3. The method according to claim 2, wherein the fluorous alcohol is selected from the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and combinations thereof.

4. The method according to claim 2 or 3, wherein the reaction is carried out at -35°C to 70°C.

5. The method according to claim 2 or 3, wherein the reaction is carried out at -30°C to -10°C.

6. The method according to any one of claims 1 to 5, wherein step I-2 comprises reacting the compound represented by formula A-10 with a strong base in the presence of an alkyl ester of perfluorocarboxylic acid to give the compound represented by formula A-11.

7. The method according to claim 6, wherein the alkyl ester of perfluorocarboxylic acid is methyl trifluoroacetate, ethyl trifluoroacetate, propyl trifluoroacetate, isopropyl trifluoroacetate, butyl trifluoroacetate, methyl pentafluoropropionate, ethyl pentafluoropropionate, propyl pentafluoropropionate, isopropyl pentafluoropropionate, butyl pentafluoropropionate, methyl heptafluorobutyrate, ethyl heptafluorobutyrate, propyl heptafluorobutyrate, isopropyl heptafluorobutyrate, butyl heptafluorobutyrate, methyl nonafluorovalerate, ethyl nonafluorovalerate, propyl nonafluorovalerate, isopropyl nonafluorovalerate, butyl nonafluorovalerate, methyl undecafluorocaproate, ethyl undecafluorocaproate, propyl undecafluorocaproate, isopropyl undecafluorocaproate, or butyl undecafluorocaproate.

8. The method according to claim 6 or 7, wherein the strong base is selected from the group consisting of sodium, lithium, and potassium salts of metal amides; sodium, lithium, potassium, cesium, and barium salts of C1-C20 alkoxides; sodium hydride, potassium hydride, lithium hydride, butyllithium, potassium carbonate, sodium carbonate, cesium carbonate, lithium carbonate, potassium phosphate, sodium phosphate, cesium phosphate, lithium phosphate, diazabicycloundecene (DBU), diazabicyclononene (DBN), and 1,1,3,3-tetramethylguanidine (TMG); and combinations thereof.

9. The method according to claim 6 or 7, wherein the strong base is potassium tert-butoxide, sodium tert-butoxide, lithium tert-butoxide, or lithium hexamethyldisilazide (LHMDS).

10. The method according to any one of claims 6 to 9, wherein the step of reacting the compound represented by formula A-10 with a strong base in the presence of an alkyl ester of perfluorocarboxylic acid to give the compound represented by formula A-11 is carried out in a C₁-C₁₀ alcohol solvent alone or in a mixture of a C₁-C₁₀ alcohol solvent and an amide-based solvent, ether-based solvent, ester-based solvent, aromatic solvent, halogen-based solvent, hydrocarbon-based solvent, or nitrile-based solvent.

11. The method according to any one of claims 1 to 10, wherein in step I-3, each amino group in the compound represented by formula A-14 is protected with an aryloxycarbonyl (COOAr) group to give the compound represented by formula A-15.

12. The method according to any one of claims 1 to 11, wherein in step I-3, the step of producing the compound represented by formula A-15 from the compound represented by formula A-14 is carried out in an aqueous solution containing sodium bicarbonate, potassium bicarbonate, disodium hydrogen phosphate, or dipotassium hydrogen phosphate.

13. The method according to any one of claims 1 to 11, wherein R¹ is isopropyl or 4-methoxyphenyl.

14. The method according to any one of claims 1 to 13, wherein the method comprises a step for producing the compound represented by formula A-16;
the step comprising:
stirring a solution containing a compound represented by the following formula G-11: 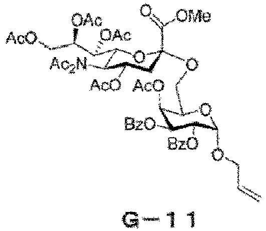
and carbonylchlorohydridotris(triphenylphosphine) ruthenium(II), and then adding water and 1,3-dibromo-5,5-dimethylhydantoin, iodide, or N-bromosuccinimide to the solution and stirring the mixture to eliminate the allyl group attached via oxygen to the carbon at the 1-position of D-galactopyranoside in the compound represented by formula G-11 to give a compound represented by the following formula G-12: 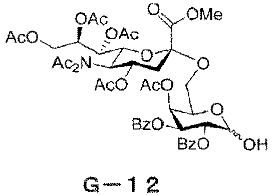
and further converting the hydroxyl group on the carbon at the 1-position of D-galactopyranoside in the compound represented by formula G-12 to a 2,2,2-trifluoro-N-phenylacetimidate group to give the compound represented by formula A-16.

15. A method for producing a compound represented by the following formula A-5: 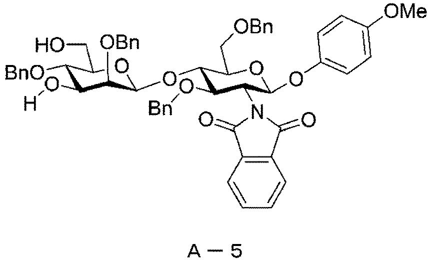 the method comprising reacting a compound represented by the following formula A-4: 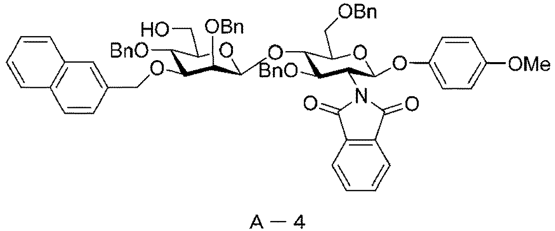 with 2,3-dichloro-5,6-dicyano-p-benzoquinone (DDQ) in a mixed solvent of fluorous alcohol and water to eliminate a 2-naphthylmethyl group in the compound represented by formula A-4 to give the compound represented by formula A-5.

16. The method according to claim 15, wherein the fluorous alcohol is selected from the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and combinations thereof.

17. The method according to claim 15 or 16, wherein the reaction is carried out at -35°C to 70°C.

18. The method according to claim 15 or 16, wherein the reaction is carried out at -30°C to -10°C.

19. A method for producing a compound represented by the following formula A-11: 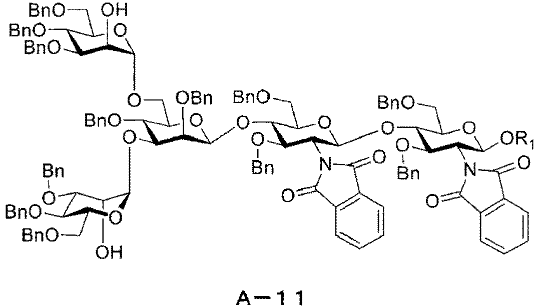
wherein R₁ is a C₁ to C₆ alkyl group or a phenyl group, and the phenyl group is optionally substituted with one to three C₁ to C₆ alkoxy groups,
the method comprising the step of:
reacting a compound represented by the following formula A-10: 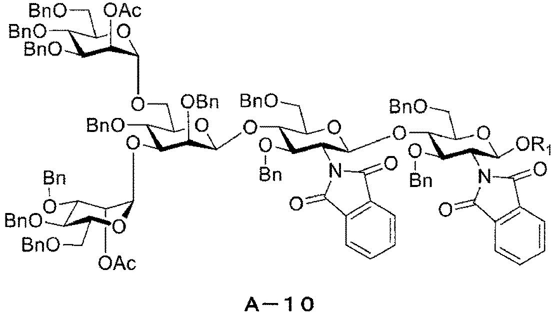
wherein R₁ is a C₁ to C₆ alkyl group or a phenyl group, and the phenyl group is optionally substituted with one to three C₁ to C₆ alkoxy groups,
with a strong base in the presence of an alkyl ester of perfluorocarboxylic acid.

20. The method according to claim 19, wherein the alkyl ester of perfluorocarboxylic acid is methyl trifluoroacetate, ethyl trifluoroacetate, propyl trifluoroacetate, isopropyl trifluoroacetate, butyl trifluoroacetate, methyl pentafluoropropionate, ethyl pentafluoropropionate, propyl pentafluoropropionate, isopropyl pentafluoropropionate, butyl pentafluoropropionate, methyl heptafluorobutyrate, ethyl heptafluorobutyrate, propyl heptafluorobutyrate, isopropyl heptafluorobutyrate, butyl heptafluorobutyrate, methyl nonafluorovalerate, ethyl nonafluorovalerate, propyl nonafluorovalerate, isopropyl nonafluorovalerate, butyl nonafluorovalerate, methyl undecafluorocaproate, ethyl undecafluorocaproate, propyl undecafluorocaproate, isopropyl undecafluorocaproate, or butyl undecafluorocaproate.

21. The method according to claim 19 or 20, wherein the strong base is selected from the group consisting of sodium, lithium, and potassium salts of metal amides; sodium, lithium, potassium, cesium, and barium salts of C1-C20 alkoxides; sodium hydride, potassium hydride, lithium hydride, butyllithium, potassium carbonate, sodium carbonate, cesium carbonate, lithium carbonate, potassium phosphate, sodium phosphate, cesium phosphate, lithium phosphate, diazabicycloundecene (DBU), diazabicyclononene (DBN), and 1,1,3,3-tetramethylguanidine (TMG); and combinations thereof.

22. The method according to claim 19 or 20, wherein the strong base is potassium tert-butoxide, sodium tert-butoxide, lithium tert-butoxide, or lithium hexamethyldisilazide (LHMDS).

23. The method according to any one of claims 19 to 22, wherein the reaction is carried out in a C₁-C₁₀ alcohol solvent alone or in a mixture of a C₁-C₁₀ alcohol solvent and an amide-based solvent, ether-based solvent, ester-based solvent, aromatic solvent, halogen-based solvent, hydrocarbon-based solvent, or nitrile-based solvent.

24. A method for producing a compound represented by the following formula A-17: 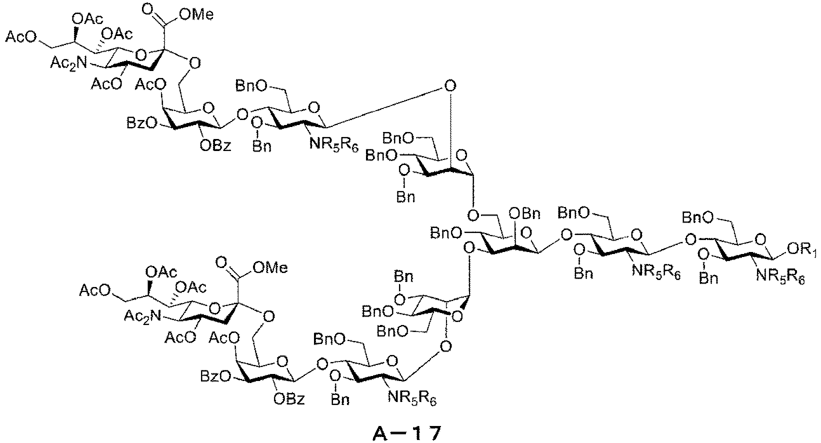
wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
the method comprising the step of:
producing a compound represented by the following formula A-15: 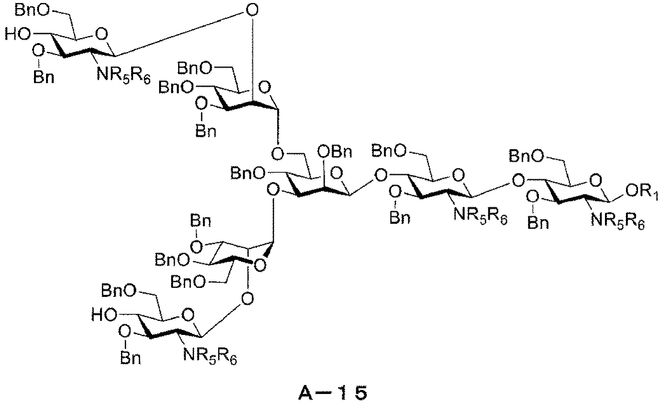
wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
and then subjecting the compound and a compound represented by the following formula A-16: 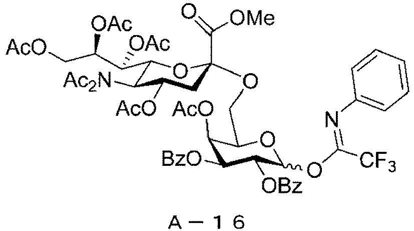
to β-1,4-glycosidic linkage to give the compound represented by formula A-17.

25. The method according to claim 24, wherein R₅ is an aryloxycarbonyl (COOAr) group.

26. A method for producing a compound represented by the following formula A-18: 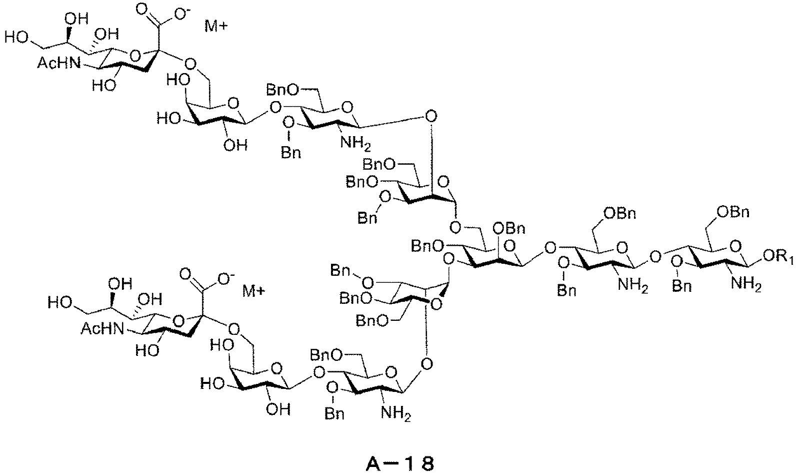
wherein R₁ is a C₁ to C₆ alkyl group or a phenyl group, the phenyl group is optionally substituted with one to three C₁ to C₆ alkoxy groups, and M⁺ is a sodium ion, lithium ion, potassium ion, or protonated triethylamine cation,
the method comprising:
eliminating each protecting group of an amino group and each acyl-based protecting group of a hydroxyl group in a compound represented by the following formula A-17: 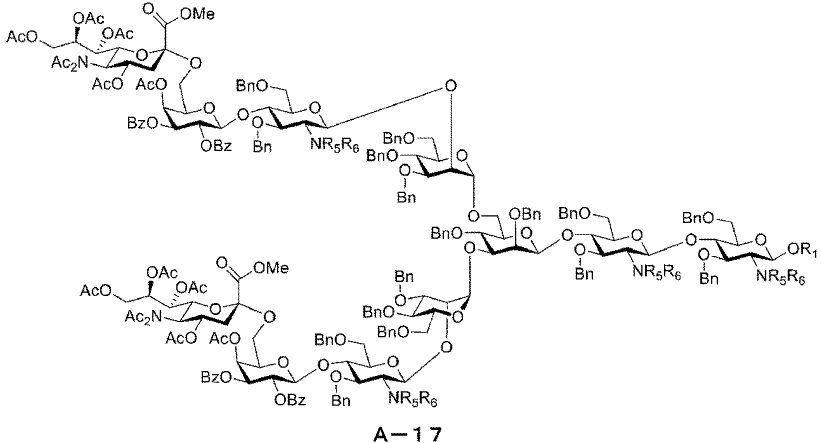
wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group.

27. An oligosaccharide represented by the following formula A-22: 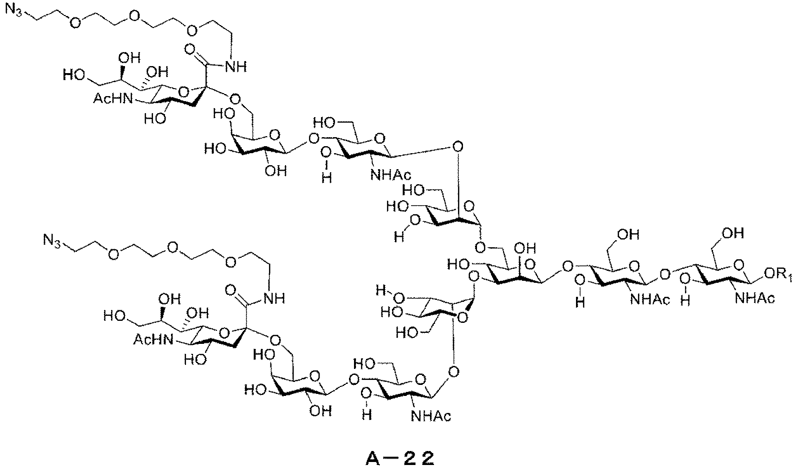 wherein R₁ is a C₁ to C₆ alkyl group or a phenyl group, and the phenyl group is optionally substituted with one to three C₁ to C₆ alkoxy groups.

28. An oligosaccharide represented by the following formula A'-22:

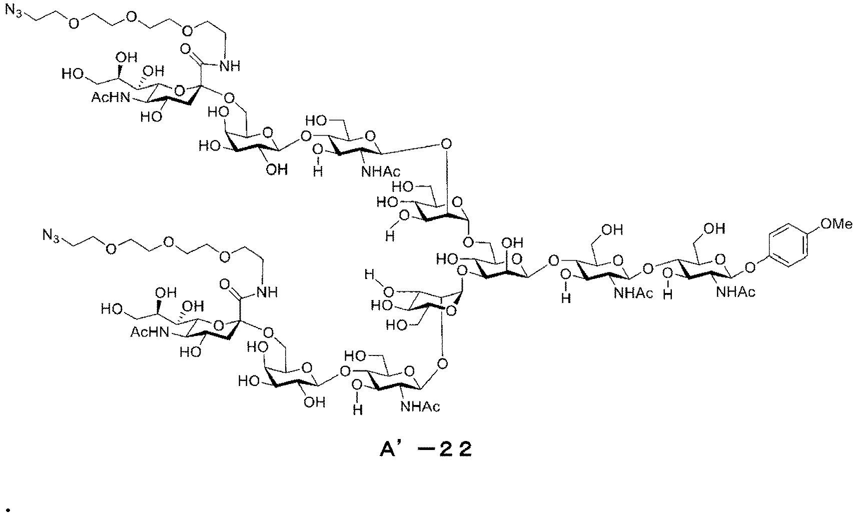

29. A compound represented by the following formula A-8:

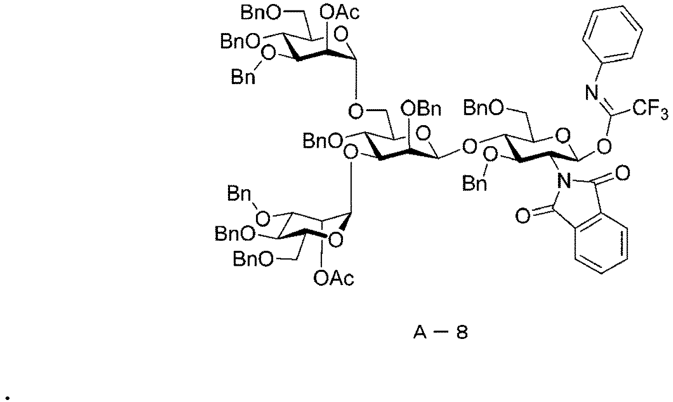

30. A compound represented by the following formula A-10: 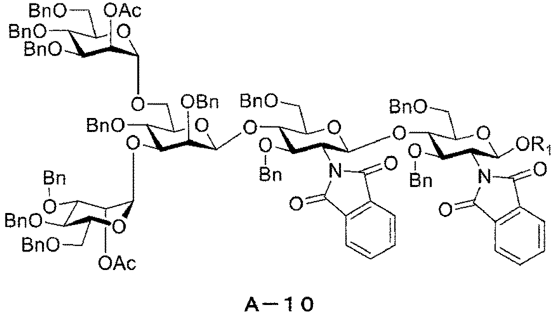 wherein R₁ is a C₁ to C₆ alkyl group or a phenyl group, and the phenyl group is optionally substituted with one to three C₁ to C₆ alkoxy groups.

31. A compound represented by the following formula A-11: 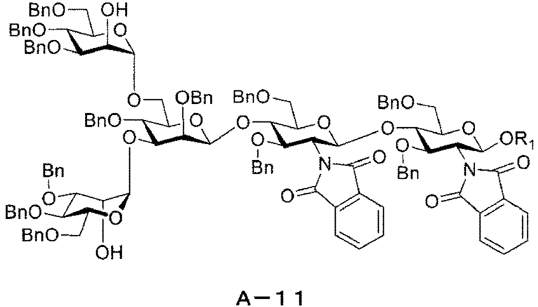 wherein R₁ is a C₁ to C₆ alkyl group or a phenyl group, and the phenyl group is optionally substituted with one to three C₁ to C₆ alkoxy groups.

32. A compound represented by the following formula A-13: 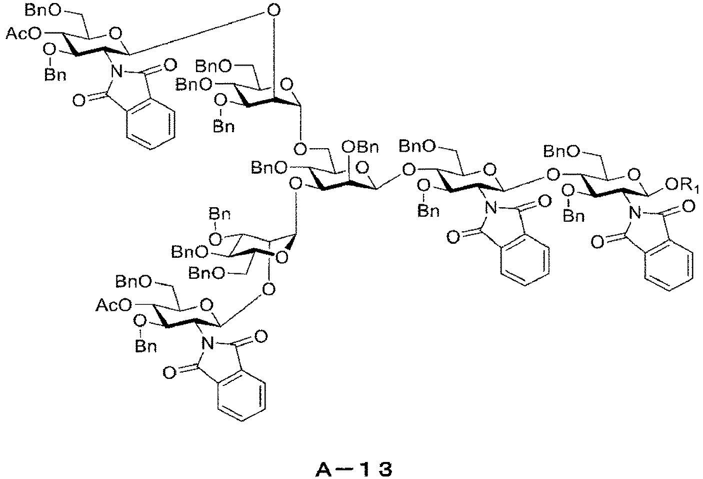 wherein R₁ is a C₁ to C₆ alkyl group or a phenyl group, and the phenyl group is optionally substituted with one to three C₁ to C₆ alkoxy groups.

33. A compound represented by the following formula A-14: 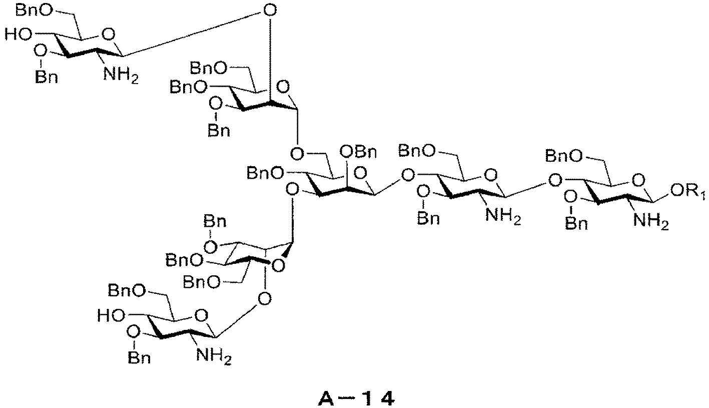 wherein R₁ is a C₁ to C₆ alkyl group or a phenyl group, and the phenyl group is optionally substituted with one to three C₁ to C₆ alkoxy groups.

34. A compound represented by the following formula A-14-FMA: 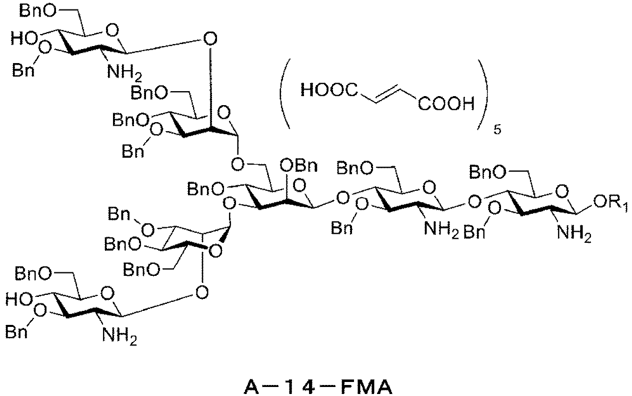 wherein R₁ is a C₁ to C₆ alkyl group or a phenyl group, and the phenyl group is optionally substituted with one to three C₁ to C₆ alkoxy groups.

35. A compound represented by the following formula A-15: 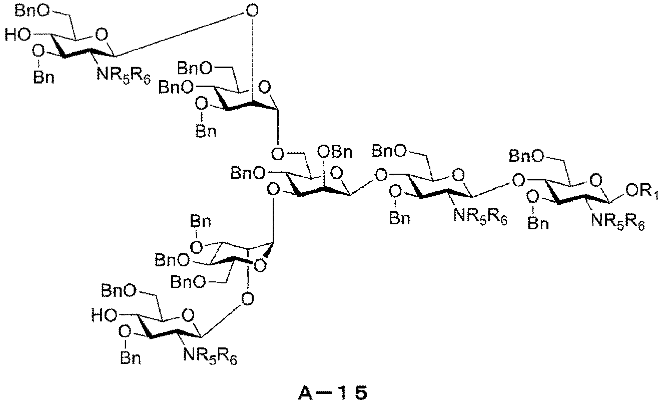 wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group.

36. A compound represented by the following formula A-17: 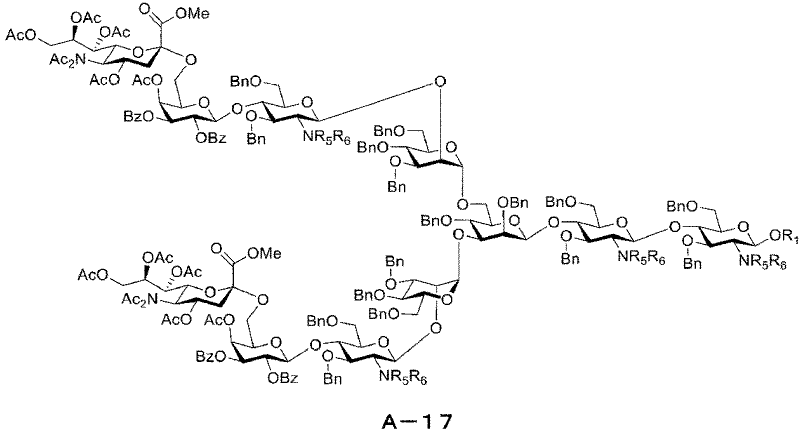 wherein R₁ is a C₁ to C₆ alkyl group or a phenyl group, the phenyl group is optionally substituted with one to three C₁ to C₆ alkoxy groups, R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group.

37. A compound represented by the following formula A-18-FF: 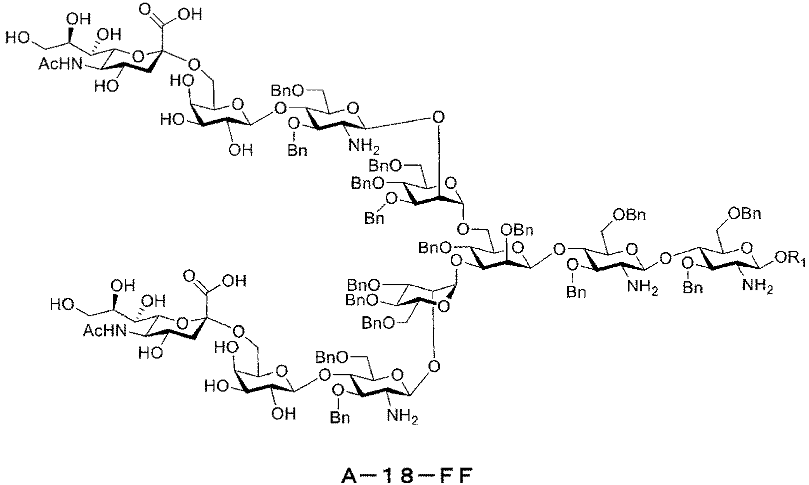
wherein R₁ is a C₁ to C₆ alkyl group or a phenyl group, and the phenyl group is optionally substituted with one to three C₁ to C₆ alkoxy groups,
or a salt thereof.

38. A compound represented by the following formula A-19-FF: 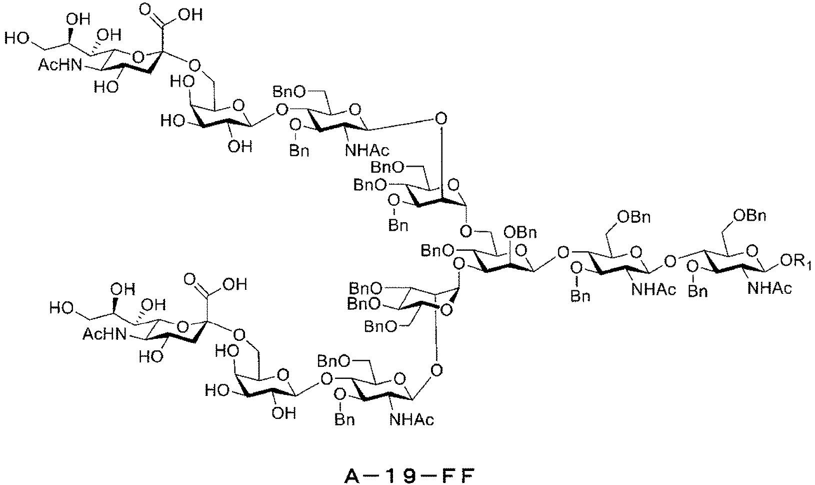
wherein R₁ is a C₁ to C₆ alkyl group or a phenyl group, and the phenyl group is optionally substituted with one to three C₁ to C₆ alkoxy groups,
or a salt thereof.

39. A compound represented by the following formula A-20-FF: 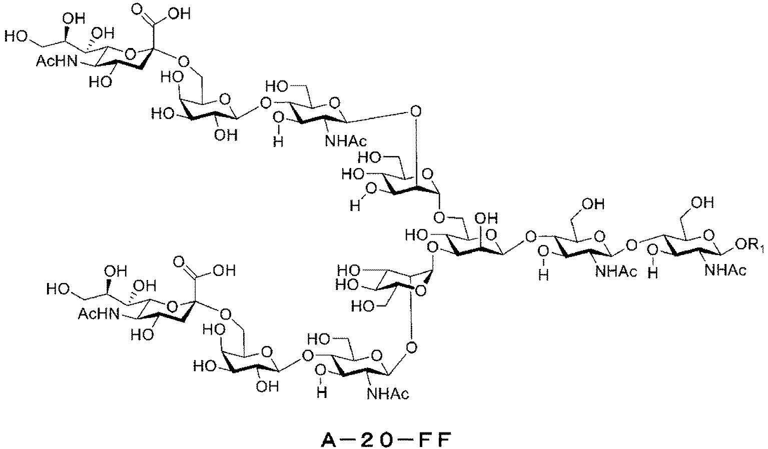
wherein R₁ is a C₁ to C₆ alkyl group or a phenyl group, and the phenyl group is optionally substituted with one to three C₁ to C₆ alkoxy groups,
or a salt thereof.

40. A compound represented by the following formula A'-20-FF: 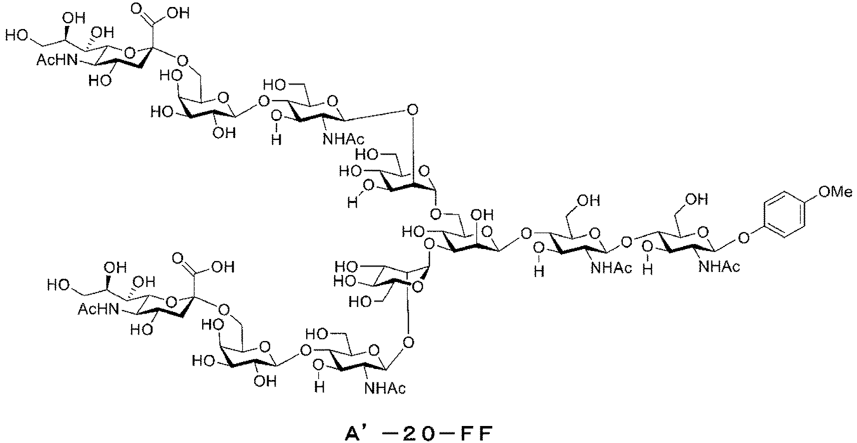 or a salt thereof.

41. A method for producing a glycan-remodeled antibody or a molecule containing an Fc region thereof, the method comprising the steps of: obtaining the oligosaccharide represented by formula A-22, the step comprising the method according to any one of claims 1 to 14; and reacting the resulting oligosaccharide represented by formula A-22 with an acceptor molecule that is an antibody having core GlcNAc to which fucose is optionally added as an N297-linked glycan or a molecule containing an Fc region thereof to obtain the glycan-remodeled antibody or molecule containing an Fc region thereof.

42. The method according to claim 41, further comprising the step of reacting an azide group (N₃-) with a molecule having an alkyne structure.

43. The method according to claim 42, wherein the molecule having an alkyne structure is selected from chemotherapeutic agents, molecular target drugs, immunostimulatory agents, toxins, antimicrobial agents, antiviral agents, diagnostic agents, proteins, peptides, amino acids, nucleic acids, antigens, vitamins, and hormones.

44. A method for producing an antibody-drug conjugate, comprising the method according to any one of claims 41 to 43.
